# EUROPEAN PATENT APPLICATION

(11) **EP 4 559 908 A1**
(43) Date of publication of application: **28.05.2025**
(21) Application number: 23843387.4
(22) Date of filing: 19.07.2023
(51) Int. Cl.: C07D 403/12, A61K 31/501, A61K 31/496, A61K 31/506, A61K 31/4439, A61K 31/497, A61K 31/53, A61K 31/5377, A61P 7/02, C07D 235/18

(54) **HETEROARYL DERIVATIVE COMPOUND AND USE THEREOF**

(30) Priority: 20.07.2022 KR 20220089413; 05.09.2022 KR 20220112256
(71) Applicant: S2CBio Inc., Incheon 21983 (KR)
(72) Inventor: JEONG, Jinah, Hwaseong-si, Gyeonggi-do 18449 (KR); KWAK, Hyunjung, Hwaseong-si, Gyeonggi-do 18449 (KR); CHOI, Jihye, Hwaseong-si, Gyeonggi-do 18449 (KR); LEE, Seolhee, Hwaseong-si, Gyeonggi-do 18449 (KR); LEE, Eunjung, Hwaseong-si, Gyeonggi-do 18449 (KR); CHO, Sungmin, Hwaseong-si, Gyeonggi-do 18449 (KR); KIM, Bokyung, Hwaseong-si, Gyeonggi-do 18449 (KR); SONG, Yoonsung, Hwaseong-si, Gyeonggi-do 18449 (KR); YOON, Hongchul, Hwaseong-si, Gyeonggi-do 18449 (KR); PARK, Joontae, Hwaseong-si, Gyeonggi-do 18449 (KR); AN, Kyungmi, Hwaseong-si, Gyeonggi-do 18449 (KR); LEE, Jungwoo, Hwaseong-si, Gyeonggi-do 18449 (KR); KIM, Gunhee, Hwaseong-si, Gyeonggi-do 18449 (KR); OH, Changmok, Hwaseong-si, Gyeonggi-do 18449 (KR); LIM, Dami, Hwaseong-si, Gyeonggi-do 18449 (KR)
(74) Representative: Fuchs Patentanwälte Partnerschaft mbB
(86) International application number: PCT/KR2023/010439
(87) International publication number: WO 2024/019541

(57) **Abstract**

The present invention relates to fused heteroaryl derivatives and uses thereof. The fused heteroaryl derivative of the present invention may exhibit excellent inhibitory activity against anoctamin-6 (ANO6), which may be usefully employed as a therapeutic agent for thrombosis or thrombosis-related diseases.

## Description

### [Technical Field]

The present invention relates to a heteroaryl derivative compound and a medicinal use thereof. Specifically, the present invention relates to a heteroaryl derivative compound having inhibitory activity against anoctamin-6 (ANO6).

### [Background Art]

Anoctamin-6 (ANO6/TMEM16F) is encoded by the TMEM16F gene, which is one of a family of transmembrane proteins belonging to the anoctamin family, and is expressed in various cells. The anoctamin-6 protein is essential for the calcium ion-dependent exposure to the outside of the cell membrane, of phosphatidylserine present inside the cell membrane, through which it is involved in blood coagulation, bone formation, and T cell activation, etc. In particular, when platelets are activated, anoctamin-6 exposes phosphatidylserine to activate the blood coagulation system. In addition, exposure of phosphatidylserine to the outside of the cell membrane by anoctamin-6 acts as a cell activation signal in various cells and is involved in cell proliferation and death, thus playing an important role in the development and progression of various diseases including hemorrhagic disease and cancer.

Therefore, there is a need to develop a composition capable of effectively improving or treating diseases such as thromboembolic diseases, inflammatory diseases, or cancers involving the functions of anoctamin-6 as an ion channel and phospholipid scramblase, through the development of anoctamin-6 activity inhibitor.

### [Disclosure of invention]

### [Technical Problem]

An object of the present invention is to provide a heteroaryl derivative having a novel structure, a tautomer thereof, a stereoisomer thereof, or a pharmaceutically acceptable salt thereof.

Another object of the present invention is to provide a method for preparing the heteroaryl derivative compound.

Another object of the present invention is to provide a pharmaceutical use of the heteroaryl derivative compound, specifically, a pharmaceutical composition for treating or preventing thrombosis or thrombosis-related diseases comprising the heteroaryl derivative compound as an active ingredient, use of the compound for treatment or prevention of thrombosis or thrombosis-related diseases, or a method for treating or preventing thrombosis or thrombosis-related diseases comprising administering the compound.

### [Technical Solution]

In one general aspect, a specific embodiment of the present invention provides a heteroaryl derivative compound having a specific structure that inhibits anoctamin-6 (ANO6) activity.

### Heteroaryl Derivative Compound

The present invention provides a compound represented by the following Chemical Formula 1, a tautomer thereof, astereoisomer thereof, or a pharmaceutically acceptable salt thereof: 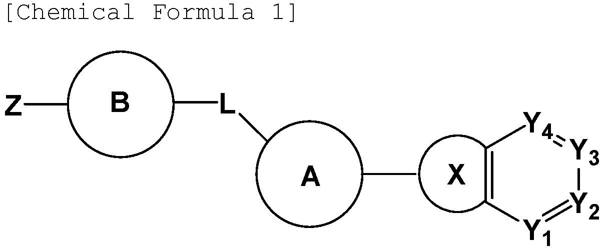 in Chemical Formula 1 above,
ring X is a 5-6 membered heteroaryl, a 5-6 membered heterocycloalkyl, or a 5-6 membered heterocycloalkenyl, wherein at least one H of the 5-6 membered heteroaryl, 5-6 membered heterocycloalkyl, or 5-6 membered heterocycloalkenyl ring may be substituted with -C₁₋₆alkyl, -benzyl, -C₁₋₆haloalkyl, -(CH₂)n-Rₑ, - O-(CH₂)n-Rₑ, or -halo;
Y₁ to Y₄ are each independently CR_{Y} or N;
R_{Y} is -H, -C₁₋₆alkyl, -C₁₋₆aminoalkyl, -C₁₋₆hydroxyalkyl, -C₁₋₆haloalkyl, -CN, -NO₂, -NRₐR_{b}, -OR_{c}, -C(=O)-R_{d}, -(CH₂)n-Rₑ, -O-(CH₂)n-Rₑ, -S(=O)₂-C₁₋₆alkyl, -halo, or 5-6 membered heteroaryl, wherein at least one H of the 5-6 membered heteroaryl ring may be substituted with -C₁₋₆alkyl;
ring A and ring B are each independently aryl, heteroaryl, cycloalkyl, or heterocycloalkyl, wherein the aryl, heteroaryl, cycloalkyl, or heterocycloalkyl is a single ring or multiple rings, and at least one H of the aryl, heteroaryl, cycloalkyl, or heterocycloalkyl ring may be substituted with -C₁₋₆alkyl, -C₁₋₆aminoalkyl, -C₁₋₆hydroxyalkyl, -C₁₋₆haloalkyl, -CN, -NO₂, -NRₐR_{b}, - OR_{c}, -C(=O)-R_{d}, -(CH₂)n-Rₑ, -O-(CH₂)n-Rₑ, -halo, or 5-6 membered heterocycloalkyl, wherein at least one H of the 5-6 membered heterocycloalkyl may be substituted with -C₁₋₆alkyl;
L is -CH₂-, -NR_{L}-, -C≡C-NR_{L}, -0-, -C(=O)-, -C(=O)O-, - OC(=O)-, -C(=O)NR_{L}-, -NR_{L}C(=O)-, -S-, -S(=O)₂-, -S(=O)₂-NR_{L}-, or - NR_{L}-S(=O)₂-;
R_{L} is -H or -C₁₋₆alkyl;
Z is -H, -C₁₋₆alkyl, -CN, -C₂alkynyl, aryl, heteroaryl, cycloalkyl, or heterocycloalkyl, wherein at least one H of the - C₂alkynyl, aryl, heteroaryl, cycloalkyl, or heterocycloalkyl ring may be substituted with -C₁₋₆alkyl, -C₁₋₆aminoalkyl, -C₁₋₆hydroxyalkyl, -C₁₋₆haloalkyl, -CN, -NO₂, -NRₐR_{b}, -OR_{c}, -C(=O)-R_{d}, - (CH₂)n-Rₑ, -O-(CH₂)n-Rₑ, -halo, or 5-6 membered heterocycloalkyl, wherein at least one H of the 5-6 membered heterocycloalkyl may be substituted with -C₁₋₆alkyl;
Rₐ and R_{b} are each independently -H, -C₁₋₆alkyl, or -benzyl;
R_{c} is -H, -C₁₋₆alkyl, -C₁₋₆haloalkyl, -(CH₂)n-Rₑ, -benzyl or heterocycloalkyl, wherein at least one H of the heterocycloalkyl ring may be substutited with -C₁₋₆alkyl;
R_{d} is -H, -OH, -O-C₁₋₆alkyl, -NH₂, -NH-C₁₋₆alkyl, -N(C₁₋₆alkyl) (C₁₋₆alkyl), -NH-C₃₋₆cycloalkyl , or -NH-aryl;
Rₑ is -C₁₋₆aminoalkyl, -NH₂, -NH-C₁₋₆alkyl, -N(C₁₋₆alkyl)(C₁₋₆alkyl), heteroaryl, heterocycloalkyl, or heterocycloalkenyl, wherein the heteroaryl, heterocycloalkyl, or heterocycloalkenyl is a single ring or multiple rings, and at least one H of the heteroaryl, heterocycloalkyl, or heterocycloalkenyl ring may be substituted with -C₁₋₆alkyl; and
n is 0, 1, 2, 3 or 4.

According to an embodiment of the present invention, 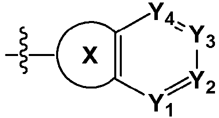 may be following range:
Ring X is a 5 membered heteroaryl, wherein at least one H of the 5 membered heteroaryl may be substituted with -C₁₋₆alkyl; and
Y₁ and Y₄ are each independently CR_{Y}, and Y₂ and Y₃ are each independently CR_{Y} or N, wherein when ring X is furan, thiophene, or thiazole, one of Y₂ and Y₃ is N.

According to an embodiment of the present invention, 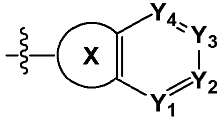 may be, for example, in the following range: 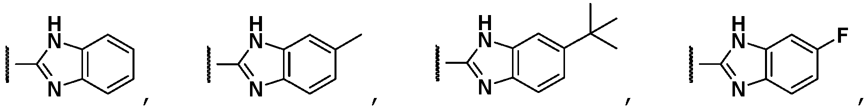 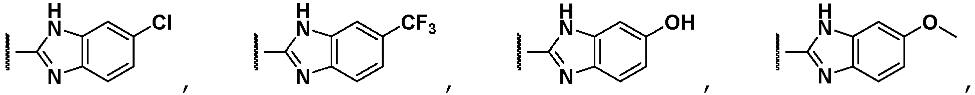 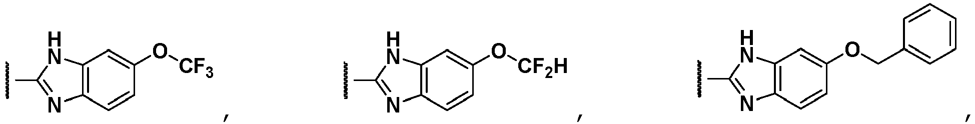 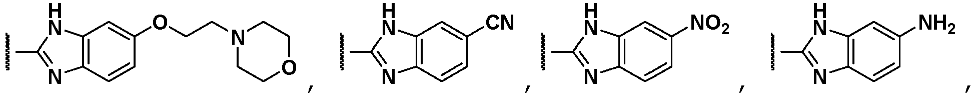 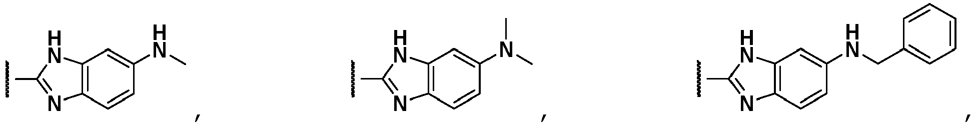 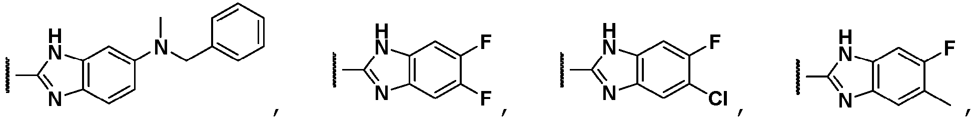 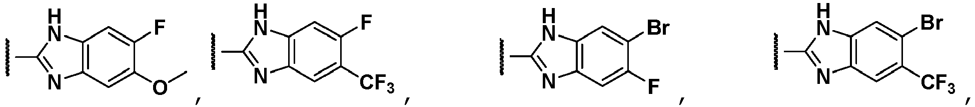 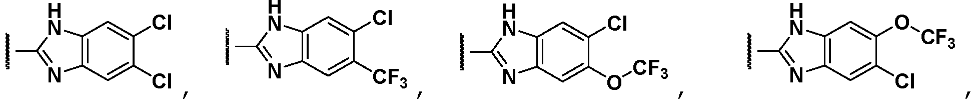 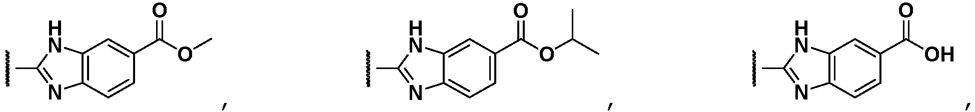 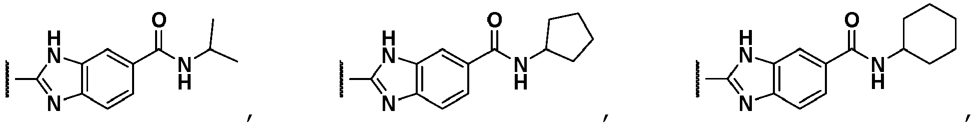 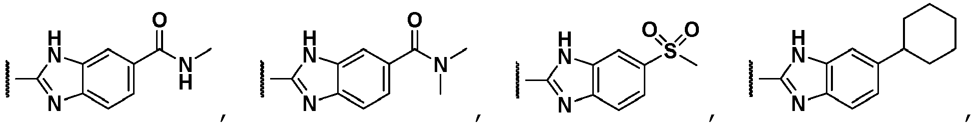 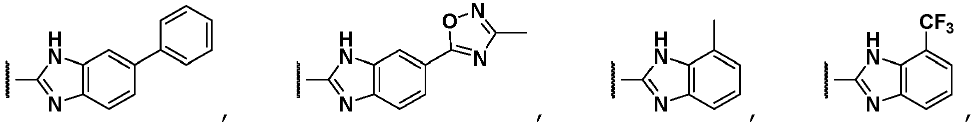 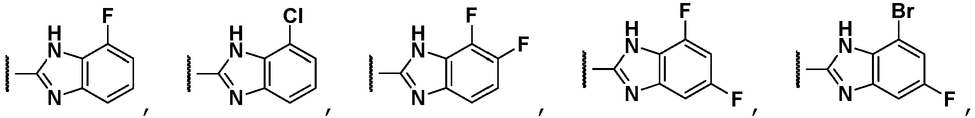 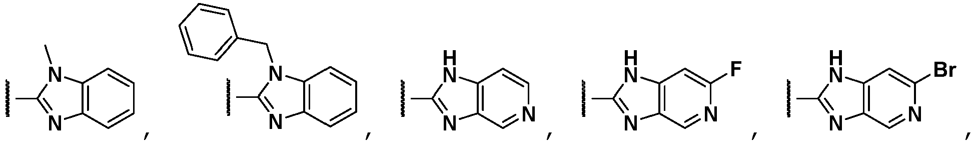 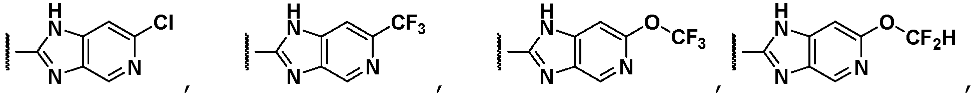 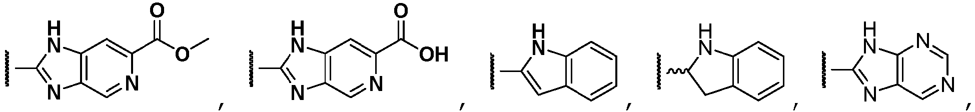 or

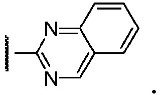

According to an embodiment of the present invention, the L may be, for example, in the following range: 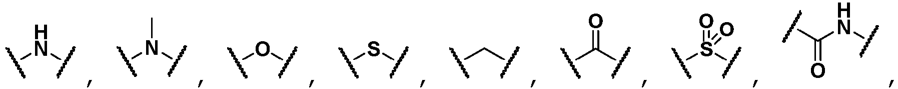 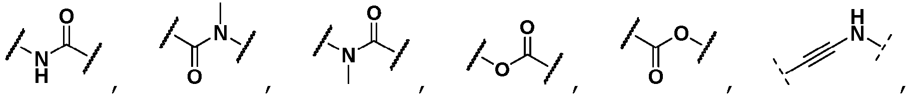 or

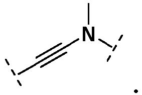

According to an embodiment of the present invention, the ring A may be, for example, in the following range: 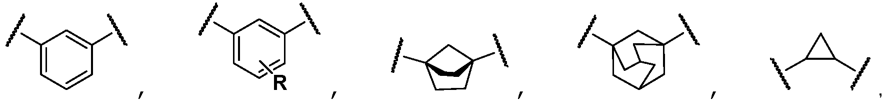 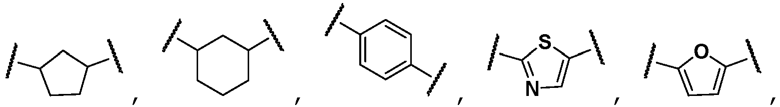 or 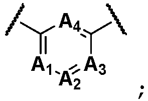
A₁ to A₄ are each independently CR or N; and
R is -CF₃, -N(CH₃)(CH₃), -OH, -OCH₃, -OCH₂CH₂-N(CH₃)(CH₃), - halo,

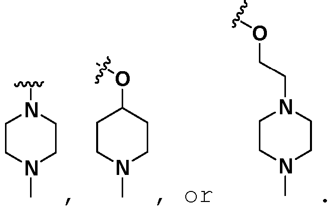

According to an embodiment of the present invention, the ring B may be, for example, in the following range: 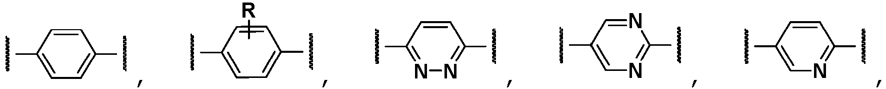 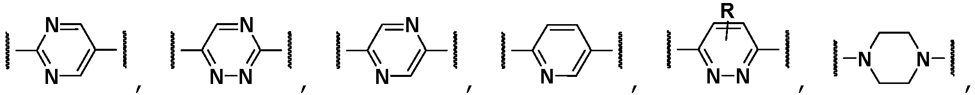 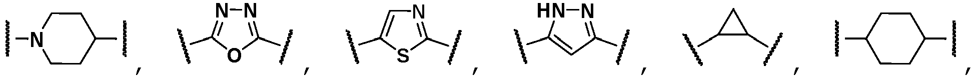 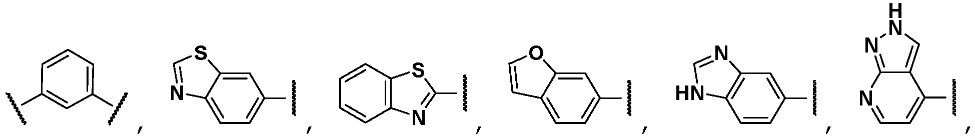 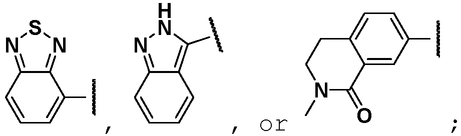 and
R is -methyl, -NO₂, -NH₂, -OCH₃, -OH, or -halo.

According to an embodiment of the present invention, Z may be, for example, in the following range: 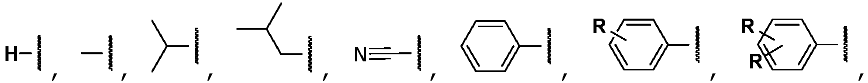 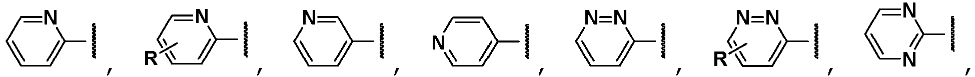 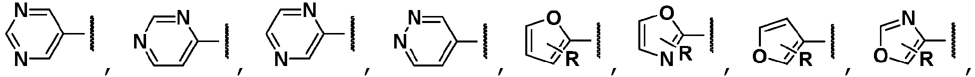 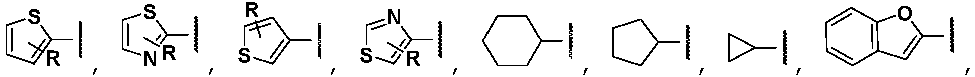 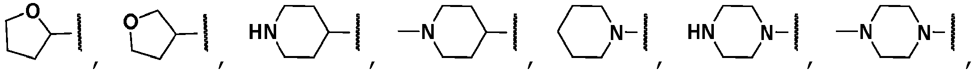 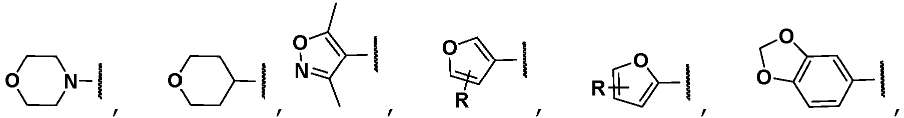 or 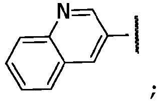 and
R is -H, -methyl, -CF₃, -CN, -CH₂-NH(CH₃), -CH₂-N(CH₃)(CH₃), -OCH₃, -OH, -C(=O)-CH₃, -C(=O)-OCH₃, -C(=O)-OH, -C(=O)-O-t-butyl, -halo, or

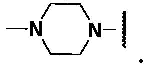

According to an embodiment of the present invention, Rₑ may be, for example, in the following range:

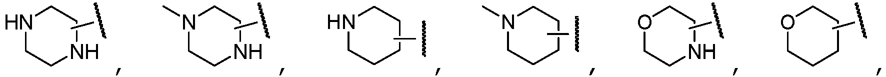

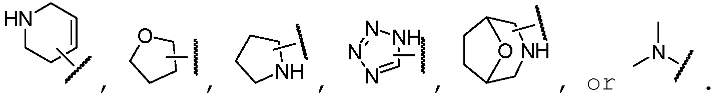

According to an embodiment of the present invention, the compound represented by Chemical Formula 1 may be a compound represented by the following Chemical Formula 1-1: 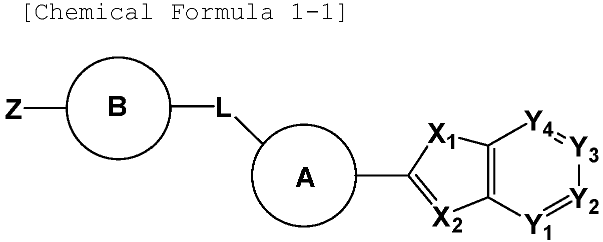 in Chemical Formula 1-1 above,
X₁ is CR₁R₂, NR₃, O, or S;
X₂ is CR₄ or N;
R₁ to R₄ are each independently -H or -C₁₋₆alkyl;
Y₁ to Y₄ are each independently CR_{Y} or N;
R_{Y} is -H, -C₁₋₆alkyl, -C₁₋₆aminoalkyl, -C₁₋₆hydroxyalkyl, -C₁₋₆haloalkyl, -CN, -NO₂, -NRₐR_{b}, -OR_{c}, -C(=O)-R_{d}, -S(=O)₂-C₁₋₆alkyl, - halo, or 5-6 membered heteroaryl, wherein at least one H of the 5-6 membered heteroaryl may be substituted with -C₁₋₆alkyl;
ring A and ring B are each independently aryl, heteroaryl, cycloalkyl, or heterocycloalkyl, wherein at least one H of the aryl, heteroaryl, cycloalkyl, or heterocycloalkyl ring may be substituted with -C₁₋₆alkyl, -C₁₋₆aminoalkyl, -C₁₋₆hydroxyalkyl, -C₁₋₆haloalkyl, -CN, -NO₂, -NRₐR_{b}, -OR_{c}, -C(=O)-R_{d}, -halo, or 5-6 membered heterocycloalkyl, wherein at least one H of the 5-6 membered heterocycloalkyl ring may be substituted with -C₁₋₆alkyl;
L is -CH₂-, -NR_{L}-, -C≡C-NR_{L}, -O-, -C(=O)-, -C(=O)O-, - OC(=O)-, -C(=O)NR_{L}-, -NR_{L}-C(=O)-, -S-, -S(=O)₂-, -S(=O)₂-NR_{L}-, or - NR_{L}-S(=O)₂-;
R_{L} is -H or -C₁₋₆alkyl;
Z is -H, -CN, -C₂alkynyl, aryl, heteroaryl, cycloalkyl, or heterocycloalkyl, wherein at least one H of the -C₂alkynyl, aryl, heteroaryl, cycloalkyl, or heterocycloalkyl ring may be substituted with -C₁₋₆alkyl, -C₁₋₆aminoalkyl, -C₁₋₆hydroxyalkyl, -C₁₋₆haloalkyl, -CN, -NO₂, -NRₐR_{b}, -OR_{c}, -C(=O)-R_{d}, -O-(CH₂)n-Rₑ, -halo, or 5-6 membered heterocycloalkyl, wherein at least one H of the 5-6 membered heterocycloalkyl may be substituted with -C₁₋₆alkyl;
Rₐ and R_{b} are each independently -H, -C₁₋₆alkyl, or -benzyl;
R_{c} is -H, -C₁₋₆alkyl, -C₁₋₆haloalkyl, -(CH₂)n-Rₑ, -benzyl, or heterocycloalkyl, wherein at least one H of the heterocycloalkyl ring may be substitutied with -C₁₋₆alkyl;
R_{d} is -H, -OH, -O-C₁₋₆alkyl, -NH₂, -NH-C₁₋₆alkyl, -N(C₁₋₆alkyl) (C₁₋₆alkyl), -NH-C₃₋₆cycloalkyl , or -NH-aryl; and
Rₑ is -C₁₋₆aminoalkyl, -NH₂, -NH-C₁₋₆alkyl, -N(C₁₋₆alkyl)(C₁₋₆alkyl), heteroaryl, heterocycloalkyl, or heterocycloalkenyl, wherein the heteroaryl, heterocycloalkyl, or heterocycloalkenyl is a single ring or multiple rings, and at least of one H of the heteroaryl, heterocycloalkyl, or heterocycloalkenyl ring may be substituted with -C₁₋₆alkyl; and
n is 0, 1, 2, 3, or 4.

According to an embodiment of the present invention, 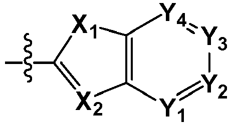 may be in the following range:
X₁ is NR₃ or O;
X₂ is CR₄ or N;
R₃ and R₄ are each independently -H or -C₁₋₆alkyl;
Y₁ and Y₄ are each independently CR_{Y}, Y₂ and Y₃ are each independently CR_{Y} or N, wherein when ring X₁ is S, one of Y₂ and Y₃ is N, when X₁ is O and X₂ is CR₄, one of Y₂ and Y₃ is N; and
R_{Y} is -H, -C₁₋₆alkyl, -C₁₋₆haloalkyl, -CN, -NO₂, -NRₐR_{b}, -OR_{c}, -C(=O)-R_{d}, -S(=O)₂-C₁₋₆alkyl, -halo, or 5-6 membered heteroaryl, wherein at least one H of the 5-6 membered heteroaryl may be substituted with -C₁₋₆alkyl.

According to an embodiment of the present invention, the ring A may be in the following range:
ring A is phenyl, 5-6 membered heteroaryl, or 5-6 membered cycloalkyl, wherein at least one H of the phenyl, 5-6 membered heteroaryl, or 5-6 membered cycloalkyl ring may be substituted with -C₁₋₆haloalkyl, -NO₂, -NRₐR_{b}, -OR_{c}, -halo, or 5-6 membered heterocycloalkyl, wherein at least one H of the heterocycloalkyl may be substituted with -C₁₋₆alkyl.

According to an embodiment of the present invention, the L may be in the following range:
L is -NR_{L}-, -C≡C-NR_{L}, -O-, or -S(=O)₂-; and
R_{L} is -H or -C₁₋₆alkyl.

According to an embodiment of the present invention, the ring B may be in the following range:
the ring B is phenyl, 5-6 membered heteroaryl, or 5-6 membered heterocycloalkyl, wherein at least one H of the phenyl, 5-6 membered heteroaryl, or 5-6 membered heterocycloalkyl ring may be substituted with -C₁₋₆alkyl, -C₁₋₆haloalkyl, -NO₂, -NRₐR_{b}, -OR_{c}, or -halo.

According to an embodiment of the present invention, Z may be in the following range:
Z is -CN, phenyl, 5-6 membered heteroaryl, 5-6 membered cycloalkyl, or 5-6 membered heterocycloalkyl, wherein at least one H of the phenyl, 5-6 membered heteroaryl, 5-6 membered cycloalkyl, or 5-6 membered heterocycloalkyl ring may be substituted with -C₁₋₆alkyl, -C₁₋₆haloalkyl, -CN, -OR_{c}, -C(=O)-R_{d}, -(CH₂)n-Rₑ, -halo, or 5-6 membered heterocycloalkyl, wherein at least one H of the 5-6 membered heterocycloalkyl may be substituted with -C₁₋₆alkyl, wherein when the ring B is phenyl, 6 membered heteroaryl, or 6 membered heterocycloalkyl, Z is connected to the p-position with respect to L.

According to an embodiment of the present invention, the compound represented by Chemical Formula 1 may be selected from the group consisting of compounds listed in Table 1 described below.

**[Table 1]**

| | |
|---|---|
| **1** | 6-Phenyl-*N*-{3-[6-(trifluoromethyl)-1*H*-benzo[*d*]imidazol-2-yl]phenyl}pyridazin-3-amine |
| **2** | *N*-[3-(1*H*-benzo[*d*]imidazol-2-yl)phenyl]-6-phenylpyridazin-3-amine |
| **3** | *N*-[3-(6-tert-butyl-1*H*-benzo[*d*]imidazol-2-yl)phenyl]-6-phenylpyridazin-3-amine |
| **4** | *N*-[3-(6-bromo-5-fluoro-1*H*-benzo[*d*]imidazol-2-yl)phenyl]-6-phenylpyridazin-3-amine |
| **5** | 5,6-Dichloro-2-{3-[(4-phenylpiperazin-1-yl)methyl]phenyl}-1*H-*benzo[*d*]imidazole |
| **6** | 6-Phenyl-*N*-{3-[6-(trifluoromethoxy)-1H-benzo[*d*]imidazol-2-yl]phenyl}pyridazin-3-amine |
| **7** | *N*-[3-(5,6-dichloro-1H-benzo[*d*]imidazol-2-yl)phenyl]-6-phenylpyridazin-3-amine |
| **8** | *N*-{3-[6-chloro-5-(trifluoromethyl)-1*H*-benzo[*d*]imidazol-2-yl]phenyl}-6-phenylpyridazin-3-amine |
| **9** | *N*-{3-[6-bromo-5-(trifluoromethyl)-1*H*-benzo[*d*]imidazol-2-yl]phenyl}-6-phenylpyridazin-3-amine |
| **10** | *N*-{3-[6-chloro-5-(trifluoromethoxy)-1*H*-benzo[*d*]imidazol-2-yl]phenyl}-6-phenylpyridazin-3-amine |
| **11** | *N*-[3-(7-bromo-5-fluoro-1H-benzo[*d*]imidazol-2-yl)phenyl]-6-phenylpyridazin-3-amine |
| **12** | *N*-[3-(6-chloro-1*H*-benzo[*d*]imidazol-2-yl)phenyl]-6-phenylpyridazin-3-amine |
| **13** | *N*-[3-(5,6-dichloro-1H-benzo[d]imidazol-2-yl)phenyl]-5-phenylpyridin-2-amine |
| **14** | 5-Phenyl-*N*-{3-[6-(trifluoromethyl)-1H-benzo[*d*]imidazol-2-yl]phenyl}pyrimidin-2-amine |
| **15** | 5-Phenyl-*N*-{3-[6-(trifluoromethyl)-1H-benzo[*d*]imidazol-2-yl]phenyl}pyridin-2-amine |
| **16** | *N*-[3-(5,6-dichloro-1H-benzo[*d*]imidazol-2-yl)phenyl]pyrimidin-2-amine |
| **17** | 5-(3-Fluorophenyl)-*N*-{3-[6-(trifluoromethyl)-1H-benzo[*d*]imidazol-2-yl]phenyl}pyrimidin-2-amine |
| **18** | 5-(3-Fluorophenyl)-*N*-{3-[6-(trifluoromethyl)-1H-benzo[*d*]imidazol-2-yl]phenyl}pyridin-2-amine |
| **19** | *N*-[3-(1H-benzo[*d*]imidazol-2-yl)phenyl]-[1,1'-biphenyl]-4-amine |
| **20** | *N*-[3-(1H-benzo[*d*]imidazol-2-yl)phenyl]-4-(pyridin-2-yl)aniline |
| **21** | *N*-[3-(1H-benzo[*d*]imidazol-2-yl)phenyl]-4-(pyridazin-3-yl)aniline |
| **22** | *N*-[3-(1H-benzo[*d*]imidazol-2-yl)phenyl]-4-(pyrimidin-4-yl)aniline |
| **23** | *N*-[4-(pyridazin-3-yl)phenyl]-3-[6-(trifluoromethyl)-1H-benzo[*d*]imidazol-2-yl]aniline |
| **24** | *N*-[4-(pyrimidin-4-yl)phenyl]-3-[6-(trifluoromethyl)-1H-benzo[*d*]imidazol-2-yl]aniline |
| **25** | 3-(6-Fluoro-1H-benzo[*d*]imidazol-2-yl)-*N*-[4-(pyridazin-3-yl)phenyl]aniline |
| **26** | *N*-[3-(1H-benzo[*d*]imidazol-2-yl)phenyl]-4-(pyrimidin-2-yl)aniline |
| **27** | *N*-[4-(pyrimidin-2-yl)phenyl]-3-[6-(trifluoromethyl)-1H-benzo[*d*]imidazol-2-yl]aniline |
| **28** | *N*-[4-(pyrimidin-5-yl)phenyl]-3-[6-(trifluoromethyl)-1H-benzo[*d*]imidazol-2-yl]aniline |
| **29** | *N*-[4-(pyrazin-2-yl)phenyl]-3-[6-(trifluoromethyl)-1H-benzo[*d*]imidazol-2-yl]aniline |
| **30** | *N*-[3-(1H-benzo[*d*]imidazol-2-yl)phenyl]-4-(pyrimidin-5-yl)aniline |
| **31** | *N*-[3-(1H-benzo[*d*]imidazol-2-yl)phenyl]-4-(pyridin-3-yl)aniline |
| **32** | *N*-[3-(1H-benzo[*d*]imidazol-2-yl)phenyl]-4-(pyrazin-2-yl)aniline |
| **33** | *N*-[3-(1H-benzo[*d*]imidazol-2-yl)phenyl]-4-(pyridin-4-yl)aniline |
| **34** | 6-Phenyl-*N*-[(1s,4s)-4-(1H-benzo[*d*]imidazol-2-yl)bicyclo[2.2.1]heptan-1-yl]pyridazin-3-amine |
| **35** | *N*-[3-(1H-benzo[*d*]imidazol-2-yl)adamantan-1-yl]-6-phenylpyridazin-3-amine |
| **36** | 6-Phenyl-*N*-{3-[6-(trifluoromethyl)-1H-benzo[*d*]imidazol-2-yl]adamantan-1-yl}pyridazin-3-amine |
| **37** | 6-Phenyl-*N*-[(1s,4s)-4-[6-(trifluoromethyl)-1H-benzo[*d*]imidazol-2-yl]bicyclo[2.2.1]heptan-1-yl]pyridazin-3-amine |
| **38** | *N*-[3-(1H-benzo[*d*]imidazol-2-yl)phenyl]-5-(3-fluorophenyl)-N-methylpyrimidin-2-amine |
| **39** | *N*-[5-(1H-benzo[*d*]imidazol-2-yl)-2-methoxyphenyl]-5-(3-fluorophenyl)pyrimidin-2-amine |
| **40** | 4-(1H-benzo[*d*]imidazol-2-yl)-2-{[5-(3-fluorophenyl)pyrimidin-2-yl]amino}phenol |
| **41** | 5-(3-Fluorophenyl)-*N*-{2-methoxy-5-[6-(trifluoromethyl)-1H-benzo[*d*]imidazol-2-yl]phenyl}pyrimidin-2-amine |
| **42** | 2-{[5-(3-Fluorophenyl)pyrimidin-2-yl]amino}-4-[6-(trifluoromethyl)-1H-benzo[*d*]imidazol-2-yl]phenol |
| **43** | *N*-[3-(1H-benzo[*d*]imidazol-2-yl)phenyl]-5-(4-methylthiophen-3-yl)pyrimidin-2-amine |
| **44** | *N*-[3-(1H-benzo[*d*]imidazol-2-yl)phenyl]-5-(pyridin-2-yl)pyrimidin-2-amine |
| **45** | *N*-[3-(6-fluoro-1H-benzo[*d*]imidazol-2-yl)phenyl]-5-(pyridin-2-yl)pyrimidin-2-amine |
| **46** | *N*-(3-{1H-imidazolo[4,5-c]pyridin-2-yl}phenyl)-4-(pyridazin-3-yl)aniline |
| **47** | *N*-(3-{1H-imidazolo[4,5-c]pyridin-2-yl}phenyl)-[2,3'-bipyridin]-6'-amine |
| **48** | *N*-(3-{1H-imidazolo[4,5-c]pyridin-2-yl}phenyl)-5-(pyridin-2-yl)pyrimidin-2-amine |
| **49** | *N*-(3-{1H-imidazolo[4,5-c]pyridin-2-yl}phenyl)-5-(pyridazin-3-yl)pyrimidin-2-amine |
| **50** | *N*-(3-{1H-imidazolo[4,5-c]pyridin-2-yl}phenyl)-5-(pyridazin-3-yl)pyridin-2-amine |
| **51** | *N*-[3-(6-fluoro-1H-benzo[*d*]imidazol-2-yl)phenyl]-5-(pyridazin-3-yl)pyrimidin-2-amine |
| **52** | 5-(Pyridazin-3-yl)-N-{3-[6-(trifluoromethyl)-1H-benzo[*d*]imidazol-2-yl]phenyl}pyrimidin-2-amine |
| **53** | *N*-[3-(1H-benzo[*d*]imidazol-2-yl)phenyl]-5-(pyridazin-3-yl)pyrimidin-2-amine |
| **54** | *N*-(3-{1H-imidazolo[4,5-c]lpyridin-2-yl}phenyl)-6-phenylpyridazin-3-amine |
| **55** | *N*-[3-(6-fluoro-1H-benzo[*d*]imidazol-2-yl)phenyl]-5-(pyridazin-3-yl)pyridin-2-amine |
| **56** | *N*-[3-(1H-benzo[*d*]imidazol-2-yl)phenyl]-5-(pyridazin-3-yl)pyridin-2-amine |
| **57** | *N*-{3-[6-(trifluoromethyl)-1H-benzo[*d*]imidazol-2-yl]phenyl}-[2,3'-bipyridin]-6'-amine |
| **58** | *N*-[3-(6-fluoro-1H-benzo[*d*]imidazol-2-yl)phenyl]-[2,3'-bipyridin]-6'-amine |
| **59** | *N*-[3-(1H-benzo[*d*]imidazol-2-yl)phenyl]-[2,3'-bipyridin]-6'-amine |
| **60** | *N*-(3-{1H-imidazolo[4,5-c]pyridin-2-yl}phenyl)-4-(pyrimidin-5-yl)aniline |
| **61** | Methyl 2-(3-{[4-(pyridazin-3-yl)phenyl]amino}phenyl)-1H-benzo[*d*]imidazol-6-carboxylate |
| **62** | 2-(3-{[4-(Pyridazin-3-yl)phenyl]amino}phenyl)-1H-benzo[*d*]imidazol-6-carboxylic acid |
| **63** | *N*-{3-[6-(trifluoromethyl)-1H-benzo[*d*]imidazol-2-yl]phenyl}-[2,5'-bipyrimidin]-2'-amine |
| **64** | *N*-(3-{6-chloro-1H-imidazolo[4,5-c]pyridin-2-yl}phenyl)-5-(pyridin-2-yl)pyrimidin-2-amine |
| **65** | *N*-(propan-2-yl)-2-(3-{[4-(pyridazin-3-yl)phenyl]amino}phenyl)-1H-benzo[*d*]imidazol-6-carboxamide |
| **66** | *N*-cyclohexyl-2-(3-{[4-(pyridazin-3-yl)phenyl]amino}phenyl)-1H-benzo[*d*]imidazol-6-carboxamide |
| **67** | *N*-cyclopentyl-2-(3-{[4- (pyridazin-3-yl)phenyl]amino}phenyl)-1H-benzo[*d*]imidazol-6-carboxamide |
| **68** | *N*-[3-(1H-benzo[*d*]imidazol-2-yl)phenyl]-5-(pyrimidin-4-yl)pyridin-2-amine |
| **69** | *N*-[3-(1H-benzo[*d*]imidazol-2-yl)phenyl]-5-(pyrimidin-2-yl)pyridin-2-amine |
| **70** | *N*-[3-(1H-benzo[*d*]imidazol-2-yl)phenyl]-[5,5'-bipyrimidin]-2-amine |
| **71** | *N*-[3-(1H-benzo[*d*]imidazol-2-yl)phenyl]-[2,5'-bipyrimidin]-2'-amine |
| **72** | *N*-[3-(6-fluoro-1H-benzo[*d*]imidazol-2-yl)phenyl]-5-(pyrimidin-4-yl)pyridin-2-amine |
| **73** | *N*-[3-(1H-benzo[*d*]imidazol-2-yl)phenyl]-5-(4-fluoropyridin-2-yl)pyrimidin-2-amine |
| **74** | *N*-[3-(1H-benzo[*d*]imidazol-2-yl)phenyl]-[1,1'-biphenyl]-3-amine |
| **75** | *N*-[3-(1H-benzo[*d*]imidazol-2-yl)phenyl]-3-(pyrimidin-5-yl)aniline |
| **76** | *N*-[3-(1H-benzo[*d*]imidazol-2-yl)phenyl]-[4,5'-bipyrimidin]-2'-amine |
| **77** | *N*-[3-(1H-benzo[*d*]imidazol-2-yl)phenyl]-2-phenylpyrimidin-5-amine |
| **78** | Methyl 2-(3-{[4-(pyrimidin-2-yl)phenyl]amino}phenyl)-1H-benzo[*d*]imidazol-6-carboxylate |
| **79** | *N*-[4-(pyridazin-3-yl)phenyl]-3-[6-(trifluoromethyl)-1H-imidazolo[4,5-c]pyridin-2-yl]aniline |
| **80** | *N*-[3-(1*H*-benzo[*d*]imidazol-2-yl)phenyl]-6-phenyl-pyridin-3-amine |
| **81** | 3-(5,6-difluoro-1*H*-benzo[*d*]imidazol-2-yl)-*N*-(4-pyridazin-3-ylphenyl)aniline |
| **82** | *N*-[3-(6-chloro-1*H*-benzo[*d*]imidazol-2-yl)phenyl]-5-(2-pyridyl)pyrimidin-2-amine |
| **83** | 5-(2-pyridyl)-*N*-[3-[6-(trifluoromethyl)-1*H*-benzo[*d*]imidazol-2-yl]phenyl]pyrimidin-2-amine |
| **84** | 3-(6-chloro-1*H*-imidazo[4,5-*c*]pyridin-2-yl)-*N*-(4-pyridazin-3-ylphenyl)aniline |
| **85** | *N,N*-dimethyl-2-(3-((4-(pyridazin-3-yl)phenyl)amino)phenyl)-1*H-*benzo[*d*]imidazole-6-carboxamide |
| **86** | *N*-(4-pyrimidin-4-ylphenyl)-3-[6-(trifluoromethyl)-1*H*-imidazo[4,5-*c*]pyridin-2-yl]aniline |
| **87** | *N*-methyl-2-(3-((4-(pyridazin-3-yl)phenyl)amino)phenyl)-1*H-*benzo[*d*]imidazole-6-carboxamide |
| **88** | 5-pyrimidin-4-yl-*N*-[3-[6-(trifluoromethyl)-1*H*-benzo[*d*]imidazol-2-yl]phenyl]pyridin-2-amine |
| **89** | 5-pyrimidin-4-yl-*N*-[3-[6-(trifluoromethyl)-1*H*-imidazo[4,5-*c*]pyridin-2-yl]phenyl]pyridin-2-amine |
| **90** | 3-(1*H*-indol-2-yl)-*N*-(4-pyridazin-3-ylphenyl)aniline |
| **91** | 3-(1-methylbenzimidazol-2-yl)-*N*-(4-pyridazin-3-ylphenyl)aniline |
| **92** | 3-(6-fluoro-1*H*-benzo[*d*]imidazol-2-yl)-N-[4-(2-pyridyl)phenyl]aniline |
| **93** | 3-[6-(3-methyl-1,2,4-oxadiazol-5-yl)-1*H*-benzo[*d*]imidazol-2-yl]-N-(4-pyridazin-3-ylphenyl)aniline |
| **94** | 3-(1*H*-benzo[*d*]imidazol-2-yl)-*N*-(4-pyridazin-4-ylphenyl)aniline |
| **95** | 3-(5,6-difluoro-1*H*-benzo[*d*]imidazol-2-yl)-*N*-(4-pyrimidin-4-ylphenyl)aniline |
| **96** | 3-(6-fluoro-1*H*-benzo[*d*]imidazol-2-yl)-*N*-(4-pyrimidin-4-ylphenyl)aniline |
| **97** | 3-(1*H*-benzo[*d*]imidazol-2-yl)-*N*-(4-cyclohexylphenyl)aniline |
| **98** | *N*-[4-(2-pyridyl)phenyl]-3-[6-(trifluoromethyl)-1*H*-benzo[d]imidazol-2-yl]aniline |
| **99** | *N*-[3-(5,6-difluoro-1*H*-benzo[*d*]imidazol-2-yl)phenyl]-5-pyridazin-3-yl-pyrimidin-2-amine |
| **100** | 5-pyrimidin-2-yl-*N*-[3-[6-(trifluoromethyl)-1*H*-benzo[*d*]imidazol-2-yl]phenyl]pyridin-2-amine |
| **101** | 3-(7-methyl-1*H*-benzo[*d*]imidazol-2-yl)-*N*-(4-pyridazin-3-ylphenyl)aniline |
| **102** | 3-(5-chloro-6-fluoro-1*H*-benzo[*d*]imidazol-2-yl)-*N*-(4-pyridazin-3-ylphenyl)aniline |
| **103** | 5-pyridazin-3-yl-*N*-[3-[6-(trifluoromethyl)-1*H*-imidazo[4,5-*c*]pyridin-2-yl]phenyl]pyrimidin-2-amine |
| **104** | 3-[6-fluoro-5-(trifluoromethyl)-1*H*-benzo[*d*]imidazol-2-yl]-*N*-(4-pyridazin-3-ylphenyl)aniline |
| **105** | 3-(6-methyl-1*H*-benzo[*d*]imidazol-2-yl)-*N*-(4-pyridazin-3-ylphenyl)aniline |
| **106** | 3-(6-methylsulfonyl-1*H*-benzo[*d*]imidazol-2-yl)-*N*-(4-pyridazin-3-ylphenyl)aniline |
| **107** | 3-(6-tert-butyl-1*H*-benzo[*d*]imidazol-2-yl)-*N*-(4-pyridazin-3-ylphenyl)aniline |
| **108** | *N*-[3-(6-methoxy-1*H*-benzo[*d*]imidazol-2-yl)phenyl]-5-pyridazin-3-yl-pyrimidin-2-amine |
| **109** | *N*-(4-pyrimidin-4-ylphenyl)-3-[6-(trifluoromethoxy)-1*H-*benzo[*d*]imidazol-2-yl]aniline |
| **111** | isopropyl 2-(3-((4-(pyridazin-3-yl)phenyl)amino)phenyl)-1*H-*benzo[*d*]imidazole-6-carboxylate |
| **112** | 4-[4-[3-[6-(trifluoromethyl)-1*H*-benzo[*d*]imidazol-2-yl]anilino]phenyl]benzonitrile |
| **113** | 6-(1-methyl-4-piperidyl)-*N*-[3-[6-(trifluoromethyl)-1*H-*benzo[*d*]imidazol-2-yl]phenyl]pyridazin-3-amine |
| **114** | 3-(6-fluoro-5-methyl-1*H*-benzo[*d*]imidazol-2-yl)-*N*-(4-pyridazin-3-ylphenyl)aniline |
| **115** | 3-(7-chloro-1*H*-benzo[*d*]imidazol-2-yl)-*N*-(4-pyridazin-3-ylphenyl)aniline |
| **116** | 3-(6-chloro-1*H*-imidazo[4,5-*c*]pyridin-2-yl)-*N*-(4-pyrimidin-2-ylphenyl)aniline |
| **117** | 3-(6-chloro-1*H*-imidazo[4,5-*c*]pyridin-2-yl)-*N*-(4-pyrimidin-4-ylphenyl)aniline |
| **118** | 5-(2-pyridyl)-*N*-[3-[6-(trifluoromethoxy)-1*H*-benzo[*d*]imidazol-2-yl]phenyl]pyrimidin-2-amine |
| **119** | 3-(6-chloro-1*H*-imidazo[4,5-*c*]pyridin-2-yl)-*N*-[4-(2-pyridyl)phenyl]aniline |
| **120** | *N*-[3-[6-chloro-5-(trifluoromethoxy)-1*H*-benzo[*d*]imidazol-2-yl]phenyl]-5-pyridazin-3-yl-pyrimidin-2-amine |
| **121** | *N*-[3-(6-fluoro-5-methoxy-1*H*-benzo[*d*]imidazol-2-yl)phenyl]-5-pyridazin-3-yl-pyrimidin-2-amine |
| **122** | 2-[3-(4-pyridazin-3-ylphenyl)sulfonylphenyl]-6-(trifluoromethyl)-1*H*-benzo[*d*]imidazole |
| **123** | *N*-[3-(1*H*-benzo[*d*]imidazol-2-yl)phenyl]-5-pyridazin-3-yl-1,3,4-oxadiazol-2-amine |
| **124** | *N*-(4-pyridazin-3-ylcyclohexyl)-3-[6-(trifluoromethyl)-1*H-*benzo[*d*]imidazol-2-yl]aniline |
| **125** | 1-pyridazin-3-yl-*N*-[3-[6-(trifluoromethyl)-1*H*-benzo[*d*]imidazol-2-yl]phenyl]piperidin-4-amine |
| **126** | 3-(5,6-difluoro-1*H*-benzo[*d*]imidazol-2-yl)-*N*-(4-(pyrimidin-2-yl)phenyl)aniline |
| **150** | 3-(6-fluoro-1*H*-benzo[*d*]imidazol-2-yl)-*N*-[4-(4-methylpiperazin-1-yl)phenyl]aniline |
| **151** | *N*-[3-(6-fluoro-1*H*-benzo[*d*]imidazol-2-yl)phenyl]-5-(4-methylpiperazin-1-yl)pyridin-2-amine |
| **152** | *N*-[4-(4-methylpiperazin-1-yl)phenyl]-3-[6-(trifluoromethyl)-1*H-*benzo[*d*]imidazol-2-yl]aniline |
| **153** | 5-(4-methylpiperazin-1-yl)-*N*-[3-[6-(trifluoromethyl)-1*H-*benzo[*d*]imidazol-2-yl]phenyl]pyridin-2-amine |
| **154** | *N*-[4-[2-(4-methylpiperazin-1-yl)pyrimidin-5-yl]phenyl]-3-[6-(trifluoromethyl)-1*H*-benzo[*d*]imidazol-2-yl]aniline |
| **155** | 3-(5-fluoro-1*H*-benzo[*d*]imidazol-2-yl)-*N*-[4-[2-(4-methylpiperazin-1-yl)pyrimidin-5-yl]phenyl]aniline |
| **156** | 5-(1*H*-benzo[*d*]imidazol-2-yl)-2-(4-methylpiperazin-1-yl)-*N*-(4-pyrimidin-5-ylphenyl)aniline |
| **157** | (1*R*,2*R*)-2-(1*H*-benzo[*d*]imidazol-2-yl)-*N*-(4-pyrazin-2-ylphenyl)cyclopropanecarboxamide |
| **158** | (1*R*,2*R*)-2-(5-fluoro-1*H*-benzo[*d*]imidazol-2-yl)-*N*-(4-pyrazin-2-ylphenyl)cyclopropanecarboxamide |
| **159** | (1*R*,2*R*)-2-(1*H*-benzo[*d*]imidazol-2-yl)-*N*-(4-pyrimidin-5-ylphenyl)cyclopropanecarboxamide |
| **160** | (1*R*,2*R*)-2-(5-fluoro-1*H*-benzo[*d*]imidazol-2-yl)-*N*-(4-pyrimidin-5-ylphenyl)cyclopropanecarboxamide |
| **161** | 5-(1*H*-benzo[*d*]imidazol-2-yl)-2-(4-methylpiperazin-1-yl)-*N*-(4-pyrazin-2-ylphenyl)aniline |
| **162** | 5-(6-fluoro-1*H*-benzo[*d*]imidazol-2-yl)-2-(4-methylpiperazin-1-yl)-*N-*(4-pyrazin-2-ylphenyl)aniline |
| **163** | *N*-[3-(1*H*-benzo[*d*]imidazol-2-yl)phenyl]-5-(3-furyl)pyrimidin-2-amine |
| **164** | *N*-[3-(1*H*-benzo[*d*]imidazol-2-yl)phenyl]-5-(3-thienyl)pyrimidin-2-amine |
| **165** | 3-(1*H*-benzo[*d*]imidazol-2-yl)-*N*-(4-oxazol-2-ylphenyl)aniline |
| **166** | 3-(1*H*-benzo[*d*]imidazol-2-yl)-*N*-(4-thiazol-4-ylphenyl)aniline |
| **167** | 2-(1*H*-benzo[*d*]imidazol-2-yl)-*N*¹,*N*¹-dimethyl-*N*⁴-(4-pyrazin-2-ylphenyl)benzene-1,4-diamine |
| **168** | 3-(1*H*-benzo[*d*]imidazol-2-yl)-4-[2-(dimethylamino)ethoxy]-*N*-(4-pyrazin-2-ylphenyl)aniline |
| **169** | 3-(1*H*-benzo[*d*]imidazol-2-yl)-4-[2-(4-methylpiperazin-1-yl)ethoxy]-*N*-(4-pyrazin-2-ylphenyl)aniline |
| **170** | 2-(1*H*-benzo[d*]*imidazol-2-yl)-*N*¹,*N*¹-dimethyl-*N*⁴-(4-pyrimidin-5-ylphenyl)benzene-1,4-diamine |
| **171** | *N*-[3-(1*H*-benzo[d]imidazol-2-yl)phenyl]-5-oxazol-2-yl-pyrimidin-2-amine |
| **172** | 3-(1*H*-benzo[*d*]imidazol-2-yl)-4-[(1-methyl-4-piperidyl)oxy]-*N*-(4-pyrazin-2-ylphenyl)aniline |
| **173** | *N*-[3-(1*H*-benzo[*d*]imidazol-2-yl)phenyl]-5-thiazol-4-yl-pyrimidin-2-amine |
| **174** | 2-[2-(dimethylamino)ethoxy]-*N*-(4-pyrazin-2-ylphenyl)-5-[6-(trifluoromethyl)-1*H*-benzo[*d*]imidazol-2-yl]aniline |
| **175** | 2-[2-(4-methylpiperazin-1-yl)ethoxy]-*N*-(4-pyrazin-2-ylphenyl)-5-[6-(trifluoromethyl)-1*H*-benzo[*d*]imidazol-2-yl]aniline |
| **176** | *N*-[2-(4-methylpiperazin-1-yl)-5-[6-(trifluoromethyl)-1*H-*benzo[*d*]imidazol-2-yl]phenyl]-5-pyridazin-3-yl-pyrimidin-2-amine |
| **177** | *N*-[2-[(1-methyl-4-piperidyl)oxy]-3-[6-(trifluoromethyl)-1*H-*benzo[*d*]imidazol-2-yl]phenyl]-5-pyridazin-3-yl-pyrimidin-2-amine |
| **178** | *N*-[3-[(1-methyl-4-piperidyl)oxy]-5-[6-(trifluoromethyl)-1*H-*benzo[*d*]imidazol-2-yl]phenyl]-5-pyridazin-3-yl-pyrimidin-2-amine |
| **179** | *N*-[3-[2-(dimethylamino)ethoxy]-5-[6-(trifluoromethyl)-1*H-*benzo[*d*]imidazol-2-yl]phenyl]-5-pyridazin-3-yl-pyrimidin-2-amine |
| **200** | *N*-(3-(1*H*-benzo[*d*]imidazol-2-yl)phenyl)-6-(pyrimidin-2-yl)pyridazin-3-amine |
| **201** | *N*-(3-(6-fluoro-1*H*-benzo[*d*]imidazol-2-yl) phenyl)-6-(pyrimidin-2-yl)pyridazin-3-amine |
| **202** | 6-(pyrimidin-2-yl)-*N*-(3-(6-(trifluoromethyl)-1*H*-benzo[*d*]imidazol-2-yl)phenyl)pyridazin-3-amine |
| **203** | *N*-(3-(1*H*-benzo[*d*]imidazol-2-yl)phenyl)-2-methyl-4-(pyridazin-3-yl)aniline |
| **204** | *N*-(3-(1*H*-benzo[d]imidazol-2-yl)phenyl)-3-methyl-4-(pyridazin-3-yl)aniline |
| **205** | *N*-(3-(1*H*-benzo[*d*]imidazol-2-yl)phenyl)-2-nitro-4-(pyridazin-3-yl)aniline |
| **206** | *N*-(3-(1*H*-benzo[*d*]imidazol-2-yl)phenyl)-3-nitro-4-(pyridazin-3-yl)aniline |
| **207** | *N*¹-(3-(1*H*-benzo[*d*]imidazol-2-yl)phenyl)-4-(pyridazin-3-yl)benzene-1,3-diamine |
| **208** | *N*-(3-(1*H*-benzo[*d*]imidazol-2-yl)phenyl)-3-methoxy-4-(pyridazin-3-yl)aniline |
| **209** | 5-((3-(1*H*-benzo[*d*]imidazol-2-yl)phenyl)amino)-2-(pyridazin-3-yl)phenol |
| **210** | *N*-(3-(1*H*-benzo[*d*]imidazol-2-yl)phenyl)-2-methoxy-4-(pyridazin-3-yl)aniline |
| **211** | 2-((3-(1*H*-benzo[*d*]imidazol-2-yl)phenyl)amino)-5-(pyridazin-3-yl)phenol |
| **212** | 3-(1*H*-benzo[*d*]imidazol-2-yl)-*N*-(4-(benzofuran-2-yl)phenyl)aniline |
| **213** | 2-(3-(4-(pyridazin-3-yl)phenoxy)phenyl)-1*H*-benzo[d]imidazole |
| **214** | 5-(1*H*-benzo[*d*]imidazol-2-yl)-*N*-(4-(pyridazin-3-yl)phenyl)thiazol-2-amine |
| **215** | 3-(1*H*-benzo[*d*]imidazol-2-yl)-*N*-(4-morpholinophenyl)aniline |
| **216** | 3-(benzo[*d*]oxazol-2-yl)-*N*-(4-(pyridazin-3-yl)phenyl)aniline |
| **217** | *N,N*-dimethyl-2-(3-((4-(pyridazin-3-yl)phenyl)amino)phenyl)-1*H-*benzo[*d*]imidazol-6-amine |
| **218** | 3-(6-bromo-1*H*-imidazo[4,5-*c*]pyridin-2-yl)-*N*-(4-(pyridazin-3-yl)phenyl)aniline |
| **219** | 3-(1*H*-imidazo[4,5-*b*]pyridin-2-yl)-*N*-(4-(pyridazin-3-yl)phenyl)aniline |
| **220** | methyl 2-(3-((4-(pyridazin-3-yl)phenyl)amino)phenyl)-1*H-*imidazo[4,5-*c*]pyridine-6-carboxylate |
| **221** | 2-(3-((4-(pyridazin-3-yl) phenyl)amino)phenyl)-1*H*-imidazo[4,5-*c*]pyridine-6-carboxylic acid |
| **222** | 3-(6-(benzyloxy)-1*H*-benzo[*d*]imidazol-2-yl)-*N*-(4-(pyridazin-3-yl)phenyl)aniline |
| **223** | 3-(6-methoxy-1*H*-benzo[*d*]imidazol-2-yl)-N-(4-(pyridazin-3-yl)phenyl)aniline |
| **224** | 2-(3-((4-(pyridazin-3-yl)phenyl)amino)phenyl)-1*H*-benzo[*d*]imidazol-6-ol |
| **225** | 3-(5,6-dichloro-1*H*-benzo[*d*]imidazol-2-yl)-*N*-(4-(pyridazin-3-yl)phenyl)aniline |
| **226** | 3-(6-chloro-1*H*-benzo[*d*]imidazol-2-yl)-*N*-(4-(pyridazin-3-yl)phenyl)aniline |
| **227** | 3-(6-nitro-1*H*-benzo[*d*]imidazol-2-yl)-*N*-(4-(pyridazin-3-yl)phenyl)aniline |
| **228** | 2-(3-((4-(pyridazin-3-yl)phenyl)amino)phenyl)-1*H*-benzo[*d*]imidazol-6-amine |
| **229** | *N*-benzyl-2-(3-((4-(pyridazin-3-yl)phenyl)amino)phenyl)-1*H-*benzo[*d*]imidazol-6-amine |
| **230** | *N*-(4-(pyridazin-3-yl)phenyl)-3-(6-(trifluoromethoxy)-1*H-*benzo[*d*]imidazol-2-yl)aniline |
| **231** | 2-(3-((4-(pyridazin-3-yl)phenyl)amino)phenyl)-1*H*-benzo[*d*]imidazole-6-carbonitrile |
| ***232*** | N-(4-(pyridazin-3-yl)phenyl)-3-(6-(trifluoromethoxy)-1*H-*imidazo[4,5-*c*]pyridin-2-yl)aniline |
| **234** | *N*-(4-(pyridazin-3-yl)phenyl)-3-(6-(trifluoromethyl)-1*H*-imidazo[4,5-*b*]pyridin-2-yl)aniline |
| **235** | *N*-(4-(pyridazin-3-yl)phenyl)-3-(5-(trifluoromethyl)-1*H*-imidazo[4,5-*b*]pyridin-2-yl)aniline |
| **238** | *N*-(3-(6-(trifluoro methyl)-1*H*-imidazo [4,5-*c*]pyridin-2-yl) phenyl)-[2,3'-bipyridin]-6'-amine |
| **242** | 5-(pyridin-2-yl)-*N*-(3-(6-(trifluoromethyl)-1*H*-imidazo[4,5-*c*]pyridin-2-yl)phenyl)pyrimidin-2-amine |
| **243** | 5-(pyridin-2-yl)-*N*-(3-(6-(trifluoromethyl)-1*H*-imidazo[4,5-*b*]pyridin-2-yl)phenyl)pyrimidin-2-amine |
| **244** | 5-(pyridin-2-yl)-*N*-(3-(5-(trifluoromethyl)-1*H*-imidazo[4,5-*b*]pyridin-2-yl)phenyl)pyrimidin-2-amine |
| **247** | *N*-(3-(6-fluoro-1*H*-benzo[*d*]imidazol-2-yl)phenyl)-[2,5'-bipyrimidin]-2'-amine |
| **249** | 3-(1*H*-benzo[*d*]imidazol-2-yl)-5-methyl-*N*-(4-(pyridazin-3-yl)phenyl)aniline |
| **250** | 3-(1*H*-benzo[*d*]imidazol-2-yl)-*N*-(4-(pyridazin-3-yl) phenyl)-5-(trifluoromethyl)aniline |
| **251** | *N*-(4-(3-fluoropyridin-2-yl)phenyl)-3-(6-(trifluoromethyl)-1*H-*benzo[*d*]imidazol-2-yl)aniline |
| **252** | *N*-(4-(6-fluoropyridin-2-yl)phenyl)-3-(6-(trifluoromethyl)-1*H-*benzo[*d*]imidazol-2-yl)aniline |
| **253** | *N*-(4-(5-fluoropyridin-2-yl)phenyl)-3-(6-(trifluoromethyl)-1*H-*benzo[*d*]imidazol-2-yl)aniline |
| **254** | 5-(pyridazin-3-yl)-*N*-(3-(6-(trifluoromethyl)-1*H*-imidazo[4,5-*c*]pyridin-2-yl)phenyl)pyridin-2-amine |
| **255** | *N*-(3-(1*H*-benzo[*d*] imidazol-2-yl)phenyl)-6-(pyridin-2-yl)pyrimidin-3-amine |
| **256** | 6-(pyridin-2-yl)-*N*-(3-(6-(trifluoromethyl)-1*H*-benzo[*d*]imidazol-2-yl)phenyl)pyridazin-3-amine |
| **257** | *N*-(3-(1*H*-benzo[*d*]imidazol-2-yl)phenyl)-6-(pyrimidin-4-yl)pyridazin-3-amine |
| **258** | 5-(6-fluoropyridin-2-yl)-*N*-(3-(6-(trifluoromethyl)-1*H-*benzo[*d*]imidazol-2-yl)phenyl)pyrimidin-2-amine |
| **259** | *N*-(3-(1*H*-benzo[*d*]imidazol-2-yl)phenyl)-5-(6-fluoropyridin-2-yl) pyrimidin-2-amine |
| **260** | 5-(5-fluoropyridin-2-yl)-*N*-(3-(6-(trifluoromethyl)-1*H-*benzo[*d*]imidazol-2-yl)phenyl)pyrimidin-2-amine |
| **261** | *N*-(4-(pyridazin-3-yl) phenyl)-4-(6-(trifluoromethyl)-1*H-*benzo[*d*]imidazol-2-yl)pyridin-2-amine |
| **262** | *N*-(4-(pyridazin-3-yl) phenyl)-5-(6-(trifluoromethyl)-1*H-*benzo[*d*]imidazol-2-yl)pyridin-3-amine |
| **263** | *N*-(4-(pyridazin-3-yl) phenyl)-2-(6-(trifluoromethyl)-1*H-*benzo[*d*]imidazol-2-yl)pyridin-4-amine |
| **264** | *N*-(4-(6-fluoropyridazin-3-yl)phenyl)-3-(6-(trifluoromethyl)-1*H-*benzo[*d*]imidazol-2-yl) aniline |
| **265** | 3-(1*H*-benzo[*d*]imidazol-2-yl)-*N*-(4-(6-fluoropyridazin-3-yl)phenyl)aniline |
| **266** | 3-(1*H*-benzo[*d*]imidazole-2-yl)-*N*-(4-(4-(trifluoromethyl)pyridazin-3-yl)phenyl)aniline |
| **267** | 3-(1*H*-benzo[*d*]imidazol-2-yl)-*N*-(4-(5-(trifluoromethyl)pyridazin-3-yl)phenyl)aniline |
| **268** | 3-(1*H*-benzo[*d*]imidazol-2-yl)-*N*-(4-(6-(trifluoromethyl)pyridazin-3-yl)phenyl)aniline |
| **269** | *N*-(3-(1*H*-benzo[*d*]imidazol-2-yl)phenyl)-2-fluoro-4-(pyridazin-3-yl)aniline |
| **270** | *N*-(3-(1*H*-benzo[*d*]imidazol-2-yl)phenyl)-3-fluoro-4-(pyridazin-3-yl)aniline |
| **271** | *N*-(3-(1*H*-benzo[*d*]imidazol-2-yl)phenyl)-6-phenyl-1,2,4-triazin-3-amine |
| **272** | *N*-(3-(1*H*-benzo[*d*]imidazol-2-yl)phenyl)-2'-(trifluoromethyl)-[1,1'-biphenyl]-4-amine |
| **273** | methyl 6-(4-((3-(1*H*-benzo[*d*]imidazol-2-yl) phenyl)amino)phenyl)pyridazine-4-carboxylate |
| **274** | 6-(4-((3-(1*H*-benzo[*d*]imidazol-2-yl)phenyl) amino)phenyl)pyridazine-4-carboxylic acid |
| **275** | 3-(1*H*-benzo[*d*] imidazol-2-yl)-*N*-(4-(6-methoxypyridazin-3-yl)phenyl)aniline |
| **276** | *N*-(3-(1*H*-benzo[*d*] imidazol-2-yl)phenyl)-5-(pyridin-2-yl)pyrazin-2-amine |
| **277** | *N*-(3-(1*H*-benzo[*d*] imidazol-2-yl)phenyl)-5-(pyrimidin-4-yl)pyrazin-2-amine |
| **278** | *N*-(4-(6-methoxypyridazin-3-yl)phenyl)-3-(6-(trifluoromethyl)-1*H-*benzo[*d*]imidazol-2-yl)aniline |
| **279** | *N*-methyl-2-(3-((4-(pyridazin-3-yl)phenyl) amino)phenyl)-1*H-*benzo[*d*]imidazol-6-amine |
| **280** | *N*-benzyl-*N*-methyl-2-(3-((4-(pyridazin-3-yl)phenyl)amino)phenyl)-1*H-*benzo[*d*]imidazol-6-amine |
| **281** | *N*-(3-(6-fluoro-1*H*-imidazo[4,5-*c*]pyridin-2-yl)phenyl)-[2,3'-bipyridin]-6'-amine |
| **282** | *N*-(3-(6-chloro-1*H*-imidazo[4,5-*c*]pyridin-2-yl)phenyl)-[2,3'-bipyridin]-6'-amine |
| **283** | *N*-(3-(5,6-difluoro-1*H*-benzo[*d*]imidazol-2-yl) phenyl)-[2,3'-bipyridin] -6'-amine |
| **284** | *N*-(3-(6-(difluoromethoxy)-1*H*-benzo[*d*]imidazol-2-yl)phenyl)-[2,3'-bipyridin]-6'-amine |
| **285** | *N*-(3-(6-(difluoromethoxy)-1*H*-benzo[*d*]imidazol-2-yl)phenyl)-5-(pyridin-2-yl)pyrimidin-2-amine |
| **286** | *N*-(3-(6-fluoro-1*H*-imidazo[4,5-*c*]pyridin-2-yl)phenyl)-5-(pyridin-2-yl)pyrimidin-2-amine |
| **287** | *N*-(3-(6-(difluoromethoxy)-1*H*-benzo[*d*]imidazol-2-yl)phenyl)-5-(pyridazin-3-yl)pyrimidin-2-amine |
| **288** | *N*-(3-(6-(2-morpholinoethoxy)-1*H*-benzo[*d*]imidazol-2-yl)phenyl)-5-(pyridazin-3-yl)pyrimidin-2-amine |
| **289** | 5-(pyridazin-3-yl)-*N*-(3-(7-(trifluoromethyl)-1*H*-benzo[*d*]imidazol-2-yl) phenyl)pyrimidin-2-amine |
| **290** | *N*-(3-(7-fluoro-1*H*-benzo[*d*]imidazol-2-yl)phenyl)-5-(pyridazin-3-yl)pyrimidin-2-amine |
| **291** | *N*-(3-(7-chloro-1*H*-benzo[*d*]imidazol-2-yl)phenyl)-5-(pyridazin-3-yl)pyrimidin-2-amine |
| **292** | *N*-(3-(5,7-difluoro-1*H*-benzo[*d*]imidazol-2-yl)phenyl)-5-(pyridazin-3-yl)pyrimidin-2-amine |
| **293** | *N*-(3-(6,7-difluoro-1*H*-benzo[*d*]imidazol-2-yl)phenyl)-5-(pyridazin-3-yl)pyrimidin-2-amine |
| **294** | *N*-(3-(6-chloro-5-(trifluoromethyl)-1*H*-benzo[*d*]imidazol-2-yl)phenyl)-5-(pyridazin-3-yl)pyrimidin-2-amine |
| **295** | 5-(pyridazin-3-yl)-*N*-(3-(6-(trifluoromethoxy)-1*H*-benzo[*d*]imidazol-2-yl)phenyl)pyrimidin-2-amine |
| **296** | 6-(pyrimidin-4-yl)-*N*-(3-(6-(trifluoromethyl)-1*H*-benzo[*d*]imidazol-2-yl)phenyl)pyridazin-3-amine |
| **297** | *N*-(3-(5,6-difluoro-1*H*-benzo[*d*]imidazol-2-yl) phenyl)-5-(pyrimidin-4-yl)pyrazin-2-amine |
| **298** | *N*-(3-(5,6-difluoro-1*H*-benzo[*d*]imidazol-2-yl) phenyl)-5-(pyridin-2-yl)pyrazin-2-amine |
| **299** | *N*-(3-(6-methoxy-1*H*-benzo[*d*]imidazol-2-yl) phenyl)-5-(pyridin-2-yl)pyrazin-2-amine |
| **300** | *N*-(3-(6-(difluoromethoxy)-1*H*-benzo[*d*]imidazol-2-yl)phenyl)-5-(pyridin-2-yl)pyridazin-2-amine |
| **301** | *N*-(3-(6-chloro-1*H*-imidazo[4,5-*c*]pyridin-2-yl)phenyl)-5-(pyridin-2-yl)pyrazin-2-amine |
| **302** | 5-(pyridin-2-yl)-*N*-(3-(6-(trifluoromethyl)-1*H*-benzo[*d*]imidazol-2-yl)phenyl)pyrazin-2-amine |
| **303** | *N*-(3-(6-chloro-1*H*-imidazo[4,5-*c*]pyridin-2-yl)phenyl)-5-(thiazol-4-yl)pyrimidin-2-amine |
| **304** | 5-(thiazol-4-yl)-*N*-(3-(6-(trifluoromethyl)-1*H*-benzo[*d*]imidazol-2-yl)phenyl)pyrimidin-2-amine |
| **305** | *N*-(4-(4-fluoropyridin-2-yl)phenyl)-3-(6-(trifluoromethyl)-1*H-*benzo[*d*]imidazol-2-yl)aniline |
| **306** | 5-(pyridin-2-yl)-*N*-(4-(6-(trifluoromethyl)-1*H*-benzo[*d*]imidazol-2-yl)pyridin-2-yl)pyrimidin-2-amine |
| **307** | 5-(pyridin-2-yl)-*N*-(5-(6-(trifluoromethyl)-1*H*-benzo[*d*]imidazol-2-yl) pyridin-3-yl)pyrimidin-2-amine |
| **308** | 3-(6-bromo-1*H*-benzo[*d*]imidazol-2-yl)-*N*-(4-(pyridazin-3-yl) phenyl)aniline |
| **309** | *N*-(3-(6-(difluoromethoxy)-1*H*-benzo[*d*]imidazol-2-yl) phenyl)-5-(pyrimidin-4-yl)pyrazin-2-amine |
| **310** | 5-(pyrimidin-4-yl)-*N*-(3-(6-(trifluoromethyl)-1*H*-imidazo[4,5-*c*] pyridin-2-yl)phenyl)pyrazin-2-amine |
| **311** | 5-(5-((methylamino)methyl)pyridin-2-yl)-*N*-(3-(6-(trifluoromethyl)-1*H*-benzo[*d*]imidazol-2-yl)phenyl)pyrimidin-2-amine |
| **312** | 5-(5-((dimethylamino)methyl)pyridin-2-yl)-*N*-(3-(6-(trifluoromethyl)-1H-benzo[*d*]imidazol-2-yl)phenyl)pyrimidin-2-amine |
| **313** | *N*-(3-(5,6-difluoro-1*H*-benzo[*d*]imidazol-2-yl)phenyl)-5-(pyridin-2-yl)pyrimidin-2-amine |
| **314** | 6-((3-(6-(trifluoromethyl)-1*H*-benzo[*d*]imidazol-2-yl)phenyl)amino)nicotinonitrile |
| **315** | 4-((3-(6-(trifluoromethyl)-1*H*-benzo[*d*]imidazol-2-yl)phenyl)amino)benzonitrile |
| **316** | *tert*-butyl 4-(6-((3-(6-(trifluoromethyl)-1*H*-benzo[*d*]imidazol-2-yl)phenyl)amino)pyridazine-3-yl)piperidine-1-carboxylate |
| **317** | 6-(piperidin-4-yl)-*N*-(3-(6-(trifluoromethyl)-1*H*-benzo[*d*]imidazol-2-yl)phenyl)pyridazin-3-amine |
| **320** | *N*-(3-(1*H*-benzo[*d*]imidazol-2-yl)phenyl)-3'-(trifluoromethyl)-[1,1'-biphenyl]-4-amine |
| **321** | methyl 4'-((3-(1*H*-benzo[*d*]imidazol-2-yl-phenyl)amino)-[1,1'-biphenyl]-3-carboxylate |
| **322** | *N*-(3-(1*H*-benzo[*d*]imidazol-2-yl)phenyl)-2'-fluoro-[1,1'-biphenyl]-4-amine |
| **323** | *N*-(3-(6-fluoro-1*H*-benzo[*d*]imidazol-2-yl)phenyl)-5-(pyridin-2-yl)pyrazin-2-amine |
| **324** | *N*-(3-(6-chloro-1*H*-benzo[*d*]imidazol-2-yl)phenyl)-5-(pyridin-2-yl)pyrazin-2-amine |
| **325** | 5-(pyridin-2-yl)-*N*-(3-(6-(trifluoromethoxy)-1*H*-benzo[*d*]imidazol-2-yl)phenyl)pyrazin-2-amine |
| **326** | 5-(pyrimidin-4-yl)-*N*-(3-(6-(trifluoromethyl)-1*H*-benzo[*d*]imidazol-2-yl)phenyl)pyrazin-2-amine |
| **327** | 5-(pyrimidin-4-yl)-*N*-(3-(6-(trifluoromethoxy)-1*H*-benzo[*d*]imidazol-2- yl)phenyl)pyrazin-2-amine |
| **328** | 5-(pyridin-2-yl)-*N*-(3-(6-(trifluoromethoxy)-1*H*-imidazo[4,5-*c*]pyridin-2-yl)phenyl)pyrazin-2-amine |
| **329** | *N*-(3-(6-(difluoromethoxy)-1*H*-imidazo[4,5-*c*]pyridin-2-yl)phenyl)-5-(pyridin-2-yl)pyrimidin-2-amine |
| **330** | *N*-(3-(6-fluoro-1*H*-imidazo[4,5-*c*]pyridin-2-yl)phenyl)-5-(pyridin-2-yl)pyrazin-2-amine |
| **331** | 3-(6-(difluoromethoxy)-1*H*-imidazo[4,5-*c*] pyridin-2-yl)-*N*-(4-(pyridazin-3-yl)phenyl)aniline |
| **332** | *N*-(3-(5,6-difluoro-1*H*-benzo[*d*]imidazol-2-yl)phenyl)-5-(thiazol-4-yl)pyrimidin-2-amine |
| **333** | *N*-(3-(6-(difluoromethoxy)-1*H*-imidazo[4,5-*c*]pyridin-2-yl)phenyl)-5-(6-fluoropyridin-2-yl)pyrimidin-2-amine |
| **334** | 5-(6-fluoropyridin-2-yl)-*N*-(3-(6-(trifluoromethyl)-1*H*-imidazo[4,5-*c*]pyridin-2-yl)phenyl)pyrimidin-2-amine |
| **335** | 3-(6-(difluoromethoxy)-1*H*-benzo[*d*]imidazol-2-yl)-*N*-(4-(pyridazin-3-yl)phenyl)aniline |
| **336** | *N*-(3-(6-(difluoromethoxy)-1*H*-imidazo[4,5-*c*]pyridin-2-yl)phenyl)-5-(pyridin-2-yl)pyrazin-2-amine |

The term "alkyl" as used herein may refer to a straightchain or branched-chain acyclic, cyclic, or saturated hydrocarbon in which the carbon atoms are connected, unless otherwise specified. For example, "C₁₋₆ alkyl" may mean an alkyl containing 1 to 6 carbon atoms, "C₁₋₄alkyl" may mean an alkyl containing 1 to 4 carbon atoms, and "C₁₋₃alkyl" may mean an alkyl containing 1 to 3 carbon atoms. The acyclic alkyl may include, for example, methyl, ethyl, n-propyl, n-butyl, isopropyl, sec-butyl, isobutyl, tert-butyl, and the like, but is not limited thereto. The cyclic alkyl may be used interchangeably with "cycloalkyl" in the present specification, and may include, as an example, cyclopropyl, cyclobutyl, cyclopentyl, cyclohexyl, cycloheptyl, cyclooctyl, and the like, but is not limited thereto.

In the present invention, "alkoxy" may refer to -(O-alkyl) as an alkyl ether group, wherein the alkyl is the same as defined above. For example, "C₁₋₆alkoxy" may mean an alkoxy containing C₁₋₆alkyl, i.e., -(O-C₁₋₆alkyl), "C₁₋₄alkoxy" may mean an alkoxy containing C₁₋₄alkyl, i.e., -(O-C₁₋₄alkyl), and "C₁₋₃alkoxy" may mean an alkoxy containing C₁₋₃alkyl, i.e., -(O-C₁₋₃alkyl). As an example, the alkoxy may include, but is not limited to, methoxy, ethoxy, n-propoxy, isopropoxy, n-butoxy, isobutoxy, sec-butoxy, tert-butoxy, and the like.

In the present invention, "halo" may be F, Cl, Br, or I.

In the present invention, the term "haloalkyl" may mean a straight or branched chain alkyl (hydrocarbon) having carbon atoms substituted with at least one halo as defined herein. Examples of the haloalkyl include, but are not limited to, methyl, ethyl, propyl, isopropyl, isobutyl or n-butyl independently substituted with at least one halogen such as F, Cl, Br, or I.

In the present specification, the term "hydroxyalkyl" may refer to a straight or branched chain alkyl (hydrocarbon) having carbon atoms substituted with hydroxy (OH). Examples of the hydroxyalkyl include, but are not limited to, methyl, ethyl, propyl, isopropyl, isobutyl or n-butyl independently substituted with at least one -OH.

In the present specification, the term "aminoalkyl" may refer to a straight or branched chain alkyl (hydrocarbon) having carbon atoms substituted with amino-(NR'R"). Here, R' and R" may each independently be selected from the group consisting of hydrogen, C₁₋₆alkyl, and an N protecting group (for example, Boc), and the selected R' and R" may each independently be substituted or unsubstituted.

In the present invention, "cycloalkyl" may refer to a hydrocarbon ring that does not contain a hetero atom (N, O, P, P(=O), S, or the like) in the ring, and may be saturated or partially unsaturated. Here, when unsaturated, the cycloalkyl may be referred to as a cycloalkenyl. Unless otherwise stated, the cycloalkyl may be a single ring or multiple rings such as spiro rings, bridged rings or fused rings.

In the present invention, "heterocycloalkyl" may refer to a ring containing at least one selected from N, O, P, P(=O), and S in the ring, and may be saturated or partially unsaturated. Herein, when unsaturated, the heterocycloalkyl may be referred to as a heterocycloalkene. Unless otherwise stated, the heterocycloalkyl may be a single ring or multiple rings such as spiro rings, bridged rings or fused rings. Further, "3- to 12-membered heterocycloalkyl" may mean a heterocycloalkyl containing 3 to 12 atoms forming a ring. Examples of the heterocycloalkyl may include, but are not limited to, pyrrolidine, piperidine, imidazolidine, pyrazolidine, butyrolactam, valerolactam, imidazolidinone, hydantoin, dioxolane, phthalimide, piperidine, pyrimidine-2,4(1H,3H)-dione, 1,4-dioxane, morpholine, thiomorpholine, thiomorpholine-S-oxide, thiomorpholine-S,S-oxide, piperazine, pyran, pyridone, 3-pyrroline, thiopyran, pyrone, tetrahydrofuran, tetrahydrothiophene, quinuclidine, tropane, 2-azaspiro[3.3]heptane, (1R,5S)-3-azabicyclo[3.2.1]octane, (1s,4s)-2-azabicyclo[2.2.2]octane, or (1R,4R)-2-oxa-5-azabicyclo[2.2.2]octane, and the like.

In the present invention, "arene" may mean an aromatic hydrocarbon ring. The arene may be a single ring or multiple rings. The number of ring carbon atoms in the arene may be 5 or more and 30 or less, 5 or more and 20 or less, or 5 or more and 15 or less. Examples of arene may include, but are not limited to, benzene, naphthalene, fluorene, anthracene, phenanthrene, bibenzene, terbenzene, quaterbenzene, quinquebenzene, sexibenzene, triphenylene, pyrene, benzofluoranthene, chrysene, and the like. In the present specification, a moiety obtained by removing one hydrogen atom from the above "arene" is referred to as "aryl".

In the present invention, "heteroarene" may be a ring containing one or more of O, N, P, Si, and S as heterogeneous elements. The heteroarene may be a single ring or multiple rings. The number of ring carbon atoms of the heteroarene may be 2 or more and 30 or less, or 2 or more and 20 or less. The heteroarene may be a monocyclic heteroarene or a polycyclic heteroarene. The polycyclic heteroarene may have, for example, a bicyclic or tricyclic structure. Examples of the heteroarene include, but not limited to, thiophene, purine, pyrrole, pyrazole, imidazole, thiazole, oxazole, isothiazole, oxadiazole, triazole, pyridine, bipyridyl, triazine, acridyl, pyridazine, pyrazine, quinoline, quinazoline, quinoxaline, phenoxazine, phthalazine, pyrimidine, pyridopyrimidine, pyridopyrazine, pyrazinopyrazine, isoquinoline, indole, carbazole, imidazopyridazine, imidazopyridine, imidazopyrimidine, pyrazolopyrimidine, imidazopyrazine or pyrazolopyridine, N-arylcarbazole, N-heteroarylcarbazole, **N-**alkylcarbazole, benzoxazole, benzoimidazole, benzothiazole, benzocarbazole, benzothiophene, dibenzothiophene, thienothiophene, benzofuran, phenanthroline, isoxazole, oxadiazole, thiadiazole, benzothiazole, tetrazole, phenothiazine, dibenzosilol, dibenzofuran, and the like. If the heteroarene could be a tautomer, the heteroarene may include the tautomer. In an embodiment of the present invention, the heteroarene may also include a bicyclic heterocyclo-arene containing an arene ring fused to a heterocycloalkyl ring or a heteroarene fused to a cycloalkyl ring. In the present specification, a moiety obtained by removing one hydrogen atom from the above "heteroarene" is referred to as "heteroaryl".

In the present invention, the term "tautomer" means an isomer that is in equilibrium with each other and whose molecular structure is transformed by the transfer of protons.

In the present invention, the term "stereoisomer" means a compound of the present invention, having the same chemical formula or molecular formula but different in spatial arrangement. In the present specification, the stereoisomer includes an optical isomer, an enantiomer, a diastereomer, cis/trans isomer, rotamer, and atropisomer. Each of these isomer, racemate and mixture thereof are also included within the scope of the present invention. For example, Chemical Formula I of the present invention may include stereoisomers of Chemical Formula I because the stereochemistry is not specified. Unless otherwise specified, a solid bond ( 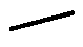 ) connected to an asymmetric carbon atom may include a wedged solid bond ( 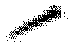 ) or wedge dashed bond ( 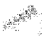 ) representing the absolute arrangement of stereocenters.

The compound represented by Chemical Formula 1 of the present invention may be present in the form of a "pharmaceutically acceptable salt". As the salt, an acid addition salt formed by a pharmaceutically acceptable free acid is useful. The term "pharmaceutically acceptable salt" as used herein refers to a concentration having a relatively non-toxic and harmless effective effect on patients, which includes any organic acid or inorganic acid addition salt of the compound represented by Chemical Formula 1 in which side effects caused by these salts do not reduce the beneficial efficacy of the compound.

Acid addition salts are prepared by conventional methods, for example, by dissolving a compound in an excess of an aqueous acid solution and precipitating the salt using a water-miscible organic solvent, such as methanol, ethanol, acetone or acetonitrile. Here, an acid or alcohol in water and the compound in equimolar amounts may be heated, and then the mixture may be evaporated to dryness, or the precipitated salt may be suction filtered.

Here, as the free acid, an organic acid or an inorganic acid may be used, wherein the inorganic acid may be hydrochloric acid, phosphoric acid, sulfuric acid, nitric acid, or the like, and the organic acid may be methanesulfonic acid, p-toluenesulfonic acid, acetic acid, trifluoroacetic acid, maleic acid, succinic acid, oxalic acid, benzoic acid, tartaric acid, fumaric acid, manderic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, or hydroiodic acid, or the like. However, the organic acid and inorganic acid are not limited thereto.

Further, a pharmaceutically acceptable metal salt may be prepared using a base. The alkali metal salt or alkaline earth metal salt is obtained, for example, by dissolving the compound in an excess of alkali metal hydroxide or alkaline earth metal hydroxide solution, filtering the undissolved compound salt, and then evaporating and drying the filtrate. Here, as the metal salt, sodium, potassium, or calcium salt is particularly suitable in a pharmaceutical aspect, but the metal salt is not limited thereto. In addition, the corresponding silver salt may be obtained by reacting an alkali metal or alkaline earth metal salt with a suitable silver salt (for example, silver nitrate).

The pharmaceutically acceptable salt of the present invention, unless otherwise indicated, includes salts of acidic or basic groups that may be present in the compounds represented by Chemical Formula 1 above. For example, the pharmaceutically acceptable salts may include sodium, calcium, and potassium salts, of hydroxy groups, and may include other pharmaceutically acceptable salts of amino groups such as hydrobromide, sulfate, hydrogen sulfate, phosphate, hydrogen phosphate, dihydrogen phosphate, acetate, succinate, citrate, tartrate, lactate, mandelate, methanesulfonate (mesylate), and p-toluenesulfonate (tosylate) salts, and the like, which may be prepared through salt preparation methods known in the art.

### Use of heteroaryl derivative compound

The present invention provides use of a compound represented by the following Chemical Formula 1, a tautomer thereof, a stereoisomer thereof, or a pharmaceutically acceptable salt thereof: 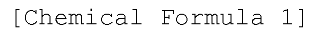 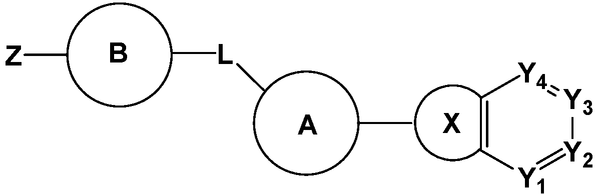 the Chemical Formula 1 is the same as defined above.

The compound represented by Chemical Formula 1 of the present invention, a tautomer thereof, a stereoisomer thereof, or a pharmaceutically acceptable salt thereof exhibits inhibitory activity against anoctamin-6 (ANO6).

According to an embodiment of the present invention, the heteroaryl derivative represented by Chemical Formula 1 may exhibit excellent inhibitory activity against anoctamin-6 (ANO6), thereby being usefully employed for treatment or prevention of thrombosis or thrombosis-related diseases.

In the present invention, the thrombosis or thrombosis-related disease may be at least one disease selected from the group consisting of acute myocardial infarction; atrial fibrillation; unstable angina; chronic stable angina; transient ischemic attack; stroke; peripheral vascular disease; pre-eclampsia; eclampsia; deep vein thrombosis; embolism; cancer-related thrombosis; disseminated intravascular coagulation; thrombotic thrombocytopenia; and thrombotic or restenotic complications that occur after invasive procedures resulting from angioplasty, carotid endarterectomy, post coronary artery bypass graft (CABG) surgery, vascular graft surgery, stent placements and insertion of endovascular devices or prostheses.

According to an embodiment of the present invention, the present invention provides a pharmaceutical composition for treating or preventing thrombosis or thrombosis-related diseases, comprising the compound represented by Chemical Formula 1, a tautomer thereof, a stereoisomer thereof, or a pharmaceutically acceptable salt thereof as described above as an active ingredient. The types of thrombosis or thrombosis-related diseases are as described above.

The pharmaceutical composition of the present invention may further comprise at least one active ingredient exhibiting the same or similar efficacy in addition to the compound represented by Chemical Formula 1, a tautomer thereof, a stereoisomer thereof, or a pharmaceutically acceptable salt thereof.

The pharmaceutical composition of the present invention may be used for clinical administration, and may be prepared to be administered in various oral and parenteral dosage forms.

In addition, according to an embodiment of the present invention, provided is a method for treating or preventing thrombosis or thrombosis-related diseases, comprising: administering a therapeutically effective amount of the compound represented by Chemical Formula 1, a tautomer thereof, a stereoisomer thereof, or a pharmaceutically acceptable salt thereof to a subject in need thereof. The subject may be a mammal including a human.

The term "therapeutically effective amount" used in the present invention refers to an amount of the compound represented by Chemical Formula 1 effective for the treatment or prevention of thrombosis or thrombosis-related diseases. Specifically, the "pharmaceutically effective amount" means an amount sufficient to treat a disease at a reasonable benefit/risk ratio applicable to medical treatment, and the effective dose level may be determined depending on the type and severity of the individual, age, sex, the type of disease, the activity of the drug, the sensitivity to the drug, the time of administration, the route of administration and the rate of excretion, the duration of treatment, factors including concomitant drugs and other factors well known in the medical field. The pharmaceutical composition of the present invention may be administered as an individual therapeutic agent or in combination with other therapeutic agents, or may be administered sequentially or simultaneously with a commercially available therapeutic agent. In addition, the pharmaceutical composition of the present invention may be administered singly or in multiple doses. In consideration of all of the above factors, it is important to administer an amount capable of obtaining the maximum effect with the minimum amount without side effects, which may be easily determined by those skilled in the art. The administration dose of the pharmaceutical composition of the present invention may be determined by specialists depending on various factors such as the patient's condition, age, sex, complications, and the like. Since the active ingredient of the pharmaceutical composition of the present invention has excellent safety, the active ingredient may be used even above the predetermined administration dose.

In addition, according to an embodiment of the present invention, the present invention provides use of the compound represented by Chemical Formula 1, a tautomer thereof, a stereoisomer thereof, or a pharmaceutically acceptable salt thereof for the manufacture of a medicament for the treatment or prevention of thrombosis or thrombosis-related diseases. The compound represented by Chemical Formula 1 for the manufacture of medicaments may be mixed with acceptable adjuvants, diluents, carriers, and the like, and may be prepared as a combined preparation with other active agents to have a synergistic effect of the active ingredients.

Matters described in the use, composition, and treatment method of the present invention are equally applied unless they contradict each other.

### [Advantageous Effects]

The heteroaryl derivative compound of the present invention may exhibit excellent inhibitory activity against ANO6, which may be usefully employed for the treatment or prevention of thrombosis or thrombosis-related diseases.

### [Brief description of Drawings]

FIG. 1 is a graph showing Inhibition rate of Compound 20 and 24 treatment groups according to Experimental Example 1.
FIG. 2 is a graph showing Inhibition rate of Compound 25 and 1 treatment groups according to Experimental Example 1.
FIG. 3 is a graph showing Inhibition rate of Compound 27 and 28 treatment groups according to Experimental Example 1.
FIG. 4 is a graph showing Inhibition rate of Compound 23, 58 and 45 treatment groups according to Experimental Example 1.
FIG. 5 is a graph showing Inhibition rate of Compound 21 treatment group according to Experimental Example 1.
FIG. 6 is a graph showing Inhibition rate of Compound 52 and 223 treatment groups according to Experimental Example 1.

### [Best Mode]

Hereinafter, the present invention will be described in detail with reference to Preparation Examples, Examples, and Experimental Examples. However, the following Preparation Examples, Examples, and Experimental Examples are only provided to illustrate the present invention, and the content of the present invention is not limited to these Examples.

### [Preparation Example] Preparation of main intermediate compounds

### Method a. Preparation of amine derivative using Suzuki reaction (Formula II-a)

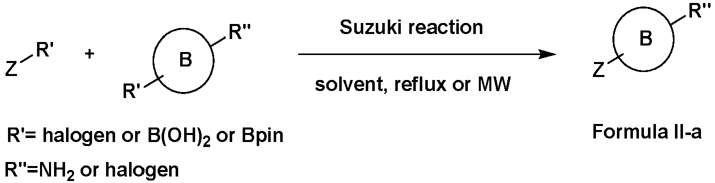

### Preparation Example 1: Synthesis of 5-(4-methylthiophen-3-yl)pyrimidin-2-amine

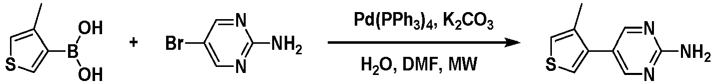

(4-Methylthiophen-3-yl)boronic acid (902 mg, 6.35 mmol), 5-bromopyrimidin-2-amine (850 mg, 4.88 mmol), Pd(PPh₃)₄ (282 mg, 0.244 mmol), and K₂CO₃ (2.03g, 14.65 mmol) were dissolved in H₂O (80 mL) and DMF (10 mL), and stirred in a microwave reactor at 110°C for 35 minutes. Pd was removed through Celite filtration, then the residue was washed with a brine solution and extracted with ethyl acetate, and the organic layer was dried over Na₂SO₄ and concentrated. The concentrated mixture was slurried with ethyl acetate and hexane and filtered to obtain the title compound (496 mg, 53%) as a beige solid as a filter cake.

¹H NMR (400 MHz, DMSO-*d*₆) δ 8.32 (s, 2H), 7.48 (d, *J* = 3.3 Hz, 1H), 7.29-7.27 (m, 1H), 6.73 (s, 2H), 2.22 (s, 3H).

### Preparation Example 2: Synthesis of 5-(pyrimidin-4-yl)pyridin-2-amine

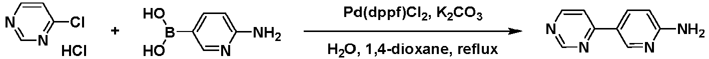

4-Chloropyrimidine hydrochloride (2 g, 13.25 mmol), (6-aminopyridin-3-yl)boronic acid (2.2 g, 15.9 mmol), Pd(dppf)Cl₂ (0.485 g, 0.66 mmol), and K₂CO₃ (11 g, 79.5 mmol) were dissolved in H₂O (13 mL) and 1,4-dioxane (26 mL), followed by stirring at reflux at 110°C for 17 hours. Pd was removed through Celite filtration, then the residue was washed with a brine solution and extracted with ethyl acetate, and the organic layer was dried over Na₂SO₄ and concentrated. The concentrated solution was purified by MPLC, then slurried with ethyl acetate and filtered to obtain the title compound (877 mg, 38%) as a gray solid as a filter cake.

¹H NMR (400 MHz, DMSO-*d*₆) δ 9.08 (d, *J* = 1.0 Hz, 1H), 8.82 (d, *J* = 2.3 Hz, 1H), 8.69 (d, *J* = 5.5 Hz, 1H), 8.18 (dd, *J* = 2.4, 8.8 Hz, 1H), 7.92 (dd, *J* = 1.3, 5.5 Hz, 1H), 6.66 (s, 2H), 6.54 (d, *J* = 8.8 Hz, 1H).

### Preparation Example 3: Synthesis of 5-(4-fluoropyridin-2-yl)pyrimidin-2-amine

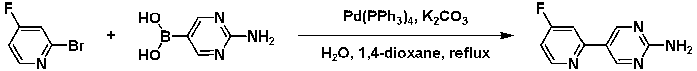

2-Bromo-4-fluoropyridine (5 g, 28.4 mmol), (2-aminopyrimidin-5-yl)boronic acid (4.7 g, 34.1 mmol), Pd(PPh₃)₄ (1.64 g, 1.42 mmol) and K₂CO₃ (11.8 g, 85.2 mmol) were dissolved in H₂O (30 mL) and 1,4-dioxane (60 mL), followed by stirring at reflux at 100°C for 6 hours. Pd was removed through Celite filtration, then the residue was washed with a brine solution and extracted with ethyl acetate, and the organic layer was dried over Na₂SO₄ and concentrated. The concentrated mixture was slurried with ethyl acetate and filtered to obtain the title compound (3.84 g, 71 %) as a beige solid as a filter cake.

¹H NMR (400 MHz, DMSO-*d*₆) δ 8.96 (s, 2H), 8.64-8.60 (m, 1H), 7.86 (dd, *J* = 2.3, 11.2 Hz, 1H), 7.25-7.18 (m, 1H), 7.10 (s, 2H).

### Preparation Example 4: Synthesis of 5-(pyridin-2-yl)pyrimidin-2-amine

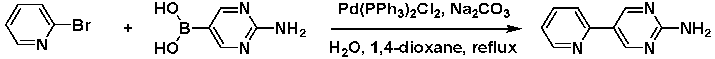

2-Bromopyridine (1.7 mL, 18.0 mmol), (2-aminopyrimidin-5-yl)boronic acid (2.08 g, 15.0 mmol), Pd(PPh₃)₂Cl₂ (1.05 g, 1.5 mmol) and Na₂CO₃ (4.77 g, 45.0 mmol) were dissolved in H₂O (18.75 mL) and 1,4-dioxane (56.25 mL), followed by stirring at reflux at 110°C for 23 hours. After confirming the completion of the reaction by LC-MS, the reaction mixture was concentrated. The concentrated solution was washed with a brine solution and extracted with ethyl acetate, and the organic layer was dried over MgSO₄ and concentrated. The concentrated mixture was slurried with ethyl acetate and filtered to obtain the title compound (1.71 g, 66.3%) as a pale yellow solid as a filter cake.

¹H NMR (400 MHz, DMSO-*d*₆) δ 8.93 (s, 2H), 8.61-8.58 (m, 1H), 7.90-7.80 (m, 2H), 7.31-7.26 (m, 1H), 6.97 (s, 2H).

### Preparation Example 5: Synthesis of 4-(pyridin-2-yl)aniline

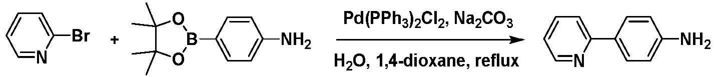

2-Bromopyridine (1.16 mL, 12 mmol), 4-(4,4,5,5-tetramethyl-1,3, 2-dioxaborolan-2-yl) aniline (2.19 g, 10 mmol), Pd(PPh₃)₂Cl₂ (0.70 g, 1 mmol) and Na₂CO₃ (3.18 g, 30 mmol) were dissolved in H₂O (12.5 mL) and 1,4-dioxane (37.5 mL), and the mixture was stirred at reflux at 100°C for 21 hours. After removing Pd and H₂O through Celite and MgSO₄ filtration, the reaction mixture was concentrated. The concentrated solution was purified by MPLC to obtain the title compound (1.57 g, 92%) as an orange solid.

¹H NMR (400 MHz, DMSO-*d*₆) δ 8.54-8.51 (m, 1H), 7.81-7.72 (m, 4H), 7.17-7.13 (m, 1H), 6.66-6.60 (m, 2H), 5.44 (s, 2H).

### Preparation Example 6: Synthesis of 4-(pyrimidin-5-yl)aniline

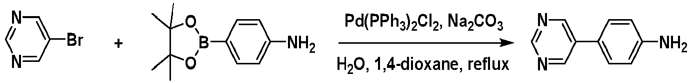

5-Bromopyrimidin (1.91 g, 12 mmol), 4-(4,4,5,5-tetramethyl-1,3, 2-dioxaborolan-2-yl) aniline (2.19 g, 10 mmol), Pd(PPh₃)₂Cl₂ (0.70 g, 1 mmol) and Na₂CO₃ (3.18 g, 30 mmol) were dissolved in H₂O (12.5 mL) and 1,4-dioxane (37.5 mL), and the mixture was stirred at reflux at 100°C for 16 hours. After removing Pd and H₂O through Celite and MgSO₄ filtration, the reaction mixture was concentrated. The concentrated solution was purified by MPLC to obtain the title compound (1.50 g, 87%) as a pale brown solid.

¹H NMR (400 MHz, DMSO-*d*₆) δ 9.02 (s, 1H), 9.01 (s, 2H), 7.53-7.48 (m, 2H), 6.71-6.66 (m, 2H), 5.49 (s, 2H).

### Preparation Example 7: Synthesis of 4-(pyridin-4-yl)aniline

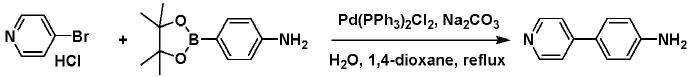

4-Bromopyridine hydrochloride (2.33 g, 12 mmol), 4-(4,4,5,5-tetramethyl-1,3,2-dioxaborolan-2-yl)aniline (2.19 g, 10 mmol), Pd(PPh₃)₂Cl₂ (0.70 g, 1 mmol) and Na₂CO₃ (3.18 g, 30 mmol) were dissolved in H₂O (12.5 mL) and 1,4-dioxane (37.5 mL), and the mixture was stirred at reflux at 100°C for 16 hours. After removing Pd and H₂O through Celite and MgSO₄ filtration, the reaction mixture was concentrated. The concentrated solution was purified by MPLC to obtain the title compound (1.16 g, 67%) as a pale brown solid.

¹H NMR (400 MHz, DMSO-*d*₆) δ 8.49-8.47 (m, 2H), 7.58-7.52 (m, 4H), 6.69-6.64 (m, 2H), 5.53 (s, 2H).

### Preparation Example 8: Synthesis of 6-phenylpyridazin-3-amine

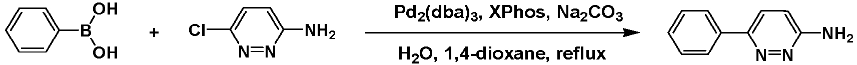

6-Chloropyridazin-3-amine (50.0 g, 386 mmol), phenylboronic acid (70.6 g, 579 mmol), XPhos (73.6 g, 154 mmol) and Na₂CO₃ (69.5 g, 656 mmol) were dissolved in H₂O (25 mL) and 1,4-dioxane (250 mL), and the dissolved solution was degassed and purged with N₂. Pd₂(dba)₃ (21.2 g, 23.2 mmol) was added to the mixed solution, and the reaction mixture was stirred at reflux at 100°C for 12 hours. After confirming the completion of the reaction by LC-MS, the reaction mixture was washed with H₂O (500 mL), extracted with ethyl acetate (250 mL x 4), and the organic layer was dried over Na₂SO₄ and concentrated. The concentrated mixture was purified by column chromatography to obtain the title compound (35.0 g, 53%) as a yellow solid.

¹H NMR (400 MHz, DMSO-*d*₆) δ 7.95 (d, *J* = 7.6 Hz, 2H), 7.82 (d, *J* = 9.2 Hz, 1H), 7.48 (t, *J* = 7.2 Hz, 2H), 7.37 (t, *J* = 7.6 Hz, 1H), 6.87 (d, *J* = 9.2 Hz, 1H), 6.53 (s, 2H).

### Preparation Example 9: Synthesis of 4-(pyridazin-3-yl)aniline

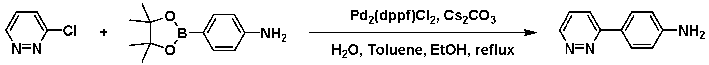

4-(4,4,5,5-Tetramethyl-1,3,2-dioxaborolan-2-yl)aniline (63.8 g, 291 mmol), 3-chloropyridazine (30.0 g, 262 mmol), and Cs₂CO₃ (30 M, 29.1 mL, 873 mmol) aqueous solution were dissolved in toluene (180 mL), H₂O (40 mL) and EtOH (60 mL), and then Pd(dppf)Cl₂ (6.39 g, 8.73 mmol) was added. The mixed solution was degassed and purged with N₂, and the reaction mixture was stirred at reflux at 110°C for 12 hours. After confirming the completion of the reaction by LC-MS, the mixture was diluted with H₂O (100 mL) and ethyl acetate (100 mL), and the suspension was filtered. The filter cake was dried under vacuum to obtain the title compound (42.0 g, 84%) as a black solid.

¹H NMR (400 MHz, DMSO-*d*₆) δ 9.00 (d, *J* = 4.8 Hz, 1H), 8.00-7.98 (m, 1H), 7.88 (d, *J* = 8.8 Hz, 2H), 7.62-7.58 (m, 1H), 6.68 (d, *J* = 8.8 Hz, 2H), 5.59 (s, 2H).

### Preparation Example 10: Synthesis of 5-(pyridazin-3-yl)pyrimidin-2-amine

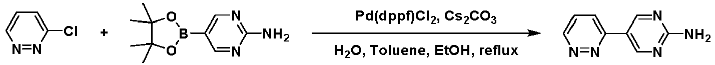

5-(4,4,5,5-Tetramethyl-1,3,2-dioxaborolan-2-yl)pyrimidin-2-amine (199.0 mg, 0.9 mmol), 3-chloropyridazine (114.5 mg , 1.0 mmol), and Cs₂CO₃ (963.2 mg, 3.0 mmol) were dissolved in toluene (3 mL), H₂O (0.7 mL) and EtOH (1 mL), and then Pd(dppf)Cl₂ (5.17 mg, 0.04 mmol) was added. The reaction mixture was stirred at reflux at 110°C for 16 hours. After confirming the completion of the reaction by LC-MS, Pd was removed through celite filtration and the reaction mixture was concentrated. The concentrated mixture was purified by MPLC to obtain the title compound (41 mg, 24%) as a brown solid.

¹H NMR (400 MHz, DMSO-*d*₆) δ 9.14 (dd, *J* = 1.4, 4.9 Hz, 1H), 9.01 (s, 2H), 8.16 (dd, *J* = 1.5, 8.8 Hz, 1H), 7.73 (dd, *J* = 4.9, 8.8 Hz, 1H), 7.15 (s, 2H).

### Preparation Example 11: Synthesis of [2,5'-bipyrimidine]-2'-amine

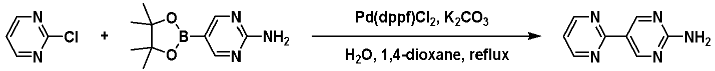

5-(4,4,5,5-Tetramethyl-1,3,2-dioxaborolan-2-yl)pyrimidin-2-amine (5.2 g, 23.7 mmol), 2-chloropyrimidine (2.8 g , 24.9 mmol) and K₂CO₃ (9.8 g, 71.1 mmol) were dissolved in 1,4-dioxane (40 mL) and H₂O (10 mL), and then Pd(dppf)Cl₂ (867 mg, 1.18 mmol) was added. The reaction mixture was stirred at reflux at 90°C for 64 hours. After confirming the completion of the reaction by LC-MS, the reaction mixture was diluted with H₂O and extracted with ethyl acetate. The organic layer was dried over MgSO₄ and concentrated, then the residue was slurried with dichloromethane and filtered. The filtrate was purified by MPLC to obtain the title compound (520 mg, 13%) as a beige solid.

¹H NMR (400 MHz, DMSO-*d*₆) δ 9.14 (dd, *J* = 1.3, 4.8 Hz, 1H), 8.99 (s, 2H), 8.15 (dd, *J* = 1.3, 8.7 Hz, 1H), 7.59-7.47 (m, 1H), 7.14 (s, 2H).

### Preparation Example 12: Synthesis of 5-(3-fluorophenyl)pyridin-2-amine

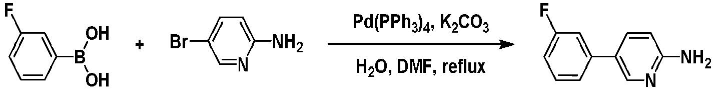

5-Bromopyridin-2-amine (900 mg, 5.2 mmol), (3-fluorophenyl) boronic acid (873 mg, 6.24 mmol), and K₂CO₃ (2.2 g, 15.6 mmol) were dissolved in DMF (10.4 mL) and H₂O (10.4 mL), then Pd(PPh₃)₄ (301 mg, 0.26 mmol) was added, and the mixture was stirred at reflux at 140°C for 12 hours. After confirming the completion of the reaction by LC-MS, the reaction mixture was diluted with H₂O and extracted with ethyl acetate. The organic layer was washed with brine, dried over Na₂SO₄, concentrated, and purified by column chromatography. The residue was slurried with MTBE at room temperature for 1 hour and filtered through MTBE. The filtercake was dried under vacuum to obtain the title compound (18.8 g, 35%) as a white solid.

¹H NMR (400 MHz, DMSO-*d*₆) δ 8.29 (d, *J* = 2.0 Hz, 1H), 7.73 (dd, *J* = 8.8, 2.4 Hz, 1H), 7.45-7.40 (m, 3H), 7.09-7.04 (m, 1H), 6.52 (d, *J* = 8.4 Hz, 1H), 6.16 (s, 2H).

### Preparation Example 13: Synthesis of 4-(pyrimidin-4-yl)aniline

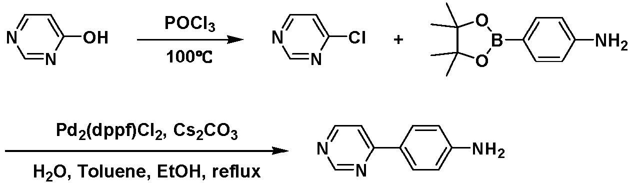

### Step 1: Synthesis of 4-chloropyrimidine

Pyrimidin-4-ol (10.0 g, 104 mmol) was dissolved in POCl₃ (100 mL, 1.08 mol) and stirred in a pressure flask at 100°C for 6 hours. The mixture was concentrated to remove POCl₃, then ethyl acetate was slowly added to the mixture, stirred for 30 minutes, and then filtered with ethyl acetate. The filtercake was dried under vacuum to obtain the title compound (3.5 g, 30%) as a brown solid.

¹H NMR (400 MHz, DMSO-*d*₆) δ 9.14 (s, 1H), 8.07 (d, *J* = 7.20 Hz, 1H), 6.62 (d, *J* = 7.60 Hz, 1H).

### Step 2: Synthesis of 4-(pyrimidin-4-yl)aniline

4-(4,4,5,5-Tetramethyl-1,3,2-dioxaborolan-2-yl)aniline (3.79 g, 17.3 mmol), 4-chloropyrimidine (1.80 g, 15.7 mmol), and Cs₂CO₃ (20.5 g, 62.9 mmol) were dissolved in toluene (12 mL), H₂O (3.6 mL) and EtOH (4 mL), then Pd(dppf)Cl₂ (575 mg, 0.786 mmol) was added. The mixed solution was stirred at reflux at 100°C for 12 hours. After confirming the completion of the reaction by TLC, the reaction product was extracted with H₂O and ethyl acetate. The organic layer was washed with brine, dried over Na₂SO₄, concentrated, and purified by column chromatography to obtain the title compound (1.7 g, 63%) as a yellow solid.

¹H NMR (400 MHz, DMSO-*d*₆) δ 9.02 (s, 1H), 8.62 (d, *J* = 5.20 Hz, 1H), 7.94 (d, *J* = 8.80 Hz, 2H), 7.83-7.79 (m, 1H), 6.65 (d, *J* = 8.80 Hz, 2H), 5.80 (s, 2H).

### Preparation Example 14: Synthesis of 4-(6-fluoropyridazine-3-yl)aniline

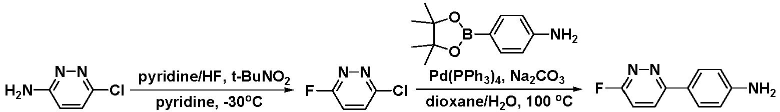

### Step 1: Synthesis of 3-chloro-6-fluoropyridazine

In a flask, pyridine (100 mL) was added slowly to pyridine/HF (200 mL) at -30°**C** and stirred at this temperature for 15 minutes. Then a mixture of tert-butyl nitrite (19.2 g, 185.26 mmol) dissoloved in pyridine (100 mL) was added to the above solution dropwise at -30°**C** and stirred at this temperature for another 15 minutes. The reaction mixture was poured into ice water (2000 mL), then extracted with EA (200 mL x3), the combined organic layer was washed with brine (2000 mL x3), dried over Na₂SO₄ and concentrated under reduced pressure, the residue was purified by column chromatography to obtain the title compound (6 g, 48.88%) as a yellow solid.

MS: m/z = 133 (M+1, ESI+).

### Step 2: Synthesis of 4-(6-fluoropyridazin-3-yl)aniline

To a solution of 3-chloro-6-fluoropyridazine (3 g, 22.64 mmol) and 4-(4,4,5,5-tetramethyl-1,3,2-dioxaborolan-2-yl)aniline (6.47 g, 29.53 mmol) in dioxane/H₂O (40 mL/8 mL) was added Na₂CO₃ (12.95 g, 45.30 mmol) and Pd(PPh₃)₄ (2.61 g, 2.26 mmol). Then the reaction mixture was stirred at 100°C for 4 hours under N₂. The reaction mixture was cooled to room temperature and poured into water (200 mL), then extracted EA (50 mL x3), the combined organic layer was washed with brine (200 mL x3), dried over Na₂SO₄ and concentrated under reduced pressure. The residue was purified by column chromatography to obtain the title compound (3.52 g, 82.24%) as a yellow solid.

¹H NMR (400 MHz, DMSO-d₆) δ 8.26 (dd, *J* = 9.3, 7.5 Hz, 1H), 7.91-7.73 (m, 2H), 7.62 (dd, *J* = 9.3, 1.7 Hz, 1H), 6.71-6.62 (m, 2H), 5.62 (s, 2H).

MS: m/z = 190 (M+1, ESI+).

### Preparation Example 15: Synthesis of 4-(4-trifluoromethyl)pyridazin-3-yl)aniline

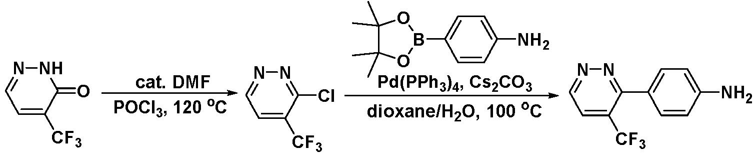

### Step 1: Synthesis of 3-chloro-4-(trifluoromethyl)pyridazine

To a mixture of 4-(trifluoromethyl)pyridazin-3(*2H*)-one (4g, 24.38 mmol) in POCl₃ (40 mL) was added DMF (0.5 mL) and stirred at **120 °C** for 4 hours. The reaction mixture was cooled to room temperature and poured into ice water (400 mL), then extracted with DCM (50 mL x3), the combined organic layer was washed with brine (200 mL x3), dried over Na₂SO₄ and concentrated under reduced pressure. The residue was purified by column chromatography to obtain the title compound (3 g, 67.87%) as a yellow solid.

¹H NMR (400 MHz, DMSO-d₆) δ 9.58 (d, *J* = 5.0 Hz, 1H), 8.30 (d, *J* = 5.1 Hz, 1H).

MS: m/z = 183 (M+1, ESI+).

### Step 2: Synthesis of 4-(4-(trifluoromethyl)pyridazin-3-yl)aniline

To a mixture of 3-chloro-4-(trifluoromethyl)pyridazine (3 g, 16.44 mmol) and 4-(4,4,5,5-tetramethyl-1,3,2-dioxaborolan-2-yl)aniline (4.38 g, 21.47 mmol) in dioxane/H₂O (60 mL/12 mL) was added Cs₂CO₃ (16.19 g, 49.64 mmol) and Pd(PPh₃)₄ (1.92 g, 1.64 mmol), the reaction mixture was stirred at 100°C for 4 hours under N₂. The reaction mixture was cooled to room temperature and poured into water (500 mL), then extracted EA (100 mL x3), the combined organic layer was washed with brine (500 mL x3), dried over Na₂SO₄ and concentrated under reduced pressure. The residue was purified by column chromatography to obtain the title compound (3 g, 76.92%) as a yellow solid.

¹H NMR (400 MHz, DMSO-d₆) δ 9.45 (d, *J* = 5.0 Hz, 1H), 8.11 (d, *J* = 5.3 Hz, 1H), 7.28 (d, *J* = 8.4 Hz, 2H), 6.76-6.57 (m, 2H), 5.57 (s, 2H).

MS: m/z = 240 (M+1, ESI+).

### Preparation Example 16: Synthesis of methyl-6-(4-aminophenyl)pyridazine-4-carboxylate

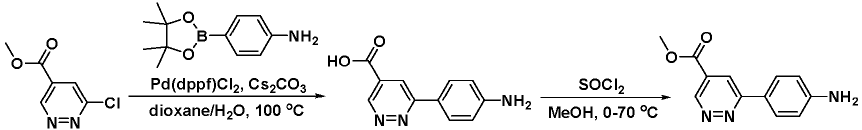

### Step 1: Synthesis of 6-(4-aminophenyl)pyridazine-4-carboxylic acid

To a solution of methyl 6-chloropyridazine-4-carboxylate (6 g, 34.77 mmol) and 4-(4,4,5,5-tetramethyl-1,3,2-dioxaborolan-2-yl)aniline (9.14 g, 41.72 mmol) in dioxane/H₂O (500 mL/100 mL) was added Cs₂CO₃ (33.98 g, 104.31 mmol) and Pd(dppf)Cl₂ (1.26 g, 1.74 mmol), the reaction mixture was stirred at 100°C for 2 hours under N₂. The reaction mixture was cooled to room temperature and poured into water (500 mL), then extracted with EA (80 mL x3), the combined organic layer was washed with brine (500 mL x3), dried over Na₂SO₄ and concentrated under reduced pressure. The residue was purified by column chromatography to obtain the title compound (12.1 g, crude) as a yellow solid.

MS: m/z = 216 (M+1, ESI+)

### Step 2: Synthesis of methyl 6-(4-aminophenyl)pyridazine-4-carboxylate

To a solution of 6-(4-aminophenyl)pyridazine-4-carboxylic acid (12.1 g, 56.28 mmol) in MeOH (200 mL) at 0°C was added SOCl₂ (13.39 g, 112.56 mmol) dropwise, then the reaction mixture was stirred at 70°C for 5 hours. The reaction mixture was cooled to room temperature and concentrated under reduced pressure, the residue was acidified to pH 8 with NaHCO₃ (aq, 500 mL), then extracted with EA (80 mL x3), the combined organic layer was washed with brine (400 mL x3), dried over Na₂SO₄ and concentrated under reduced pressure. The residue was purified by column chromatography to obtain the title compound (4 g, 31.05%) as a yellow solid.

¹H NMR (400 MHz, DMSO-*d*₆) δ 9.33 (d, *J* = 2.0 Hz, 1H), 8.28 (d, *J* = 2.0 Hz, 1H), 7.95 (d, *J* = 8.4 Hz, 2H), 6.70 (d, *J* = 8.4 Hz, 2H), 5.72 (s, 2H), 3.95 (s, 3H).

MS: m/z = 230 (M+1, ESI+).

### Preparation Example 17: Synthesis of 4-(2-(4-methylpiperazin-1-yl)pyrimidin-5-yl) aniline

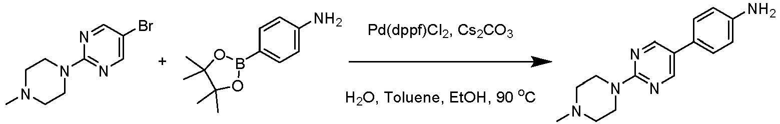

To a solution of 4-(4,4,5,5-tetramethyl-1,3,2-dioxaborolan-2-yl)aniline (500 mg, 2.3 mmol), 5-bromo-2-(4-methypiperazin-1-yl)pyrimidine (593 mg, 2.3 mmol) in H₂O/toluene/EtOH (3 mL/ 12 mL/ 6 mL) was added Cs₂CO₃ (1.9 g, 5.7 mmol) and Pd(dppf)Cl₂ (130 mg, 0.07 mmol), the reaction mixture was stirred at 90°C for 4 hours. The reaction mixture was poured into water, then extracted with DCM. The combined organic layer was dried over MgSO₄ and concentrated under reduced pressure and purified by MPLC. The crude mixture was solidified using DCM and hexane to obtain the title compound (232 mg, 38%) as a beige solid.

¹H NMR (400 MHz, DMSO-*d*₆) δ 8.58-8.54 (m, 2H), 7.29 (d, *J* = 8.5 Hz, 2H), 6.63 (d, *J* = 8.5 Hz, 2H), 5.21 (s, 2H), 3.76-3.69 (m, 4H), 2.39-2.32 (m, 4H), 2.21 (s, 3H).

### Method b. Preparation of carboxylic acid derivative using Buchwald reaction (Formula III-a)

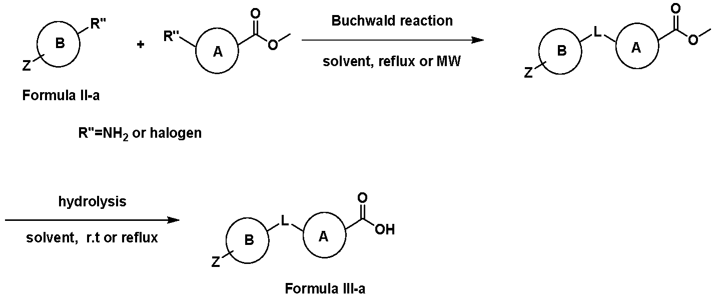

### Preparation Example 18: Synthesis of 3-((5-(4-methylthiophen-3-yl)pyrimidin-2-yl)amino)benzoic acid

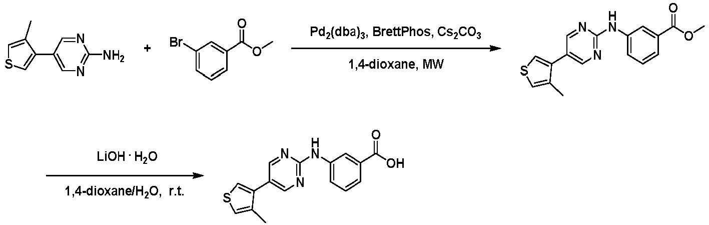

### Step 1: Synthesis of methyl 3-((5-(4-methylthiophen-3-yl)pyrimidin-2-yl)amino)benzoate

5-(4-Methylthiophen-3-yl)pyrimidin-2-amine (490 mg, 2.56 mmol), methyl 3-bromobenzoate (661 mg, 3.07 mmol), Pd₂(dba)₃ (235 mg , 0.0256 mmol), BrettPhos (275 mg, 0.512 mmol) and Cs₂CO₃ (1.67 g, 5.12 mmol) were dissolved in 1,4-dioxane (13 mL), and stirred at 120°C for 90 minutes in a microwave reactor. After confirming the completion of the reaction by LC-MS, the mixture was washed with a brine solution and extracted with ethyl acetate, and the organic layer was dried over Na₂SO₄ and concentrated. The concentrated mixture was slurried with ethyl acetate and hexane and filtered to obtain the title compound (364 mg, 44 %) as a white solid as a filter cake.

¹H NMR (400 MHz, DMSO-*d*₆) δ 10.00 (s, 1H), 8.64 (s, 2H), 8.45 (t, *J* = 1.9 Hz, 1H), 8.10-8.06 (m, 1H), 7.64 (d, *J* = 3.3 Hz, 1H), 7.58-7.55 (m, 1H), 7.45 (t, *J* = 7.9 Hz, 1H), 7.34-7.32 (m, 1H), 3.87 (s, 3H), 2.29 (s, 3H).

### Step 2: Synthesis of 3-((5-(4-methylthiophen-3-yl)pyrimidin-2-yl)amino)benzoic acid

Methyl 3-((5-(4-methylthiophen-3-yl)pyrimidin-2-yl)amino)benzoate (350 mg, 1.08 mmol) prepared in step 1 above and LiOH·H₂O (451 mg, 10.8 mmol) were dissolved in H₂O (4.5 mL) and 1,4-dioxane (21.5 mL), and the reaction mixture was stirred overnight at room temperature. After confirming the completion of the reaction by LC-MS, the reaction product was acidified to pH 3 with 1N-HCl (aq), extracted with ethyl acetate, and washed with brine. Then, the organic layer was dried over Na₂SO₄ and concentrated. The concentrated mixture was slurried with ethyl acetate and hexane and filtered to obtain the title compound (311 mg, 93 %) as a white solid as a filter cake.

¹H NMR (400 MHz, DMSO-*d*₆) δ 12.92 (s, 1H), 9.97 (s, 1H), 8.64 (s, 2H), 8.44 (t, *J* = 1.8 Hz, 1H), 8.05-8.01 (m, 1H), 7.63 (d, *J* = 3.1 Hz, 1H), 7.55 (td, *J* = 1.2, 7.6 Hz, 1H), 7.42 (t, *J* = 7.9 Hz, 1H), 7.34-7.32 (m, 1H), 2.29 (s, 3H).

### Preparation Example 19: Synthesis of 3-((5-(pyridin-2-yl)pyrimidin-2-yl)amino)benzoic acid

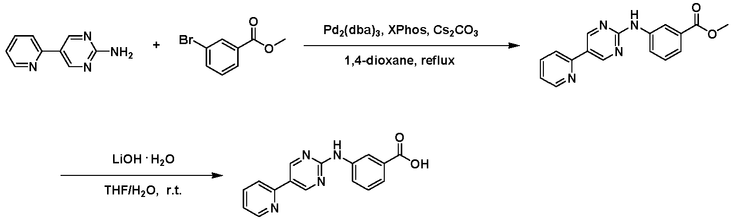

### Step 1: Synthesis of methyl 3-((5-(pyridin-2-yl)pyrimidin-2-yl)amino)benzoate

5-(Pyridin-2-yl)pyrimidin-2-amine (861 mg, 5.0 mmol), methyl 3-bromobenzoate (2.69 g, 12.5 mmol), Pd₂(dba)₃ (366 mg, 0.4 mmol), XPhos (405 mg, 0.85 mmol) and Cs₂CO₃ (3.26 g, 10.0 mmol) were dissolved in 1,4-dioxane (25 mL), and the mixture was stirred at reflux at 110°C for 19.5 hours. After confirming the completion of the reaction by LC-MS, Pd was removed through celite filtration, and the reaction mixture was concentrated. The concentrated solution was purified by MPLC, concentrated, slurried with ethyl acetate and filtered to obtain the title compound (829 mg, 54.1%) as a pale yellow solid as a filter cake.

¹H NMR (400 MHz, DMSO-*d*₆) δ 10.18 (s, 1H), 9.21 (s, 2H), 8.68-8.65 (m, 1H), 8.49 (t, *J* = 1.9 Hz, 1H), 8.11-8.07 (m, 1H), 8.04-8.00 (m, 1H), 7.93-7.88 (m, 1H), 7.61-7.57 (m, 1H), 7.48 (t, J = 7.9 Hz, 1H), 7.39-7.35 (m, 1H), 3.88 (s, 3H).

### Step 2: Synthesis of 3-((5-(pyridin-2-yl)pyrimidin-2-yl)amino)benzoic acid

Methyl 3-((5-(pyridin-2-yl)pyrimidin-2-yl)amino)benzoate (820 mg, 0.59 mmol) prepared in step 1 above and LiOH·H₂O (449 mg, 10.7 mmol) were dissolved in H₂O (8.9 mL) and THF (17.8 mL), and the reaction mixture was stirred at room temperature for 22 hours. To the reaction mixture, 1N-HCl (aq) was added to crystallize and filtered to obtain the title compound (763 mg, 97.5%) as a white solid as a filter cake.

¹H NMR (400 MHz, DMSO-*d*₆) δ 12.91 (s, 1H), 10.14 (s, 1H), 9.21 (s, 2H), 8.68-8.65 (m, 1H), 8.47-8.45 (m, 1H), 8.07-8.03 (m, 1H), 8.03-8.00 (m, 1H), 7.92-7.87 (m, 1H), 7.59-7.56 (m, 1H), 7.44 (t, *J* = 7.9 Hz, 1H), 7.38-7.34 (m, 1H).

### Preparation Example 20: Synthesis of 3-((4-(pyridin-2-yl)phenyl)amino)benzoic acid

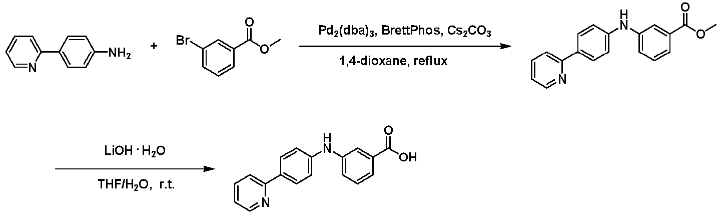

### Step 1: Synthesis of methyl 3-((4-(pyridin-2-yl)phenyl)amino)benzoate

4-(Pyridin-2-yl)aniline (1.52 g, 8.93 mmol), methyl 3-bromobenzoate (2.88 g, 13.4 mmol), Pd₂(dba)₃ (0.82 g, 0.89 mmol), BrettPhos (0.96 g , 1.79 mmol) and Cs₂CO₃ (11.64 g, 35.7 mmol) were dissolved in 1,4-dioxane (45 mL), and the mixture was stirred at reflux at 100°C for 15 hours. After removing Pd through Celite filtration, the reaction product was concentrated, and the concentrated solution was purified by MPLC to obtain the title compound (1.36 g, 49%) as a yellow solid.

¹H NMR (400 MHz, DMSO-*d*₆) δ 8.73-8.71 (m, 1H), 8.61-8.59 (m, 1H), 8.05-8.01 (m, 2H), 7.91-7.87 (m, 1H), 7.86-7.80 (m, 1H), 7.74-7.71 (m, 1H), 7.46-7.40 (m, 3H), 7.28-7.24 (m, 1H), 7.21-7.17 (m, 2H), 3.85 (s, 3H).

### Step 2: Synthesis of 3-((4-(pyridin-2-yl)phenyl)amino)benzoic acid

Methyl 3-((4-(pyridin-2-yl)phenyl)amino)benzoate (1.35 g, 4.43 mmol) prepared in step 1 above and LiOH·H₂O (0.75 g, 17.74 mmol) were dissolved in H₂O (15 mL) and THF (30 mL), and the reaction mixture was stirred at room temperature for 117 hours. The reaction product was acidified to pH 3 with 1N-HCl (aq), extracted with ethyl acetate, and washed with brine. Then, the organic layer was dried over MgSO₄ and concentrated. The concentrated mixture was purified by MPLC to obtain the title compound (321 mg, 25%) as a pale yellow solid.

¹H NMR (400 MHz, DMSO-*d*₆) δ 12.93 (s, 1H), 8.67 (s, 1H), 8.61-8.60 (m, 1H), 8.05-8.00 (m, 2H), 7.90-7.86 (m, 1H), 7.85-7.80 (m, 1H), 7.73-7.71 (m, 1H), 7.45-7.37 (m, 3H), 7.28-7.24 (m, 1H), 7.21-7.16 (m, 2H).

### Preparation Example 21: Synthesis of 3-((4-(pyrimidin-5-yl)phenyl)amino)benzoic acid

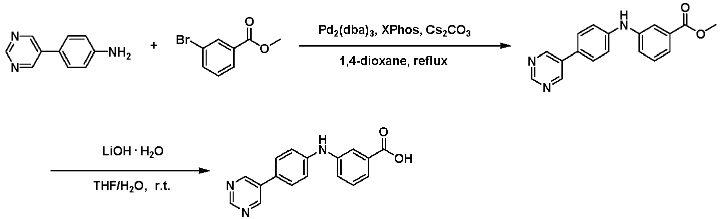

### Step 1: Synthesis of methyl 3-((4-(pyrimidin-5-yl)phenyl)amino)benzoate

4-(Pyrimidin-5-yl)aniline (1.45 g, 8.47 mmol), methyl 3-bromobenzoate (2.0 g, 9.32 mmol), Pd₂(dba)₃ (0.62 g, 0.68 mmol), XPhos (0.69 g, 1.44 mmol) and Cs₂CO₃ (5.52 g, 16.94 mmol) were dissolved in 1,4-dioxane (43 mL), and the mixture was stirred at reflux at 100°C for 16 hours. Pd was removed through celite filtration, and the reaction mixture was concentrated. The concentrated solution was purified by MPLC, concentrated, then slurried with acetone and filtered to obtain the title compound (0.93 g, 36%) as a pale yellow solid as a filter cake.

¹H NMR (400 MHz, DMSO-*d*₆) δ 9.12 (s, 3H), 8.75 (s, 1H), 7.79-7.72 (m, 3H), 7.50-7.36 (m, 3H), 7.25-7.22 (m, 2H), 3.85 (s, 3H).

### Step 2: Synthesis of 3-((4-(pyrimidin-5-yl)phenyl)amino)benzoic acid

Methyl 3-((4-(pyrimidin-5-yl)phenyl)amino)benzoate (0.90 g, 2.95 mmol) prepared in step 1 above and LiOH·H₂O (0.5 g, 11.8 mmol) were dissolved in H₂O (10 mL) and THF (20 mL), and the reaction mixture was stirred at room temperature for 64 hours. After acidifying to pH 3 with 1N-HCl (aq), the mixture was extracted with ethyl acetate. The extract was washed with brine, and the organic layer was dried over MgSO₄ and concentrated. The concentrated mixture was purified by MPLC to obtain the title compound (706 mg, 82%) as a yellow solid.

¹H NMR (400 MHz, DMSO-*d*₆) δ 12.91 (br s, 1H), 9.12-9.11 (m, 3H), 8.70 (s, 1H), 7.79-7.70 (m, 3H), 7.49-7.33 (m, 3H), 7.26-7.20 (m, 2H).

### Preparation Example 22: Synthesis of 3-((4-(pyridin-4-yl)phenyl)amino)benzoic acid

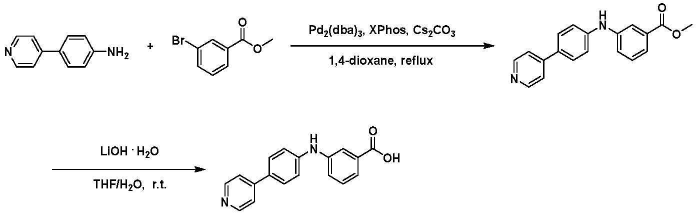

### Step 1: Synthesis of methyl 3-((4-(pyridin-4-yl)phenyl)amino)benzoate

4-(Pyridin-4-yl)aniline (1.12 g, 6.58 mmol), methyl 3-bromobenzoate (1.56 g, 7.24 mmol), Pd₂(dba)₃ (0.48 g, 0.53 mmol), XPhos (0.53 g, 1.12 mmol) and Cs₂CO₃ (4.29 g, 18.44 mmol) were dissolved in 1,4-dioxane (33 mL), and stirred at reflux at 100°C for 16 hours. Pd was removed through celite filtration, and the reaction mixture was concentrated. The concentrated solution was purified by MPLC, concentrated, then slurried with acetone and filtered to obtain the title compound (0.79 g, 40%) as a white solid as a filter cake.

¹H NMR (400 MHz, DMSO-*d*₆) δ 8.79-8.77 (m, 1H), 8.59-8.56 (m, 2H), 7.80-7.76 (m, 2H), 7.74-7.71 (m, 1H), 7.69-7.66 (m, 2H), 7.48-7.41 (m, 3H), 7.23-7.19 (m, 2H), 3.86-3.85 (m, 3H).

### Step 2: Synthesis of 3-((4-(pyridin-4-yl)phenyl)amino)benzoic acid

Methyl 3-((4-(pyridin-4-yl)phenyl)amino)benzoate (0.76 g, 2.5 mmol) prepared in step 1 above and LiOH·H₂O (0.42 g, 10 mmol) were dissolved in H₂O (8.5 mL) and THF (17 mL), and the reaction mixture was stirred at room temperature for 40 hours. The reaction product was acidified to pH 3 with 1N-HCl (aq), extracted with ethyl acetate, and washed with brine. Then, the organic layer was dried over MgSO₄ and concentrated. The concentrated mixture was slurried with ethyl acetate and acetone and filtered to obtain the title compound (244 mg, 34%) as a yellow solid.

¹H NMR (400 MHz, DMSO-*d*₆) δ 12.99 (br s, 1H), 9.24 (s, 1H), 8.75 (s, 2H), 8.23-8.16 (m, 2H), 7.99 (d, J = 8.8 Hz, 2H), 7.79 (s, 1H), 7.61-7.40 (m, 3H), 7.26 (d, J = 42.4 Hz, 2H).

### Preparation Example 23: Synthesis of 3-((6-phenylpyridazin-3-yl)amino)benzoic acid

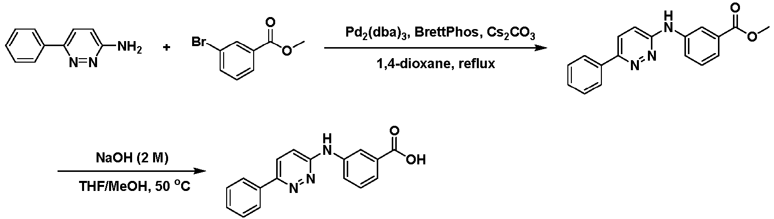

### Step 1: Synthesis of methyl 3-((6-phenylpyridazin-3-yl)amino)benzoate

Methyl 3-bromobenzoate (35.9 g, 167 mmol), 6-phenylpyridazin-3-amine (30.0 g, 175 mmol), BrettPhos (18.0 g, 33.4 mmol) and Cs₂CO₃ (136 g, 417 mmol) were dissolved in 1,4-dioxane (150 mL), and the dissolved solution was degassed and purged with N₂. Pd₂(dba)₃ (4.58 g, 5.01 mmol) was added to the mixed solution, and the reaction mixture was stirred at reflux at 100°C for 6 hours. After confirming the completion of the reaction by LC-MS, the reaction solution was filtered. The filter cake was dried under vacuum and slurried with THF (300 mL) and MeOH (60 mL) at 25°C for 12 hours. The suspension was filtered and the filtrate was concentrated to obtain the title compound as a yellow solid (30.0 g, 98.3 mmol, 59%).

¹H NMR (400 MHz, DMSO-*d*₆) δ 8.65 (s, 1H), 8.55 (s, 1H), 8.07 (d, *J* = 6.0 Hz, 1H), 7.97 (d, *J* = 5.6 Hz 3H), 7.49-7.47 (m, 1H), 7.46-7.44 (m, 4H), 7.44-7.42 (m, 1H), 3.87 (s, 3H).

### Step 2: Synthesis of 3-((6-phenylpyridazin-3-yl)amino)benzoic acid

Methyl 3-((6-phenylpyridazin-3-yl)amino)benzoate (20.0 g, 65.5 mmol) prepared in step 1 above and NaOH (2 M, 131 mL, 262 mmol) were dissolved in THF (100 mL) and MeOH (20 mL), and the reaction mixture was stirred at 50°C for 12 hours. After confirming the completion of the reaction by LC-MS, the reaction solution was dried under vacuum, and H₂O was added, followed by acidification to pH 3 with 1N-HCl (aq). The suspension was filtered with H₂O, and the filter cake was dried under vacuum to obtain the title compound (10.0 g, 34.3 mmol, 52%) as an off-white solid.

¹H NMR (400 MHz, DMSO-*d*₆) δ 12.97 (s, 1H), 9.60 (s, 1H), 8.49 (s, 1H), 8.08 (t, *J* = 9.2 Hz, 4H), 7.55-7.46 (m, 5H), 7.25 (d, *J* = 9.2 Hz, 1H).

### Preparation Example 24: Synthesis of 3-((4-(pyridazin-3-yl)phenyl)amino)benzoic acid

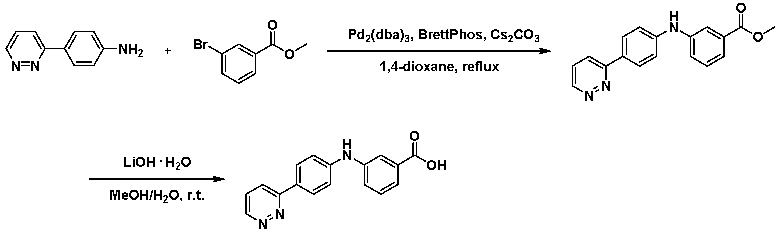

### Step 1: Synthesis of methyl 3-((4-(pyridazin-3-yl)phenyl)amino)benzoate

Methyl 3-bromobenzoate (57.1 g, 265 mmol), 4-(pyridazin-3-yl)aniline (45.0 g, 263 mmol), BrettPhos (9.88 g, 18.4 mmol), Cs₂CO₃ (214 g, 657 mmol) and Pd₂(dba)₃ (12.0 g, 13.1 mmol) were dissolved in 1,4-dioxane (350 mL), and the dissolved solution was degassed and purged with N₂, followed by stirring at reflux at 90°C for 12 hours. After confirming the completion of the reaction by LC-MS, the reaction solution was diluted with H₂O, extracted with ethyl acetate, and washed with brine. Then, the organic layer was dried over Na₂SO₄ and concentrated. The concentrated mixture was purified by column chromatography to obtain the title compound (32.0 g, 40%) as a brown solid.

¹H NMR (400 MHz, DMSO-*d*₆) δ 9.11 (d, *J* = 3.2 Hz, 1H), 8.80 (s, 1H), 8.14-8.09 (m, 3H), 7.75-7.69 (m, 2H), 7.47-7.44 (m, 3H), 7.23 (d, *J* = 8.4 Hz, 2H), 3.85 (s, 3H).

### Step 2: Synthesis of 3-((4-(pyridazin-3-yl)phenyl)amino)benzoic acid

Methyl 3-((4-(pyridazin-3-yl)phenyl)amino)benzoate (30.0 g, 98.3 mmol) prepared in Step 1 and LiOH·H₂O (8.25 g, 197 mmol) were dissolved in H₂O (60 mL) and MeOH (120 mL), and the reaction mixture was stirred at room temperature for 12 hours. After confirming the completion of the reaction by LC-MS, the reaction solution was dried under vacuum, and H₂O was added, followed by acidification to pH 5 to 6 with 0.5N-HCl (aq). The suspension was filtered with H₂O, and the filter cake was dried under vacuum to obtain the title compound (25 g, 95%) as a brown solid.

¹H NMR (400 MHz, DMSO-*d*₆) δ 12.98 (s, 1H), 9.12 (d, *J* = 4.0 Hz, 1H), 8.79 (s, 1H), 8.17-8.15 (m, 1H), 8.11-8.08 (m, 2H), 7.74-7.72 (m, 2H), 7.47-7.46 (m, 1H), 7.41-7.40 (m, 2H), 7.23 (d, *J* = 8.4 Hz, 2H).

### Preparation Example 25: Synthesis of 3-((5-(3-fluorophenyl)pyrimidin-2-yl)amino)benzoic acid

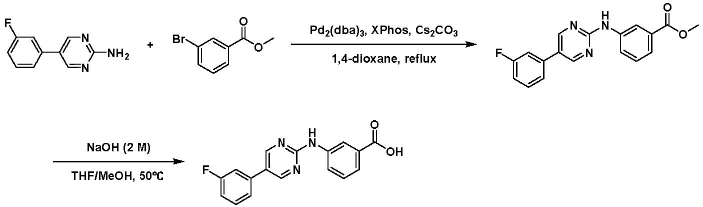

### Step 1: Synthesis of methyl 3-((5-(3-fluorophenyl)pyrimidin-2-yl)amino)benzoate

5-(3-Fluorophenyl)pyrimidin-2-amine (30.0 g, 158 mmol), methyl 3-bromobenzoate (31.0 g, 144 mmol), XPhos (20.6 g, 43.3 mmol), and Cs₂CO₃ (141 g, 432 mmol) were dissolved in 1,4-dioxane (210 mL), and the dissolved solution was degassed and purged with N₂. Pd₂(dba)₃ (3.96 g, 4.32 mmol) was added to the mixed solution, and the reaction mixture was stirred at reflux at 100°C for 12 hours. After confirming the completion of the reaction by TLC, the reaction solution was diluted with H₂O and the suspension was filtered with H₂O. The filtercake was dried under vacuum, then THF was added, and the suspension was filtered with THF. The filtrate was purified by column chromatography to obtain the title compound (10.0 g, 22%) as a white solid.

¹H NMR (400 MHz, DMSO-*d*₆) δ 10.10 (s, 1H), 8.93 (s, 2H), 8.49 (s, 1H), 8.05 (dd, *J* = 1.2 Hz, 1H), 7.67 - 7.43 (m, 5H), 7.20 (s, 1H), 3.86 (s, 3H).

### Step 2: Synthesis of 3-((5-(3-fluorophenyl)pyrimidin-2-yl)amino)benzoic acid

Methyl 3-((5-(3-fluorophenyl)pyrimidin-2-yl)amino)benzoate (10 g, 30.9 mmol) prepared in step 1 above and NaOH (2 M, 30.9 mL) were dissolved in THF (70 mL) and MeOH (50 mL), and the reaction mixture was then stirred at 50°C for 12 hours. After confirming the completion of the reaction by TLC, the reaction solution was dried under vacuum, and H₂O was added, followed by acidification to pH 1 to 2 with 1N-HCl (aq). The suspension was filtered with H₂O, and the filter cake was dried under vacuum to obtain the title compound (5.0 g, 52%) as a white solid.

¹H NMR (400 MHz, DMSO-*d*₆) δ 10.04 (s, 1H), 8.92 (s, 2H), 8.79 (s, 1H), 8.46 (s, 1H), 8.00 (d, *J* = 8.0 Hz, 1H), 7.66-7.50 (m, 4H), 7.42 (t, *J* = 9.2 Hz, 1H), 7.22-7.17 (m, 1H).

### Preparation Example 26: Synthesis of 3-((5-(3-fluorophenyl)pyridin-2-yl)amino)benzoic acid

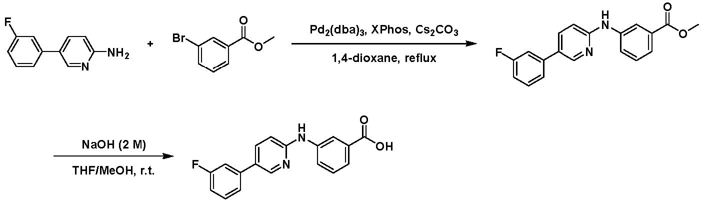

### Step 1: Synthesis of methyl 3-((5-(3-fluorophenyl)pyridin-2-yl)amino) benzoate

5-(3-Fluorophenyl)pyridin-2-amine (20.0 g, 93.0 mmol), methyl 3-bromobenzoate (18.4 g, 97.6 mmol), XPhos (13.30 g, 27.9 mmol), and Cs₂CO₃ (90.9 g, 279 mmol) were dissolved in 1,4-dioxane (100 mL), and the dissolved solution was degassed and purged with N₂. Pd₂(dba)₃ (2.55 g, 2.79 mmol) was added to the mixed solution, and the reaction mixture was stirred at reflux at 100°C for 12 hours. After confirming the completion of the reaction by LC-MS, the reaction solution was filtered with ethyl acetate, and the organic layer was dried over Na₂SO₄ and concentrated. The residue was slurried with MTBE at room temperature for 1 hour and filtered through MTBE. The filtercake was dried under vacuum to obtain the title compound (16.5 g, 55%) as a white solid.

¹H NMR (400 MHz, DMSO-*d*₆) δ 9.51 (s, 1H), 8.58 (d, J = 2.4 Hz, 1H), 8.35 (t, J = 1.6 Hz, 1H), 8.07-8.05 (m, 1H), 7.98 (dd, J = 8.8, 2.4 Hz, 1H), 7.55-7.40 (m, 5H), 7.17-7.12 (m, 1H), 6.93 (d, J = 8.4 Hz, 1H), 3.86 (s, 3H).

### Step 2: Synthesis of 3-((5-(3-fluorophenyl)pyridin-2-yl)amino)benzoic acid

Methyl 3-((5-(3-fluorophenyl)pyridin-2-yl)amino)benzoate (18 g, 55.8 mmol) prepared in step 1 above and NaOH (2 M, 55.8 mL) were dissolved in THF (18 mL) and MeOH (90 mL), and the reaction mixture was then stirred at room temperature for 8 hours. After confirming the completion of the reaction by LC-MS, the reaction solution was concentrated and then H₂O was added. The residue was acidified to pH 5 with 6N-HCl (aq). The suspension was filtered, and the filter cake was dried under vacuum to obtain the title compound (10.0 g, 58%) as a bright yellow solid.

¹H NMR (400 MHz, DMSO-*d*₆) δ 12.85 (s, 1H), 9.49 (s, 1H), 8.56 (d, J = 2.0 Hz, 1H), 8.33-8.32 (m, 1H), 7.99-7.95 (m, 2H), 7.54-7.36 (m, 5H), 7.15-7.10 (m, 1H), 6.93 (d, J = 8.8 Hz, 1H).

### Preparation Example 27: Synthesis of 3-((4-(pyrimidin-4-yl)phenyl)amino)benzoic acid

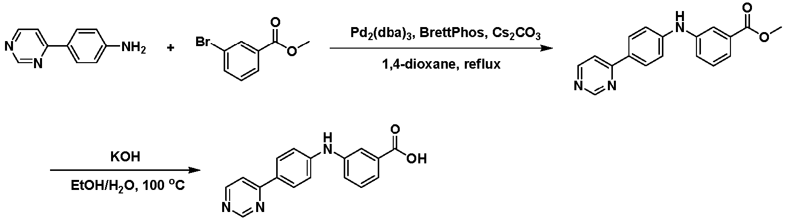

### Step 1: Synthesis of methyl 3-((4-(pyrimidin-4-yl)phenyl)amino)benzoate

4-(Pyrimidin-4-ylaniline) (1.70 g, 9.93 mmol), methyl 3-bromobenzoate (2.14 g, 9.93 mmol), BrettPhos (1.07 g, 1.99 mmol), Cs₂CO₃ (8.09 g, 24.8 mmol) were dissolved in 1,4-dioxane (15 mL), and the dissolved solution was degassed and purged with N₂. Pd₂(dba)₃ (909 mg, 0.993 mmol) was added to the mixed solution, followed by stirring at reflux at 100°C for 12 hours. After confirming the completion of the reaction by TLC, the reaction solution was diluted with H₂O, extracted with ethyl acetate, and washed with brine. Then, the organic layer was dried over Na₂SO₄ and concentrated. The residue was purified by column chromatography to obtain the title compound (1.30 g, 43%) as a yellow solid.

¹H NMR (400 MHz, DMSO-*d*₆) δ 9.13 (s, 1H), 8.91 (s, 1H), 8.74 (d, *J* = 5.20 Hz, 1H), 8.15 (d, *J* = 8.80 Hz, 2H), 7.96 (d, *J* = 5.20 Hz, 1H), 7.76 (s, 1H), 7.52-7.49 (m, 1H), 7.46-7.43 (m, 2H), 7.20 (d, *J* = 8.80 Hz, 2H), 3.85 (s, 3H).

### Step 2: Synthesis of 3-((4-(pyrimidin-4-yl)phenyl)amino)benzoic acid

Methyl 3-((4-(pyrimidin-4-yl)phenyl)amino)benzoate (1.30 g, 4.26 mmol) prepared in step 1 above and KOH (478 mg, 8.52 mmol) were dissolved in H₂O (5 mL) and EtOH (7 mL), and the reaction mixture was stirred at 100°C for 4 hours. After confirming the completion of the reaction by TLC, H₂O was added to the reaction solution, followed by extraction with 2-MeTHF and acidification to pH 5 to 6 with 0.5N-HCl (aq). The mixed solution was extracted with 2-MeTHF, washed with brine, and the organic layer was dried over Na₂SO₄ and concentrated. The residue was slurried with CH₃CN at room temperature for 12 hours to obtain the title compound (1.01 g, 81%) as a yellow solid.

¹H NMR (400 MHz, DMSO-*d*₆) δ 12.96 (s, 1H), 9.13 (s, 1H), 8.88 (s, 1 H), 8.74 (d, *J* = 5.60 Hz, 1H), 8.15 (d, *J* = 8.80 Hz, 2H), 7.97-7.94 (m, 1H), 7.75 (s, 1H), 7.52-7.48 (m, 1H), 7.43-7.41 (m, 2H), 7.20 (d, *J* = 8.80 Hz, 2H).

### Preparation Example 28: Synthesis of 3-((5-(3-fluorophenyl)pyrimidin-2-yl)amino)-4-methoxybenzoic acid

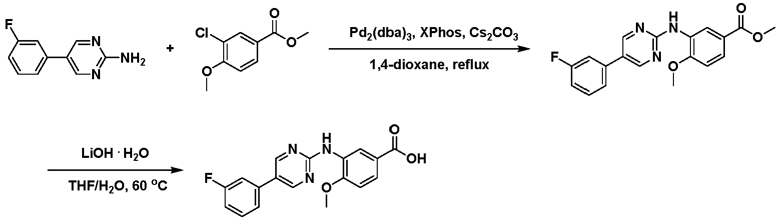

### Step 1: Synthesis of methyl 3-((5-(3-fluorophenyl)pyrimidin-2-yl)amino)-4-methoxybenzoate

5-(3-Fluorophenyl)pyrimidin-2-amine (1.13 g, 6.0 mmol), methyl 3-chloro-4-methoxybenzoate (1.81 g, 9.00 mmol), XPhos (572 mg, 1.2 mmol), Cs₂CO₃ (3.90 g, 12.0 mmol), and Pd₂(dba)₃ (824 mg, 0.90 mmol) were added to 1,4-dioxane (50 mL) and stirred at reflux at 100°C for 12 hours. After confirming the completion of the reaction by LC-MS, a saturated NaHCO₃ solution was added to the reaction solution, followed by extraction with ethyl acetate and concentration. The residue was purified by MPLC to obtain the title compound (1.43 g, 68%) as an orange solid.

¹H NMR (400 MHz, DMSO) δ 8.91 (s, 2H), 8.73 (s, 1H), 8.52 (s, 1H), 7.76 - 7.59 (m, 3H), 7.56 - 7.48 (m, 1H), 7.24 - 7.17 (m, 2H), 3.95 (s, 3H), 3.84 (s, 3H).

### Step 2: Synthesis of 3-((5-(3-fluorophenyl)pyrimidin-2-yl)amino)-4-methoxybenzoic acid

Methyl 3-((5-(3-fluorophenyl)pyrimidin-2-yl)amino)-4-methoxybenzoate (706 mg, 2.00 mmol) prepared in step 1 above and LiOH·H₂O (1.68 g, 40.0 mmol) were dissolved in H₂O (10 mL) and THF (20 mL), and the reaction mixture was then stirred at 60°C for 48 hours. The completion of the reaction was confirmed by LC-MS, and the reaction product was acidified up to pH 2 with 1N-HCl (aq). The suspension was filtered with H₂O, and dried under vacuum to obtain the title compound (540 mg, 80%) as an orange solid.

¹H NMR (400 MHz, DMSO-*d*₆) δ 13.12 (s, 1H), 8.80 (s, 2H), 7.92 (s, 1H), 7.81 (d, *J* = 8.1 Hz, 1H), 7.66 (d, *J* = 8.9 Hz, 1H), 7.61-7.48 (m, 4H), 7.22-7.15 (m, 1H), 3.56 (s, 3H).

### Preparation Example 29: Synthesis of 3-((5-(pyridazin-3-yl)pyrimidin-2-yl)aminobenzoic acid

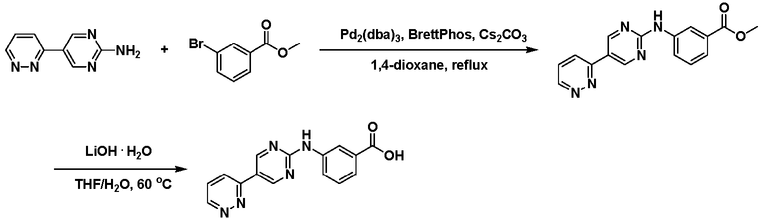

### Step 1: Synthesis of methyl 3-((5-(pyridazin-3-yl)pyrimidin-2-yl)amino)benzoate

5-(Pyridazin-3-yl)pyrimidin-2-amine (2.34 g, 13.5 mmol), methyl 3-bromobenzoate (3.05 g, 14.2 mmol), BrettPhos (870 mg, 1.6 mmol), Cs₂CO₃ (11.0 g, 33.8 mmol), and Pd₂(dba)₃ (1.09 g, 1.35 mmol) were added to 1,4-dioxane (40 mL) and stirred at reflux at 90°C for 12 hours. After confirming the completion of the reaction by LC-MS, the reaction solution was diluted with H₂O, slurried with MeOH, and filtered. The resulting filter cake was slurried with dichloromethane and dried in vacuum to obtain the title compound (2.19 g, 53%) as a beige solid.

¹H NMR (400 MHz, DMSO-*d*₆) δ 10.30 (s, 1H), 9.29 (s, 2H), 9.22 (dd, *J* = 1.7, 5.0 Hz, 1H), 8.50 (s, 1H), 8.30 (dd, *J* = 1.4, 8.7 Hz, 1H), 8.09 (dd, *J* = 1.2, 8.2 Hz, 1H), 7.81 (dd, *J* = 4.9, 8.6 Hz, 1H), 7.64-7.59 (m, 1H), 7.49 (t, = 7.9 Hz, 1H), 3.88 (s, 3H).

### Step 2: Synthesis of 3-((5-(pyridazin-3-yl)pyrimidin-2-yl)amino)benzoic acid

Methyl 3-((5-(pyridazin-3-yl)pyrimidin-2-yl)amino)benzoate (2.18 g, 7.09 mmol) prepared in step 1 above and LiOH·H₂O (1.20 g, 28.38 mmol) were dissolved in H₂O (20 mL) and THF (40 mL), and the reaction mixture was stirred at 60°C for 13.5 hours. The completion of the reaction was confirmed by LC-MS, and the reaction product was neutralized with 1N-HCl (aq). The suspension was filtered with H₂O to obtain the title compound (1.8 g, 84%) as a beige solid.

¹H NMR (400 MHz, DMSO-*d*₆) δ 12.96 (s, 1H), 10.28 (s, 1H), 9.29 (d, *J* = 2.5 Hz, 2H), 9.25-9.21 (m, 1H), 8.50-8.46 (m, 1H), 8.33-8.28 (m, 1H), 8.08-8.04 (m, 1H), 7.85-7.79 (m, 1H), 7.60 (d, *J* = 7.6 Hz, 1H), 7.49-7.43 (m, 1H).

### Preparation Example 30: Synthesis of 3-([2,5'-bipyrimidine]-2'-ylamino)benzoic acid

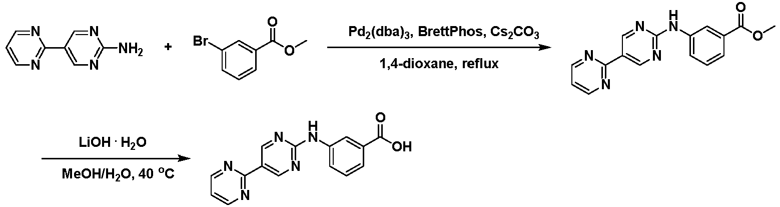

### Step 1: Synthesis of methyl 3-([2,5'-bipyrimidine]-2'-ylamino)benzoate

[2,5'-Bipyrimidin]-2'-amine (500 mg, 2.9 mmol), methyl 3-bromobenzoate (627 mg, 2.91 mmol), BrettPhos (108 mg, 0.2 mmol), Cs₂CO₃ (2.35 g, 7.21 mmol) and Pd₂(dba)₃ (116 mg, 0.14 mmol) were added to 1,4-dioxane (60 mL) and stirred at reflux at 90°C for 23 hours. After confirming the completion of the reaction by LC-MS, H₂O was added to the reaction solution and the reaction solution was extracted with ethyl acetate. The organic layer was dried over MgSO₄ and concentrated. The residue was purified by MPLC to obtain the title compound (180 mg, 20%) as a beige solid.

¹H NMR (400 MHz, DMSO-*d*₆) δ 10.34 (s, 1H), 9.36 (s, 2H), 8.90 (d, *J* = 4.9 Hz, 2H), 8.44 (t, *J* = 1.8 Hz, 1H), 8.12 (dd, *J* = 1.3, 8.1 Hz, 1H), 7.64-7.60 (m, 1H), 7.50 (d, *J* = 7.9 Hz, 1H), 7.48-7.44 (m, 1H), 3.88 (s, 3H).

### Step 2: Synthesis of 3-([2,5'-bipyrimidine]-2'-ylamino)benzoic acid

Methyl 3-([2,5'-bipyrimidin]-2'-ylamino)benzoate (180 mg, 0.59 mmol) prepared in step 1 above and LiOH·H₂O (49 mg, 1.17 mmol) were dissolved in H₂O (3 mL) and MeOH (6 mL), and the reaction mixture was stirred at 40°C for 16 hours. The completion of the reaction was confirmed by LC-MS, and the reaction product was acidified up to pH 2 with 1N-HCl (aq). The suspension was filtered with H₂O, the resulting filter cake was slurried with H₂O, and the filter cake was dried in vacuum to obtain the title compound (110 mg, 64%) as a beige solid.

MS: m/z = 294 (M+1,ESI+).

### Method a+b. Preparation of carboxylic acid derivative using Suzuki reaction and Buchwald reaction (Formula III-a)

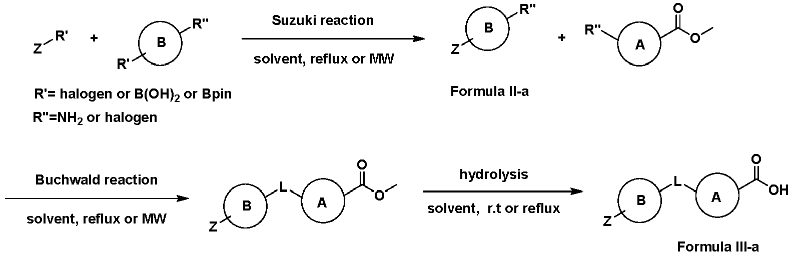

### Preparation Example 31: Synthesis of 3-((3-nitro-4-(pyridazin-3-yl)phenyl)amino)benzoic acid

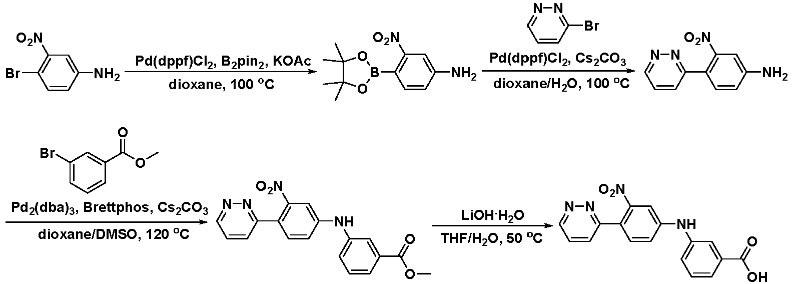

### Step 1: Synthesis of 3-nitro-4(4,4,5,5-tetramethyl-1,3,2-dioxaborolan-2-yl)aniline

To a solution of 4-bromo-3-nitroanili (5 g, 23.04 mmol) and 4,4,4',4',5,5,5',5'-octamethyl-2,2'-bi(1,3,2-dioxaborolane) (8.78 g, 34.5 mmol) in dioxane (50 mL) was added KOAc (5.65 g, 57.60 mmol) and Pd(dppf)Cl₂ (1.67 g, 2.30 mmol), the reactioni mixture was stirred at 100°C for 2 hours under N₂. The reaction mixture was cooled to room temperature and poured into water (300 mL), then extracted with EA (60 mL x3), the combined organic layer was washed with brine (300 mL x3), dried over Na₂SO₄ and concentrated under reduced pressure. The residue was purified by column chromatography to obtain the title compound (3 g, 49.3%) as a yellow solid.

MS: m/z = 265 (M+1, ESI+).

### Step 2: Synthesis of 3-nitro-4-(pyridazin-3-yl)aniline

To a solution of 3-nitro-4-(4,4,5,5-tetramethyl-1,3,2-dioxaborolan-2-yl)aniline (3 g, 11.36 mmol) and 3-bromopyridazine (2.35 g, 14.77 mmol) in dioxane/H₂O (50 mL/ 10 mL) was added Cs₂CO₃ (7.40 g, 22.72 mmol) and Pd(dppf)Cl₂ (921 mg, 1.14 mmol), the treaction mixture was stirred at 100°C for 2 hours under N₂. The reaction mixture was cooled to room temperature and poured into water (300 mL), then extracted with EA (60 mL x3), the combined organic layer was washed with brine (300 mL x3), dried over Na₂SO₄ and concentrated under reduced pressure. The residue was purified by column chromatography to obtain the title compound (1.1 g, 44.8%) as a yellow solid.

MS: m/z = 217 (M+1, ESI+).

### Step 3: Synthesis of methyl 3-((3-nitro-4-(pyridazin-3-yl)phenyl)amino)benzoate

To a solution of 3-nitro-4-(pyridazin-3-yl)aniline (1.1 g, 5.09 mmol) and methyl 3-bromobenzoate (1.29 g, 6.62 mmol) in dioxane/DMSO (50 mL/ 4 mL) was added Cs₂CO₃ (3.02 g, 10.18 mmol), Brettphos (745 mg, 1.53 mmol) and Pd₂(dba)₃ (636 mg, 0.76 mmol), the reaction mixture was stirred at 120°C for 16 hours under N₂. The reaction mixture was cooled to room temperature and poured into water (300 mL), then extracted with EA (60 mL x3), the combined organic layer was washed with brine (300 mL x3), dried over Na₂SO₄ and concentrated under reduced pressure. The residue was purified by column chromatography to obtain the title compound (800 mg, 44.9%) as a pale yellow solid.

MS: m/z = 351 (M+1, ESI+).

### Step 4: Synthesis of 3-((3-nitro-4-(pyridazin-3-yl)phenyl)amino)benzoic acid

To a solution of methyl 3-((3-nitro-4-(pyridazin-3-yl)phenyl)amino)benzoate (800 mg, 2.29 mmol) in THF/H₂O (20 mL/ 4 mL) was added LiOH·H₂O (480 mg, 11.45 mmol), the reaction mixture was stirred at 50 °C for 16 hours. The reaction mixture was acidified to pH 2 with 2N-HCl (aq) (30 mL), then extracted with EA (10 mL x3), the combined organic layer was washed with brine (30 mL x3), dried over Na₂SO₄ and concentrated under reduced pressure to obtain the title compound (700 mg, 91.14%) as a yellow solid.

¹H NMR (400 MHz, DMSO-*d*₆) δ 9.49 (s, 1H), 9.18 (s, 1H), 7.99 (d, J = 13.1 Hz, 1H), 7.82 (s, 2H), 7.68 (s, 1H), 7.63 (d, J = 2.2 Hz, 1H), 7.58 (d, J = 6.8 Hz, 1H), 7.48 (s, 3H).

MS: m/z = 337 (M+1, ESI+).

### Preparation Example 32: Synthesis of 3-((3-methoxy-4-(pyridazin-3-yl)phenyl)amino)benzoic acid

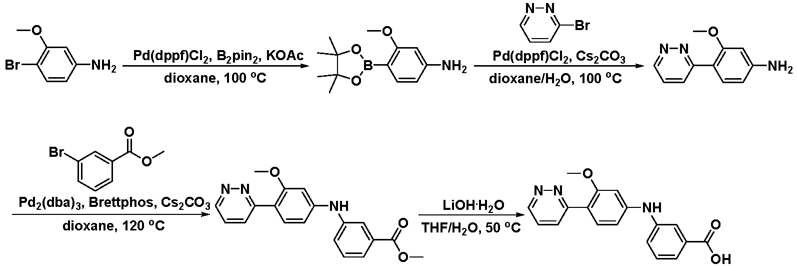

### Step 1: Synthesis of 3-methoxy-4-(4,4,5,5-tetramethyl-1,3,2-dioxaborolan-2-yl)aniline

To a solution of 4-bromo-3-methoxyaniline (5 g, 24.74 mmol) and 4,4,4',4',5,5,5',5'-octamethyl-2.2'-bi(1,3,2-dioxaborolane) (9.43 g, 37.11 mmol) in dioxane (50 mL) was added KOAc (6.06 g, 61.87 mmol) and Pd(dppf)Cl₂ (898 mg, 1.21 mmol), the reaction mixture was stirred at 100°C for 2 hours under N₂. The reaction mixture was cooled to room temperature and poured into water (300 mL), then extracted with EA (60 mL x3), the combined organic layer was washed with brine (300 mL x3), dried over Na₂SO₄ and concentrated under reduced pressure. The residue was purified by column chromatography to obtain the title compound (2.4 g, 38.96%) as a yellow solid.

¹H NMR (400 MHz, DMSO-*d*₆) δ 7.25 (d, *J* = 7.8 Hz, 1H), 6.15-6.07 (m, 2H), 5.48 (s, 2H), 3.64 (d, *J* = 6.2 Hz, 3H), 1.22 (s, 12H).

MS: m/z = 250 (M+1, ESI+).

### Step 2: Synthesis of 3-methoxy-4-(pyridazin-3-yl)aniline

To a solution of 3-bromopyridazine (1.99 g, 12.53 mmol) and 3-methoxy-4-(4,4,5,5-tetramethyl-1,3,2-dioxaborolan-2-yl)aniline (2,4 g, 9.64 mmol) in dioxane/H₂O (50 mL/ 10 mL) was added Cs₂CO₃ (6.28 g, 19.28 mmol) and Pd(dppf)Cl₂ (697 mg, 0.96 mmol), the reaction mixture was stirred at 100°C for 2 hours under N₂. The reaction mixture was cooled to room temperature and poured into water (300 mL), then extracted with EA (60 mL x3), the combined organic layer was washed with brine (300 mL x3), dried over Na₂SO₄ and concentrated under reduced pressure. The residue was purified by column chromatography to obtain the title compound (1.4 g, 72.27%) as a yellow solid.

MS: m/z = 202 (M+1, ESI+).

### Step 3: Synthesis of methyl 3-((3-methoxy-4-(pyridazin-3-yl)phenyl)amino)benzoate

To a solution of 3-methoxy-4-(pyridazin-3-yl)aniline (1.4 g, 6.97 mmol) and methyl 3-bromobenzoate (1.95 g, 9.05 mmol) in dioxane (20 mL) was added Cs₂CO₃ (4.54 g, 13.94 mmol), Brettphos (697 mg, 1.30 mmol) and Pd₂(dba)₃ (595 mg, 0.65 mmol), the reaction mixture was stirred at 120°C under N₂ for 16 hours. The reaction mixture was cooled to room temperature and poured into water (300 mL), then extracted with EA (60 mL x3), the combined organic layer was washed with brine (300 mL x3), dried over Na₂SO₄ and concentrated under reduced pressure. The residue was purified by column chromatography to obtain the title compound (1.1 g, 47.21%) as a pale yellow solid.

¹H NMR (400 MHz, DMSO-*d*₆) δ 9.08 (dd, *J* = 4.8, 1.6 Hz, 1H), 8.84-8.68 (m, 1H), 8.15-7.94 (m, 1H), 7.81-7.74 (m, 2H), 7.64 (dd, *J* = 8.7, 4.9 Hz, 1H), 7.51-7.39 (m, 3H), 6.89-6.81 (m, 2H), 3.86 (s, 3H), 3.82 (s, 3H).

MS: m/z = 336 (M+1, ESI+).

### Step 4: Synthesis of 3-((3-methoxy-4-(pyridazin-3-yl)phenyl)amino)benzoic acid

To a solution of methyl 3-((3-methoxy-4-(pyridazin-3-yl)phenyl)amino)benzoate (1.1 g, 3.28 mmol) in THF/H₂O (20 mL/ 4 mL) was added LiOH·H₂O (689 mg, 16.42 mmol), the reaction mixture was stirred at 50 °C for 16 hours. The reaction mixture was acidified to pH 2 with 2N-HCl (aq) (20 mL), then extracted with EA (10 mL x3), the combined organic layer was washed with brine (30 mL x3), dried over Na₂SO₄ and concentrated under reduced pressure to obtain the title compound (900 mg, 85.71%) as a yellow solid.

¹H NMR (400 MHz, DMSO-*d*₆) δ 9.33 (dd, *J* = 4.9, 1.4 Hz, 1H), 8.68 (d, *J* = 8.9 Hz, 1H), 8.28 (dd, *J* = 8.9, 4.9 Hz, 1H), 7.79 (dd, *J* = 12.9, 5.5 Hz, 2H), 7.61-7.41 (m, 3H), 6.89 (dd, *J* = 6.6, 2.1 Hz, 2H).

MS: m/z = 322 (M+1, ESI+).

### Preparation Example 33: Synthesis of 3-((5-(6-fluoropyridin-2-yl)pyrimidin-2-yl)amino)benzoic acid

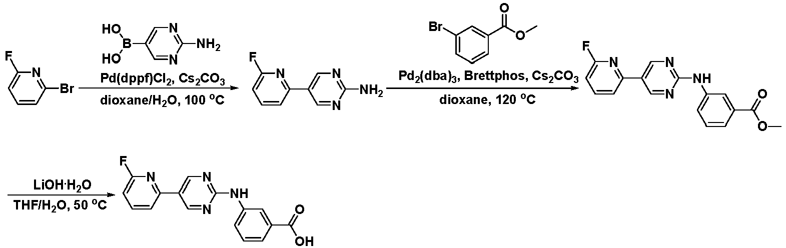

### Step 1: Synthesis of 5-(6-fluoropyridin-2-yl)pyrimidin-2-amine

To a solution of 2-bromo-6-fluoropyridine (3 g, 17.05 mmol) and (2-aminopyrimidin-5-yl) boronic acid (2.26 g, 16.23 mmol) in dioxane/H₂O (50 mL/ 10 mL) was added Cs₂CO₃ (10.58 g, 32.46 mmol) and Pd(dppf)Cl₂ (1.18 g, 1.62 mmol), the reaction mixture was sitrred at 100°C for 16 hours under N₂. The reaction mixture was cooled to room temperature and poured into water (100 mL), then extracted with EA (60 mL x3), the combined organic layer was washed with brine (100 mL x3), dried over Na₂SO₄ and concentrated under reduced pressure. The residue was purified by column chromatography to obtain the title compound (3.10 g, 92.50%) as a brown solid.

MS: m/z = 191 (M+1, ESI+).

### Step 2: Synthesis of methyl 3-((5-(6-fluoropyridin-2-yl)pyrimidin-2-yl)amino)benzoate

To a solution of 5-(6-fluoropyridin-2-yl)pyrimidin-2-amine (3 g, 15.70 mmol) and methyl 3-bromobenzoate (3.39 g, 15.70 mmol) in dioxane (80 mL) was added Cs₂CO₃ (10.26 g, 31.40 mmol), Brettphos (847 mg, 1.57 mmol) and Pd₂(dba)₃ (723 mg, 1.57 mmol), the reaction mixture was stirred at 120°C under N₂ for 16 hours. The reaction mixture was cooled to room temperature and poured into water (200 mL), then extracted with EA (80 mL x3), the combined organic layer was washed with brine (200 mL x3), dried over Na₂SO₄ and concentrated under reduced pressure. The residue was purified by column chromatography to obtain the title compound (3.8 g, 71. 96%) as a brown solid.

¹H NMR (400 MHz, DMSO-*d*₆) δ 10.22 (d, *J* = 24.0 Hz, 1H), 9.19 (d, *J* = 16.1 Hz, 2H), 8.53-8.41 (m, 1H), 8.15-8.01 (m, 2H), 7.97 (dd, *J* = 7.5, 2.5 Hz, 1H), 7.60 (d, *J* = 7.7 Hz, 1H), 7.51-7.40 (m, 1H), 7.18-7.08 (m, 1H), 3.87 (s, 3H).

MS: m/z = 325 (M+1, ESI+).

### Step 3: Synthesis of 3-((5-(6-fluoropyridin-2-yl)pyrimidin-2-yl)amino)benzoic acid

To a solution of methyl 3-((5-(6-fluoropyridin-2-yl)pyrimidin-2-yl)amino)benzoate (3.8 g, 11.73 mmol) in THF/H₂O (50 mL/ 10 mL) was added LiOH·H₂O (2.46 g, 58.64 mmol), the reaction mixture was stirred at 50°C for 16 hours. The reaction mixture was acidified to pH 2 with 2N-HCl (aq) (80 mL), then extracted with EA (40 mL x3), the combined organic layer was washed with brine (80 mL x3), dried over Na₂SO₄ and concentrated under reduced pressure to obtain the title compound (3.2 g, 88.89%) as a brown solid.

¹H NMR (400 MHz, DMSO-*d*₆) δ 9.97 (d, *J* = 39.6 Hz, 1H), 9.22-9.08 (m, 2H), 8.30 (d, *J* = 14.8 Hz, 1H), 8.06 (q, *J* = 8.1 Hz, 1H), 7.99-7.90 (m, 1H), 7.85 (d, *J*=8.0 Hz, 1H), 7.59 (d, *J* = 7.5 Hz, 1H), 7.29 (t, *J* = 7.8 Hz, 1H), 7.10 (dd, *J* = 8.0, 2.2 Hz, 1H).

MS: m/z = 311 (M+1, ESI+).

### Preparation Example 34: Synthesis of 3-((4-(6-fluoropyridin-2-yl)phenyl)amino)benzoic acid

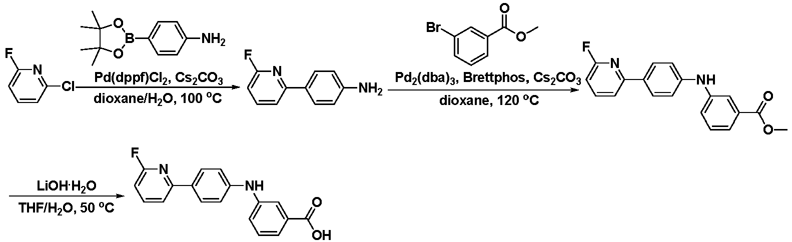

### Step 1: Synthesis of 4-(6-fluoropyridin-2-yl)aniline

To a solution of 2-chloro-6-fluoropyridine (1 g, 7.60 mmol) and 4-(4,4,5,5-tetramethyl-1,3,2-dioxaborolan-2-yl)aniline (2.0 g, 9.12 mmol) in dioxane/H₂O (50 mL/ 10 mL) was added Cs₂CO₃ (4.95 g, 15.20 mmol) and Pd(dppf)Cl₂ (616 mg, 0.76 mmol), the reaction mixture was stirred at 100°C for 2 hours under N₂. The reaction mixture was cooled to room temperature and poured into water (100 mL), then extracted with EA (60 mL x3), the combined organic layer was washed with brine (100 mL x3), dried over Na₂SO₄ and concentrated under reduced pressure. The residue was purified by column chromatogaphy to obtain the title compound (1.4 g, 88.60%) as a brown solid.

MS: m/z = 189 (M+1, ESI+)

### Step 2: Synthesis of 3-((4-(6-fluoropyridin-2-yl)phenyl)amino)benzoate

To a solution of 4-(6-fluoropyridin-2-yl)aniline (1.4 g, 7.45 mmol) and methyl 3-bromobenzoate (1.92 g, 8.94 mmol) in dioxane (30 mL) was added Cs₂CO₃ (4.84 g, 14.90 mmol), Brettphos (403 mg, 0.75 mmol) and Pd₂(dba)₃ (687 mg, 0.75 mmol), the reaction mixture was stirred at 120°C under N₂ for 16 hours. The reaction mixture was cooled to room temperature and poured into water (100 mL), then extracted with EA (40 mL x3), the combined organic layer was washed with brine (100 mL x3), dried over Na₂SO₄ and concentrated under reduced pressure. The residue was purified by column chromatography to obtain the title compound (1.1 g, 45.83%) as a brown solid.

¹H NMR (400 MHz, DMSO-*d*₆) δ 8.78 (s, 1H), 8.04-7.95 (m, 3H), 7.83 (dd, *J* = 7.6, 2.7 Hz, 1H), 7.73 (s, 1H), 7.51-7.39 (m, 3H), 7.19 (d, *J* = 8.8 Hz, 2H), 7.01 (dd, *J* = 8.0, 2.8 Hz, 1H), 3.84 (d, *J* = 9.8 Hz, 3H).

MS: m/z = 323 (M+1, ESI+).

### Step 3: Synthesis of 3-((4-(6-fluoropyridin-2-yl)phenyl)amino)benzoic acid

To a solution of methyl 3-((4-(6-fluoropyridin-2-yl)phenyl)amino)benzoate (1.1 g, 3.42 mmol) in THF/H₂O (25 mL/ 5 mL) was added LiOH·H₂O (718 mg, 17.10 mmol), the reaction mixture was stirred at 50°C for 16 hours. The reaction mixture was acidified to pH 2 with 2N-HCl (aq) (40 mL), then extracted with EA (15 mL x3), the combined organic layer was washed with brine (60 mL x3), dried over Na₂SO₄ and concentrated under reduced pressure to obtain the title compound (900 mg, 85.70%) as a yellow solid.

¹H NMR (400 MHz, DMSO-*d*₆) δ 8.68 (s, 1H), 8.13-8.00 (m, 3H), 7.90 (ddd, *J* = 21.2, 7.6, 2.5 Hz, 1H), 7.79 (s, 1H), 7.76-7.63 (m, 1H), 7.57-7.49 (m, 1H), 7.48-7.38 (m, 2H), 7.28-7.10 (m, 2H), 7.05 (dd, J = 8.0, 2.7 Hz, 1H).

MS: m/z = 309 (M+1, ESI+).

### Preparation Example 35: Syntheseis of 2-(dimethylamin)-5-((4-(pyrazin-2-yl)phenyl)amino)benzoic acid

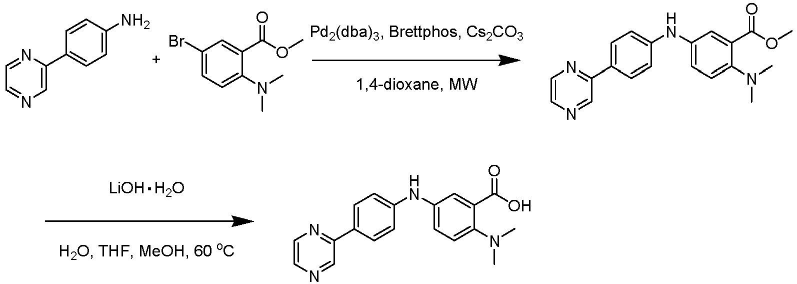

### Step 1: Synthesis of methyl 2-(dimethylamine)-5-((4-(pyrazin-2-yl)phenyl)amino)benzoate

To a solution of 4-(pyrazin-2-yl)aniline (150 mg, 0.88 mmol), methyl 5-bromo-2-(dimethylamino)benzoate (271 mg, 1.05 mmol) in 1,4-dioxane (9 mL) was added Pd₂(dba)₃ (71 mg, 0.0876 mmol), Brettphos (235 mg, 0.438 mmol) and Cs₂CO₃ (563 mg, 1.75 mmol). The mixture was stirred in microwave at 150°C for 2 hours. The reaction mixture was cooled to room temperature and poured into water, then extracted with DCM, the combined organic layer was washed with brine, dried over MgSO₄ and concentrated under reduced pressure. The residue was purified by MPLC. The crude mixture was solidified using EA and hexane to obtain the title compound (173 mg, 57%) as a yellow solid.

¹H NMR (400 MHz, DMSO-*d*₆) δ 9.13 (d, *J* = 1.6 Hz, 1H), 8.62-8.57 (m, 1H), 8.45 (d, J = 2.5 Hz, 1H), 8.38 (s, 1H), 8.04-7.97 (m, 2H), 7.34 (d, *J* = 2.8 Hz, 1H), 7.26 (dd, *J* = 2.8, 8.8 Hz, 1H), 7.07-6.98 (m, 3H), 3.81 (s, 3H), 2.72 (s, 6H).

### Step 2: Synthesis of 2-(dimethylamine)-5-((4-(pyrazin-2-yl)phenyl)amino)benzoic acid

To a solution of methyl 2-(dimethylamino)-5-((4-(pyrazin-2-yl)phenyl)amino)benzoate (130 mg, 0.37 mmol) in H₂O/THF/MeOH (1 mL/ 3 mL/ 1 mL) was added LiOH·H₂O (94 mg, 2.24 mmol), the reaction mixture was stirred at 60°C for 5 hours. The reaction mixture was acidified to pH 3 with 1N-HCl (aq). Then the solid was filtered by using H₂O. The filtrate was solidified using DCM and MeOH to obtain the title compound (82 mg, 66%) as a yellow solid.

¹H NMR (400 MHz, DMSO-*d*₆) δ 9.20 (d, J = 1.6 Hz, 1H), 8.93 (s, 1H), 8.66-8.64 (m, 1H), 8.52 (d, J = 2.5 Hz, 1H), 8.13-8.07 (m, 2H), 7.82-7.76 (m, 2H), 7.45 (dd, J = 2.8, 8.8 Hz, 1H), 7.26-7.21 (m, 2H), 2.96 (s, 6H).

### Preparation Example 36: Synthesis of 4-(4-methylpiperazin-1-yl)-3-((5-(pyridazin-3-yl)pyrimidin-2-yl)amino)benzoic acid

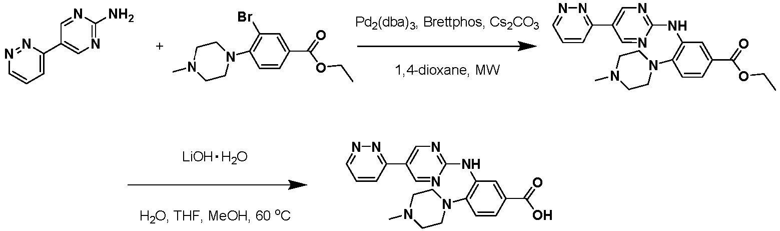

### Step 1: Synthesis of ethyl 4-(4-methylpiperazin-1-yl)-3-((5-(pyridazin-3-yl)pyrimidin-2-yl)amino)benzoate

To a solution of 5-(pyridazin-3-yl)pyrimidin-2-amine (400 mg, 2.31 mmol), ethyl 3-bromo-4-(4-methylpiperazin-1-yl)benzoate (907 mg, 2.77 mmol) in 1,4-dioxane (20 mL) was added Pd₂(dba)₃ (467 mg, 0.58 mmol), Brettphos (620 mg, 1.15 mmol) and Cs₂CO₃ (1483 mg, 4.62 mmol), the reaction mixture was stirred in microwave at 150°C for 6 hours. The reaction mixture was cooled to room temperature and poured into water, then extracted with DCM, the combined organic layer was washed with brine, dried over MgSO₄ and concentrated under reduced pressure. The residue was purified by MPLC. The crude mixture was solidified using EA and hexane to obtain the title compound (388 mg, 40%) as a brown solid.

MS: m/z = 420 (M+1,ESI+).

### Step 2: Synthesis of 4-(4-methylpiperazin-1-yl)-3-((5-(pyridazin-3-yl)pyrimidin-2-yl)amino)benzoic acid

To a solution of ethyl 4-(4-methylpiperazin-1-yl)-3-((5-(pyridazin-3-yl)pyrimidin-2-yl)amino)benzoate (387 mg, 0.95 mmol) in H₂O/THF/MeOH (1.5 mL/ 9 mL/ 3 mL) was added LiOH·H₂O (240 mg, 5.73 mmol), the reaction mixture was stirred at 60°C for 6 hours. The reaction mixture was acidified to pH 3 with 1N-HCl (aq), then extracted with DCM, the combined organic layer was concentrated under reduced pressure. The residue was filtrated and washed with MeOH to obtain the title compound (285 mg, 78%) as a yellow solid.

¹H NMR (400 MHz, DMSO-*d*₆) δ 10.79 (s, 1H), 9.30 (s, 2H), 9.24 (dd, *J* = 4.9, 1.5 Hz, 1H), 8.86 (s, 1H), 8.76 (d, *J* = 2.0 Hz, 1H), 8.35 (dd, J = 1.5, 8.7 Hz, 1H), 7.89-7.81 (m, 1H), 7.70 (dd, *J* = 2.0, 8.3 Hz, 1H), 7.29 (d, *J* = 8.3 Hz, 1H), 3.38-3.11 (m, 8H), 2.84 (m, 3H).

### Method c. Preparation of carboxylic acid derivative using other method (Formula III-a)

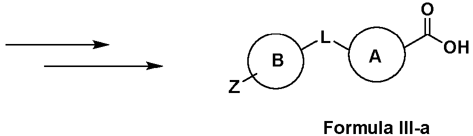

### Preparation Example 37: Synthesis of (1s,4s)-4-((6-phenylpyridazin-3-yl)amino)bicyclo[2.2.1]heptane-1-carboxylic acid

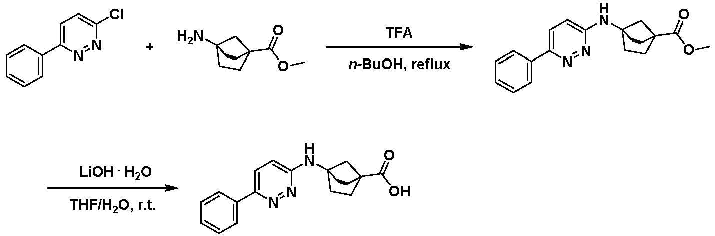

### Step 1: Synthesis of methyl (1s,4s)-4-((6-phenylpyridazin-3-yl)amino)bicyclo[2.2.1]heptane-1-carboxylate

In a sealed tube, 3-chloro-6-phenylpyridazine (0.5 g, 2.6 mmol) and methyl (1s,4s)-4-aminobicyclo[2.2.1]heptane-1-carboxylate (0.578 g, 3.4 mol) were dissolved in n-butanol (10 mL), and trifluoroacetic acid (0.148 g, 13 mmol) was added to the solution. Then, the resulting mixture was stirred at reflux at 150°C for 72 hours. After confirming the completion of the reaction by TLC, the reaction solution was cooled at room temperature, diluted with H₂O, extracted with ethyl acetate, washed with brine, and the organic layer was dried over Na₂SO₄ and concentrated. The residue was purified by column chromatography to obtain the title compound (170 mg, 20%) as a white solid.

¹H NMR (400 MHz, methanol-*d*₄) δ 7.87 (d, J = 7.6 Hz, 2H), 7.72 (d, J = 9.6, 1H), 7.50-7.40 (m, 3H), 6.95 (d, J = 9.6, 1H), 3.67 (s, 3H), 2.25-2.10 (m, 6H), 2.03-1.94 (m, 2H), 1.83-1.78 (m, 2H) .

### Step 2: Synthesis of (1s,4s)-4-((6-phenylpyridazin-3-yl)amino)bicyclo[2.2.1]heptane-1-carboxylic acid

Methyl (1s,4s)-4-((6-phenylpyridazin-3-yl)amino)bicyclo[2.2.1]heptane-1-carboxylate (1.4 g, 4.3 mol) prepared in step 1 above and LiOH·H₂O (0.541 g, 12.9 mol) were dissolved in H₂O (5 mL) and THF (10 mL), and stirred at room temperature for 6 hours. The completion of the reaction was confirmed by TLC, and the reaction product was acidified up to pH 4 with 2N-HCl (aq). The suspension was filtered with H₂O, and the filter cake was dried under vacuum to obtain the title compound (1.05 g, 80%) as an off-white solid.

¹H NMR (400 MHz, DMSO-*d*₆) δ 12.15 (s, 1H), 7.98 (d, *J* = 7.2 Hz, 2H), 7.87 (d, *J* = 9.2 Hz, 1H), 7.51-7.43 (m, 4H), 7.00 (d, *J* = 9.2 Hz, 1H), 2.13-2.04 (m, 6H), 1.83 (m, 2H), 1.70 (m, 2H).

### Preparation Example 38: Synthesis of 3-((6-phenylpyridazin-3-yl)amino)adamantane-1-carboxylic acid

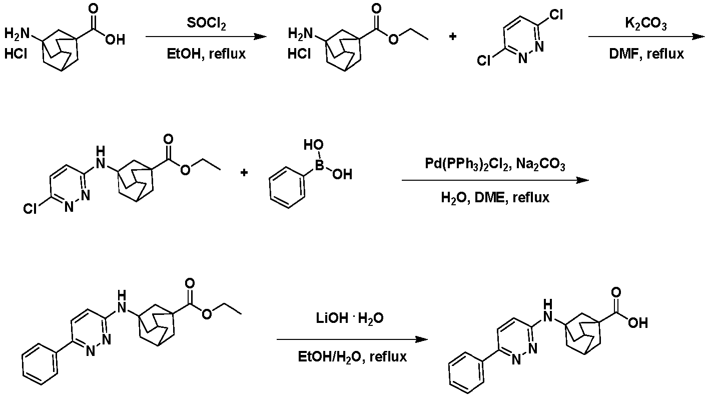

### Step 1: Synthesis of ethyl 3-aminoadamantane-1-carboxylate hydrochloride

To a solution in which 3-aminoadamantane-1-carboxylic acid hydrochloride (20.0 g, 86.3 mmol) was dissolved in EtOH (140 mL), SOCl₂ (10.3 g, 86.3 mmol) was added and stirred at reflux at 80°C for 4 hours. After confirming the completion of the reaction by LC-MS, the reaction solution was concentrated. Petroleum ether was added to the residue and the pressure was reduced until precipitation occurred. After repeating this process three times, the resulting product was slurried with petroleum ether at room temperature for 30 minutes and filtered to obtain the title compound (21.0 g, 94%) as a white solid.

¹H NMR (400 MHz, DMSO-*d*₆) δ 8.28 (s, 3H), 4.06 (q, *J* = 7.2 Hz, 2H), 2.18 (s, 2H), 1.90 (s, 2H), 1.77 (s, 6H), 1.67 - 1.55 (m, 4H), 1.17 (t, *J* = 7.2 Hz, 3H).

### Step 2: Synthesis of ethyl 3-((6-chloropyridazin-3-yl)amino)adamantane-1-carboxylate

Ethyl 3-aminoadamantane-1-carboxylate hydrochloride (21.0 g, 80.8 mmol) prepared in step 1 above and K₂CO₃ (33.5 g, 243 mmol) were added to a solution in which 3,6-dichloropyridazine (24.1 g, 162 mmol) was dissolved in DMF (147 mL), followed by stirring at reflux at 135°C for 12 hours. After confirming the completion of the reaction by TLC, the reaction solution was diluted with H₂O, extracted with ethyl acetate, and washed with brine. Then, the organic layer was dried over Na₂SO₄ and concentrated. The residue was purified by column chromatography to obtain the title compound (1.80 g, 7%) as a white solid.

¹H NMR (400 MHz, CDCl₃) δ 8.28 (s, 3H), 4.06 (q, J = 7.2 Hz, 2H), 2.18 (s, 2H), 1.90 (s, 2H), 1.77 (s, 6H), 1.67 - 1.55 (m, 4H), 1.17 (t, J = 7.2 Hz, 3H).

### Step 3: Synthesis of ethyl 3-((6-phenylpyridazin-3-yl)amino)adamantane-1-carboxylate

To a solution in which ethyl 3-((6-chloropyridazin-3-yl)amino)adamantane-1-carboxylate (1.80 g, 5.36 mmol) prepared in step 2 above was dissolved in DME (9 mL) and H₂O (1.8 mL), phenylboronic acid (719 mg, 5.90 mmol) and Na₂CO₃ (2.84 g, 26.8 mmol) were added. Pd(PPh₃)₂Cl₂ (376 mg, 0.536 mmol) was added to the mixed solution, followed by degassing and purging with N₂, and the mixture was stirred at reflux at 80°C for 12 hours. After confirming the completion of the reaction by TLC, the reaction solution was diluted with H₂O, extracted with ethyl acetate, and washed with brine. Then, the organic layer was dried over Na₂SO₄ and concentrated. The residue was purified by column chromatography and prep-HPLC to obtain the title compound (800 mg, 40%) as a white solid.

¹H NMR (400 MHz, CDCl₃) δ 7.97 (d, *J* = 7.2 Hz, 2H), 7.56 (d, *J* = 9.6 Hz, 1H), 7.48 - 7.44 (m 2H), 7.42 - 7.40 (m 1H), 6.70 (d, *J* = 9.2 Hz, 1H), 4.15 - 4.09 (m, 2H), 2.33 (s, 2H), 2.28 (s, 2H), 2.19 (s, 3H), 1.90 (q, *J* = 12 Hz, 3H), 1.75 - 1.70 (m, 2H), 1.59 (s, 2H), 1.29 - 1.23 (m, 3H).

### Step 4: Synthesis of 3-((6-phenylpyridazin-3-yl)amino)adamantane-1-carboxylic acid

Ethyl 3-((6-phenylpyridazin-3-yl)amino)adamantane-1-carboxylate (800 mg, 2.12 mmol) prepared in step 3 above and LiOH·H₂O (445 mg, 10.6 mmol) were dissolved in H₂O (1.6 mL) and EtOH (3.2 mL), and the reaction mixture was stirred at reflux at 45°C for 12 hours. After confirming the completion of the reaction by LC-MS, EtOH was removed by concentration. The residue was acidified to pH 5 with citric acid (aq). The suspension was filtered with H₂O, and the filter cake was dried under vacuum to obtain the title compound (450 mg, 60%) as a white solid.

¹H NMR (400 MHz, DMSO-*d*₆) δ 12.11 (s, 1H), 7.96 (d, *J* = 7.2 Hz, 2H), 7.75 (d, *J* = 9.2 Hz, 1H), 7.48 - 7.44 (m, 2H), 7.40 - 7.38 (m, 1H), 6.90 (d, *J* = 9.6 Hz, 1H), 6.60 (s, 1H), 2.26 (s, 2H), 2.18 - 2.16 (m, 4H), 2.05 - 2.03 (m, 2H), 1.82 - 1.79 (s, 4H), 1.66 - 1.63 (m, 2H).

### Preparation Example 39: Synthesis of 3-((4-phenylpiperazin-1-yl)methyl)benzoic acid

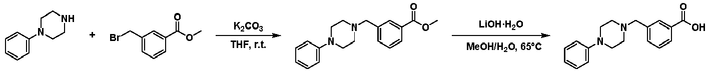

### Step 1: Synthesis of methyl 3-((4-phenylpiperazin-1-yl)methyl)benzoate

1-Phenylpiperazine (648 mg, 4.0 mmol), methyl 3-(bromomethyl)benzoate (458 mg, 2.0 mmol) and K₂CO₃ (552 mg, 4.0 mmol) were dissolved in THF (18 mL) and stirred at room temperature for 39.5 hours. After confirming the completion of the reaction by TLC, the reaction solution was concentrated. The residue was slurried with ethyl acetate and filtered, and the filter cake was purified by MPLC to obtain the title compound (532 mg, 85%) as a white solid.

MS: m/z = 311 (M+1, ESI+).

### Step 2: Synthesis of 3-((4-phenylpiperazin-1-yl)methyl)benzoic acid

To a solution in which methyl 3-((4-phenylpiperazin-1-yl)methyl)benzoate (310 mg, 1.0 mmol) prepared in step 1 above was dissolved in MeOH (10 mL) and H₂O (2 mL), LiOH·H₂O (422 mg, 10.0 mmol) was added and stirred at 65°C for 2 hours. The completion of the reaction was confirmed by LC-MS, and the reaction product was neutralized up to pH 6 with 6N-HCl (aq). The suspension was slurried with H₂O and filtered to obtain the title compound (221 mg, 75%) as a white solid.

MS: m/z = 297 (M+1, ESI+).

### Preparation Example 40: Synthesis of 3-(4-(pyridazin-3-yl)phenoxy)benzoic acid

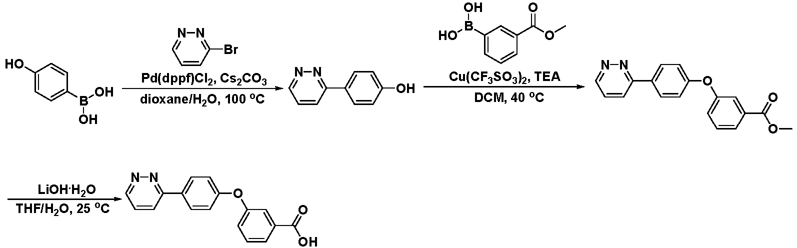

### Step 1: Synthesis of 4-(pyridazin-3-yl)phenol

To a solution of (4-hydroxyphenyl)boronic acid (2.5 g, 18.13 mmol) and 3-bromopyridazine (3.75 g, 23.56 mmol) in dioxane/H₂O (50 mL/ 10 mL) was added Cs₂CO₃ (11.81 g, 36.26 mmol) and Pd(dppf)Cl₂ (1.31 g, 1.81 mmol), the reaction mixture was stirred at 100°C for 16 hours under N₂. The reaction mixture was cooled to room temperature and poured into water (300 mL), then extracted with EA (60 mL x3), the combined organic layer was washed with brine (300 mL x3), dried over Na₂SO₄ and concentrated under reduced pressure. The residue was purified by column chromatography to obtain the title compound (1.9 g, 61.29%) as a yellow solid.

¹H NMR (400 MHz, CDCl₃) δ 7.65-7.31 (m, 3H), 7.26 (dd, *J* = 15.2, 5.7 Hz, 2H), 6.97-6.76 (m, 2H).

MS: m/z = 173 (M+1, ESI+).

### Step 2: Synthesis of methyl 3-(4-(pyridazin-3-yl)phenoxy)benzoate

To a solution of 4-(pyridazin-3-yl)phenol (1.6 g, 9.30 mmol) and (3-(methoxycarbonyl) phenyl) boronic acid (2.17 g, 12.09 mmol) in DCM (30 mL) was added Cu(CF₃SO₃)₂ (6.73 g, 18.60 mmol) and TEA (2.82 g, 27.90 mmol), the reaction mixture was stirred at 40°C for 72 hours under N₂. The reaction mixture was cooled to room temperature and poured into water (150 mL), then extracted with DCM (30 mL x3), the combined organic layer was washed with brine (150 mL x3), dried over Na₂SO₄ and concentrated under reduced pressure. The residue was purified by column chromatography to obtain the title compound (650 mg, 22.41%) as a yellow solid.

MS: m/z = 307 (M+1, ESI+).

### Step 3: Synthesis of 3-(4-(pyridazin-3-yl)phenoxy)benzoic acid

To a solution of methyl 3-(4-(pyridazin-3-yl)phenoxy)benzoate (650 mg, 2.12 mmol) in THF/H₂O (20 mL/ 4 mL) was added LiOH·H₂O (445 mg, 10.62 mmol), the reaction mixture was stirred at 25°C for 16 hours. The reaction mixture was acidified to pH 2 with 2N-HCl (aq) (20 mL), then extracted with EA (10 mL x3), the combined organic layer was washed with brine (30 mL x3), dried over Na₂SO₄ and concentrated under reduced pressure to obtain the title compound (600 mg, 96.77%) as a yellow solid.

MS: m/z = 293 (M+1, ESI+).

### Method d. Preparation of benzimidazole derivative using cyclization (Formula IV-a)

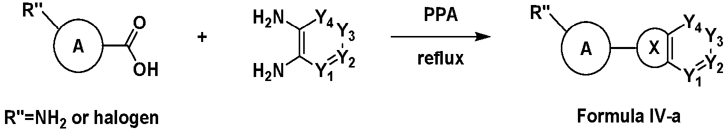

### Preparation Example 41: Synthesis of 2-(3-bromophenyl)-6-fluoro-1H-benzo[d]imidazole

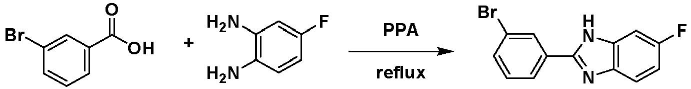

3-Bromobenzoic acid (10.0 g, 49.8 mmol) and 4-fluorobenzene-1,2-diamine (6.27 g, 49.8 mmol) were dissolved in polyphosphoric acid (75 mL) and stirred at reflux at 190°C for 5 hours. The completion of the reaction was confirmed by TLC, and the reaction product was cooled to room temperature and basified up to pH 9 with 2N-KOH (aq). The suspension was slurried with H₂O, filtered and dried in vacuum to obtain the title compound (10.5 g, 71%) as a purple solid.

¹H NMR (400 MHz, DMSO-*d*₆) δ 13.2 (s, 1H), 8.35 (s, 1H), 8.16 (d, J = 6.4 Hz, 1H), 7.54 - 7.50 (m, 4H), 7.09 (s, 1H).

### Preparation Example 42: Synthesis of 3-(5,6-dichloro-1H-benzo[d]imidazol-2-yl)aniline

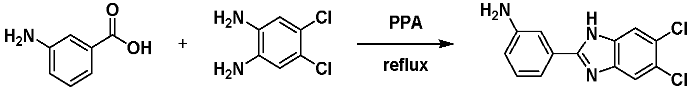

3-Aminobenzoic acid (411 mg, 3.0 mmol) and 4,5-dichlorobenzene-1,2-diamine (531 mg, 3.0 mmol) were dissolved in polyphosphoric acid (10 mL) and stirred at reflux at 200°C for 1 hour. The completion of the reaction was confirmed by LC-MS, and the reaction product was cooled to room temperature and neutralized up to pH 6 with 2N-NaOH (aq). The suspension was diluted with H₂O and extracted with ethyl acetate. The organic layer was dried over Na₂SO₄ and concentrated, separated by MPLC, slurried with ethyl acetate and diethyl ether, and filtered to obtain the title compound (190 mg, 68%) as a dark pink solid.

¹H NMR (400 MHz, DMSO-*d*₆) δ 13.07 (s, 1H), 7.91 (s, 1H), 7.70 (s, 1H), 7.41 (t, *J* = 1.9 Hz, 1H), 7.27 (d, *J* = 8.0 Hz, 1H), 7.19 (t, *J* = 7.8 Hz, 1H), 6.73 - 6.70 (m, 1H), 5.37 (s, 2H).

### Method e. Preparation of amine derivative using Stille reaction (Formula II-a)

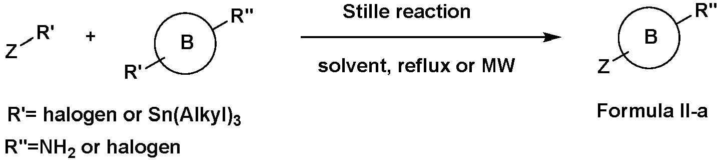

### Method e+b. Preparation of carboxylic acid derivative using Stille reaction and Buchwald reaction (Formula III-a)

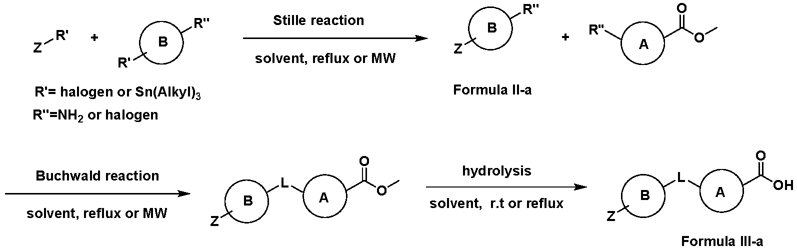

### Preparation Example 43: Synthesis of 3-((5-(pyrimidin-4-yl)pyridin-2-yl)amino)benzoic acid

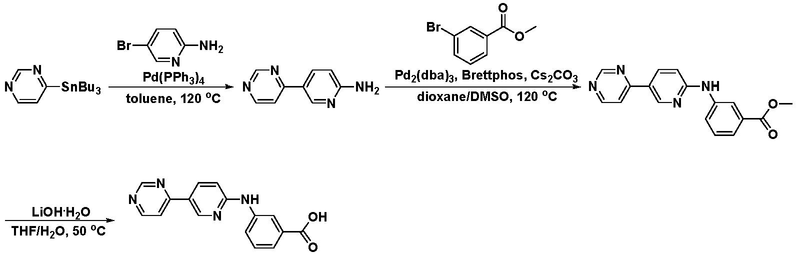

### Step 1: Synthesis of 5-(pyrimidin-4-yl)pyridin-2-amine

To a solution of 4-(tributylstannyl)pyrimidine (4g, 10.84 mmol) and 5-bromopyridin-2-amine (1.55 g, 7.06 mmol) in toluene (60 mL) was added Pd(PPh₃)₄ (1.25 g, 1.08 mmol), the reaction mixture was stirred at 120°**C** for 48 hours under N₂. The reaction mixture was cooled to room temperature and poured into water (100 mL), then extracted with EA (30 mL x3), the combined organic layer was washed with brine (100 mL x3), dried over Na₂SO₄ and concentrated under reduced pressure. The residue was purified by column chromatography to obtain the title compound (1.10 g, 58.9%) as a yellow solid.

MS: m/z = 173 (M+1, ESI+).

### Step 2: Synthesis of methyl 3-((5-(pyrimidin-4-yl)pyridin-2-yl)amino) benzoate

To a solution of 5-(pyrimidin-4-yl)pyridin-2-amin (1g, 5.81 mmol) and methyl 3-bromobenzoate (1.63 g, 7.56 mmol) in dioxane/DMSO (15 mL/ 3 mL) was added Cs₂CO₃ (3.79 g, 11.62 mmol), Brettphos (936 mg, 1.74 mmol) and Pd₂(dba)₃ (1.59 g, 1.74 mmol), the reaction mixture was stirred at 120°**C** for 16 hours under N₂. The reaction mixture was cooled to room temperature and poured into water (100 mL), then extracted with EA (30 mL x3), the combined organic layer was washed with brine (100 mL x3), dried over Na₂SO₄ and concentrated under reduced pressure. The residue was purified by column chromatography to obtain the title compound (800 mg, 44.9%) as a pale yellow solid.

MS: m/z = 307 (M+1, ESI+).

### Step 3: Synthesis of 3-((5-(pyrimidin-4-yl)pyridin-2-yl)amino)benzoic acid

To a solution of methyl 3-((5-(pyrimidin-4-yl)pyridin-2-yl)amino)benzoate (800 mg, 2.61 mmol) in THF/H₂O (10 mL/ 2 mL) was added LiOH·H₂O (547 mg, 13.04 mmol), the reaction mixture was stirred at 50°**C** for 16 hours. The reaction mixture was acidified to pH 2 with 2N-HCl (aq) (20 mL), then extracted with EA (10 mL x3), the combined organic layer was washed with brine (30 mL x3), dried over Na₂SO₄ and concentrated under reduced pressure to obtain the title compound (700 mg, 91.7%) as a pale yellow solid.

¹H NMR (400 MHz, DMSO-*d*₆) δ 9.73 (s, 1H), 9.25-9.13 (m, 1H), 9.07 (d, *J* = 2.3 Hz, 1H), 8.78 (d, *J* = 5.4 Hz, 1H), 8.48-8.28 (m, 3H), 8.11-7.96 (m, 2H), 7.54 (t, *J* = 10.1 Hz, 1H), 7.40 (t, *J* = 7.9 Hz, 1H), 6.99 (d, *J* = 8.8 Hz, 1H).

MS: m/z = 293 (M+1, ESI+).

### Preparation Example 44: Synthesis of 3-((6-(pyridin-2-yl)pyridazin-3-yl)amino)benzoic acid

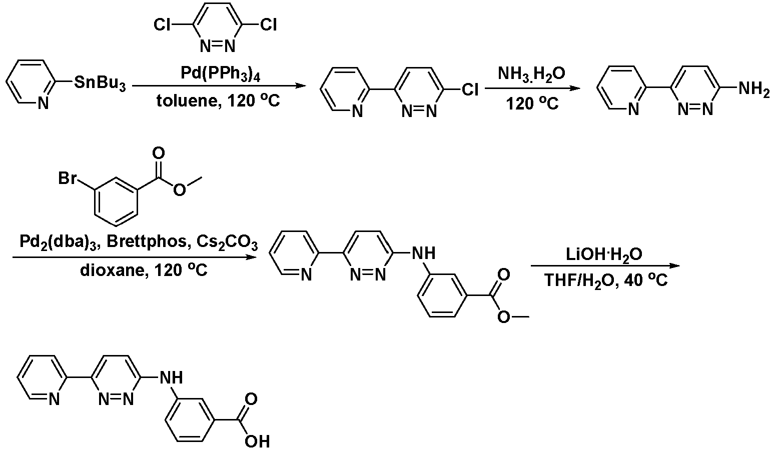

### Step 1: Synthesis of 3-chloro-6-(pyridin-2-yl)pyridazine

To a solution of 2-(tributylstannyl)pyridine (14 g, 38.03 mmol) and 3,6-dichloropyridazine (7.4 g, 49.44 mmol) in toluene (200 mL) was added Pd(PPh₃)₄ (2.2 g, 1.90 mmol), the reaction mixture was stirred at 120°**C** for 48 hours under N₂. The reaction mixture was cooled to room temperature and poured into water (500 mL), then extracted with EA (100 mL x3), the combined organic layer was washed with brine (500 mL x3), dried over Na₂SO₄ and concentrated under reduced pressure. The residue was purified by column chromatography to obtain the title compound (3.2 g, 43.9%) as a yellow solid.

¹H NMR (400 MHz, DMSO-*d*₆) δ 8.77 (t, *J* = 8.7 Hz, 1H), 8.55 (dd, *J* = 19.6, 8.5 Hz, 2H), 8.05 (dd, *J* = 11.8, 5.4 Hz, 2H), 7.66-7.54 (m, 1H).

MS: m/z = 192 (M+1, ESI+).

### Step 2: Synthesis of 6-(pyridin-2-yl)pyridazin-3-amine

A mixture of 3-chloro-6-(pyridin-2-yl)pyridazine (3.2 g, 16.7 mmol) in NH₃·H₂O (35 mL) was stirred at 120°**C** for 16 hours. The reaction mixture was cooled to room temperature and concentrated under reduced pressure to obtain the title compound (3 g, crude) as a white solid.

MS: m/z = 173 (M+1, ESI+)

### Step 3: Synthesis of methyl 3-((6-(pyridin-2-yl)pyridazin-3-yl)amino)benzoate

To a solution of 6-(pyridin-2-yl)pyridazin-3-amine (2 g, 11.62 mmol) and methyl 3-bromobenzoate (5 g, 23.23 mmol) in dioxane (50 mL) was added Cs₂CO₃ (7.6 g, 23.23 mmol), Brettphos (623 mg, 1.16 mmol) and Pd₂(dba)₃ (532 mg, 0.58 mmol), the reaction mixture was stirred at 120°**C** for 16 hours under N₂. The reaction mixture was cooled to room temperature and poured into water (500 mL), then extracted with EA (80 mL x3), the combined organic layer was washed with brine (500 mL x3), dried over Na₂SO₄ and concentrated under reduced pressure. The residue was purified by column chromatography to obtain the title compound (1.1 g, 30.38%) as a yellow solid.

MS: m/z = 307 (M+1, ESI+).

### Step 4: Synthesis of 3-((6-(pyridin-2-yl)pyridazin-3-yl)amino)benzoic acid

To a solution of methyl 3-((6-(pyridin-2-yl)pyridazin-3-yl)amino)benzoate (1.1 g, 3.59 mmol) in THF/H₂O (12 mL/ 3 mL) was added LiOH·H₂O (301 mg, 7.18 mmol), the reaction mixture was stirred at 40°**C** for 16 hours. The reaction mixture was acidified to pH 2 with 2N-HCl (aq) (100 mL), then extracted with EA (40 mL x3), the combined organic layer was washed with brine (100 mL x3), dried over Na₂SO₄ and concentrated under reduced pressure to obtain the title compound (900 mg, 85.71%) as a yellow solid.

¹H NMR (400 MHz, DMSO-*d*₆) δ 9.74 (s, 1H), 8.70 (d, *J* = 4.1 Hz, 1H), 8.59-8.43 (m, 2H), 8.36 (d, *J* = 9.3 Hz, 1H), 8.12-7.92 (m, 2H), 7.54 (dd, *J* = 39.6, 7.6 Hz, 3H), 7.30 (d, *J* = 9.3 Hz, 1H).

MS: m/z = 293 (M+1, ESI+).

### Method f. Preparation of benzimidazole derivative using cyclization reaction(acetic acid) (Formula IV-a)

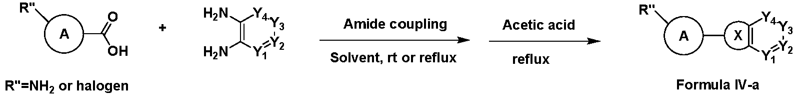

### Preparation Example 45: Synthesis of 2-(2-bromopyridin-4-yl)-6-(trifluoromethyl)-1H-benzo[d]imidazole

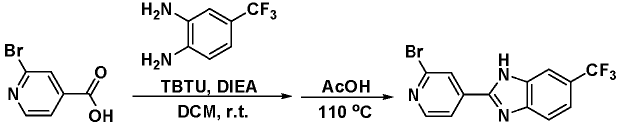

To a solution of 2-bromoisonicotinic acid (5.00 g, 24.75 mmol) and TBTU (11.92 g, 37.12 mmol) in DCM (300 mL) was added 4-(trifluoromethyl)benzene-1,2-diamine (5.67 g, 32.18 mmol) and DIEA (9.60 g, 74.25 mmol), the reaction mixture was stirred at room temperature for 16 hours. The reaction mixture was poured into water (200 mL), then extracted with DCM (50 mL x3), the combined organic layer was washed with brine (200 mL x3), dried over Na₂SO₄ and concentrated under reduced pressure to obtain *N*-(2-amino-5-(trifluoromethyl)phenyl)-2-bromoisonicotinamide (4.4 g, 49.4%) as a yellow solid. MS: m/z = 362 (M+1, ESI+). And a mixture of *N*-(2-amino-5-(trifluoromethyl)phenyl)-2-bromoisonicotinamide (4.4 g, 12.22 mmol) in AcOH (50 mL) was stirred at 110°**C** for 16 hours. The reaction mixture was cooled to room temperature and concentrated under reduced pressure, the residue was purified by column chromatography to obtain the title compound (3.0 g, 71.56%) as a white solid.

¹H NMR (400 MHz, DMSO-*d*₆) δ 13.78 (s, 1H), 8.62 (d, *J* = 5.1 Hz, 1H), 8.36 (s, 1H), 8.18 (dd, *J* = 5.1, 1.2 Hz, 1H), 8.06 (s, 1H), 7.87 (d, *J* = 8.3 Hz, 1H), 7.61 (d, *J* = 8.2 Hz, 1H).

MS: m/z = 343 (M+1, ESI+).

### Preparation Example 46: Synthesis of 2-(3-bromophenyl)-6-(trifluoromethyl)-1H-benzo[d]imidazole

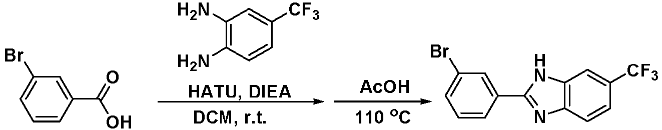

To a solution of 3-bromobenzoic acid (16.5 g, 82.09 mmol) and HATU (37.4 g, 98.51 mmol) in DCM (300 mL) was added 4-(trifluoromethyl)benzene-1,2-diamine (15.17 g, 86.20 mmol) and DIEA (31.77 g, 246.27 mmol), the reaction mixture was stirred at room temperature for 16 hours. The reaction mixture was poured into water (800 mL), then extracted with DCM (100 mL x3), the combined organic layer was washed with brine (800 mL x3), dried over Na₂SO₄ and concentrated under reduced pressure, the residue was purified by column chromatography to obtain *N*-(2-amino-4-(trifluoromethyl)phenyl)-3-bromobenzamide (21 g, 71.19%) as a yellow solid. MS: m/z = 360 (M+1, ESI+). And a mixture of N-(2-amino-4-(trifluoromethyl)phenyl)-3-bromobezamide (21 g, 58.50 mmol) in AcOH (200 mL) was stirred at 110°**C** for 16 hours. The reaction mixture was cooled to room temperature and concentrated under reduced pressure, the residue was purified by column chromatography to obtain the title compound (16 g, 80.21%) as a yellow solid.

¹H NMR (400 MHz, DMSO-*d*₆) δ 13.47 (s, 1H), 8.40 (s, 1H), 8.22 (d, *J* = 7.5 Hz, 1H), 8.05 (s, 1H), 7.88 (d, *J* = 13.1 Hz, 1H), 7.76 (d, *J* = 7.9 Hz, 1H), 7.56 (t, *J* = 7.4 Hz, 2H).

MS: m/z = 341 (M+1, ESI+).

### Method g. Preparation of bezimidazole derivative using Mitsunobu reaction and cyclization reaction(acetic acid) (Formula IV-b)

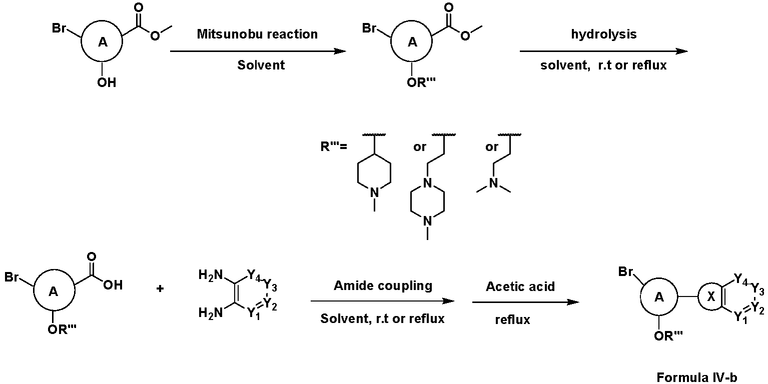

### Preparation Example 47: Synthesis of 2-(2-(1H-benzo[d]imidazol-2-yl)-4-bromophenoxy)-N,N-dimethylethan-1-amine

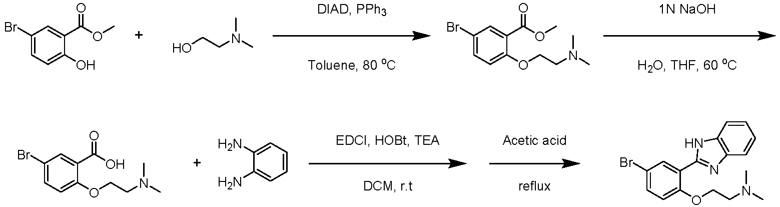

### Step 1: Synthesis of methyl 5-bromo-2-(2-(dimethylamine)ethoxy)benzoate

To a solution of methyl 5-bromo-2-hydroxybenzoate (300 mg, 1.30 mmol), 2-(dimethylamino)ethan-1-ol (0.13 mL, 1.30 mmol) in toluene (3 mL) was added PPh₃ (375 mg, 1.43 mmol) and DIAD (0.28 mL, 1.43 mmol), the reaction mixture was stirred 80°**C** for 7 hours. The reaction mixture was cooled to room temperature and poured into water, then extracted with DCM, the combined organic layer was dried over MgSO₄ and concentrated under reduced pressure. The residue was purified by MPLC to obtain the title compound (258 mg, 66%) as a yellow oil.

¹H NMR (400 MHz, DMSO-*d*₆) δ 7.74 (d, *J* = 2.6 Hz, 1H), 7.68 (dd, *J* = 2.7, 8.9 Hz, 1H), 7.15 (d, *J* = 8.9 Hz, 1H), 4.11 (t, *J* = 5.7 Hz, 2H), 3.78 (s, 3H), 2.62 (t, *J* = 5.7 Hz, 2H), 2.21 (s, 6H).

### Step 2: Synthesis of 5-bromo-2-(2-(dimethylamino)ethoxy)benzoic acid

To a solution of methyl 5-bromo-2-(2-(dimethylamino)ethoxy)benzoate (200 mg, 0.662 mmol) in H₂O/THF (1 mL/ 4 mL) was added 1N-NaOH (2 mL), the reaction mixture was stirred at 60°**C** for 5 hours. The reaction mixture was acidified to pH 3 with 1N-HCl (aq), then extracted with DCM, the combined organic layer was dried over MgSO₄ and concentrated under reduced pressure to obtain the title compound (245 mg, > 100%) as a white solid.

¹H NMR (400 MHz, DMSO-*d*₆) δ 7.80 (d, *J* = 2.6 Hz, 1H), 7.72 (dd, *J* = 2.7, 8.9 Hz, 1H), 7.19 (d, *J* = 8.9 Hz, 1H), 4.46-4.42 (m, 2H), 3.52-3.48 (m, 2H), 2.87 (s, 6H).

### Step 3: Synthesis of 2-(2-(1H-benzo[d]imidazol-2-yl)-4-bromophenoxy)-N,N-dimethylethan-1-amine

To a solution of 5-bromo-2-(2-(dimethylamino)ethoxy)benzoic acid (130 mg, 0.451 mmol), benzene-1,2-diamine (54 mg, 0.496 mmol) in DCM (5 mL) was added EDCI (95 mg, 0.496 mmol), HOBt (122 mg, 0.902 mmol) and TEA (0.189 mL, 1.354 mmol), the reaction mixture was stirred at room temperature for 96 hours. The reaction mixture was concentrated under reduced pressure, AcOH (5 mL) was added, and the residue was stirred at 100°**C** for 8 hours. The reaction mixture was washed with NaHCO₃ (aq), then extracted with DCM, the combined organic layer was dried over MgSO₄ and concentrated under reduced pressure. The residue was purified by MPLC to obtain the title compound (69 mg, 42%) as a yellow solid.

¹H NMR (400 MHz, DMSO-*d*₆) δ 13.26 (s, 1H), 8.38 (d, *J* = 2.6 Hz, 1H), 7.69-7.52 (m, 3H), 7.32 (d, *J* = 8.8 Hz, 1H), 7.27-7.19 (m, 2H), 4.40 (t, *J* = 5.3 Hz, 2H), 2.83 (t, *J* = 5.3 Hz, 2H), 2.43 (s, 6H).

### Preparation Example 48: Synthesis of 2-(5-bromo-2-(2-(4-methylpiperazin-1-yl)ethoxy)phenyl)-1H-benzo[d]imidazole

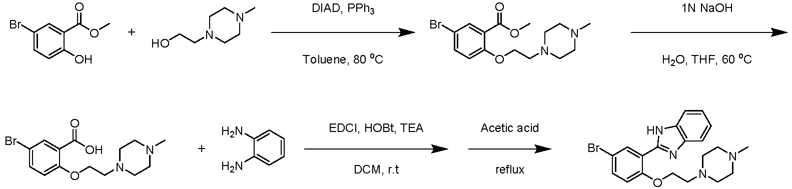

### Step 1: Synthesis of methyl 5-bromo-2-(2-(4-methylpiperazin-1-yl)ethoxy)benzoate

To a solution of methyl 5-bromo-2-hydroxybenzoate (300 mg, 1.30 mmol), 2-(4-methylpiperazin-1-yl)ethan-1-ol (0.187 mL, 1.30 mmol) in toluene (3 mL) was added PPh₃ (375 mg, 1.43 mmol) and DIAD (0.28 mL, 1.43 mmol), the reaction mixture was stirred at 80°**C** for 7 hours. The reaction mixture was cooled to room temperature and poured into water, then extracted with DCM. The combined organic layer was washed with brine, dried over MgSO₄ and concentrated under reduced pressure. The residue was purified by MPLC to obtain the title compound (341 mg, 73%) as a yellow oil.

¹H NMR (400 MHz, DMSO-*d*₆) δ 7.74 (d, *J* = 2.6 Hz, 1H), 7.68 (dd, *J* = 2.6, 8.9 Hz, 1H), 7.16 (d, *J* = 9.0 Hz, 1H), 4.13 (t, *J* = 5.6 Hz, 2H), 2.68 (t, *J* = 5.6 Hz, 2H), 2.47-2.21 (m, 8H), 2.14 (s, 3H).

### Step 2: Synthesis of 5-bromo-2-(2-(4-methylpiperazin-1-yl)ethoxy)benzoic acid

To a solution of methyl 5-bromo-2-(2-(4-methylpiperazin-1-yl)ethoxy)benzoate (290 mg, 0.812 mmol) in H₂O/THF (2 mL/ 8 mL) was added 1N-NaOH (2.4 mL), the reaction mixture was stirred at 60°**C** for 5 hours. The reaction mixture was acidified to pH 3 with 1N-HCl (aq), then extracted with DCM. The combined organic layer was dried over MgSO₄ and concentrated under reduced pressure to obtain the title compound (223 mg, 80%) as a white solid.

¹H NMR (400 MHz, DMSO-*d*₆) δ 11.11 (s, 1H), 7.79 (d, *J* = 2.6 Hz, 1H), 7.71 (dd, *J* = 2.7, 8.9 Hz, 1H), 7.17 (d, *J* = 8.9 Hz, 1H), 4.38 (s, 2H), 3.33-3.05 (m, 10H), 2.79 (s, 3H).

### Step 3: Synthesis of 2-(5-bromo-2-(2-(4-methylpiperazin-1-yl)ethoxy)phenyl)-1H-benzo[d]imidazole

To a solution of 5-bromo-2-(2-(4-methylpiperazin-1-yl)ethoxy)benzoic acid (130 mg, 0.379 mmol), benzene-1,2-diamine (45 mg, 0.417 mmol) in DCM was added EDCI (80 mg, 0.417 mmol), HOBt (102 mg, 0.758 mmol) and TEA (0.158 mL, 1.136 mmol), the reaction mixture was stirred at room temperature for 96 hours. The reaction mixture was concentrated under reduced pressure, AcOH (5 mL) was added, and the residue was stirred at 100°**C** for 8 hours. The reaction mixture was washed with NaHCO₃ (aq), then extracted with DCM, the combined organic layer was dried over MgSO₄ and concentrated under reduced pressure. The residue was purified by MPLC to obtain the title compound (67 mg, 43%) as a yellow solid.

¹H NMR (400 MHz, DMSO-*d*₆) δ 12.11 (s, 1H), 8.39 (d, *J* = 2.6 Hz, 1H), 7.71-7.61 (m, 3H), 7.32-7.22 (m, 3H), 4.40 (t, *J* = 5.5 Hz, 2H), 2.85 (t, *J* = 5.5 Hz, 2H), 2.66-2.54 (m, 4H), 2.45-2.35 (m, 4H), 2.15 (s, 3H).

### Preparation Example 49: Synthesis of 2-(5-bromo-2-((1-methylpiperidin-4-yl)oxy)phenyl)-1H-benzo[d]imidazole

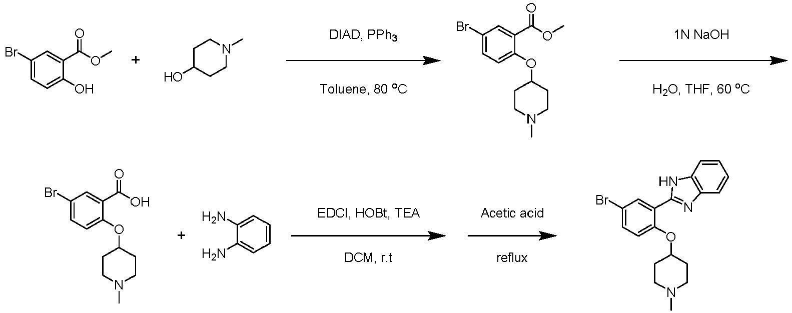

### Step 1: Synthesis of methyl 5-bromo-2-((1-methylpiperidin-4-yl)oxy)benzoate

To a solution of methyl 5-bromo-2-hydroxybenzoate (300 mg, 1.30 mmol), 1-methylpiperidin-4-ol (0.153 mL, 1.30 mmol) in toluene (3 mL) was added PPh₃ (375 mg, 1.43 mmol) and DIAD (0.28 mL, 1.43 mmol), the reaction mixture was stirred at 80°**C** for 4 hours. The reaction mixture was cooled to room temperature and poured into water, then extracted with DCM. The combined organic layer was washed with brine, dried over MgSO₄ and concentrated under reduced pressure. The residue was purified by MPLC to obtain the title compound (224 mg, 52%) as a yellow oil.

¹H NMR (400 MHz, CDCl₃) δ 7.89 (d, *J* = 2.6 Hz, 1H), 7.51 (dd, *J* = 2.6, 8.9 Hz, 1H), 6.87 (d, *J* = 8.9 Hz, 1H), 4.42 (s, 1H), 3.89 (s, 3H), 2.70 - 2.59 (m, 2H), 2.43 - 2.34 (m, 2H), 2.32 (s, 3H), 2.03 - 1.86 (m, 4H).

### Step 2: Synthesis of 5-bromo-2-((1-methylpiperidin-4-yl)oxy)benzoic acid

To a solution of methyl 5-bromo-2-((1-methylpiperidin-4-yl)oxy)benzoate (223 mg, 0.680 mmol) in H₂O/THF (1.5 mL/ 6 mL) was added 1N-NaOH (2.0 mL), the reaction mixture was stirred at 60°**C** for 5 hours. The reaction mixture was acidified to pH 3 with 1N-HCl (aq), then extracted with DCM. The combined organic layer was dried over MgSO₄ and concentrated under reduced pressure to obtain the title compound (121 mg, 56%) as a brown oil.

¹H NMR (400 MHz, DMSO-*d*₆) δ 7.79 (d, *J* = 2.6 Hz, 1H), 7.68 (dd, *J* = 2.7, 8.9 Hz, 1H), 7.24 (d, *J* = 9.0 Hz, 1H), 4.95-4.70 (m, 1H), 4.18-3.98 (m, 2H), 3.27-3.21 (m, 2H), 2.72 (s, 3H), 2.19-1.95 (m, 4H).

### Step 3: Synthesis of 2-(5-bromo-2-((1-methylpiperidin-4-yl)oxy)phenyl)-1H-benzo[d]imidazole

To a solurion of 5-bromo-2-((1-methylpiperidin-4-yl)oxy)benzoic acid (65 mg, 0.207 mmol), benzene-1,2-diamine (25 mg, 0.228 mmol) in DCM (2 mL) was added EDCI (44 mg, 0.228 mmol), HOBt (56 mg, 0.414 mmol) and TEA (0.087 mL, 0.621 mmol), the reaction mixture was stirred at room temperature for 93 hours. The reaction mixture was concentrated under reduced pressure, AcOH (5 mL) was added, and the residue was stirred at 100°**C** for 8 hours. The reaction mixture was washed with NaHCO₃ (aq), then extracted with DCM. The combined organic layer was dried over MgSO₄ and concentrated under reduced pressure. The residue was purified by MPLC to obtain the title compound (23 mg, 28%) as a yellow solid.

¹H NMR (400 MHz, DMSO-*d*₆) δ 11.86 (s, 1H), 8.26 (d, *J* = 2.6 Hz, 1H), 7.69-7.57 (m, 3H), 7.31 (d, *J* = 9.0 Hz, 1H), 7.26-7.18 (m, 2H), 4.66-4.48 (m, 1H), 2.79-2.57 (m, 3H), 2.24-2.11 (m, 4H), 2.03-1.85 (m, 4H).

### Method h. Synthesis of carboxylic acid derivative using amide coupling reaction

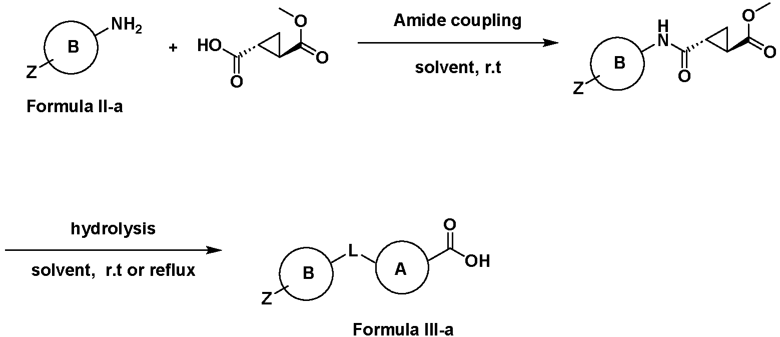

### Preparation Example 50: Synthesis of (1R,2R)-2-((4-(pyrazin-2-yl)phenyl)carbamoyl)cyclopropane-1-carboxylic acid

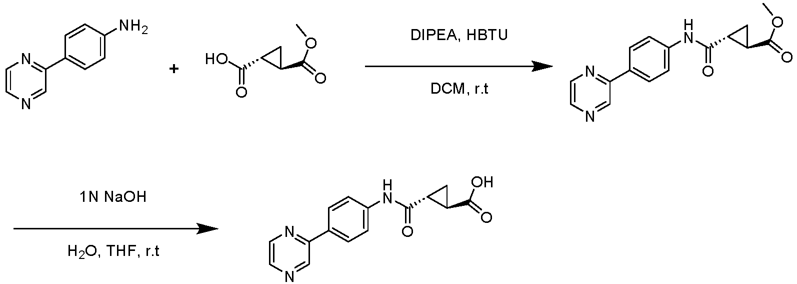

### Step 1: Synthesis of methyl (1R,2R)-2-((4-(pyrazin-2-yl)phenyl) carbamoyl)cyclopropane-1-carboxylate

To a solution of (1R,2R)-2-(methoxycarbonyl)cyclopropane-1-carboxylic acid (0.060 mL, 0.449 mmol), 4-(pyrazin-2-yl)aniline (64 mg, 0.374 mmol) in DCM (4 mL) was added HBTU (213 mg, 0.561 mmol) and DIPEA (0.326 mL, 1.869 mmol), the reaction mixture was stirred at room temperature for 24 hours. The reaction mixture was washed with NaHCO₃ (aq), then extracted with DCM, the combined organic layer was dried over MgSO₄ and concentrated under reduced pressure. The residue was solidified using EA and hexane to obtain the title compound (100 mg, 90%) as a beige solid.

¹H NMR (400 MHz, DMSO-*d*₆) δ 10.64 (s, 1H), 9.22 (d, *J* = 1.6 Hz, 1H), 8.70-8.66 (m, 1H), 8.56 (d, *J* = 2.5 Hz, 1H), 8.14-8.09 (m, 2H), 7.77-7.72 (m, 2H), 3.66 (s, 3H), 2.42-2.35 (m, 1H), 2.07-2.01 (m, 1H), 1.44-1.37 (m, 1H), 1.37-1.31 (m, 1H).

### Step 2: Synthesis of (1R,2R)-2-((4-(pyrazin-2-yl)phenyl)carbamoyl)cyclopropane-1-carboxylic acid

To a solution of (1*R*,2*R*)-2-((4-(pyrazin-2-yl)phenyl)carbamoyl)cyclopropane-1-carboxylate (89 mg, 0.299 mmol) in H₂O/THF (1 mL/ 3 mL) was added 1N-NaOH (0.9 mL), the reaction mixture was stirred at room temperature for 5 hours. The reaction mixture was acidified to pH 3 with 1N-HCl (aq), then extracted with DCM. The combined organic layer was dried over MgSO₄ and concentrated under pressure to obtain the title compound (61 mg, 71%) as a beige solid.

¹H NMR (400 MHz, DMSO-*d*₆) δ 12.63 (s, 1H), 10.64 (s, 1H), 9.23 (d, *J* = 1.5 Hz, 1H), 8.72-8.66 (m, 1H), 8.56 (d, *J* = 2.5 Hz, 1H), 8.17-8.09 (m, 2H), 7.78-7.71 (m, 2H), 2.40-2.29 (m, 1H), 1.99-1.89 (m, 1H), 1.41-1.27 (m, 2H).

### [Example] Preparation of compound represented by Chemical Formula 1 according to the present invention

### Method A. Preparation through amide reaction and cyclization reaction

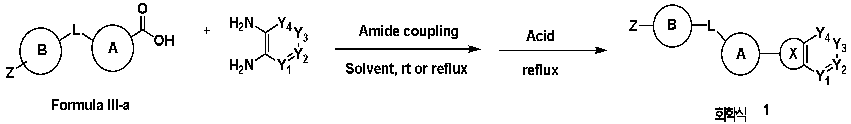

### Example 1: Synthesis of 6-phenyl-N-(3-(6-(trifluoromethyl)-1H-benzo[d]imidazol-2-yl)phenyl)pyridazin-3-amine

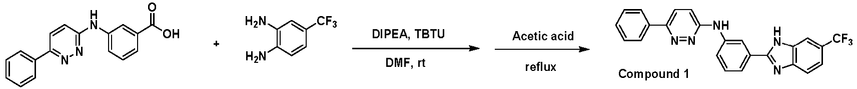

3-((6-Phenylpyridazin-3-yl)amino)benzoic acid (1.75 g, 6.0 mmol), 4-(trifluoromethyl)benzene-1,2-diamine (1.11 g, 6.3 mmol), and TBTU (2.89 g, 9.0 mmol) were dissolved in DMF (20 mL), and DIPEA (2.09 mL, 12.0 mmol) was then added thereto, followed by stirring overnight at room temperature. After confirming the completion of the reaction by LC-MS, the resulting mixture was concentrated to remove DMF, and acetic acid (20 mL) was added thereto, followed by stirring at reflux at 120°**C** for 3 hours. After confirming the completion of the reaction through LC-MS, the resulting mixture was cooled to room temperature. The mixture was washed with saturated NaHCO₃ solution and extracted with ethyl acetate, and then the organic layer was dried over MgSO₄ and concentrated. The concentrated solution was separated by MPLC, slurried with dichloromethane, and filtered to obtain the title compound (154 mg, 42%) as a white solid.

### Example 2: Synthesis of N-(3-(1H-benzo[d]imidazol-2-yl)phenyl)-6-phenylpyridazin-3-amine

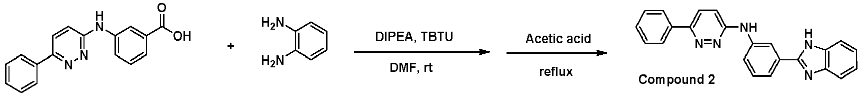

3-((6-Phenylpyridazin-3-yl)amino)benzoic acid (524 mg, 1.8 mmol), benzene-1,2-diamine (162 mg, 1.5 mmol) and TBTU (963 mg, 3.0 mmol) were dissolved in DMF (20 mL), then DIPEA (0.784 mL, 4.5 mmol) was added, and the mixture was stirred overnight at room temperature. After confirming the completion of the reaction by LC-MS, the resulting mixture was washed with 5% LiCl aqueous solution and extracted with ethyl acetate. The organic layer was concentrated to remove DMF, then acetic acid (20 mL) was added, and the mixture was stirred at reflux at 120°**C** for 5 hours. After confirming the completion of the reaction through LC-MS, the resulting mixture was cooled to room temperature. The mixture was washed with saturated NaHCO₃ solution and extracted with ethyl acetate, and then the organic layer was dried over MgSO₄ and concentrated. The concentrated solution was separated by MPLC, slurried with dichloromethane, and filtered to obtain the title compound (154 mg, 42%) as a white solid.

### Example 5: Synthesis of 5,6-dichloro-2-(3-((4-phenylpiperazin-1-yl)methyl)phenyl)-1H-benzo[d]imidazole

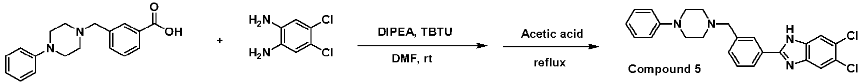

3-((4-Phenylpiperazin-1-yl)methyl)benzoic acid (221 mg, 0.747 mmol), 4,5-dichlorobenzene-1,2-diamine (217 mg, 0.821 mmol) and TBTU (360 mg, 1.12 mmol) were dissolved in DMF (5 mL), then DIPEA (0.260 mL, 1.49 mmol) was added, and the mixture was stirred at room temperature for 2.5 hours. After confirming the completion of the reaction by LC-MS, the resulting mixture was washed with 5% LiCl aqueous solution and extracted with ethyl acetate. The organic layer was concentrated to remove DMF, then acetic acid (5 mL) was added, and the mixture was stirred at reflux at 120°**C** for 1.5 hours. After confirming the completion of the reaction through LC-MS, the resulting mixture was cooled to room temperature. The mixture was washed with saturated NaHCO₃ solution and extracted with ethyl acetate, and then the organic layer was dried over Na₂SO₄ and concentrated. The concentrated solution was purified by MPLC to obtain the title compound (190 mg, 43%) as a pale yellow solid.

### Example 6: Synthesis of 6-phenyl-N-(3-(6-(trifluoromethoxy)-1H-benzo[d]imidazol-2-yl)phenyl)pyridazin-3-amine

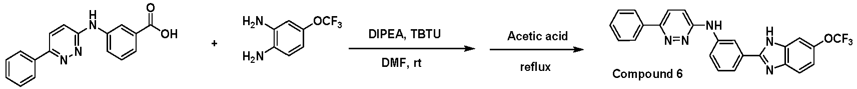

3-((6-Phenylpyridazin-3-yl)amino)benzoic acid (73 mg, 0.25 mmol), 4-(trifluoromethoxy)benzene-1,2-diamine (48 mg, 0.25 mmol) and TBTU (160 mg, 0.5 mmol) were dissolved in DMF (5 mL), and DIPEA (0.130 mL, 0.75 mmol) was then added thereto, followed by stirring overnight at room temperature. After confirming the completion of the reaction by LC-MS, the resulting mixture was washed with 5% LiCl aqueous solution and extracted with ethyl acetate. The organic layer was concentrated to remove DMF, then acetic acid (5 mL) was added, and the mixture was stirred at reflux at 120°**C** for 2 hours. After confirming the completion of the reaction through LC-MS, the resulting mixture was cooled to room temperature. The mixture was washed with saturated NaHCO₃ solution and extracted with ethyl acetate, and then the organic layer was dried over Na₂SO₄ and concentrated. The concentrated solution was separated by MPLC, slurried with dichloromethane, and filtered to obtain the title compound (16 mg, 14%) as a white solid.

### Example 7: Synthesis of N-(3-(5,6-dichloro-1H-benzo[d]imidazol-2-yl)phenyl)-6-phenylpyridazin-3-amine

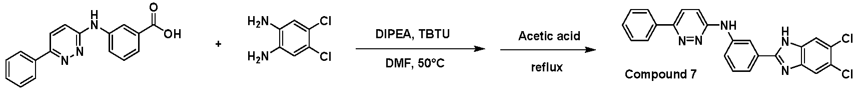

3-((6-Phenylpyridazin-3-yl)amino)benzoic acid (96 mg, 0.33 mmol), 4,5-dichlorobenzene-1,2-diamine (53 mg, 0.3 mmol) and TBTU (193 mg, 0.6 mmol) were dissolved in DMF (5 mL), then DIPEA (0.157 mL, 0.9 mmol) was added, and the mixture was stirred overnight at 50°C. After confirming the completion of the reaction by LC-MS, the resulting mixture was washed with 5% LiCl aqueous solution and extracted with ethyl acetate. The organic layer was concentrated to remove DMF, then acetic acid (5 mL) was added, and the mixture was stirred at reflux overnight at 120°**C**. After confirming the completion of the reaction through LC-MS, the resulting mixture was cooled to room temperature. The mixture was washed with saturated NaHCO₃ solution and extracted with ethyl acetate, and then the organic layer was dried over Na₂SO₄ and concentrated. The concentrated solution was purified by MPLC to obtain the title compound (45 mg, 35%) as a white solid.

### Example 11: Synthesis of N-(3-(7-bromo-5-fluoro-1H-benzo[d]imidazol-2-yl)phenyl)-6-phenylpyridazin-3-amine

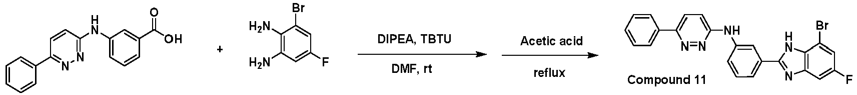

3-((6-Phenylpyridazin-3-yl)amino)benzoic acid (250 mg, 0.859 mmol), 3-bromo-5-fluorobenzene-1,2-diamine (176 mg, 0.859 mmol) and TBTU (551 mg, 1.72 mmol) were dissolved in DMF (5 mL), and DIPEA (0.450 mL, 2.58 mmol) was then added and stirred overnight at room temperature. After confirming the completion of the reaction by TLC, the resulting mixture was diluted with 5% LiCl aqueous solution and extracted with ethyl acetate. The organic layer was concentrated to remove DMF, then acetic acid (5 mL) was added, and the mixture was stirred at reflux at 120°C for 2 hours. After confirming the completion of the reaction through LC-MS, the resulting mixture was cooled to room temperature. The mixture was washed with saturated NaHCO₃ solution and extracted with ethyl acetate, and then the organic layer was dried over MgSO₄ and concentrated. The concentrated solution was separated by MPLC, filtered with dichloromethane, and dried to obtain the title compound (45 mg, 11%) as a white solid.

### Example 14: Synthesis of 5-phenyl-N-(3-(6-(trifluoromethyl)-1H-benzo[d]imidazol-2-yl)phenyl)pyrimidin-2-amine

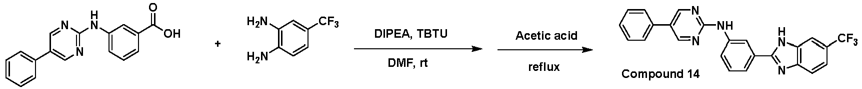

3-((5-Phenylpyrimidin-2-yl)amino)benzoic acid (873 mg, 3.0 mmol), 4-(trifluoromethyl)benzene-1,2-diamine (528 mg, 3.0 mmol) and TBTU (1.93 g, 6.0 mmol) were dissolved in DMF (30 mL), and DIPEA (1.569 mL, 9.0 mmol) was then added thereto, followed by stirring overnight at room temperature. The reaction solution was washed with 5% LiCl aqueous solution and extracted with ethyl acetate. The organic layer was concentrated to remove DMF, then acetic acid (5 mL) was added, and the mixture was stirred at reflux at 120°C for 4 hours. After confirming the completion of the reaction by TLC, the resulting mixture was cooled to room temperature. The mixture was washed with saturated NaHCO₃ solution and extracted with ethyl acetate, and then the organic layer was dried over Na₂SO₄ and concentrated. The concentrated solution was separated by MPLC, filtered with dichloromethane, and dried to obtain the title compound (475 mg, 37%) as a white solid.

### Example 18: Synthesis of 5-(3-fluorophenyl)-N-(3-(6-(trifluoromethyl)-1H-benzo[d]imidazol-2-yl)phenyl)pyridin-2-amine

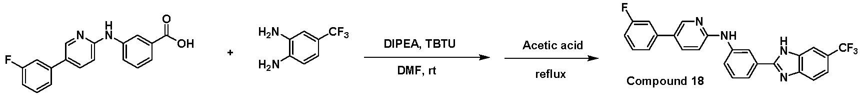

3-((5-(3-Fluorophenyl)pyridin-2-yl)amino)benzoic acid (616 mg, 2.0 mmol), 4-(trifluoromethyl)benzene-1,2-diamine (352 mg, 2.0 mmol) and TBTU (1.28 g, 4.0 mmol) were dissolved in DMF (20 mL), and DIPEA (1.045 mL, 6.0 mmol) was then added thereto, followed by stirring overnight at room temperature. The reaction solution was washed with 5% LiCl aqueous solution and extracted with ethyl acetate. The organic layer was concentrated to remove DMF, then acetic acid (20 mL) was added, and the mixture was stirred at reflux at 120°**C** for 2 hours. After confirming the completion of the reaction by TLC, the resulting mixture was cooled to room temperature. The mixture was washed with saturated NaHCO₃ solution and extracted with ethyl acetate, and then the organic layer was dried over Na₂SO₄ and concentrated. The concentrated solution was separated by MPLC, filtered with dichloromethane, and dried to obtain the title compound (69 mg, 8%) as a white solid.

### Example 20: Synthesis of 3-(1H-benzo[d]imidazol-2-yl)-N-(4-(pyridin-2-yl)phenyl)aniline

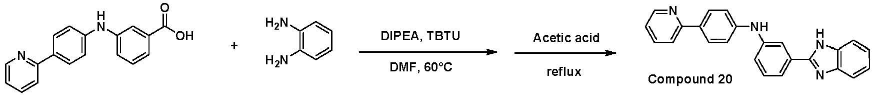

3-((4-(Pyridin-2-yl)phenyl)amino)benzoic acid (100 mg, 0.34 mmol), benzene-1,2-diamine (37 mg, 0.34 mmol) and TBTU (133 mg, 0.41 mmol) were dissolved in DMF (10 mL), then DIPEA (0.180 mL, 1.03 mmol) was added, and the mixture was stirred overnight at 60°**C**. The reaction solution was washed with 5% LiCl aqueous solution and extracted with ethyl acetate, and the organic layer was concentrated to remove DMF, and acetic acid (10 mL) was added thereto, followed by stirring at reflux at 120°**C** for 1 hour. After confirming the completion of the reaction by TLC, the resulting mixture was cooled to room temperature. The mixture was washed with saturated NaHCO₃ solution and extracted with ethyl acetate, and then the organic layer was dried over Na₂SO₄ and concentrated. The concentrated solution was separated by MPLC, slurried with dichloromethane and ethyl acetate, and filtered to obtain the title compound (51 mg, 41%) as a white solid.

### Example 21: Synthesis of 3-(1H-benzo[d]imidazol-2-yl)-N-(4-(pyridazin-3-yl)phenyl)aniline

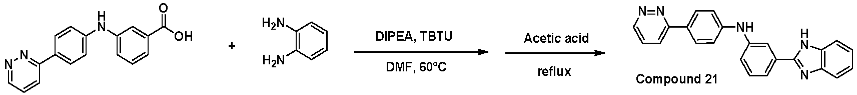

3-((4-(Pyridazin-3-yl)phenyl)amino)benzoic acid (437 mg, 1.5 mmol), benzene-1,2-diamine (162 mg, 1.5 mmol) and TBTU (722 mg, 1.5 mmol) were dissolved in DMF (10 mL), then DIPEA (0.523 mL, 3.0 mmol) was added, and the mixture was stirred overnight at 60°**C**. The reaction solution was washed with 5% LiCl aqueous solution and extracted with ethyl acetate, and the organic layer was concentrated to remove DMF, and acetic acid (10 mL) was added thereto, followed by stirring at reflux at 120°**C** for 1 hour. After confirming the completion of the reaction by TLC, the resulting mixture was cooled to room temperature. The mixture was washed with saturated NaHCO₃ solution and extracted with ethyl acetate, and then the organic layer was dried over Na₂SO₄ and concentrated. The concentrated solution was separated by MPLC, slurried with dichloromethane, and filtered to obtain the title compound (103 mg, 19%) as a pale yellow solid.

### Example 23: Synthesis of N-(4-(pyridazin-3-yl)phenyl)-3-(6-(trifluoromethyl)-1H-benzo[d]imidazol-2-yl)aniline

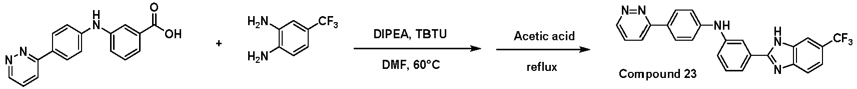

3-((4-(Pyridazin-3-yl)phenyl)amino)benzoic acid (232 mg, 0.8 mmol), 4-(trifluoromethyl)benzene-1,2-diamine (141 mg, 0.8 mmol) and TBTU (283 mg, 0.88 mmol) were dissolved in DMF (10 mL), then DIPEA (0.279 mL, 1.6 mmol) was added, and the mixture was stirred overnight at 60°**C**. The reaction solution was washed with 5% LiCl aqueous solution and extracted with ethyl acetate, and the organic layer was concentrated to remove DMF, and acetic acid (10 mL) was added thereto, followed by stirring at reflux at 120°**C** for 1 hour. After confirming the completion of the reaction by TLC, the resulting mixture was cooled to room temperature. The mixture was washed with saturated NaHCO₃ solution and extracted with ethyl acetate, and then the organic layer was dried over MgSO₄ and concentrated. The concentrated solution was separated by MPLC, slurried with dichloromethane/ethyl acetate, and filtered to obtain the title compound (190 mg, 55%) as a beige solid.

### Example 24: Synthesis of N-(4-(pyrimidin-4-yl)phenyl)-3-(6-(trifluoromethyl)-1H-benzo[d]imidazol-2-yl)aniline

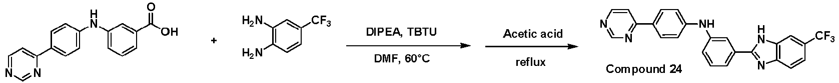

3-((4-(Pyrimidin-4-yl)phenyl)amino)benzoic acid (437 mg, 1.5 mmol), 4-(trifluoromethyl)benzene-1,2-diamine (264 mg, 1.5 mmol) and TBTU (578 mg, 1.8 mmol) were dissolved in DMF (10 mL), then DIPEA (0.523 mL, 3.0 mmol) was added, and the mixture was stirred overnight at 60°**C**. The reaction solution was washed with 5% LiCl aqueous solution and extracted with ethyl acetate, and the organic layer was concentrated to remove DMF, and acetic acid (10 mL) was added thereto, followed by stirring at reflux at 120°**C** for 1 hour. After confirming the completion of the reaction by TLC, the resulting mixture was cooled to room temperature. The mixture was washed with saturated NaHCO₃ solution and extracted with ethyl acetate, and then the organic layer was dried over MgSO₄ and concentrated. The concentrated solution was separated by MPLC, slurried with dichloromethane, and filtered to obtain the title compound (167 mg, 26%) as a beige solid.

### Example 25: Synthesis of 3-(6-fluoro-1H-benzo[d]imidazol-2-yl)-N-(4-(pyridazin-3-yl)phenyl)aniline

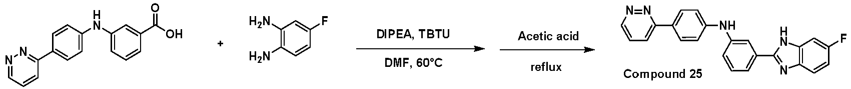

3-((4-(Pyridazin-3-yl)phenyl)amino)benzoic acid (437 mg, 1.5 mmol), 4-fluorobenzene-1,2-diamine (246 mg, 1.5 mmol) and TBTU (530 mg, 1.65 mmol) were dissolved in DMF (10 mL), then DIPEA (0.523 mL, 3.0 mmol) was added, and the mixture was stirred overnight at 60°**C**. The reaction solution was washed with 5% LiCl aqueous solution and extracted with ethyl acetate, and the organic layer was concentrated to remove DMF, and acetic acid (10 mL) was added thereto, followed by stirring at reflux at 120°**C** for 1 hour. After confirming the completion of the reaction by TLC, the resulting mixture was cooled to room temperature. The mixture was washed with saturated NaHCO₃ solution and extracted with ethyl acetate, and then the organic layer was dried over MgSO₄ and concentrated. The concentrated solution was separated by MPLC, slurried with dichloromethane and ethyl acetate, and filtered to obtain the title compound (250 mg, 44%) as a beige solid.

### Example 30: Synthesis of 3-(1H-benzo[d]imidazol-2-yl)-N-(4-(pyrimidin-5-yl)phenyl)aniline

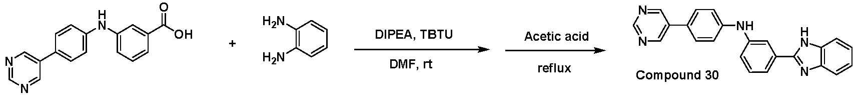

3-((4-(Pyrimidin-5-yl)phenyl)amino)benzoic acid (146 mg, 0.5 mmol), benzene-1,2-diamine (54 mg, 0.5 mmol) and TBTU (177 mg, 0.55 mmol) were dissolved in DMF (5 mL), then DIPEA (0.174 mL, 1.0 mmol) was added, and the mixture was stirred overnight at room temperature. The reaction solution was washed with 5% LiCl aqueous solution and extracted with ethyl acetate, and the organic layer was concentrated to remove DMF, and acetic acid (5 mL) was added thereto, followed by stirring at reflux at 120°**C** for 2 hours. After confirming the completion of the reaction through LC-MS, the resulting mixture was cooled to room temperature. The mixture was washed with saturated NaHCO₃ solution and extracted with ethyl acetate, and then the organic layer was dried over MgSO₄ and concentrated. The concentrated solution was separated by MPLC, slurried with dichloromethane/ethyl acetate, and filtered to obtain the title compound (35 mg, 19%) as a beige solid.

### Example 32: Synthesis of 3-(1H-benzo[d]imidazol-2-yl)-N-(4-(pyrazin-2-yl)phenyl)aniline

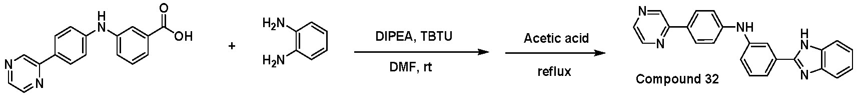

3-((4-(Pyrazin-2-yl)phenyl)amino)benzoic acid (146 mg, 0.5 mmol), benzene-1,2-diamine (54 mg, 0.5 mmol) and TBTU (177 mg, 0.55 mmol) were dissolved in DMF (5 mL), then DIPEA (0.174 mL, 1.0 mmol) was added, and the mixture was stirred overnight at room temperature. The reaction solution was washed with 5% LiCl aqueous solution and extracted with ethyl acetate, and the organic layer was concentrated to remove DMF, and acetic acid (5 mL) was added thereto, followed by stirring at reflux at 120°**C** for 2 hours. After confirming the completion of the reaction through LC-MS, the resulting mixture was cooled to room temperature. The mixture was washed with saturated NaHCO₃ solution and extracted with ethyl acetate, and then the organic layer was dried over MgSO₄ and concentrated. The concentrated solution was separated by MPLC, slurried with dichloromethane/ethyl acetate, and filtered to obtain the title compound (19 mg, 10%) as a beige solid.

### Example 34: Synthesis of N-((1s,4s)-4-(1H-benzo[d]imidazol-2-yl)bicyclo[2.2.1]heptan-1-yl)-6-phenylpyridazin-3-amine

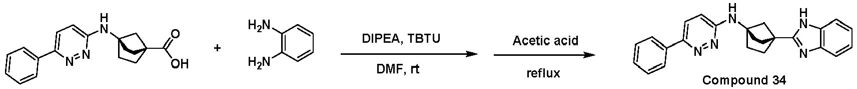

In DMF (5 mL), (1s,4s)-4-((6-phenylpyridazin-3-yl)amino)bicyclo[2.2.1]heptane-1-carboxylic acid (102 mg, 0.33 mmol), benzene-1,2-diamine (36 mg, 0.33 mmol) and TBTU (116 mg, 0.36 mmol) were dissolved, and DIPEA (0.115 mL, 0.66 mmol) was then added thereto, followed by stirring overnight at room temperature. After confirming the completion of the reaction by LC-MS, the reaction solution was concentrated, and acetic acid (5 mL) was added thereto, followed by stirring at reflux at 120°**C** for 4 hours. After confirming the completion of the reaction by TLC, the resulting mixture was cooled to room temperature. The mixture was washed with saturated NaHCO₃ solution and extracted with ethyl acetate, and then the organic layer was dried over MgSO₄ and concentrated. The concentrated solution was separated by MPLC, slurried with dichloromethane/diethylether/ethylacetate, and filtered to obtain the title compound (41 mg, 33%) as a beige solid.

### Example 35: Synthesis of N-(3-(1H-benzo[d]imidazol-2-yl)adamantan-1-yl)-6-phenylpyridazin-3-amine

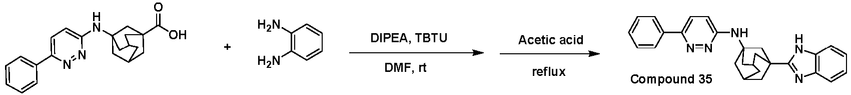

3-((6-Phenylpyridazin-3-yl)amino)adamantane-1-carboxylic acid (115 mg, 0.33 mmol), benzene-1,2-diamine (36 mg, 0.33 mmol) and TBTU (116 mg, 0.36 mmol) were dissolved in DMF (5 mL), and DIPEA (0.115 mL, 0.66 mmol) was then added thereto, followed by stirring at room temperature overnight. After confirming the completion of the reaction by LC-MS, the reaction solution was concentrated, and acetic acid (5 mL) was added thereto, followed by stirring at reflux at 120°C for 4 hours. After confirming the completion of the reaction through TLC, the reaction solution was concentrated, slurried with dichloromethane/ethyl acetate, and filtered to obtain the title compound (100 mg, 72%) as a white solid.

### Example 38: Synthesis of N-(3-(1H-benzo[d]imidazol-2-yl)phenyl)-5-(3-fluorophenyl)-N-methylpyrimidin-2-amine

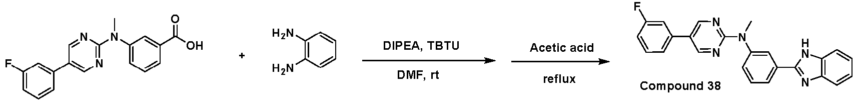

3-((5-(3-Fluorophenyl)pyrimidin-2-yl) (methyl)amino)benzoic acid (162 mg, 0.5 mmol), benzene-1,2-diamine (54 mg, 0.5 mmol) and TBTU (177 mg, 0.5 mmol) were dissolved in DMF (5 mL), then DIPEA (0.174 mL, 1.0 mmol) was added, and the mixture was stirred overnight at room temperature. After confirming the completion of the reaction through LC-MS, the reaction solution was washed with 5% LiCl aqueous solution, extracted with ethyl acetate, and the organic layer was concentrated to remove DMF. Then, acetic acid (5 mL) was added thereto, followed by stirring at reflux at 120°**C** for 2 hours. After confirming the completion of the reaction through LC-MS, the resulting mixture was cooled to room temperature. The mixture was washed with saturated NaHCO₃ solution and extracted with ethyl acetate, and then the organic layer was dried over MgSO₄ and concentrated. The concentrated solution was purified by MPLC to obtain the title compound (80 mg, 42%) as a beige solid.

### Example 39: Synthesis of N-(5-(1H-benzo[d]imidazol-2-yl)-2-methoxyphenyl)-5-(3-fluorophenyl)pyrimidin-2-amine

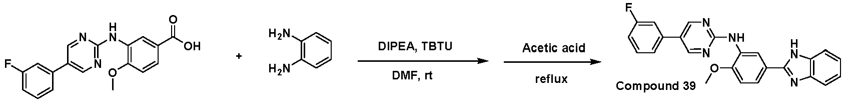

3-((5-(3-Fluorophenyl)pyrimidin-2-yl)amino)-4-methoxybenzoic acid (170 mg, 0.5 mmol), benzene-1,2-diamine (54 mg, 0.5 mmol) and TBTU (177 mg, 0.5 mmol) were dissolved in DMF (5 mL), then DIPEA (0.174 mL, 1.0 mmol) was added, and the mixture was stirred overnight at room temperature. After confirming the completion of the reaction by LC-MS, the reaction solution was concentrated, and acetic acid (5 mL) was added thereto, followed by stirring at reflux at 120°**C** for 2 hours. After confirming the completion of the reaction through LC-MS, the resulting mixture was cooled to room temperature. The mixture was washed with saturated NaHCO₃ solution and extracted with ethyl acetate, and then the organic layer was dried over MgSO₄ and concentrated. The concentrated solution was separated by MPLC, slurried with dichloromethane, and filtered to obtain the title compound (80 mg, 39%) as a beige solid.

### Example 43: Synthesis of N-(3-(1H-benzo[d]imidazol-2-yl)phenyl)-5-(4-methylthiophen-3-yl)pyrimidin-2-amine

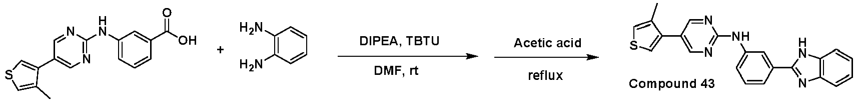

3-((5-(4-Methylthiophen-3-yl)pyrimidin-2-yl)amino)benzoic acid (100 mg, 0.32 mmol), benzene-1,2-diamine (38 mg, 0.35 mmol) and TBTU (124 mg, 0.39 mmol) were dissolved in DMF (2.1 mL), then DIPEA (67µL, 0.39 mmol) was added, and the mixture was stirred overnight at room temperature. After confirming the completion of the reaction by LC-MS, the resulting mixture was concentrated to remove DMF, and acetic acid (2.9 mL) was added thereto, followed by stirring at reflux at 100°**C** for 3 hours. After confirming the completion of the reaction through LC-MS, the resulting mixture was cooled to room temperature. The mixture was washed with saturated NaHCO₃ solution and extracted with ethyl acetate, and then the organic layer was dried over Na₂SO₄ and concentrated. The concentrated solution was purified by MPLC to obtain the title compound (91 mg, 74 %) as a yellow foam.

### Example 44: Synthesis of N-(3-(1H-benzo[d]imidazol-2-yl)phenyl)-5-(pyridin-2-yl)pyrimidin-2-amine

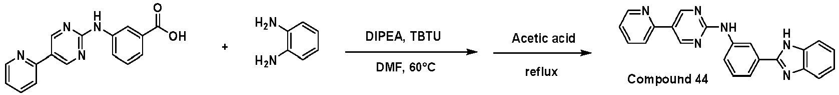

3-((5-(Pyridin-2-yl)pyrimidin-2-yl)amino)benzoic acid (130 mg, 0.44 mmol), benzene-1,2-diamine (48 mg, 0.44 mmol) and TBTU (171 mg, 0.53 mmol) were dissolved in DMF (4 mL), then DIPEA (0.155 mL, 0.89 mmol) was added, and the mixture was stirred at 60°**C** for 20 hours. After confirming the completion of the reaction by LC-MS, the reaction solution was concentrated, and acetic acid (5 mL) was added thereto, followed by stirring at reflux at 120°**C** for 1 hour. After confirming the completion of the reaction through LC-MS, the resulting mixture was cooled to room temperature. The mixture was washed with saturated NaHCO₃ solution and extracted with ethyl acetate, and then the organic layer was dried over MgSO₄ and concentrated. The concentrated solution was slurried with ethyl acetate and filtered to obtain the title compound (86 mg, 54%) as a beige solid.

### Example 46: Synthesis of 3-(1H-imidazolo[4,5-c]pyridin-2-yl)-N-(4-(pyridazin-3-yl)phenyl)aniline

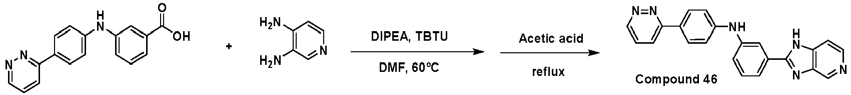

3-((4-(Pyridazin-3-yl)phenyl)amino)benzoic acid (291 mg, 1.0 mmol), pyridine-3,4-diamine (109 mg, 1.0 mmol) and TBTU (385 mg, 1.2 mmol) were dissolved in DMF (5 mL), then DIPEA (0.348 mL, 2.0 mmol) was added, and the mixture was stirred at 60°**C** for 7.5 hours. After confirming the completion of the reaction by LC-MS, the reaction solution was concentrated, and acetic acid (5 mL) was added thereto, followed by stirring at reflux at 120°**C** for 16 hours. After confirming the completion of the reaction through LC-MS, the resulting mixture was cooled to room temperature. The reaction solution was washed with saturated NaHCO₃ solution, acidified up to pH 2 with 1N-HCl (aq), extracted with dichloromethane, and concentrated. The concentrated solution was slurried with dichloromethane/diethylether/ethylacetate and filtered to obtain the title compound (84 mg, 23%) as a beige solid.

### Example 48: Synthesis of N-(3-(1H-imidazolo[4,5-c]pyridin-2-yl)phenyl)-5-(pyridin-2-yl)pyrimidin-2-amine

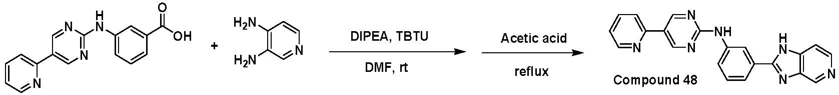

3-((5-(Pyridin-2-yl)pyrimidin-2-yl)amino)benzoic acid (100 mg, 0.34 mmol), pyridine-3,4-diamine (37 mg, 0.34 mmol) and TBTU (132 mg, 0.41 mmol) were dissolved in DMF (3.4 mL), then DIPEA (0.12 mL, 0.68 mmol) was added, and the mixture was stirred at room temperature for 21 hours. After confirming the completion of the reaction by LC-MS, the resulting mixture was concentrated to remove DMF, and acetic acid (2 mL) was added thereto, followed by stirring at reflux at 100°**C** for 18 hours. After confirming the completion of the reaction by LC-MS, the mixture was concentrated to remove acetic acid. The resulting product was extracted with ethyl acetate and washed with brine, and the organic layer was dried over MgSO₄ and concentrated. The concentrated solution was purified by MPLC, concentrated, then slurried with a small amount of MeOH and filtered to obtain the title compound (7 mg, 5.6%) as a beige solid as a filter cake.

### Example 51: Synthesis of N-(3-(6-fluoro-1H-benzo[d]imidazol-2-yl)phenyl)-5-(pyridazin-3-yl)pyrimidin-2-amine

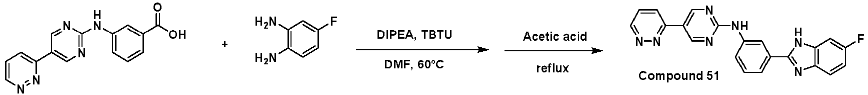

3-((5-(Pyridazin-3-yl)pyrimidin-2-yl)amino)benzoic acid (146 mg, 0.5 mmol), 4-fluorobenzene-1,2-diamine (63 mg, 0.5 mmol) and TBTU (176 mg, 0.55 mmol) were dissolved in DMF (5 mL), then DIPEA (0.174 mL, 1.0 mmol) was added, and the mixture was stirred at 60°C for 16 hours. After confirming the completion of the reaction by LC-MS, the reaction solution was concentrated, and acetic acid (5 mL) was added thereto, followed by stirring at reflux at 120°**C** for 19 hours. After confirming the completion of the reaction through LC-MS, the resulting mixture was cooled to room temperature. The mixture was washed with 1N-NaOH solution and extracted with ethyl acetate, and then the organic layer was dried over MgSO₄ and concentrated. The concentrated solution was slurried with ethyl acetate and filtered to obtain the title compound (77 mg, 40%) as a white solid.

### Example 52: Synthesis of 5-(pyridazin-3-yl)-N-(3-(6-(trifluoromethyl)-1H-benzo[d]imidazol-2-yl)phenyl)pyrimidin-2-amine

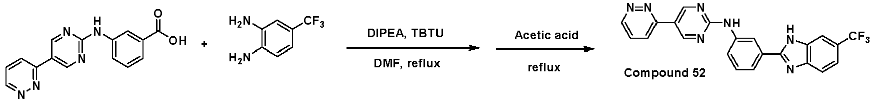

3-((5-(Pyridazin-3-yl)pyrimidin-2-yl)amino)benzoic acid (200 mg, 0.68 mmol), 4-(trifluoromethyl)benzene-1,2-diamine (120 mg, 0.68 mmol) and TBTU (263 mg, 0.82 mmol) were dissolved in DMF (7 mL), then DIPEA (238 µL, 1.36 mmol) was added, and the mixture was stirred at room temperature for 2 hours and at 50°**C** for 17 hours. After confirming the completion of the reaction by LC-MS, the resulting mixture was concentrated to remove DMF, and acetic acid (8 mL) was added thereto, followed by stirring at reflux at 110°C for 18 hours. After confirming the completion of the reaction by LC-MS, the mixture was cooled to room temperature and concentrated to remove acetic acid. The mixture was washed with saturated NaHCO₃ solution and extracted with ethyl acetate, and then the organic layer was dried over Na₂SO₄ and concentrated. The concentrated solution was slurried with dichloromethane and filtered to obtain the title compound (91 mg, 31%) as a brown solid as a filter cake.

### Example 54: Synthesis of N-(3-(1H-imidazolo[4,5-c]pyridin-2-yl)phenyl)-6-phenylpyridazin-3-amine

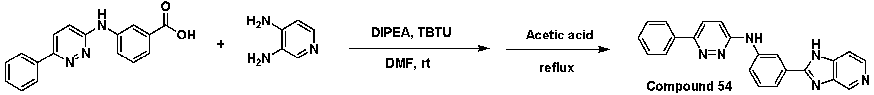

3-((6-Phenylpyridazin-3-yl)amino)benzoic acid (200 mg, 0.68 mmol), pyridine-3,4-diamine (75 mg, 0.69 mmol) and TBTU (263 mg, 0.82 mmol) were dissolved in DMF (7 mL), then DIPEA (238 µL, 1.36 mmol) was added, and the mixture was stirred at room temperature for 67 hours. After confirming the completion of the reaction by LC-MS, the resulting mixture was concentrated to remove DMF, and acetic acid (6 mL) was added thereto, followed by stirring at reflux at 110°**C** for 17 hours. After confirming the completion of the reaction through LC-MS, the resulting mixture was cooled to room temperature and concentrated. The resulting concentrate was adjusted to pH 8 using saturated NaHCO₃ solution, 1N-HCl (aq) and water, extracted with ethyl acetate, and the organic layer was dried over Na₂SO₄ and concentrated. The concentrated solution was slurried with ethyl acetate and filtered, and the resulting filter cake was lyophilized to obtain the title compound (144 mg, 58%) as a beige solid.

### Example 55: Synthesis of N-(3-(6-fluoro-1H-benzo[d]imidazol-2-yl)phenyl)-5-(pyridazin-3-yl)pyrimidin-2-amine

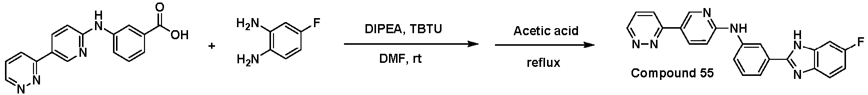

3-((5-(Pyridazin-3-yl)pyridin-2-yl)amino)benzoic acid (100 mg, 0.34 mmol), 4-fluorobenzene-1,2-diamine (48 mg, 0.38 mmol) and TBTU (132 mg, 0.41 mmol) were dissolved in DMF (4 mL), then DIPEA (119 µL, 0.68 mmol) was added, and the mixture was stirred at room temperature for 17 hours. After confirming the completion of the reaction by LC-MS, the resulting mixture was concentrated to remove DMF, and acetic acid (4 mL) was added thereto, followed by stirring at reflux at 110°**C** for 4 hours. After confirming the completion of the reaction through LC-MS, the resulting mixture was cooled to room temperature. The resulting solid was slurried with ethyl acetate and filtered to obtain the title compound (48 mg, 37 %) as a brown solid as a filter cake.

### Example 58: Synthesis of N-(3-(6-fluoro-1H-benzo[d]imidazol-2-yl)phenyl)-[2,3'-bipyridine]-6'-amine

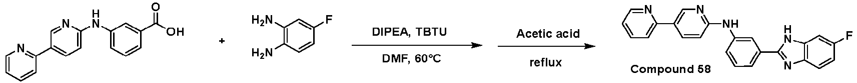

3-([2,3'-Bipyridin]-6'-ylamino)benzoic acid (190 mg, 0.65 mmol), 4-fluorobenzene-1,2-diamine (82 mg, 0.65 mmol) and TBTU (230 mg, 0.72 mmol) were dissolved in DMF (5 mL), then DIPEA (0.227 mL, 1.3 mmol) was added, and the mixture was stirred at 60°**C** for 1 hour. After confirming the completion of the reaction by LC-MS, the reaction solution was concentrated, and acetic acid (5 mL) was added thereto, followed by stirring at reflux at 120°**C** for 1 hour. After confirming the completion of the reaction through LC-MS, the resulting mixture was cooled to room temperature. The mixture was washed with 1N-NaOH solution and extracted with ethyl acetate, and then the organic layer was dried over MgSO₄ and concentrated. The concentrated solution was separated by MPLC, slurried with dichloromethane, and filtered to obtain the title compound (80 mg, 32%) as a white solid.

### Example 61: Synthesis of methyl 2-(3-((4-(pyridazin-3-yl)phenyl)amino)phenyl)-1H-benzo[d]imidazol-6-carboxylate

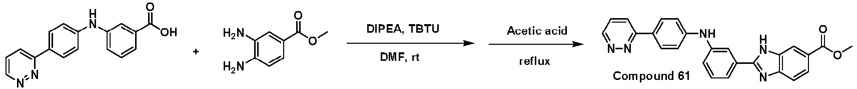

3-((4-(Pyridazin-3-yl)phenyl)amino)benzoic acid (500 mg, 1.72 mmol), methyl 3,4-diaminobenzoate (314 mg, 1.89 mmol) and TBTU (661 mg, 2.06 mmol) were dissolved in DMF (17 mL), then DIPEA (0.6 mL, 3.4 mmol) was added, and the mixture was stirred at room temperature for 17 hours. After confirming the completion of the reaction by LC-MS, the resulting mixture was concentrated to remove DMF, and acetic acid (17 mL) was added thereto, followed by stirring at reflux at 110°**C** for 2 hours. After confirming the completion of the reaction through LC-MS, the resulting mixture was cooled to room temperature. The resulting solid was slurried with ethyl acetate and filtered, and the resulting filter cake was lyophilized to obtain the title compound (476 mg, 65%) as a yellow solid.

### Example 64: Synthesis of N-(3-(6-chloro-1H-imidazolo[4,5-c]pyridin-2-yl)phenyl)-5-(pyridin-2-yl)pyrimidin-2-amine

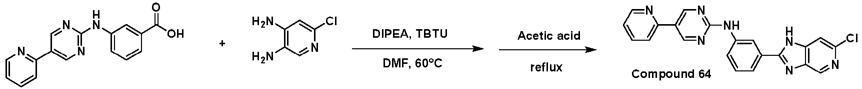

3-((5-(Pyridin-2-yl)pyrimidin-2-yl)amino)benzoic acid (146 mg, 0.5 mmol), 6-chloropyridin-3,4-amine (72 mg, 0.5 mmol) and TBTU (177 mg, 0.55 mmol) were dissolved in DMF (5 mL), then DIPEA (0.174 mL, 1.0 mmol) was added, and the mixture was stirred at 60°**C** for 18 hours. After confirming the completion of the reaction by LC-MS, the reaction solution was concentrated, and acetic acid (5 mL) was added thereto, followed by stirring at reflux at 130°**C** for 25 hours. After confirming the completion of the reaction by LC-MS, the reaction solution was concentrated. MeOH was added to the residue, and the resulting mixture was concentrated, separated by MPLC, slurried with dichloromethane, and filtered to obtain the title compound (80 mg, 32%) as a yellow solid.

### Example 78: Synthesis of methyl 2-(3-((4-(pyrimidin-2-yl)phenyl)amino)phenyl)-1H-benzo[d]imidazol-6-carboxylate

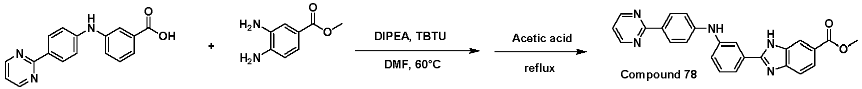

3-((4-(Pyrimidin-2-yl)phenyl)amino)benzoic acid (3.5 g, 12.0 mmol), methyl 3,4-diaminobenzoate (2.2 g, 13.2 mmol) and TBTU (4.6 g, 14.4 mmol) were dissolved in DMF (40 mL), then DIPEA (3.1 mL, 24.0 mmol) was added, and the mixture was stirred at 60°**C** for 3 hours. After confirming the completion of the reaction by LC-MS, the reaction solution was concentrated, and acetic acid (40 mL) was added thereto, followed by stirring at reflux at 120°**C** for 1 hour. After confirming the completion of the reaction through LC-MS, the resulting mixture was cooled to room temperature. The resulting mixture was slurried with saturated NaHCO₃ solution and ethyl acetate and filtered. The resulting filter cake was slurried with dichloromethane and filtered to obtain the title compound (4.5 g, 89%) as a beige solid.

### Example 79: Synthesis of N-(4-(pyridazin-3-yl)phenyl)-3-(6-(trifluoromethyl)-1H-imidazolo[4,5-c]pyridin-2-yl)aniline

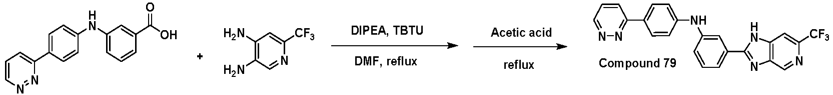

3-((4-(Pyridazin-3-yl)phenyl)amino)benzoic acid (200 mg, 0.69 mmol), 6-(trifluoromethyl)pyridine-3,4-diamine (134 mg, 0.76 mmol), and TBTU (265 mg, 0.83 mmol) were dissolved in DMF (7 mL), then DIPEA (239 µL, 1.37 mmol) was added, and the mixture was stirred at room temperature for 362 hours and stirred at 40°**C** for 16 hours. After confirming the completion of the reaction by LC-MS, the resulting mixture was concentrated to remove DMF, and acetic acid (7 mL) was added thereto, followed by stirring at reflux at 110°**C** for 111 hours. After confirming the completion of the reaction through LC-MS, the resulting mixture was cooled to room temperature. The mixture was washed with saturated NaHCO₃ solution and extracted with ethyl acetate, and then the organic layer was dried over Na₂SO₄ and concentrated. The concentrated solution was slurried with ethyl acetate and filtered, and the resulting filter cake was further slurried with 10% MeOH in DCM solution to obtain the title compound (79 mg, 27%) as a dark beige solid as a filter cake.

### Example 91: Synthesis of 3-(1-methyl-1H-benzo[d]imidazol-2-yl)-N-(4-(pyrazin-3-yl)phenyl)aniline

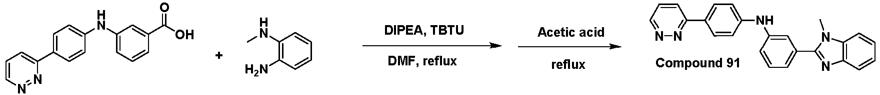

3-((4-(pyridazin-3-yl)phenyl)amino)benzoic acid (200 mg, 0.69 mmol), *N¹*-methylbenzene-1,2-diamine (92 mg, 0.76 mmol), and TBTU (265 mg, 0.83 mmol) were dissolved in DMF (7 mL), then DIPEA (239 ***µ***L, 1.37 mmol) was added, and the mixture was stirred at room temperature for 24 hours. After confirming the completion of the reaction by LC-MS, the resulting mixture was concentrated to remove DMF, and acetic acid (7 mL) was added thereto, followed by stirring at reflux at 110°**C** for 30 hours. After confirming the completion of the reaction through LC-MS, the resulting mixture was cooled to room temperature. The mixture was washed with saturated NaHCO₃ solution and extracted with ethyl acetate, and then the organic layer was dried over Na₂SO₄ and concentrated. The concentrated solution was separated by MPLC, slurried with ethyl acetate, and filtered to obtain the title compound (92 mg, 36%) as a beige solid.

### Example 200: Synthesis of N-(3-(1H-benzo[d]imidazol-2-yl)phenyl)-6-(pyrimidin-2-yl)pyridazin-3-amine

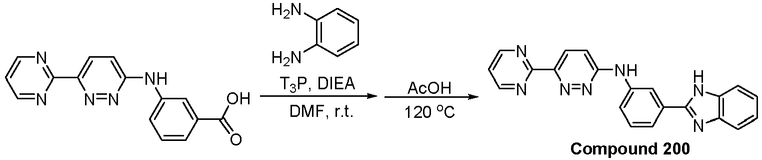

To a solution of 3-((6-(pyrimidin-2-yl)pyridazin-3-yl)amino)benzoic acid (350 mg, 1.19 mmol) and T₃P (760 mg, 2.39 mmol) in DMF (10 mL) was added benzene-1,2-diamine (167 mg, 1.55 mmol) and DIEA (461 mg, 3.57 mmol), the reaction mixture was stirred at room temperature for 16 hours. The reaction mixture was poured into water (100 mL), then extracted with EA (30 mL x3), the combined organic layer was washed with brine (100 mL x3), dried over Na₂SO₄ and concentrated under reduced pressure to obtain N-(2-aminophenyl)-3-((6(pyrimidin-2-yl)pyridazin-3-yl)amino)benzamide (400 mg, crude) as a yellow solid. This crude product was dissolved in AcOH (5 mL) and stirred at 120°**C** for 16 hours. The reaction mixture was cooled to room temperature and concentrated under reduced pressure, the residue was recrystallized with EA to obtain the title compound (35 mg, 8.04%) as a yellow solid.

### Example 213: Synthesis of 2-(3-(4-(pyridazin-3-yl)phenoxy)phenyl)-1H-benzo[d]imidazole

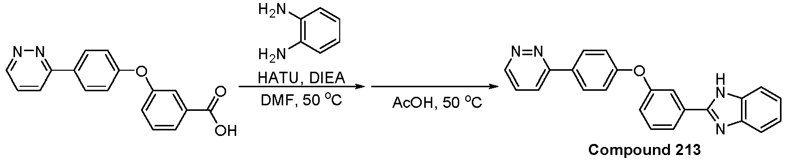

To a solution of 3-(4-(pyridazin-3-yl)phenoxy)benzoic acid (600 mg, 2.05 mmol) and HATU (1.17 g, 3.44 mmol) in DMF (20 mL) was added benzene-1,2-diamine (288 mg, 2.67 mmol) and DIEA (1.32 g, 10.25 mmol), the reaction mixture was stirred at 50°**C** for 16 hours. The reaction mixture was poured into water (100 mL), then extracted with EA (30 mL x3), the combined organic layer was washed with brine (100 mL x3), dried over Na₂SO₄ and concentrated under reduced pressure to obtain *N*-(2-aminophenyl)-3-(4-(pyridazin-3-yl)phenoxy)benzamide (600 mg, crude) as a yellow solid. This crude product was dissolved in AcOH (10 mL) and stirred at 50°**C** for 2 hours. The reaction mixture was cooled to room temperature and concentrated under reduced pressure, the residue was recrystallized with EA to obtain the title compound (160 mg, 47.97%) as a white solid.

### Example 214: Synthesis of 5-(1H-benzo[d]imidazol-2-yl)-N-(4-(pyridazin-3-yl)phenyl)thiazol-2-amine

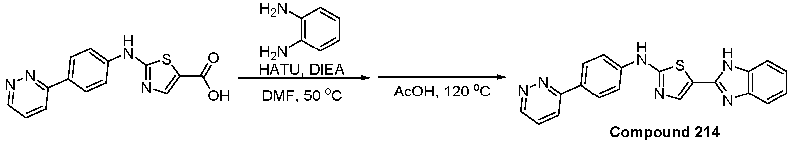

To a solution of 2-((4-(pyridazin-3-yl)phenyl)amino)thiazole-5-carboxylic acid (950 mg, 3.18 mmol) and HATU (1.45 g, 3.82 mmol) in DMF (50 mL) was added benzene-1,2-diamine (379 mg, 3.50 mmol) and DIEA (1.23 g, 9.55 mmol), the reaction mixture was stirred at 50°**C** for 16 hours. The reaction mixture was poured into water (100 mL), then extracted with EA (30 mL x3), the combined organic layer was washed with brine (100 mL x3), dried over Na₂SO₄ and concentrated under reduced pressure, the residue was purified by column chramatography to obtain the title compound (400 mg, 30.77%) as a brown solid.

### Example 218: Synthesis of 3-(6-bromo-1H-imidazo[4,5-c]pyridin-2-yl)-N-(4-(pyridazin-3-yl)phenyl)aniline

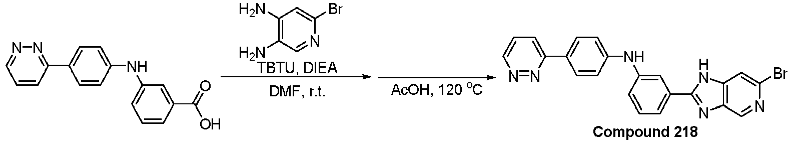

To a solution of 3-((4-(pyridazin-3-yl)phenyl)amino)benzoic acid (900 mg, 4.80 mmol) and TBTU (1.85 g, 5.76 mmol) in DMF (15 mL) was added 6-bromopyridine-3,4-diamine (1.46 g, 1.03 mmol) and DIEA (1.86 g, 14.4 mmol), the reaction mixture was stirred at room temperature for 16 hours. The reaction mixture was poured into water (150 mL), then extracted with EA (40 mL x3), the combined organic layer was washed with brine (150 mL x3), dried over Na₂SO₄ and concentrated under reduced pressure to obtain *N*-(4-amino-6-bromopyridin-3-yl)-3-((4-(pyridazin-3-yl)phenyl)amino)benzamide (1.6 g, crude) as a yellow solid. This crude product was dissolved in AcOH (30 mL) and stirred at 120°**C** for 36 hours. The reaction mixture was cooled to room temperature and concentrated under reduced pressure, the residue was recrystallized with EA to obtain the title compound (80 mg, 5.33%) as a beige solid.

### Example 222: Synthesis of 3-(6-(benzyloxy)-1H-benzo[d]imidazol-2-yl)-N-(4-(pyridazin-3-yl)phenyl)aniline

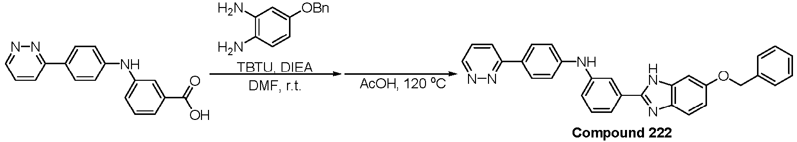

To a solution of 3-((4-(pyridazin-3-yl)phenyl) amino) benzoic acid (300 mg, 1.03 mmol) and TBTU (397 mg, 1.24 mmol) in DMF (15 mL) was added 4-(benzyloxy)benzene-1,2-diamine (232 mg, 1.08 mmol) and DIEA (402 mg, 3.09 mmol), the reaction mixture was stirred at room temperature for 16 hours. The reaction mixture was poured into water (150 mL), then extracted with EA (30 mL x3), the combined organic layer was washed with brine (150 mL x3), dried over Na₂SO₄ and concentrated under reduced pressure to obtain *N*-(3-aminopyridin-2-yl)-3-((4-(pyridazin-3-yl)phenyl)amino)benzamide (400 mg, crude) as a yellow solid. This crude product was dissolved in AcOH (10 mL) and stirred at 120°**C** for 36 hours. The reaction mixture was cooled to room temperature, concentrated under reduced pressure, and purified by Prep-HPLC to obtain the title compound (35 mg, 17.5%) as a yellow solid.

### Example 232: Synthesis of N-(4-(pyridazin-3-yl)phenyl)-3-(6-(trifluoromethoxy)-1H-imidazo[4,5-c]pyridin-2-yl)aniline

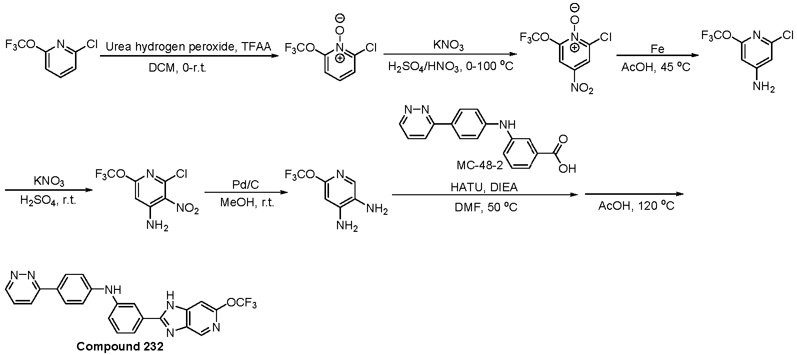

### Step 1: Synthesis of 2-chloro-6-(trifluoromethoxy)pyridine 1-oxide

To a stirred solution of 2-chloro-6-(trifluoromethoxy)pyridine (10 g, 58.94 mmol) in the DCM (500 mL) at 0°**C** were added urea hydrogen peroxide (19.41 g, 206.31 mmol) and TFAA (24.76 g, 117.89 mmol), the reaction mixture was stirred at room temperature for 16 hours. The reaction mixture was poured into water (500 mL), then extracted with DCM (80 mL x3), the combined organic layer was washed with brine (500 mL x3), dried over Na₂SO₄ and concentrated under reduced pressure, the residue was purified by column chromatography to obtain the title compound (600 mg, 4.78%) as a yellow oil.

¹H NMR (400 MHz, DMSO-*d*₆) δ 7.91 (dd, J = 8.3, 1.8 Hz, 1H), 7.77 (dd, J = 8.3, 1.0 Hz, 1H), 7.47 (t, J = 8.3 Hz, 1H).

MS: m/z = 214 (M+1, ESI+).

### Step 2: Synthesis of 2-chloro-4-nitro-6-(trifluoromethoxy)pyridine 1-oxide

To a solution of 2-chloro-6-(trifluoromethoxy)pyridine 1-oxide (550 mg, 2.58 mmol) in H₂SO₄/HNO₃ (8 mL/4 mL) at 0°C was added KNO₃ (391 mg, 3.86 mmol), then the reaction mixture was stirred at 100 °C for 16 hours. The reaction mixture was cooled to room temperature and poured into water (50 mL), then extracted with EA (15 mL x3), the combined organic layer was washed with brine (50 mL x3), dried over Na₂SO₄ and concentrated under reduced pressure, the residue was purified by column chromatography to obtain the title compound (445 mg, 66.83%) as a yellow oil.

¹H NMR (400 MHz, DMSO-*d*₆) δ 8.87 (d, J = 3.1 Hz, 1H), 8.67 (d, J = 3.0 Hz, 1H).

MS: m/z = 259 (M+1, ESI+).

### Step 3: Synthesis of 2-chloro-6-(trifluoromethoxy)pyridin-4-amine

To a solution of 2-chloro-4-nitro-6-(trifluoromethoxy)pyridine 1-oxide (445 mg, 1.72 mmol) in AcOH (10 mL) was added Fe (288 mg, 5.16 mmol). Then the mixture was stirred at 45°C for 3 hours. The reaction mixture was cooled to room temperature and poured into water (50 mL), then extracted with EA (15 mL x3), the combined organic layer was washed with brine (50 mL x3), dried over Na₂SO₄ and concentrated under reduced pressure, the residue was purified by column chromatography to obtain the title compound (160 mg, 43.73%) as a yellow solid.

MS: m/z = 213 (M+1, ESI+).

### Step 4: Synthesis of 2-chloro-3-nitro-6-(trifluoromethoxy)pyridin-4-amine

To a solution of 2-chloro-6-(trifluoromethoxy)pyridin-4-amine (160 mg, 0.75 mmol) in H₂SO₄ (5 mL) was added KNO₃ (152 mg, 1.51 mmol), reaction mixture was stirred at room temperature for 5 hours. The reaction mixture was cooled to room temperature and poured into water (50 mL), then extracted with EA (15 mL x3), the combined organic layer was washed with brine (50 mL x3), dried over Na₂SO₄ and concentrated under reduced pressure, the residue was purified by column chromatography to obtain the title compound (175 mg, 90.3%) as a yellow solid.

¹H NMR (400 MHz, DMSO-*d*₆) δ 7.74 (s, 2H), 6.59 (s, 1H).

MS: m/z = 257 (M+1, ESI+).

### Step 5: Synthesis of 6-(trifluoromethoxy)pyridine-3,4-diamine

To a solution of 2-chloro-3-nitro-6-(trifluoromethoxy)pyridin-4-amine (175 mg, 0.68 mmol) in MeOH (5 mL) was added Pd/C (36 mg, 0.34 mmol), the reaction mixture was stirred at room temperature for 16 hours. Filtered and the filtrate was purified by column chromatography to obtain the title compound (60 mg, 45.72%) as a brown solid.

¹H NMR (400 MHz, DMSO-*d*₆) δ 7.29 (s, 1H), 6.21 (s, 1H), 5.85 (s, 2H), 4.64 (s, 2H).

MS: m/z = 194 (M+1, ESI+).

### Step 6: Synthesis of N-(4-(pyrazin-3-yl)phenyl)-3-(6-(trifluoromethoxy)-1H-imidazo[4,5-c]pyridin-2-yl)aniline

To a solution of 3-((4-(pyridazin-3-yl)phenyl)amino)benzoic acid (82 mg, 0.28 mmol) and HATU (129 mg, 0.34 mmol) in DMF (10 mL) was added 6-(trifluoromethoxy)pyridine-3,4-diamine (60 mg, 0.31 mmol) and DIEA (110 mg, 0.85 mmol), the reaction mixture was stirred at 50°C for 16 hours. The reaction mixture was poured into water (100 mL), then extracted with DCM (30 mL x3), the combined organic layer was washed with brine (100 mL x3), dried over Na₂SO₄ and concentrated under reduced pressure to obtain *N*-(4-amino-6-(trifluoromethoxy)pyridin-3-yl)-3-((4-(pyridazin-3-yl)phenyl)amino)benzamide (95 mg, 78.68%) as a brown solid. MS: m/z = 467 (M+1, ESI+). The above obtained brown solid was dissolved in AcOH (2mL) and stirred at 120°C for 4 hours under microwave. The reaction mixture was cooled to room temperature and concentrated under reduced pressure, the residue was recrystallized with MeOH to obtain the title compound (10 mg, 12.24%) as an off-white solid.

### Example 234: Synthesis of N-(4-(pyridazin-3-yl)phenyl)-3-(6-(trifluoromethyl)-1H-imidazo[4,5-b]pyridin-2-yl)aniline

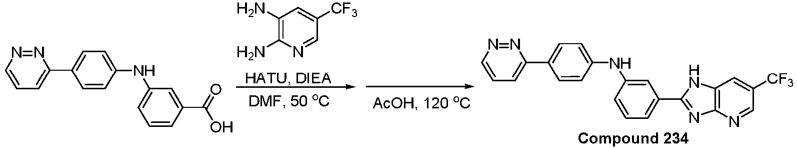

To a solution of 3-((4-(pyridazin-3-yl)phenyl)amino) benzoic acid (550 mg, 1.89 mmol) and HATU (862 mg, 2.27 mmol) in DMF (50 ml) was added 5-(trifluoromethyl)pyridine-2,3-diamine (368 mg, 2.08 mmol) and DIEA (732 mg, 5.66 mmol), the reaction mixture was stirred at 50°C for 48 hours. The reaction mixture was poured into water (500 mL), then extracted with EA (80 mL x3), the combined organic layer was washed with brine (500 mL x3), dried over Na₂SO₄ and concentrated under reduced pressure to obtain N-(2-amino-5- (trifluoromethyl) pyridin-3-yl)-3-((4-(pyridazin-3-yl)phenyl)amino)benzamide (700 mg, crude) as a brown solid. MS: m/z =451 (M+1, ESI+). This crude product was dissolved in AcOH (10 mL) and stirred at 120°C for 48 hours. The reaction mixture was cooled to room temperature and concentrated under reduced pressure, the residue was purified by reversed-column chromatography to obtain the title compound (45 mg, 5.51%) as a yellow solid.

### Example 244: Synthesis of 5-(pyridin-2-yl)-N-(3-(5-(trifluoromethyl)-1H-imidazo[4,5-b]pyridin-2-yl)phenyl)pyrimidin-2-amine

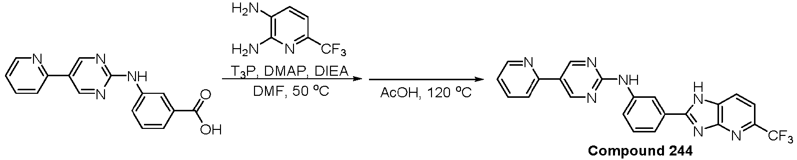

To a solution of 3-((5-(pyridin-2-yl)pyrimidin-2-yl)amino) benzoic acid (650 mg, 2.21 mmol) and T₃P (850 mg, 2.67 mmol) in DMF (30 mL) was added 6-(trifluoromethyl)pyridine-2,3-diamine (414 mg, 2.34 mmol), DIEA (863 mg, 6.68 mmol) and DMAP (134 mg, 1.11 mmol), the reaction mixture was stirred at 50°C for 16 hours. The reaction mixture was poured into water (100 mL), then extracted with DCM (40 mL x3), the combined organic layer was washed with brine (100 mL x3), dried over Na₂SO₄ and concentrated under reduced pressure to obtain *N*-(2-amino-6-(trifluoromethyl)pyridin-3-yl)-3-((5-(pyridin-2-yl)pyrimidin-2-yl)amino)benzamide (800 mg, crude) as a yellow solid. MS: m/z = 452 (M+1, ESI+). This crude product was dissolved in AcOH (8 mL) and stirred at 120°C for 16 hours. The reaction mixture was cooled to room temperature and concentrated under reduced pressure, the residue was recrystallized with MeOH to obtain the title compound (330 mg, 34.23%) as a beige solid.

### Example 250: Synthesis of 3-(1H-benzo[d]imidazol-2-yl)-N-(4-(pyridazin-3-yl)phenyl)-5-(trifluoromethyl)aniline

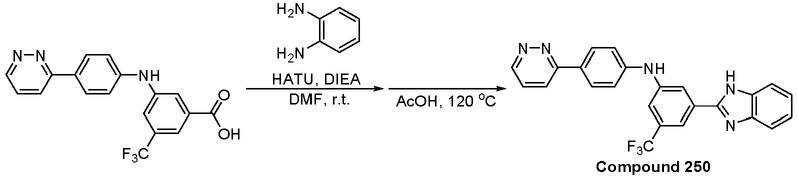

To a solution of 3-((4-(pyridazin-3-yl)phenyl)amino)-5-(trifluoromethyl)benzoic acid (940 mg, 2.62 mmol) and HATU (1.19 g, 3.14 mmol) in DMF (15 mL) was added benzene-1,2-diamine (340 mg, 3.14 mmol) and DIEA (1.01 g, 7.86mmol), the reaction mixture was stirred at room temperature for 16 hours. The reaction mixture was poured into water (150 mL), then extracted with EA (40 mL x3), the combined organic layer was washed with brine (150 mL x3), dried over Na₂SO₄ and concentrated under reduced pressure to obtain *N*-(2-aminophenyl)-3-((4-(pyridazin-3-yl)phenyl)amino)-5-(trifluoromethyl)benzamide (1.2 g, crude) as an off-white solid. This crude product was dissolved in AcOH (25 mL) and stirred at 120°C for 16 hours. The reaction mixture was cooled to room temperature and concentrated under reduced pressure, the residue was recrystallized with EA to obtain the title compound (190 mg, 16.52%) as an off-white solid.

### Example 252: Synthesis of N-(4-(6-fluoropyridin-2-yl)phenyl)-3-(6-(trifluoromethyl)-1H-benzo[d]imidazol-2-yl)aniline

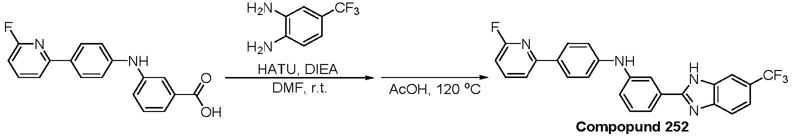

To a solution of 3-((4-(6-fluoropyridin-2-yl)phenyl)amino) benzoic acid (500 mg, 1.70 mmol) and HATU (775 mg, 2.05 mmol) in DMF (15 mL) was added 4-(trifluoromethyl)benzene-1,2-diamine (300 mg, 2.05 mmol) and DIEA (665 mg, 5.11 mmol), the reaction mixture was stirred at room temperature for 16 hours. The reaction mixture was poured into water (150 mL), then extracted with EA (50 mL x3), the combined organic layer was washed with brine (150 mL x3), dried over Na₂SO₄ and concentrated under reduced pressure to obtain *N*-(2-amino-5-(trifluoromethyl) phenyl)-3-((4-(6-fluoropyridin-2-yl)phenyl)amino)benzamide (800 mg, crude) as a brown solid. This crude product was dissolved in AcOH (20 mL) and stirred at 120°C for 16 hours. The reaction mixture was cooled to room temperature and concentrated under reduced pressure, the residue was recrystallized with EA/MeOH to obtain the title compound (130 mg, 16.95%) as a white solid.

### Example 279: Synthesis of N-methyl-2-(3-((4-(pyridazin-3-yl)phenyl)amino)phenyl)-1H-benzo[d]imidazol-6-amine

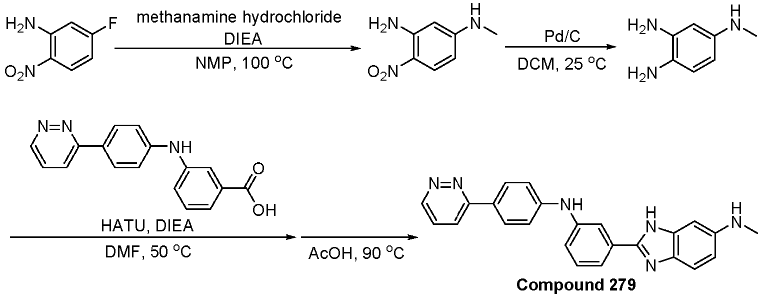

### Step 1: Synthesis of N1-methyl-4-nitrobenzene-1,3-diamine

To a solution of 5-fluoro-2-nitroaniline (5 g, 32.03 mmol) and methanamine hydrochloride (4.33 g, 64.06 mmol) in NMP (100 mL) was added to DIEA (20.66 g, 160.14 mmol), the reaction mixture was stirred at 100°C for 4 hours. The reaction mixture was cooled to room temperature and poured into water (500 mL), then extracted with EA (100 mL x3), the combined organic layer was washed with brine (500 mL x3), dried over Na₂SO₄ and concentrated under reduced pressure. The residue was purified by column chromatography to obtain the title compound (4 g, 74.77%) as a yellow solid.

MS: m/z = 168 (M+1, ESI+).

### Step 2: Synthesis of N4-methylbenzene-1,2,4-triamine

To a solution of *N*¹-methyl-4-nitrobenzene-1,3-diamine (500 mg, 2.99 mmol) in DCM (10 mL) was added Pd/C (50 mg), the reaction mixture was stirred at 25°C for 6 hours under H₂. Filtered and the filtrate was concentrated under reduced pressure to obtain the title compound (500 mg, crude) as a brown solid.

MS: m/z = 138 (M+1, ESI+).

### Step 3: Synthesis of N-methyl-2-(3-((4-(pyridazin-3-yl)phenyl)amino)phenyl)-1H-benzo[d] imidazol-6-amine

To a solution of 3-((4-(pyridazin-3-yl)phenyl)amino)benzoic acid (817 mg, 2.81mmol) and HATU (1.60 g, 4.21 mmol) in DMF (20 mL) was added *N*⁴-methylbenzene-1,2,4-triamine (500 mg, 3.65 mmol) and DIEA (1.09 g, 8.42 mmol), the reaction mixture was stirred at 50°C for 16 hours. The reaction mixture was poured into water (200 mL), then extracted with EA (50 mL x3), the combined organic layer was washed with brine (200 mL x3), dried over Na₂SO₄ and concentrated under reduced pressure to obtain N-(2-amino-5-(methylamino)phenyl)-3-((4-(pyridazin-3-yl)phenyl)amino)benzamide (250 mg, crude) as a yellow solid. This crude product was dissolved in AcOH (20 mL) and stirred at 90°C for 16 hours. The reaction mixture was cooled to room temperature and concentrated under reduced pressure, the residue was recrystallized with MeOH to obtain the title compound (8 mg, 0.56%) as a yellow solid.

### Example 280: Synthesis of N-benzyl-N-methyl-2-(3-((4-(pyridazin-3-yl)phenyl)amino)phenyl)-1H-benzo[d]imidazol-6-amine

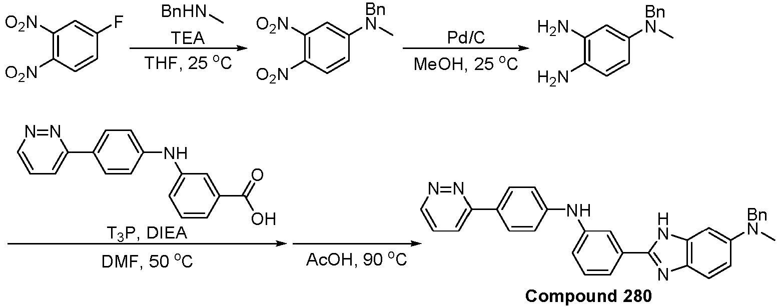

### Step 1: Synthesis of N -benzyl-N-methyl-3,4-dinitroaniline

To a solution of 4-fluoro-1,2-dinitrobenzene (10 g, 53.73 mmol) and N-methyl-1-phenylmethanamine (8.47 g, 69.85 mmol) in THF (100 mL) was added to TEA (10.86 g, 107.46 mmol), the reaction mixture was stirred at 25°C for 16 hours. The reaction mixture was poured into water (300 mL), then extracted with EA (100 mL x3), the combined organic layer was washed with brine (300 mL x3), dried over Na₂SO₄ and concentrated under reduced pressure. The residue was purified by column chromatography to obtain the title compound (9 g, 58.44%) as a yellow solid.

MS: m/z = 288 (M+1, ESI+).

### Step 2: Synthesis of N4-benzyl-N4-methylbenzene-1,2,4-triamine

To a solution of N-benzyl-N-methyl-3,4-dinitroaniline (9 g, 31.35 mmol) in MeOH (100 mL) was added Pd/C (1 g), the reaction mixture was stirred at 25°C for 16 hours under H₂. Filtered and the filtrate was concentrated under reduced pressure to obtain the title compound (1.2 g, 16.9%) as a brown solid.

¹H NMR (400 MHz, DMSO-*d*₆) δ 7.31-7.20 (m, 5H), 6.14 (d, *J* = 2.4 Hz, 1H), 5.92 (dd, *J* = 8.4, 2.8 Hz, 1H), 5.76 (s, 1H), 4.33 (s, 2H), 4.29 (s, 2H), 3.84 (s, 2H), 2.72 (s, 3H).

MS: m/z = 228 (M+1, ESI+).

### Step 3: Synthesis of N-benzyl-N-methyl-2-(3-((4-(pyridazin-3-yl)phenyl)amino)phenyl)-1H-benzo[d]imidazol-6-amine

To a solution of 3-((4-(pyridazin-3-yl)phenyl)amino) benzoic acid (1.40 g, 4.81 mmol) and T₃P (2.74 g, 7.21 mmol) in DMF (20 mL) was added *N*⁴-benzyl-*N*⁴-methylbenzene-1,2,4-triamine (1.2 g, 5.28 mmol) and DIEA (1.86 g, 14.42 mmol), the reaction mixture was stirred at 50°C for 16 h. The reaction mixture was poured into water (200 mL), then extracted with EA (50 mL x3), the combined organic layer was washed with brine (200 mL x3), dried over Na₂SO₄ and concentrated under reduced pressure to afford *N*-(2-amino-5-(benzyl(methyl)amino)phenyl)-3-((4-(pyridazin-3-yl)phenyl)amino)benzamide (1.4 g, crude) as a yellow solid. This crude product was dissolved in AcOH (20 mL) and stirred at 90°C for 16 hours. The reaction mixture was cooled to room temperature and concentrated under reduced pressure, the residue was recrystallized with EA to afford *N*-benzyl-N-methyl-2-(3-((4-(pyridazin-3-yl)phenyl)amino)phenyl)-1*H*-benzo[*d*]imidazol-6-amine (36 mg, 6.8%) as an off-white solid.

### Example 288: Synthesis of N-(3-(6-(2-morpholinoethoxy)-1H-benzo[d]imidazol-2-yl)phenyl)-5-(pyridazin-3-yl)pyrimidin-2-amine

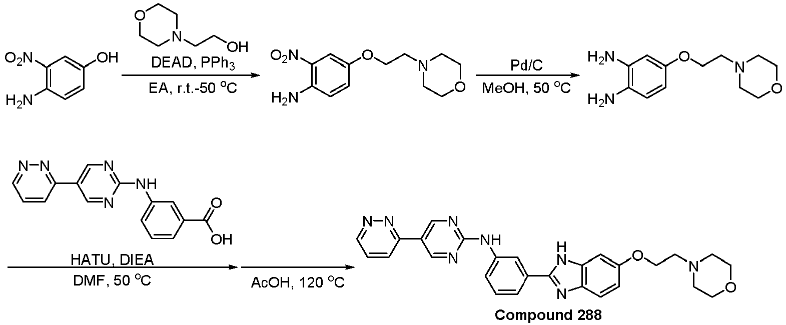

### Step 1: Synthesis of 4-(2-morpholinoethoxy)-2-nitroaniline

To a solution of 4-amino-3-nitrophenol (2 g, 12.98 mmol) and 2-morpholinoethan-1-ol (1.87 g, 14.27 mmol) in EA (150 mL) was added PPh₃ (4.08 g, 15.57 mmol) and DEAD (3.39 g, 19.47 mmol) at room temperature, then the reaction mixture was stirred at 50°C for 16 hours. The reaction mixture was cooled to room temperature and poured into water (800 mL), then extracted with EA (300 mL x3), the combined organic layer was washed with brine (800 mL x3), dried over Na₂SO₄ and concentrated under reduced pressure. The residue was purified by column chromatography to obtain the title compound (2 g, 55.5%) as a black oil.

### Step 2: Synthesis of 4-(2-morpholinoethoxy)benzene-1,2-diamine

To a solution of 4-(2-morpholinoethoxy)-2-nitroaniline (1 g, 3.74 mmol) in MeOH (10 mL) was added Pd/C (100 mg), the reaction mixture was stirred at 50°C for 16 hours. The reaction mixture was filtered and concentrated under reduced pressure to obtain the title compound (800 mg, 85.83%) as a brown solid.

### Step 3: Synthesis of N-(3-(6-(2-morpholinoethoxy)-1H-benzo[d]imidazol-2-yl)phenyl)-5-(pyridazin-3-yl)pyrimidin-2-amine

To a solution of 3-((5-(pyridazin-3-yl)pyrimidin-2-yl)amino)benzoic acid (300 mg, 1.02 mmol) and HATU (467 mg, 1.23 mmol) in DMF (10 mL) was added 4-(2-morpholinoethoxy)benzene-1,2-diamine (316 mg, 1.33 mmol) and DIEA (397 mg, 3.07 mmol), the reaction mixture was stirred at 50°C for 16 hours. The reaction mixture was poured into water (100 mL), then extracted with EA (30 mL x3), the combined organic layer was washed with brine (100 mL x3), dried over Na₂SO₄ and concentrated under reduced pressure to obtain *N*-(2-amino-5-(2-morpholinoethoxy)phenyl)-3-((5-(pyridazin-3-yl)pyrimidin-2-yl)amino)benzamide (600 mg, crude) as a brown solid. This crude product was dissolved in AcOH (5 mL) and stirred at 120°C for 16 hours. The reaction mixture was cooled to room temperature and concentrated under reduced pressure, the residue was purified by reversed-column chromatography to obtain the title compound (160 mg, 21.95%) as a white solid.

### Example 300: Synthesis of N-(3-(6-(difluoromethoxy)-1H-benzo[d]imidazol-2-yl)phenyl)-5-(pyridin-2-yl) pyrazin-2-amine

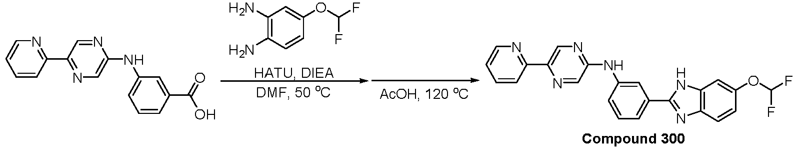

To a solution of 3-((5-(pyridin-2-yl)pyrazin-2-yl)amino)benzoic acid (270 mg, 0.92 mmol) and HATU (422 mg, 1.11 mmol) in DMF (10 mL) was added 4-(difluoromethoxy)benzene-1,2-diamine (177 mg, 1.02 mmol) and DIEA (358 mg, 2.77 mmol), the reaction mixture was stirred at 50°C for 16 hours. The reaction mixture was poured into water (100 mL), then extracted with EA (30 mL x3), the combined organic layer was washed with brine (100 mL x3), dried over Na₂SO₄ and concentrated under reduced pressure to obtain N-(2-amino-5-(difluoromethoxy) phenyl)-3-((5-(pyridin-2-yl)pyrazin-2-yl)amino)benzamide (500 mg, crude) as a yellow solid. This crude product was dissolved in AcOH (5 mL) and stirred at 120°C for 16 hours. The reaction mixture was cooled to room temperature and concentrated under reduced pressure, the residue was recrystallized with MeOH to obtain the title compound (170 mg, 38.55%) as a yellow solid.

### Example 303: Synthesis of N-(3-(6-chloro-1H-imidazo[4,5-c]pyridin-2-yl)phenyl)-5-(thiazol-4-yl)pyrimidin-2-amine

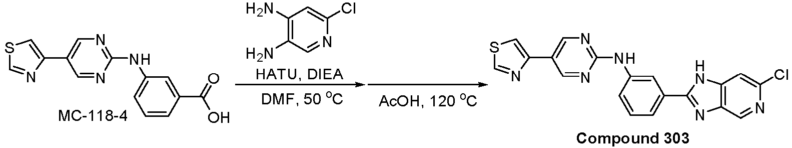

To a solution of 3-((5-(thiazol-4-yl)pyrimidin-2-yl)amino)benzoic acid (600 mg, 2.05 mmol) and HATU (935 mg, 2.45 mmol) in DMF (30 mL) was added 6-chloropyridine-3,4-diamine (353 mg, 2.45 mmol) and DIEA (793 mg, 6.15 mmol), the reaction mixture was stirred at 50°C for 16 hours. The reaction mixture was poured into water (100 mL), then extracted with EA (30 mL x3), the combined organic layer was washed with brine (100 mL x3), dried over Na₂SO₄ and concentrated under reduced pressure to obtain *N*-(5-amino-2-chloropyridin-4-yl)-3-((5-(thiazol-4-yl)pyrimidin-2-yl)amino)benzamide (700 mg, crude) as a yellow solid. This crude product was dissolved in AcOH (10 mL) and stirred at 120°C for 72 hours. The reaction mixture was cooled to room temperature and concentrated under reduced pressure, the residue was recrystallized with EA to obtain the title compound (50 mg, 6.13%) as a yellow solid.

### Example 158: Synthesis of (1R,2R)-2-(6-fluoro-1H-benzo[d]imidazol-2-yl)-N-(4-(pyrazin-2-yl)phenyl)cyclopropane-1-carboxamide

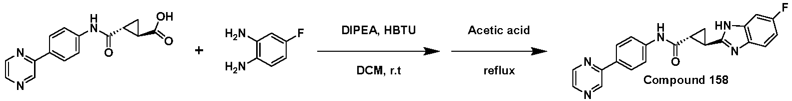

To a solution of (1*R*,2*R*)-2-((4-(pyrazin-2-yl)phenyl)carbamoyl)cyclopropane-1-carboxylic acid (20 mg, 0.0706 mmol), 4-fluorobenzene-1,2-diamine (10 mg, 0.0777 mmol) in DCM (1 mL) was added HBTU (40mg, 0.106 mmol) and DIPEA (0.037 mL, 0.212 mmol), the reaction mixture was stirred at room temperature for 22 hours. The reaction mixture was concentrated under reduced pressure, AcOH (1 mL) was added, and the residue was stirred at 100°C for 22 hours. The reaction mixture was washed with NaHCO₃ (aq), then extracted with DCM, the combined organic layer was dried over MgSO₄ and concentrated under reduced pressure. The residue was purified by MPLC to obtain the title compound (4.4 mg, 17%) as a yellow solid.

### Example 167: Synthesis of 2-(1H-benzo[d]imidazol-2-yl)-N1,N1-dimethyl-N4-(4-(pyrazin-2-yl)phenyl)benzene-1,4-diamine

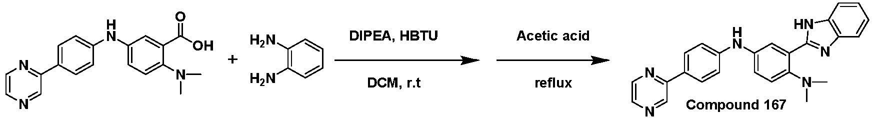

To a solution of 2-(dimethylamino)-5-((4-(pyrazin-2-yl)phenyl)amino)benzoic acid (50 mg, 0.150 mmol), benzene-1,2-diamine (16 mg, 0.150 mmol) in DCM (1.5 mL) was added HBTU (68 mg, 0.179 mmol) and DIPEA (0.052 mL, 0.299 mmol), the reaction mixture was stirred at room temperature for 7 hours. The reaction mixture was concentrated under reduced pressure, AcOH (1.5 mL) was added, and the residue was stirred at 100°C for 20 hours. The reaction mixture was washed with NaHCO₃ (aq), then extracted with DCM. The combined organic layer was dried over MgSO₄ and concentrated under reduced pressure. The residue was purified by MPLC to obtain the title compound (25 mg, 41 %) as a yellow solid.

### Example 176: Synthesis of N-(2-(4-methylpiperazin-1-yl)-5-(6-(trifluoromethyl)-lH-benzo[d]imidazol-2-yl)phenyl)-5-(pyridazin-3-yl)pyrimidin-2-amine

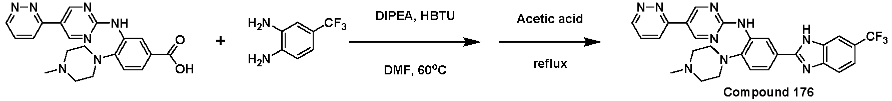

To a solution of 4-(4-methylpiperazin-1-yl)-3-((5-(pyridazin-3-yl)pyrimidin-2-yl)amino)benzoic acid (150 mg, 0.383 mmol), 4-(trifluoromethyl)benzene-1,2-diamine (67 mg, 0.383 mmol) in DMF (4 mL) was added HBTU (174 mg, 0.460 mmol) and DIPEA (0.134 mL, 0.766 mmol), the reaction mixture was stirred at 60°C for 22 hours. The reaction mixture was concentrated under reduced pressure, AcOH (1.5 mL) was added, and the residue was stirred at 100°C for 7 hours. The reaction mixture was washed with NaHCO₃ (aq), then extracted with DCM, the combined organic layer was dried over MgSO₄ and concentrated under reduced pressure. The residue was purified by MPLC. The crude mixture was solidified using EA and hexane to obtain the title compound (61 mg, 30 %) as a beige solid.

### Example 328: Synthesis of 5-(pyridin-2-yl)-N-(3-(6-(trifluoromethyl)-lH-imidazo[4,5-c]pyridin-2-yl)phenyl)pyrazin-2-amine

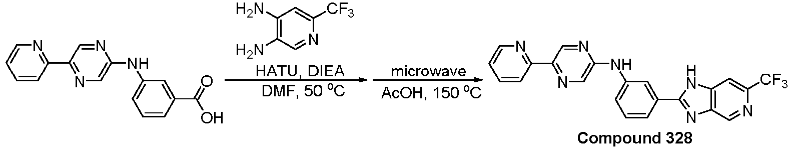

To a solution of 3-((5-(pyridin-2-yl)pyrazin-2-yl)amino)benzoic acid (300 mg, 1.03 mmol) and 6-(trifluoromethyl)pyridine-3,4-diamine (182 mg, 1.13 mmol) in DMF (10 mL) was added HATU (472 mg, 1.24 mmol) and DIEA (399 mg, 3.09 mmol), the reaction mixture was stirred at 50°C for 16 hours. The reaction mixture was poured into water (100 mL), then extracted with EA (30 mL x3), the combined organic layer was washed with brine (100 mL x3), dried over Na₂SO₄ and concentrated under reduced pressure to obtain N-(5-amino-2-(trifluoromethyl)pyridin-4-yl)-3-((5-(pyridin-2-yl)pyrazin-2-yl)amino)benzamide (350 mg, crude) as a yellow solid. This crude product was dissolved in AcOH (5 mL) and stirred at 150°C under microwave for 2 hours. The reaction mixture was cooled to room temperature and concentrated under reduced pressure, the residue was recrystallized with MeOH to obtain the title compound (78 mg, 16.46%) as a yellow solid.

### Example 329: Synthesis of N-(3-(6-(difluoromethoxy)-1H-imidazo[4,5-c]pyridin-2-yl)phenyl)-5-(pyridin-2-yl)pyrimidin-2-amine

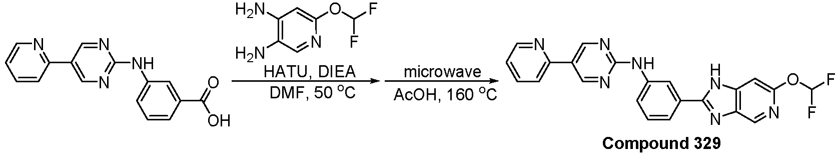

To a solution of 3-((5-(pyridin-2-yl)pyrimidin-2-yl)amino)benzoic acid (300 mg, 1.03 mmol) and 6-(difluoromethoxy)pyridine-3,4-diamine (198 mg, 1.13 mmol) in DMF (10 mL) was added HATU (472 mg, 1.24 mmol) and DIEA (399 mg, 3.09 mmol), the reaction mixture was stirred at 50°C for 16 hours. The reaction mixture was poured into water (100 mL), then extracted with EA (30 mL x3), the combined organic layer was washed with brine (100 mL x3), dried over Na₂SO₄ and concentrated under reduced pressure to obtain N-(5-amino-2-(difluoromethoxy)pyridin-4-yl)-3-((5-(pyridin-2-yl)pyrimidin-2-yl)amino)benzamide (400 mg, crude) as a yellow solid. This crude product was dissolved in AcOH (6 mL) and stirred at 160°C under microwave for 2 hours. The reaction mixture was cooled to room temperature and concentrated under reduced pressure, the residue was purified by reversed-column chromatography to obtain the title compound (34 mg, 7.2%) as a yellow solid.

### Method B. Preparation using Buchwald reaction

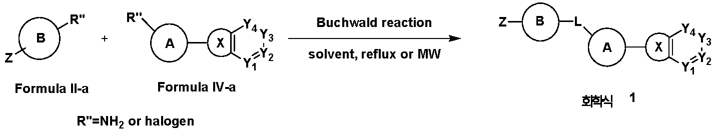

### Example 69: Synthesis of N-(3-(lH-benzo[d]imidazol-2-yl)phenyl)-5-(pyrimidin-2-yl)pyridin-2-amine

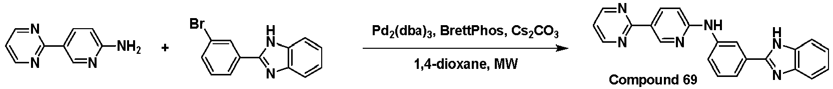

5-(Pyrimidin-2-yl)pyridin-2-amine (100 mg, 0.58 mmol), 2-(3-bromophenyl)-1H-benzo[d]imidazole (174 mg, 0.64 mmol), Pd₂(dba)₃ (53 mg, 0.058 mmol), BrettPhos (62 mg, 0.116 mmol) and Cs₂CO₃ (378 mg, 1.16 mmol) were dissolved in 1,4-dioxane (5 mL), and then the mixture was stirred in a microwave reactor at 120°C for 2 hours. After confirming the completion of the reaction by LC-MS, the reaction solution was purified by MPLC, concentrated, slurried with dichloromethane, and filtered to obtain the title compound (85 mg, 40.2%) as a beige solid as a filter cake.

### Example 72: Synthesis of N-(3-(6-fluoro-1H-benzo[d]imidazol-2-yl)phenyl)-5-(pyrimidin-4-yl)pyridin-2-amine

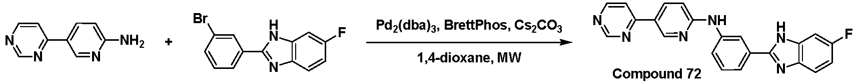

5-(Pyrimidin-4-yl)pyridin-2-amine (100 mg, 0.58 mmol), 2-(3-bromophenyl)-6-fluoro-1H-benzo[d]imidazole (186 mg, 0.64 mmol), Pd₂(dba)₃ (53 mg, 0.058 mmol), BrettPhos (62 mg, 0.116 mmol) and Cs₂CO₃ (378 mg, 1.16 mmol) were dissolved in 1,4-dioxane (5 mL), and then the mixture was stirred in a microwave reactor at 120°C for 2 hours. Pd₂(dba)₃ (106 mg, 0.116 mmol) and BrettPhos (124 mg, 0.232 mmol) were added, and the mixture was further stirred in a microwave reactor at 120°C for 1 hour. After confirming the completion of the reaction by LC-MS, the reaction solution was purified by MPLC to obtain the title compound (37 mg, 17%) as a yellow solid.

### Example 73: Synthesis of N-(3-(1H-benzo[d]imidazol-2-yl)phenyl)-5-(4-fluoropyridin-2-yl)pyridimin-2-amine

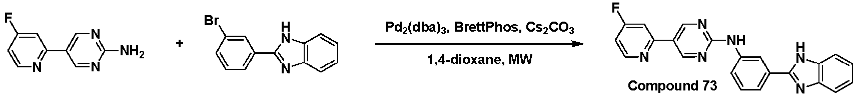

5-(4-Fluoropyridin-2-yl)pyrimidin-2-amine (100 mg, 0.53 mmol), 2-(3-bromophenyl)-1H-benzo[d]imidazole (158 mg, 0.58 mmol), Pd₂(dba)₃ (241 mg, 0.26 mmol), BrettPhos (282 mg, 0.53 mmol) and Cs₂CO₃ (343 mg, 1.05 mmol) were dissolved in 1,4-dioxane (5 mL), and then the mixture was stirred in a microwave reactor at 120°C for 1 hour. Pd₂(dba)₃ (120 mg, 0.13 mmol) and BrettPhos (141 mg, 0.26 mmol) were added, and the mixture was further stirred in a microwave reactor at 120°C for 1 hour. After confirming the completion of the reaction by LC-MS, the reaction solution was purified by MPLC to obtain the title compound (30 mg, 15 %) as a white solid.

### Example 16: Synthesis of N-(3-(5,6-dichloro-1H-benzo[d]imidazol-2-yl)phenyl)pyrimidin-2-amine

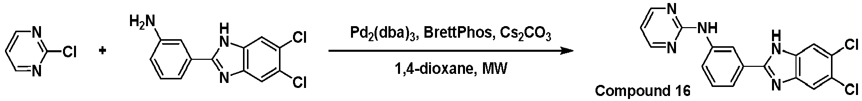

2-Chloropyrimidine (334 mg, 3.0 mmol), 3-(5,6-dichloro-1H-benzo[d]imidazol-2-yl)aniline (831 mg, 3.0 mmol), Pd₂(dba)₃ (275 mg, 0.3 mmol), BrettPhos (242 mg, 0.45 mmol) and Cs₂CO₃ (2.9 g, 9.0 mmol) were dissolved in 1,4-dioxane (10 mL), and then the mixture was stirred in a microwave reactor at 150°C for 40 minutes. After confirming the completion of the reaction by TLC, the resulting mixture was cooled to room temperature. After removing Pd through celite filtration, the resulting mixture was separated by MPLC, slurried with dichloromethane/ethyl acetate, and filtered to obtain the title compound (180 mg, 17%) as a beige solid.

### Example 75: Synthesis of N-(3-(1H-benzo[d]imidazol-2-yl)phenyl)-3-(pyrimidin-5-yl)aniline

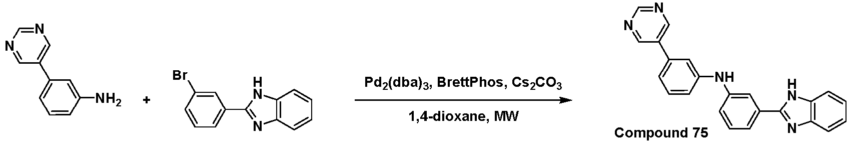

3-(Pyrimidin-5-yl)aniline (99 mg, 0.58 mmol), 2-(3-bromophenyl)-1H-benzo[d]imidazole (174 mg, 0.64 mmol), Pd₂(dba)₃ (53 mg, 0.058 mmol), BrettPhos (62 mg, 0.116 mmol) and Cs₂CO₃ (378 mg, 1.16 mmol) were dissolved in 1,4-dioxane (5 mL), and then stirred in a microwave reactor at 120°C for 2 hours. After confirming the completion of the reaction by LC-MS, the reaction solution was purified by MPLC, concentrated, slurried with ethyl acetate and filtered to obtain the title compound (115 mg, 54.5%) as a white solid as a filter cake.

### Example 77: Synthesis of N-(3-(lH-benzo[d]imidazol-2-yl)phenyl)-2-phenylpyrimidin-5-amine

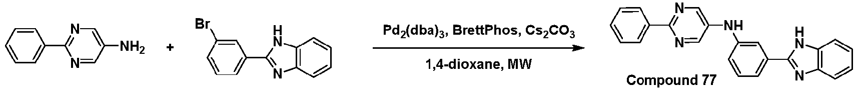

2-Phenylpyrimidin-5-amine (103 mg, 0.60 mmol), 2-(3-bromophenyl) -1H-benzo[d]imidazole (164 mg, 0.60 mmol), Pd₂(dba)₃ (49 mg, 0.053 mmol), BrettPhos (64 mg, 0.12 mmol) and Cs₂CO₃ (391 mg, 1.2 mmol) were dissolved in 1,4-dioxane (3 mL), and then stirred in a microwave reactor at 120°C for 1.5 hours. After confirming the completion of the reaction through LC-MS, the resulting mixture was cooled to room temperature. The mixture was washed with H₂O and extracted with ethyl acetate, and then the organic layer was dried over MgSO₄ and concentrated. The residue was separated by MPLC, slurried with dichloromethane/hexane, and filtered to obtain the title compound (21 mg, 9%) as a white solid.

### Example 113: Synthesis of 6-(1-methyl-4-piperidyl)-N-[3-[6-(trifluoromethyl)-1H-benzo[d]imidazol-2-yl]phenyl]pyridazin-3-amine

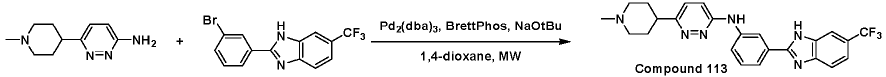

To a solution of 6-(1-methyl-4-piperidyl)pyridazin-3-amine(100 mg, 0.52 mmol) and 2-(3-bromophenyl)-6-(trifluoromethyl)-1H-benzo[d]imidazole (213 mg, 0.62 mmol) in dioxane (5 mL) was added Pd₂(dba)₃ (47.6 mg, 0.052 mmol), BrettPhos (69.8 mg, 0.13 mmol) and NaOtBu (100 mg, 1.04 mmol), the reaction mixture was stirred at 150°C under microwave for 1.5 hours. After confirming the completion of the reaction through LC-MS, the resulting mixture was cooled to room temperature. The reaction mixture was washed with brine, then extracted with 10% MeOH in DCM solution, dried over Na₂SO₄ and concentrated under reduced pressure. The residue was slurried with ethyl acetate/hexane and concentrated under reduced pressure. The residue was slurried with DCM and filtered to obtain the title compound (81 mg, 34%) as a white solid.

### Example 261: Synthesis of N-(4-(pyridazin-3-yl)phenyl)-4-(6-(trifluoromethyl)-1H-benzo[d]imidazol-2-yl) pyridin-2-amine

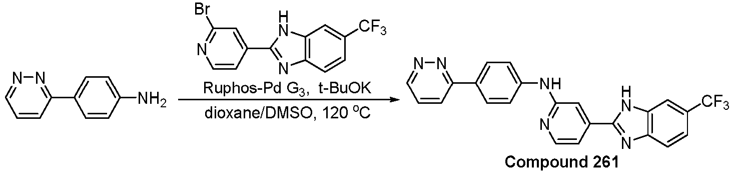

To a solution of 2-(2-bromopyridin-4-yl)-6-(trifluoromethyl)-1H-benzo[d]imidazole (1.45 g, 4.24 mmol) and 4-(pyridazin-3-yl)aniline (943 mg, 5.51 mmol) in dioxane/DMSO (30 mL/6 mL) was added t-BuOK (950 mg, 8.48 mmol) and Ruphos-Pd G₃ (706 mg, 0.85 mmol), the reaction mixture was stirred at 120°C for 16 hours under N₂. The reaction mixture was cooled to room temperature and poured into water (200 mL), then extracted with DCM (50 mL x3), the combined organic layer was washed with brine (200 mL x3), dried over Na₂SO₄ and concentrated under reduced pressure. The residue was purified by column chromatography to obtain the title compound (48 mg, 2.6%) as a yellow solid.

### Example 265: Synthesis of 3-(1H-benzo[d]imidazol-2-yl)-N-(4-(6-fluoropyridazin-3-yl)phenyl)aniline

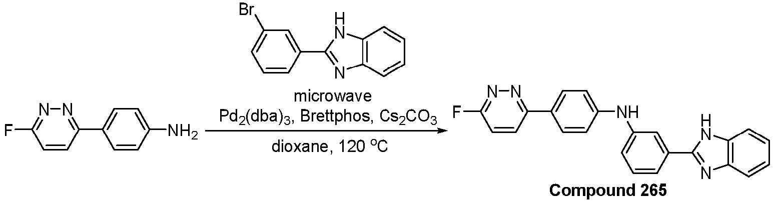

To a solution of 4-(6-fluoropyridazin-3-yl)aniline (100 mg, 0.53 mmol) and 2-(3-bromophenyl)-1H-benzo[d]imidazole (188 mg, 0.69 mmol) in dioxane (5 mL) was added Cs₂CO₃ (346 mg, 1.06 mmol), Brettphos (142 mg, 0.27 mmol) and Pd₂(dba)₃ (247 mg, 0.27 mmol), the reaction mixture was stirred at 120°C under microwave for 4 hours. The reaction mixture was cooled to room temperature and poured into water (50 mL), then extracted with DCM (15 mL x3), the combined organic layer was washed with brine (50 mL x3), dried over Na₂SO₄ and concentrated under reduced pressure. The residue was purified by column chromatography to obtain the title compound (20 mg, 9.90%) as a pale yellow solid.

### Example 154: Synthesis of N-(4-(2-(4-methylpiperazin-1-yl)pyrimidin-5-yl)phenyl)-3-(6-(trifluoromethyl)-1H-benzo[d]imidazol-2-yl)aniline

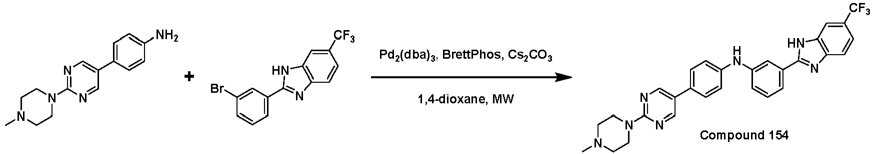

To a solution of 4-(2-(4-methylpiperazin-1-yl)pyrimidin-5-yl)aniline (56 mg, 0.206 mmol), 2-(3-bromophenyl)-6-(trifluoromethyl)-1H-benzo[d]imidazole (85 mg, 0.248 mmol) in 1,4-dioxane (3 mL) was added Pd₂(dba)₃ (42 mg, 0.0516 mmol), BrettPhos (55 mg, 0.103 mmol) and Cs₂CO₃ (133 mg, 0.413 mmol). The reaction mixture was stirried in microwave at 150°C for 2 hours. The reaction mixture was purified by MPLC. The crude mixture was solidified using DCM and hexane to obtain the title compound (10 mg, 9 %) as a beige solid.

### Example 168: Synthesis of 3-(1H-benzo[d]imidazol-2-yl)-4-(2-(dimethylamino)ethoxy)-N-(4-(pyrazin-2-yl)phenyl)aniline

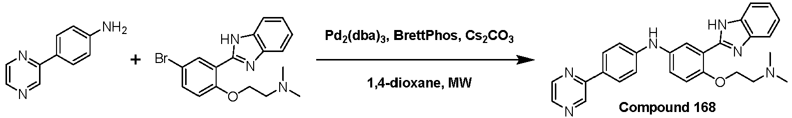

To a solution of 4-(pyrazin-2-yl)aniline (15 mg, 0.0916 mmol), 2-(2-(1*H*-benzo[*d*]imidazol-2-yl)-4-bromophenoxy)-*N,N-*dimethylethan-1-amine (30 mg 0.0833 mmol) in 1,4-dioxane (1 mL) was added Pd₂(dba)₃ (7 mg, 0.0083 mmol), BrettPhos (22 mg, 0.0416 mmol) and Cs₂CO₃ (53 mg, 0.167 mmol), the reaction mixture was stirred in microwave at 150°C for 2 hours. The reaction mixture was purified by MPLC. The crude mixture was solidified using EA and hexane to obtain the title compound (6 mg, 16 %) as a yellow solid.

### Example 169: Synthesis of 3-(lH-benzo[d]imidazol-2-yl)-4-(2-(4-methylpiperazin-1-yl)ethoxy)-N-(4-(pyrazin-2-yl)phenyl)aniline

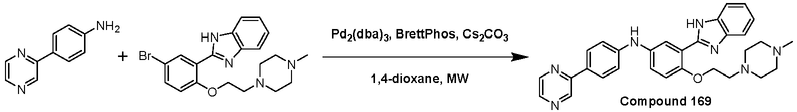

To a solution of 4-(pyrazin-2-yl)aniline (14 mg, 0.0795 mmol), 2-(5-bromo-2-(2-(4-methylpiperazin-1-yl)ethoxy)phenyl)-1H-benzo[*d*]imidazole (30 mg 0.0722 mmol) in 1,4-dioxane (1 mL) was added Pd₂(dba)₃ (6 mg, 0.0072 mmol), BrettPhos (19 mg, 0.0361 mmol) and Cs₂CO₃ (46 mg, 0.145 mmol), the reaction mixture was stirried in microwave at 150°C for 2 hours. The reaction mixture was purified. The crude mixture was solidified using EA and hexane to obtain the title compound (12 mg, 31 %) as a yellow solid.

### Example 172: Synthesis of 3-(1H-benzo[d]imidazol-2-yl)-4-((1-methylpiperidin-4-yl)oxy)-N-(4-(pyrazin-2-yl)phenyl)aniline

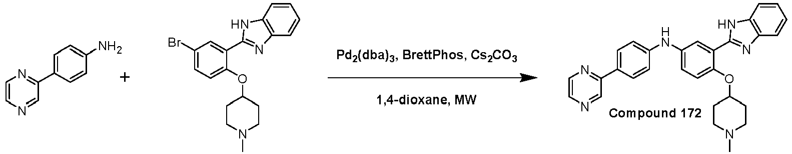

To a solution of 4-(pyrazin-2-yl)aniline (11 mg, 0.0626 mmol), 2-(5-bromo-2-((1-methylpiperidin-4-yl)oxy)phenyl)-1*H-*benzo[*d*]imidazole (22 mg, 0.0570 mmol) in 1,4-dioxane (1 mL) was added Pd₂(dba)₃ (5 mg, 0.0057 mmol), BrettPhos (15 mg, 0.0285 mmol) and Cs₂CO₃ (37 mg, 0.114 mmol), the reaction mixture was stirred in microwave at 150°C for 2 hours. The reaction mixture was purified by MPLC. The crude mixture was solidified using EA and hexane to obtain the title compound (4 mg, 16 %) as a yellow solid.

### Example 177: Synthesis of N-(2-((1-methylpiperidin-4-yl)oxy)-3-(6-(trifluoromethyl)-1H-benzo[d]imidazol-2-yl)phenyl)-5-(pyridazin-3-yl)pyrimidin-2-amine

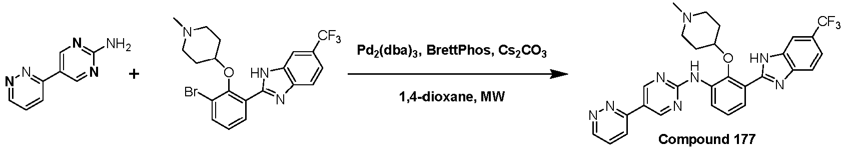

To a solution of 5-(pyridazin-3-yl)pyrimidin-2-amine (77 mg, 0.444 mmol), 2-(3-bromo-2-((1-methylpiperidin-4-yl)oxy)phenyl)-6-(trifluoromethyl)-1*H*-benzo[*d*]imidazole (184 mg, 0.404 mmol) in 1,4-dioxane (4 mL) was added Pd₂(dba)₃ (33 mg, 0.0404 mmol), BrettPhos (108 mg, 0.202 mmol) and Cs₂CO₃ (259 mg, 0.808 mmol), the reaction mixture was stirred in microwave at 150°C for 2 hours. The reaction mixture was purified by MPLC. The crude mixture was solidified using EA and hexane to obtain the title compound (6 mg, 16 %) as a yellow solid.

### Example 178: Synthesis of N-(3-((1-methylpiperidin-4-yl)oxy)-5-(6-(trifluoromethyl)-1H-benzo[d]imidazol-2-yl)phenyl)-5-(pyrazin-3-yl)pyrimidin-2-amine

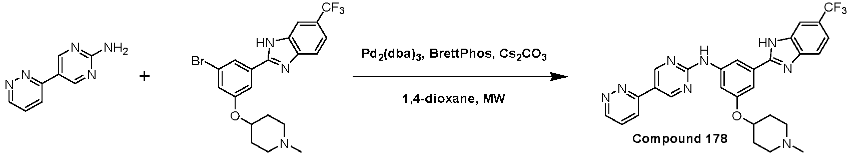

To a solution of 5-(pyridazin-3-yl)pyrimidin-2-amine (39 mg, 0.223 mmol), 2-(3-bromo-5-((1-methylpiperidin-4-yl)oxy)phenyl)-6-(trifluoromethyl)-1*H*-benzo[*d*]imidazole (92 mg, 0.203 mmol) in 1,4-dioxane (2 mL) was added Pd₂(dba)₃ (16 mg, 0.0203 mmol), BrettPhos (54 mg, 0.101 mmol) and Cs₂CO₃ (130 mg, 0.405 mmol), the reaction mixture was stirred in microwave at 150°C for 3 hours. The reaction mixture was purified by MPLC. The crude mixture was solidified using EA and hexane to obtain the title compound (10 mg, 9%) as a pink solid.

### Example 179: Synthesis of N-(3-(2-(dimethylamino)ethoxy)-5-(6-(trifluoromethyl)-1H-benzo[d]imidazol-2-yl)phenyl)-5-(pyridazin-3-yl)pyrimidin-2-amine

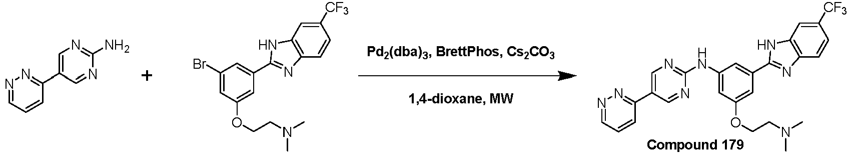

To a solution of 5-(pyridazin-3-yl)pyrimidin-2-amine (111 mg, 0.642 mmol), 2-(3-bromo-5-(6-(trifluoromethyl)-1H-benzo[d]imidazol-2-yl)phenoxy)-N,N-dimethylethan-1-amine (250 mg, 0.584 mmol) in 1, 4-dioxane (6 mL) was added Pd₂(dba)₃ (47 mg, 0.0584 mmol), BrettPhos (157 mg, 0.292 mmol) and Cs₂CO₃ (375 mg, 1.168 mmol), the reaction mixture was stirred in microwave at 160°C for 3 hours. The reaction mixture was purified by MPLC. The crude mixture was solidified using EA and hexane to obtain the title compound (58 mg, 19 %) as a yellow solid.

### Example 311: Synthesis of 5-(5-((methylamino)methyl)pyridin-2-yl)-N-(3-(6-(trifluoromethyl)-1H-benzo[d]imidazol-2-yl)phenyl)pyrimidin-2-amine

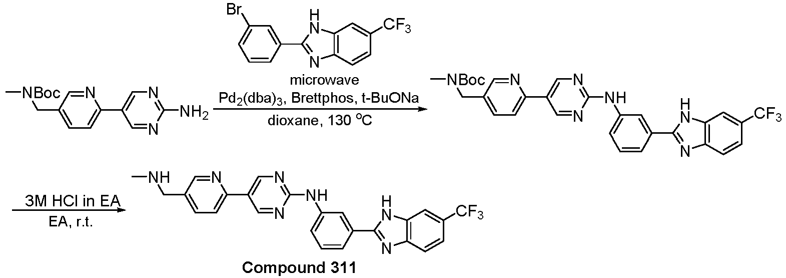

### Step 1: Synthesis of tert-butyl methyl((6-(2-((3-(6-(trifluoromethyl)-1H-benzo[d]imidazol-2-yl)phenyl) amino)pyrimidin-5-yl)pyridin-3-yl)methyl)carbamate

To a solution of tert-butyl ((6-(2-aminopyrimidin-5-yl)pyridin-3-yl)methyl)(methyl) carbamate (320 mg, 1.86 mmol) and 2-(3-bromophenyl)-6-(trifluoromethyl)-1*H*-benzo[*d*] imidazole (395 mg, 1.77 mmol) in dioxane (8 mL) was added Pd₂(dba)₃ (146 mg, 0.16 mmol), Brettphos (170 mg, 0.32 mmol) and t-BuONa (305 mg, 3.17 mmol), the reaction mixture was stirred at 130°C under microwave for 4 hours. The reaction mixture was cooled to room temperature and poured into water (30 mL), then extracted with DCM (10 mL x3), the combined organic layer was washed with brine (30 mL x3), dried over Na₂SO₄ and concentrated under reduced pressure. The residue was purified by column chromatography to obtain the title compound (700 mg, crude) as a yellow solid.

MS: m/z = 576 (M+1, ESI+).

### Step 2: Synthesis of 5-(5-((methylamino)methyl)pyridin-2-yl)-N-(3-(6-(trifluoromethyl)-1H-benzo[d]imidazol-2-yl)phenyl)pyrimidin-2-amine

A mixture of tert-butyl methyl((6-(2-((3-(6-(trifluoromethyl)-1*H*-benzo[*d*]imidazol-2-yl)phenyl)amino)pyrimidin-5-yl)pyridin-3-yl)methyl)carbamate (320 mg, 1.86 mmol) in EA (8 mL) was added 3M HCl in ethylacetate (2 mL), the reaction mixture was stirred at room temperature for 4 hours. The reaction mixture was concentrated under reduced pressure, the residue was purified by reversed-column chromatography to obtain the title compound (70 mg, 8.93%) as a white solid.

### Example 321: Synthesis of methyl 4'-((3-(1H-benzo[d]imidazol-2-yl)phenyl)amino)-[1,1'-biphenyl]-3-carboxylate

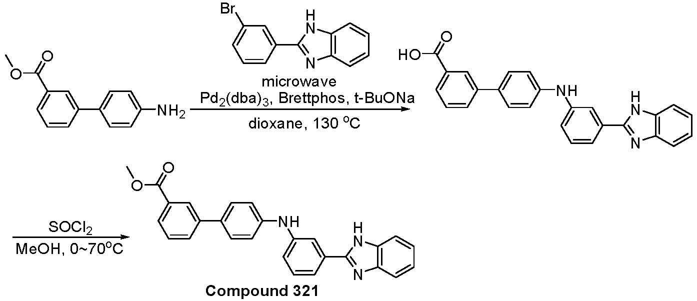

### Step 1: Synthesis of 4'-((3-(1H-benzo[d]imidazol-2-yl)phenyl)amino)-[1,1'-biphenyl]-3-carboxylic acid

To a solution of methyl 4'-amino-[1,1'-biphenyl]-3-carboxylate (320 mg, 1.41 mmol) and 2-(3-bromophenyl)-1H-benzo[d]imidazole (571 mg, 1.93 mmol) in dioxane (4 mL) was added Pd₂(dba)₃ (128 mg, 0.141 mmol), Brettphos (75 mg, 0.141 mmol) and t-BuONa (269 mg, 2.82 mmol), the reaction mixture was stirred at 130°C under microwave for 4 hours. The reaction mixture was cooled to room temperature and poured into water (20 mL), then extracted with DCM (10 mL x3), the combined organic layer was washed with brine (20 mL x3), dried over Na₂SO₄ and concentrated under reduced pressure. The residue was purified by column chromatography to obtain the title compound (110 mg, 19.30%) as a yellow solid.

### Step 2: Synthesis of methyl 4'-((3-(1H-benzo[d]imidazol-2-yl)phenyl)amino)-[1,1'-biphenyl]-3-carboxylate

To a solution of 4'-((3-(1H-benzo[d]imidazol-2-yl)phenyl)amino)-[1,1'-biphenyl]-3-carboxylic acid (110 mg, 0.27 mmol) in MeOH (10 mL) at 0°C was added SOCl₂ (64 mg, 0.54 mmol) dropwise, the reaction mixture was stirred at 70°C for 16 hours. The reaction mixture was cooled to room temperature and concentrated under reduced pressure. The residue was purified by Prep-HPLC to obtain the title compound (30 mg, 32.26%) as a white solid.

### Method C. Preparation of carboxylic acid derivative through hydrolysis reaction

### Example 62: Synthesis of 2-(3-((4-(pyridazin-3-yl)phenyl)amino)phenyl)-1H-benzo[d]imidazol-6-carboxylic acid

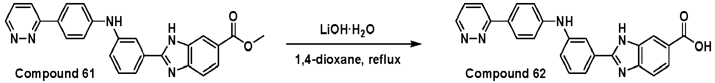

Methyl 2-(3-((4-(pyridazin-3-yl)phenyl)amino)phenyl)-1H-benzo[d]imidazol-6-carboxylate (50 mg, 0.12 mmol) prepared in Example 61 above and LiOH·H₂O (50 mg, 1.186 mmol) were dissolved in 1,4-dioxane (1 mL) and H₂O (0.2 mL), stirred at room temperature for 1.5 hours, and stirred at reflux at 50°C for 19 hours. After confirming the completion of the reaction by LC-MS, the reaction solution was adjusted to pH 5 with 1N-HCl solution, extracted with ethyl acetate, and the organic layer was dried over Na₂SO₄ and concentrated. The concentrated solution was slurried with ethyl acetate and filtered to obtain the title compound (13 mg, 27 %) as a yellow solid as a filter cake.

### Method D. Preparation of amide derivative through amide linkage reaction

### Example 65: Synthesis of N-isopropyl-2-(3-((4-(pyridazin-3-yl)phenyl)amino)phenyl)-1H-benzo[d]imidazol-6-carboxamide

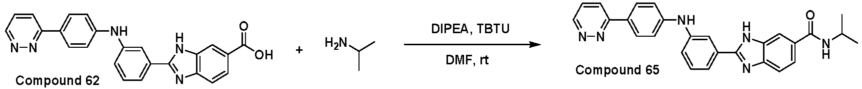

2-(3-((4-(Pyridazin-3-yl)phenyl)amino)phenyl)-1H-benzo[d]imidazol-6-carboxylic acid (50 mg, 0.12 mmol) prepared in Example 62 above, propan-2-amine (9 mg, 0.013 mmol), and TBTU (47 mg, 0.15 mmol) were dissolved in DMF (2 mL), then DIPEA (13 µL, 0.69 mmol) was added, and the mixture was stirred at room temperature for 67 hours. After confirming the completion of the reaction by LC-MS, the mixture was washed with a brine solution and extracted with ethyl acetate, and the organic layer was dried over Na₂SO₄ and concentrated. The concentrated mixture was slurried with ethyl acetate and filtered to obtain the title compound (8.4 mg, 15 %) as a beige solid as a filter cake.

### Example 66: Synthesis of N-cyclohexyl-2-(3-((4-(pyridazin-3-yl)phenyl)amino)phenyl)-1H-benzo[d]imidazol-6-carboxamide

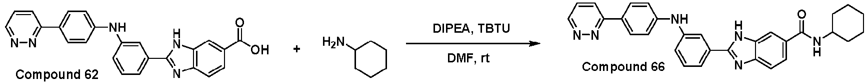

2-(3-((4-(Pyridazin-3-yl)phenyl)amino)phenyl)-1H-benzo[d]imidazol-6-carboxylic acid (50 mg, 0.12 mmol) prepared in Example 62 above, cyclohexanamine (15 mg, 0.017 mmol) and TBTU (47 mg, 0.15 mmol) were dissolved in DMF (2 mL), then DIPEA (13 µL, 0.69 mmol) was added, and the mixture was stirred at room temperature for 17 hours. After confirming the completion of the reaction by LC-MS, the mixture was washed with a brine solution and extracted with ethyl acetate, and the organic layer was dried over Na₂SO₄ and concentrated. The concentrated mixture was slurried with dichloromethane and filtered to obtain the title compound (41 mg, 68 %) as a beige solid as a filter cake.

### Method E. Preparation through additional reaction after amide reaction and cyclization reaction

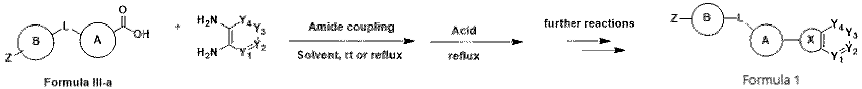

### Example 40: Synthesis of 4-(1H-benzo[d]imidazol-2-yl)-2-((5-(3-fluorophenyl)pyrimidin-2-yl)amino)phenol

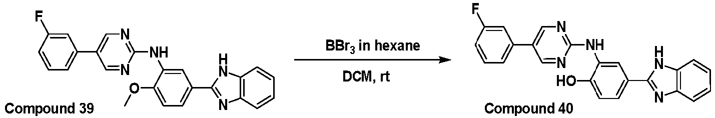

N-(5-(1H-benzo[d]imidazol-2-yl)-2-methoxyphenyl)-5-(3-fluorophenyl)pyrimidin-2-amine (41 mg, 0.10 mmol) prepared in Example 39 above was dissolved in dichloromethane, and BBr₃ in hexane (1M, 0.2 mL, 0.2 mmol) was added at 0°C. Then, the mixture was stirred at room temperature for 8 hours. After confirming the completion of the reaction through LC-MS, the residue was slurried with dichloromethane and filtered to obtain the title compound (21 mg, 9%) as a beige solid.

### Example 93: Synthesis of 3-(6-(3-methyl-1,2,4-oxadiazol-5-yl)-1H-benzo[d]imidazol-2-yl)-N-(4-(pyridazin-3-yl)phenyl)aniline

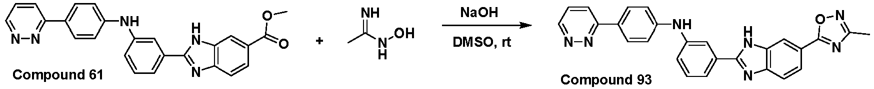

To a solution of methyl 2-(3-((4-(pyridazin-3-yl)phenyl)amino)phenyl)imidazole-6-carboxylate (102 mg, 0.24 mmol) prepared in Example 61 and N-hydroxyacetamidine (24 mg, 0.32 mmol) in DMSO (0.5 mL) was added NaOH (16 mg, 0.45 mmol), the reaction mixture was stirred at room temperature for 49 hours. After confirming the completion of the reaction through LC-MS, the resulting mixture was washed with brine. After extraction with 10% MeOH in DCM solution, the precipitated solid was once again slurried with ethyl acetate and filtered to obtain the title compound (16 mg, 22%) as a beige solid.

### Method F. Preparation by other reactions

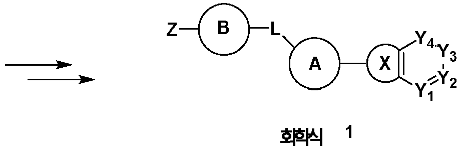

### Example 122: Synthesis of 2-(3-((4-(pyridazin-3-yl)phenyl)sulfonyl)phenyl)-6-(trifluoromethyl)-1H-benzo[d]imidazole

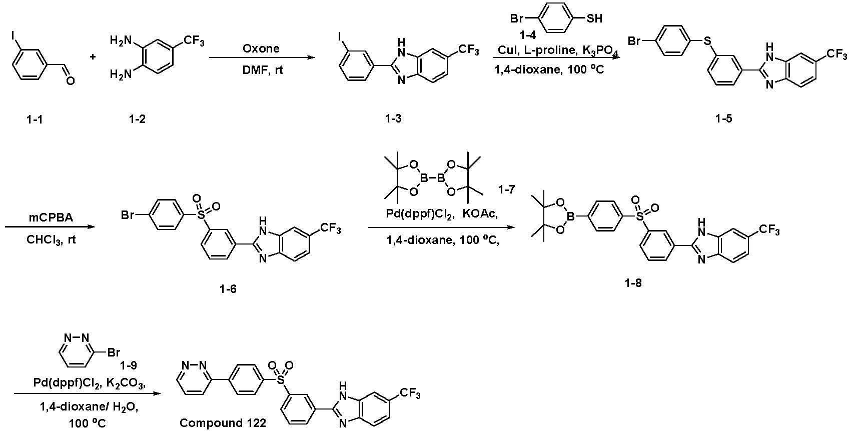

### Step 1: Synthesis of 2-(3-iodophenyl)-6-(trifluoromethyl)-1H-benzo[d]imidazole

To a solution of 3-iodobenzaldehyde (1-1, 3 g, 12.9 mmol) in DMF (20 mL) was added 4-(trifluoromethyl)benzene-1,2-diamine (1-2, 2.06 g, 11.7 mmol) and oxone (5.03 g, 8.19 mmol). After the mixture was stirred at room temperature for 2 hours, the solvent was removed in vacuo and the residue was purified by column chromatography on silica gel (PE : EtOAc = 10 : 1) to obtain the title compound **(1-3,** 3.7 g, 81%) as a white solid.

¹H-NMR (400 MHz, DMSO-*d*₆) δ: 13.45 (s, 1H), 8.59 (s, 1H), 8.24 (d, *J* = 7.6 Hz, 1H), 8.05-7.75 (m, 3H), 7.56 (d, *J* = 6.4 Hz, 1H), 7.40 (t, *J* = 8.0 Hz, 1H).

¹⁹F-NMR (377 MHz, DMSO-*d*₆) : δ -58.91.

### Step 2: Synthesis of 2-(3-((4-bromophenyl)thio)phenyl)-6-(trifluoromethyl) -1H-benzo[d]imidazole

A mixture of 2-(3-iodophenyl)-6-(trifluoromethyl)-1*H-*benzo[d]imidazole (**1-3, 1** g, 2.57 mmol), 4-bromobenzenethiol (**1-4,** 400 mg, 2.14 mmol), CuI (408 mg, 2.14 mmol), L-proline (246 mg, 2.14 mmol) and K₃PO₄ (1.36 g, 6.42 mmol) in 1,4-dioxane (10 mL) was degassed and backfilled with N₂ (three times). The mixture was heated to 100°C and stirred at this temperature overnight. After cooling down, the reaction mixture was filtered and the filtrate was concentrated to obtain the crude product, which was purified by column chromatography on silica gel (PE : EtOAc = 4 : 1) to obtain the title compound **(1-5,** 1.02 g, 88%) as a white solid.

MS: m/z = 449 (M+1, ESI+).

### Step 3: Synthesis of 2-(3-((4-bromophenyl)sulfonyl)phenyl)-6-(trifluoromethyl)-1H-benzo[d] imidazole

To a solution of 2-(3-((4-bromophenyl)thio)phenyl)-6-(trifluoromethyl)-1*H*-benzo[*d*]imidazole (**1-5,** 800 mg, 1.78 mmol) in CHCl₃ (20 mL) was added mCPBA (645 mg, 3.75 mmol), and the mixture was stirred at room temperature overnight. The solvent was removed in vacuo and the residue was purified by column chromatography on silica gel (PE : EtOAc = 4 : 1) to obtain the title compound **(1-6,** 652 mg, 76%) as a white solid.

¹H-NMR (400 MHz, DMSO-*d*₆) δ 13.68 (s, 1H), 8.81 (s, 1H), 8.52 (d, *J* = 8.0 Hz, 1H), 8.14 (d, *J* = 8.0 Hz, 1H), 8.10 (s, 0.5H), 7.98 (d, *J* = 8.4 Hz, 2H), 7.90-7.85 (m, 4H), 7.79 (d, J = 8.0 Hz, 0.5H), 7.60-7.55 (m, 1H).

¹⁹F-NMR (377 MHz, DMSO-*d*₆): δ -58.94, -59.01.

### Step 4: Synthesis of 2-(3-((4-(4,4,5,5-tetramethyl-1,3,2-dioxaborolan-2-yl)phenyl)sulfonyl)phenyl)-6-(trifluoromethyl)-1H-benzo-[d]imidazole

A mixture of 2-(3-((4-bromophenyl)sulfonyl)phenyl)-6-(trifluoromethyl)-1H-benzo[d]imidazole **(1-6,** 350 mg, 0.73 mmol), 4,4,4',4',5,5,5',5'-octamethyl-2,2'-bi(1,3,2-dioxaborolane) **(1-7,** 278 mg, 1.1 mmol), Pd(dppf)Cl₂ (22 mg, 0.03 mmol) and KOAc (214 mg, 2.19 mmol) in 1,4-dioxane (10 mL) was degassed and backfilled with N₂ (three times). The mixture was heated to 100°C overnight. LCMS showed that the reaction was completed. After cooling down, the product was used in the next directly.

MS: m/z = 529 (M+1, ESI+).

### Step 5: Synthesis of 2-(3-((4-(pyridazin-3-yl)phenyl)sulfonyl)phenyl)-6-(trifluoromethyl)-1H-benzo[d]imidazole

To a solution of 2-(3-((4-(4,4,5,5-tetramethyl-1,3,2-dioxaborolan-2-yl)phenyl)sulfonyl)phenyl)-6-(trifluoromethyl)-1H-benzo[d]imidazole in 1,4-dioxane (10 mL) **(1-8,** 0.73 mmol) which was prepared in the previous step was added 3-bromopyridazine **(1-9,** 139 mg, 0.87 mmol), H₂O (1 mL), K₂CO₃ (302 mg, 2.19 mmol) and Pd(dppf)Cl₂ (22 mg, 0.03 mmol). The mixture was degassed and backfilled with N₂ (three times), and then heated to 100°C overnight. After cooling down, the reaction mixture was filtered and the filtrate was concentrated to obtain the crude product, which was purified by column chromatography on silica gel (PE : EtOAc = 1 : 2) to obtain the title compound (150 mg, 42%) as a white solid.

### Example 123: Synthesis of N-(3-(1H-benzo[d]imidazol-2-yl)phenyl)-5-(pyridazin-3-yl)-1,3,4-oxadizol-2-amine

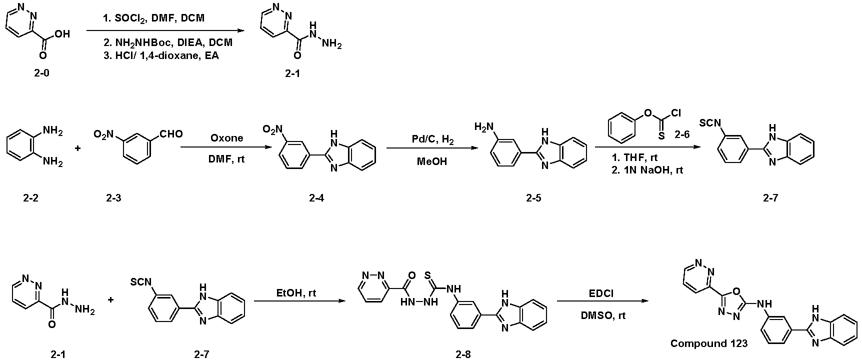

### Step 1: Synthesis of pyridazine-3-carbohydrazide

Pyridazine-3-carboxylic acid **(2-0)** (1.0 g, 8 mmol) was dissolved in DCM (20 mL) at 0°C. SOCl₂ (1.2 g, 9.6 mmol) and a drop of DMF was added to the solution. The mixture was stirred at room temperature for 2 hours. The solvent was removed in vacuo. The residue was dissolved in DCM (20 mL), then NH₂NHBoc (1.2 g, 8.8 mmol) and DIEA (2 g, 16 mmol) were added. The mixture was stirred at room temperature for 3 hours and then concentrated in vacuo. The residue was partitioned between ethyl acetate (100 mL) and aqueous NaHCO₃ (50 mL). The aqueous phase was extracted with ethyl acetate, and the combined organic extracts were washed with brine, dried over Na₂SO₄, filtered and concentrated in vacuo. The crude product was then dissolved in ethyl acetate (10 mL). HCl/1,4-dioxane (4 mol/L, 10 mL) was added and the mixture was stirred at room temperature for 2 hours. The solvent was removed under reduced pressure. The residue was partitioned between ethyl acetate (100 mL) and aqueous NaHCO₃ (100 mL). The aqueous phase was extracted with ethyl acetate and the combined organic extracts were washed with brine, dried over Na₂SO₄, filtered and concentrated in vacuo. The residue was purified by silica-gel column chromatography (DCM : MeOH = 20 : 1) to obtain the title compound (2-1, 790 mg, 71%) as a white solid.

MS: m/z = 139 (M+1, ESI+).

### Step 2: Synthesis of 2-(3-nitrophenyl)-1H-benzo[d]imidazole

Benzene-1,2-diamine (**2-2,** 3.2 g, 30 mmol) was dissolved in DMF (30 mL). 3-nitrobenzaldehyde (**2-3,** 5 g, 33 mmol) was added followed by Oxone (12.8 g, 21 mmol). The mixture was stirred at room temperature for 2 hours after which HPLC analysis indicated completion of the reaction. The reaction was quenched by aqueous K₂CO₃ solution (1 M, 10 mL), followed by dropwise addition of H₂O (120 mL) to the mixture with vigorous stirring. The resulting precipitated was collected by filtration, washed with H₂O and dried to obtain the title compound **(2-4,** 7.5 g, crude), which was used for the next step without further purification.

MS: m/z = 240 (M+1, ESI+).

### Step 3: Synthesis of 3-(1H-benzo[d]imidazol-2-yl)aniline

A mixture of 2-(3-nitrophenyl)-1H-benzo[d]imidazole (**2-4,** 7.5 g, crude) and 750 mg of Pd/C in MeOH (100 mL) was stirred at room temperature under H₂ atmosphere for 16 hours. Then, the reaction mixture was filtered through celite which was subsequently washed with MeOH. The combined filtrate was concentrated, and the residue was purified by silica-gel column chromatography (DCM : MeOH = 60 : 1) to obtain the title compound (2-5, 3.8 g, 61%) as a yellow solid.

MS: m/z = 210 (M+1, ESI+)

### Step 4: Synthesis of 2-(3-isothiocyanatophenyl)-1H-benzo[d]imidazole

To a solution of 3-(1H-benzo[d]imidazol-2-yl)aniline (**2-5,** 0.7 g, 3.3 mmol) in THF (10 mL) was added O-phenyl carbonochloridothioate (**2-6,** 633 mg, 3.7 mmol). The resulting mixture was stirred at room temperature under N₂ atmosphere for 2 hours and then 1N NaOH solution (10 mL) was added. The resulting mixture was stirred at room temperature under N₂ atmosphere for 16 hours, then diluted with H₂O, extracted with ethyl acetate. The combined organic layers were washed with brine, dried over Na₂SO₄, filtered and concentrated under reduced pressure. The residue was purified by silica-gel column chromatography (PE : EtOAc = 3 : 1) to obtain the title compound (**2-7,** 180 mg, 22%) as a yellow solid.

MS: m/z = 252 (M+1, ESI+).

### Step 5: Synthesis of N-(3-(1H-benzo[d]imidazol-2-yl)phenyl)-2-(pyridazine-3-carbonyl)hydrazine-1-carbothioamide

A mixture of 2-(3-isothiocyanatophenyl)-1H-benzo[d]imidazole (2-7, 180 mg, 0.7 mmol) and pyridazine-3-carbohydrazide (2-1, 97 mg, 0.7 mmol) in EtOH (30 mL) was stirred at room temperature for 16 hours under N₂ atmosphere. The resulting precipitate was collected by filtration, washed with EtOH and dried. The residue was purified by Prep-HPLC to obtain the title compound (**2-8**, 190 mg, 70%) as a yellow solid.

MS: m/z = 390 (M+1, ESI+).

### Step 6: Synthesis of N-(3-(1H-benzo[d]imidazol-2-yl)phenyl)-5-(pyridazin-3-yl)-1,3,4-oxadiazol-2-amine

To a solution of *N*-(3-(1*H*-benzo[d]imidazol-2-yl)phenyl)-2-(pyridazine-3-carbonyl)hydrazine-1-carbothioamide (**2-8,** 100 mg, 0.25 mmol) in DMSO (3 mL) was added EDCI (72 mg, 0.37 mmol). The resulting mixture was stirred at room temperature under N₂ atmosphere for 16 hours. The reaction was diluted with H₂O, extracted with ethyl acetate. The combined organic layers were washed with brine, dried over Na₂SO₄, filtered and concentrated under reduced pressure. The residue was purified by Prep-HPLC to obtain the title compound (50 mg, 56%) as a white solid.

### Example 124: Synthesis of N-(4-(pyridazin-3-yl)cyclohexyl)-3-(6-(trifluoromethyl)-1H-benzo[d]imidazol-2-yl)aniline

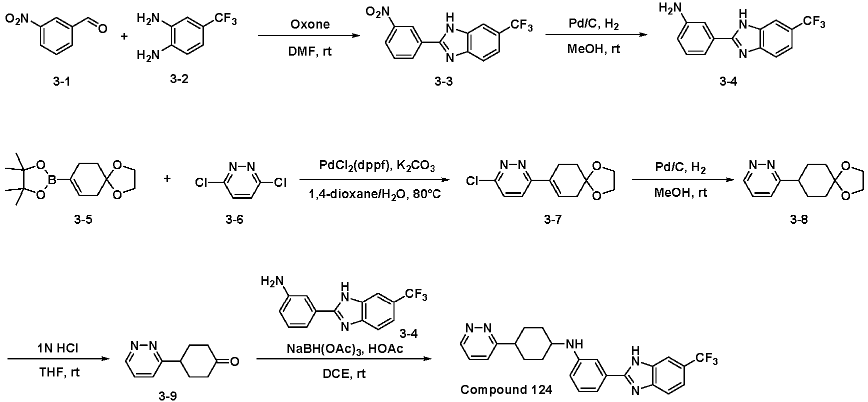

### Step 1: Synthesis of 2-(3-nitrophenyl)-6-(trifluoromethyl) -1H-benzo[d]imidazole

To a solution of 4-(trifluoromethyl)benzene-1,2-diamine (**3-2, 821** mg, 4.7 mmol) was dissolved in DMF (10 mL), was added 3-nitrobenzaldehyde (**3-1,** 774 mg, 5.1 mmol) followed by Oxone (2.0 g, 3.3 mmol). The mixture was stirred at room temperature for 2 hours after which LCMS analysis indicated completion of the reaction. The reaction was quenched by aqueous K₂CO₃ solution (1 M, 25 mL), followed by dropwise addition of H₂O (60 mL) to the mixture with vigorous stirring. The resulting precipitated was collected by filtration, washed with H₂O and dried to obtain the title compound **(3-3,** 1.5 g, crude) was obtained, which was used for the next step without further purification.

MS: m/z = 308 (M+1, ESI+).

### Step 2: Synthesis of 3-(6-(trifluoromethyl)-1H-benzo[d]imidazol-2-yl)aniline

A mixture of 2-(3-nitrophenyl)-6-(trifluoromethyl)-1H-benzo[d]imidazole **(3-3,** 1.5 g, crude) and 150 mg of Pd/C in MeOH (30 mL) was stirred at room temperature under H₂ atmosphere for 2 hours. Then, the reaction mixture was filtered through celite which was subsequently washed with MeOH. The combined filtrate was concentrated, and the residue was purified by silica-gel column chromatography (DCM : MeOH = 60 : 1) to obtain the title compound **(3-4,** 410 mg, 31%) as a brown solid.

MS: m/z = 278 (M+1, ESI+).

### Step 3: Synthesis of 3-chloro-6-(1,4-dioxaspiro[4.5]dec-7-en-8-yl)pyridazine

A flamed-dried flask was charged with PdCl₂(dppf) (250 mg, 0.3 mmol), 4,4,5,5-tetramethyl-2-(1,4-dioxaspiro[4.5]dec-7-en-8-yl)-1,3,2-dioxaborolane **(3-5,** 2.1 g, 8.0 mmol), 3,6-dichloropyridazine **(3-6,** 1.0 g, 6.7 mmol), and K₂CO₃ (2.8 g, 20.1 mmol). The flask was evacuated and backfilled with N₂. 1,4-dioxane/H₂O (3:1, 30 mL) was added via syringe. The reaction mixture was heated at 80°C for 16 hours. The mixture was allowed to cool to room temperature after the starting material was fully consumed as determined by monitoring with TLC. The reaction mixture was diluted with H₂O and extracted with EtOAc. The organic layer was dried over Na₂SO₄, filtered, and concentrated in vacuo. The crude residue was purified by column chromatography on silica gel, eluting with (PE: EtOAc = 2:1) to obtain the title compound **(3-7,** 1.2 g, 71%) as an off-white solid.

MS: m/z = 253 (M+1, ESI+).

### Step 4: Synthesis of 3-(1,4-dioxaspiro[4.5]decan-8-yl)pyridazine

A mixture of 3-chloro-6-(1,4-dioxaspiro[4.5]dec-7-en-8-yl)pyridazine **(3-7,** 1.2 g, 4.7 mmol ) and 200 mg of Pd/C in MeOH (20 mL) was stirred at room temperature under H₂ atmosphere for 16 hours. Then the reaction mixture was filtered through celite which was subsequently washed with MeOH. The combined filtrates were concentrated to obtain the title compound **(3-8,** 0.95 g, crude) as a yellow solid, which was used for the next step without further purification.

MS: m/z = 221 (M+1, ESI+).

### Step 5: Synthesis of 4-(pyridazin-3-yl)cyclohexanone

To a stirred solution of 3-(1,4-dioxaspiro[4.5]decan-8-yl)pyridazine **(3-8,** 0.95 g, crude) in THF (20 mL) was added 1N-HCl (aq) (10 mL) at room temperature. The resulting mixture was stirred at room temperature for 2 hours. After the disappearance of starting material was confirmed by TLC, the reaction mixture was extracted with ethyl acetate three times. The combined organic layers were washed with brine, dried over MgSO₄, filtered and concentrated. The residue was purified by silica-gel column chromatography (DCM : MeOH = 60 : 1) to obtain the title compound **(3-9,** 480 mg, 57%) as a white solid.

MS: m/z = 177 (M+1, ESI+).

### Step 6: Synthesis of N-(4-(pyridazin-3-yl)cyclohexyl)-3-(6-(trifluoromethyl)-1H-benzo[d]imidazol-2-yl)aniline

To a solution of 3-(6-(trifluoromethyl)-1*H-*benzo[*d*]imidazol-2-yl)aniline (3-4, 150 mg, 0.54 mmol), 4-(pyridazin-3-yl)cyclohexanone **(3-9,** 95 mg, 0.54 mmol), and HOAc (65 mg, 1.1 mmol) in DCE (10 mL) was added sodium triacetoxyborohydride (229 mg, 1.1 mmol). The resulting mixture was stirred at room temperature under N₂ atmosphere for 16 hours. The reaction was quenched by aqueous NaHCO₃ solution, and extracted with ethyl acetate. The combined organic layers were washed with brine, dried over Na₂SO₄, filtered, and concentrated under reduced pressure. The residue was purified by Prep-HPLC to obtain the title compound (78 mg, mixture of two isomers, 31%) as a white solid.

¹⁹F NMR (376 MHz, DMSO-*d*₆) : δ -58.78.

### Example 125: Synthesis of 1-(pyridazin-3-yl)-N-(3-(6-(trifluoromethyl)-1H-benzo[d]imidazol-2-yl)phenyl)piperidin-4-amine

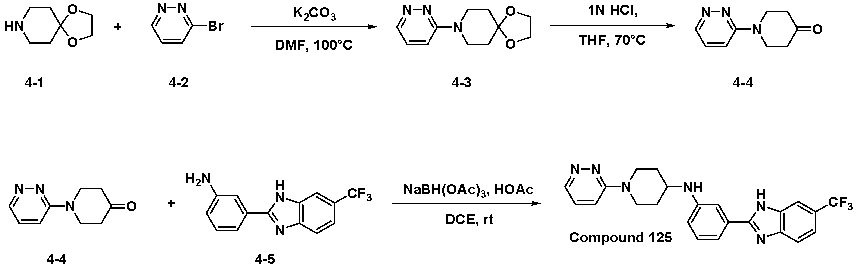

### Step 1: Synthesis of 8-(pyridazin-3-yl)-1,4-dioxa-8-azaspiro[4.5]decane

A mixture of 1,4-dioxa-8-azaspiro[4.5]decane **(4-1,** 1.4 g, 9.8 mmol), 3-bromopyridazine **(4-2,** 2.3 g, 14.7 mmol) and K₂CO₃ (2.7 g, 19.6 mmol) in 30 mL of DMF was stirred at 100 °C for 16 hours and monitored by TLC. After the reaction was complete, the mixture was cooled down, and 100 mL of water was added. The mixture was extracted with ethyl acetate. The organic layers were washed with brine, dried over Na₂SO₄, filtered, and concentrated under reduced pressure to obtain the title compound **(4-3,** 2.5 g, crude) which was used without further purification.

MS: m/z = 222 (M+1, ESI+).

### Step 2: Synthesis of 1-(pyridazin-3-yl)piperidin-4-one

To a stirred solution of 8-(pyridazin-3-yl)-1,4-dioxa-8-azaspiro[4.5]decane **(4-3,** 2.5 g, crude) in THF (20 mL) was added 1N-HCl (aq) (10 mL) at room temperature. The resulting mixture was stirred at 70°C for 2 hours. After the reaction was complete confirming by TLC, the mixture was extracted with ethyl acetate three times. The combined organic layers were washed with brine, dried over Na₂SO₄, filtered and concentrated. The residue was purified by silica-gel column chromatography (DCM : MeOH = 60 : 1) to obtain the title compound **(4-4,** 490 mg, 27%) as a brown oil.

MS: m/z = 178 (M+1, ESI+).

### Step 3: Synthesis of 1-(pyridazin-3-yl)-N-(3-(6-(trifluoromethyl)-1H-benzo[d]imidazol-2-yl)phenyl)piperidin-4-amine

To a solution of 1-(pyridazin-3-yl)piperidin-4-one **(4-4,** 100 mg, 0.56 mmol), 3-(6-(trifluoromethyl)-1H-benzo[d]imidazol-2-yl)aniline (4-5, 155 mg, 0.56 mmol), and HOAc (67 mg, 1.1 mmol) in DCE (10 mL) was added sodium triacetoxyborohydride (239 mg, 1.1 mmol). The resulting mixture was stirred at room temperature under N₂ atmosphere for 16 hours. The reaction was quenched by aqueous NaHCO₃ solution, and extracted with ethyl acetate. The combined organic layers were washed with brine, dried over Na₂SO₄, filtered, and concentrated under reduced pressure. The residue was purified by Prep-HPLC to obtain the title compound (77 mg, 31%) as a white solid.

¹⁹F NMR (376 MHz, DMSO-*d*₆) : δ -58.81.

### Example 312: Synthesis of 5-(5-((dimethylamino)methyl)pyridin-2-yl)-N-(3-(6-(trifluoromethyl)-1H-benzo[d]imidazol-2-yl)phenyl)pyrimidin-2-amine

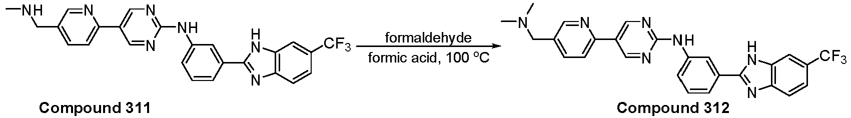

A mixture of 5-(5-((methylamino)methyl)pyridin-2-yl)-*N*-(3-(6-(trifluoromethyl)-1*H*-benzo[*d*] imidazol-2-yl)phenyl)pyrimidin-2-amine (100 mg, 0.17 mmol) prepared in Example 311 in formic acid (5 mL) was added formaldehyde (5 mL), the reaction mixture was stirred at 100°C for 4 hours. The reaction mixture was cooled to room temperature and concentrated under reduced pressure, the residue was purified by reversed-column chromatography to obtaini the title compound (17 mg, 13.82%) as a white solid.

### Example 317: Synthesis of 6-(piperidin-4-yl)-N-(3-(6-(trifluoromethyl)-1H-benzo[d]imidazol-2-yl)phenyl)pyridazin-3-amine

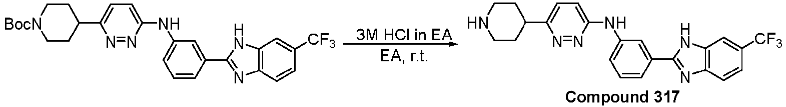

A mixture of tert-butyl 4-(6-((3-(6-(trifluoromethyl)-1H-benzo[d]imidazol-2-yl)phenyl)amino)pyridazin-3-yl)piperidine-1-carboxylate (200 mg, 0.37 mmol) in EA (8 mL) was added 3M-HCl in ethylacetate (4 mL), the reaction mixture was stirred at room temperature for 4 hours. The reaction mixture was concentrated under reduced pressure, the residue was purified by reversed-column chromatography to obtain the title compound (23 mg, 8.07%) as a white solid.

Compounds other than those according to the Examples described above were prepared using Methods A to F as described above.

Chemical structural formulas, NMR and LC-MS analysis results of the compounds of Examples 1 to 336 are summarized in Table 2 below.

**[Table 2]**

| **Ex. Nos.** | **Structural Formula** | **¹H NMR; ¹⁹F NMR** | **LCMS [m/z]** | **Method** |
|---|---|---|---|---|
| **1** | 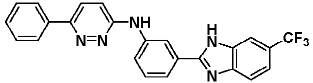 | ¹H NMR (400 MHz, DMSO-*d₆*) δ 13.35 (s, 1H), 9.67 (s, 1H), 8.81 (s, 1H), 8.11-8.05 (m, 4H), 7.96-7.85 (m, 1H), 7.81-7.74 (m, 1H), 7.67-7.61 (m, 1H), 7.61-7.51 (m, 4H), 7.50-7.44 (m, 1H), 7.32-7.28 (m, 1H). | 432 | **A** |
| **2** | 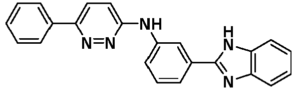 | ¹H NMR (400 MHz, DMSO-*d₆*) δ 12.91 (s, 1H), 9.64 (s, 1H), 8.77 (s, 1H), 8.17-8.04 (m, 3H), 7.90-7.85 (m, 1H), 7.77-7.69 (m, 2H), 7.60-7.51 (m, 4H), 7.49-7.43 (m, 1H), 7.30 (d, *J =* 9.3 Hz, 1H), 7.26-7.18 (m, 2H). | 364 | **A** |
| **3** | 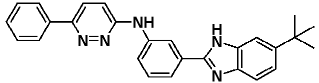 | ¹H NMR (400 MHz, DMSO-*d₆*) δ 12.79 (s, 1H), 9.62 (s, 1H), 8.76 (s, 1H), 8.20-7.97 (m, 3H), 7.84 (d, *J* = 7.8 Hz, 1H), 7.73 (d, *J =* 7.8 Hz, 1H), 7.63-7.40 (m, 6H), 7.37-7.26 (m, 2H), 1.38 (s, 9H). | 420 | **A** |
| **4** | 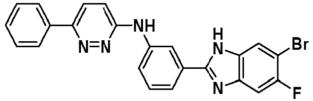 | ¹H NMR (400 MHz, DMSO-*d₆*) δ 13.23 (s, 1H), 9.66 (s, 1H), 8.78 (s, 1H), 8.10-8.08 (m, 3H), 7.90-7.72 (m, 3H), 7.56-7.47 (m, 5H), 7.29 (d, *J =* 9.2 Hz, 1H). | 460 | **A** |
| **5** | 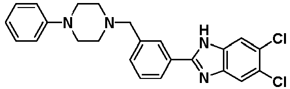 | ¹H NMR (400 MHz, DMSO-*d₆*) δ 13.31 (s, 1H), 8.17 (s, 1H), 8.08 (d, *J* = 7.2 Hz, 1H), 7.86 (s, 2H), 7.57-7.49 (m, 2H), 7.22-7.18 (m, 2H), 6.93-6.91 (m, 2H), 6.77 (t, *J* = 3.6 Hz, 1H), 3.62 (s, 2H), 3.15 (s, 4H), 2.56 (s, 4H). | 437 | **A** |
| **6** | 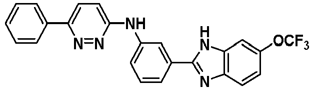 | ¹H NMR (400 MHz, DMSO-*d₆*) δ 13.23 (s, 1H), 9.68 (s, 1H), 8.81 (s, 1H), 8.09-8.08 (m, 3H), 7.88 (t, *J* = 7.6 Hz, 1H), 7.82-7.72 (m, 2H), 7.64-7.45 (m, 5H), 7.29 (d, *J =* 9.6 Hz, 1H), 7.25-7.20 (m, 1H). | 448 | **A** |
| **7** | 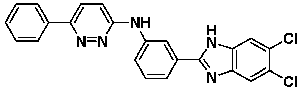 | ¹H NMR (400 MHz, DMSO-*d₆*) δ 13.29 (s, 1H), 9.68 (s, 1H), 8.81 (dd, *J =* 1.8, 1.8 Hz, 1H), 8.18-8.06 (m, 3H), 8.06-7.65 (m, 4H), 7.65-7.50 (m, 3H), 7.49-7.45 (m, 1H), 7.29 (d, *J =* 9.4 Hz, 1H). | 432 | **A** |
| **8** | 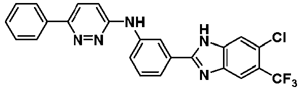 | ¹H NMR (400 MHz, DMSO-*d₆*) δ 13.55 (s, 1H), 9.71 (s, 1H), 8.85 (s, 1H), 8.22-7.76 (m, 7H), 7.59-7.49 (m, 3H), 7.47-7.45 (m, 1H), 7.29 (d, *J =* 9.2 Hz, 1H). | 466 | **A** |
| **9** | 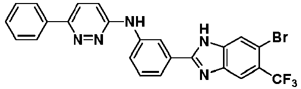 | ¹H NMR (400 MHz, DMSO-*d₆*) δ 13.52 (s, 1H), 9.70 (s, 1H), 8.84 (s, 1H), 8.27-8.05 (m, 5H), 7.93-7.91 (m, 1H), 7.77 (d, *J =* 8.1 Hz, 1H), 7.59-7.52 (m, 3H), 7.49-7.45 (m, 1H), 7.29 (d, *J =* 9.4 Hz, 1H). | 511 | **A** |
| **10** | 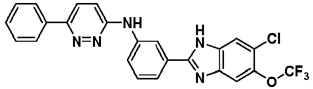 | ¹H NMR (400 MHz, DMSO-*d₆*) δ 13.38 (s, 1H), 9.68 (s, 1H), 8.81 (s, 1H), 8.11-8.08 (m, 3H), 7.90-7.73 (m, 4H), 7.58-7.51 (m, 3H), 7.47 (t, *J =* 7.3 Hz, 1H), 7.29 (d, *J =* 9.4 Hz, 1H). | 482 | **A** |
| **11** | 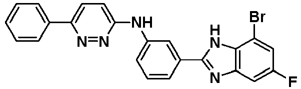 | ¹H NMR (400 MHz, DMSO-*d₆*) δ 13.41 (s, 1H), 9.73 (s, 1H), 8.55 (s, 1H), 8.18-8.16 (m, 1H), 8.10-8.07 (m, 3H), 7.74-7.72 (m, 1H), 7.64-7.52 (m, 3H), 7.49-7.40 (m, 3H), 7.29 (d, *J =* 9.3 Hz, 1H). | 462 | **A** |
| **12** | 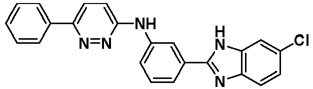 | ¹H NMR (400 MHz, DMSO-*d₆*) δ 13.12 (s, 1H), 9.66 (s, 1H), 8.78 (s, 1H), 8.24-7.97 (m, 3H), 7.90-7.85 (m, 1H), 7.75-7.73 (m, 1H), 7.71-7.58 (m, 1H), 7.58-7.41 (m, 5H), 7.41-7.16 (m, 2H). | 398 | **A** |
| **13** | 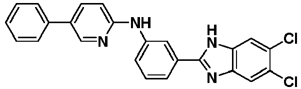 | ¹H NMR (400 MHz, DMSO-*d₆*) δ 13.24 (s, 1H), 9.47 (s, 1H), 8.60-8.48 (m, 2H), 8.01-7.72 (m, 4H), 7.72-7.61 (m, 3H), 7.53-7.42 (m, 3H), 7.34 (t, *J =* 7.4 Hz, 1H), 6.99 (d, *J* = 8.6 Hz, 1H). | 431 | **A** |
| **14** | 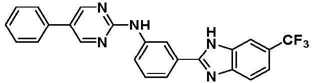 | ¹H NMR (400 MHz, DMSO-*d₆*) δ 13.35 (s, 1H), 10.06 (s, 1H), 8.91 (s, 2H), 8.71 (s, 1H), 8.05-7.93 (m, 2H), 7.92-7.64 (m, 4H), 7.64-7.45 (m, 4H), 7.40 (t, *J =* 7.4 Hz, 1H). | 432 | **A** |
| **15** | 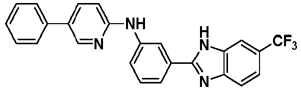 | ¹H NMR (400 MHz, DMSO-*d₆*) δ 13.33 (s, 1H), 9.48 (s, 1H), 8.57-8.51 (d, *J* = 2.4 Hz, 2H), 7.98-7.90 (m, 3H), 7.82-7.78 (m, 1H), 7.74-7.64 (m, 3H), 7.61-7.42 (m, 4H), 7.34 (t, *J =* 7.4 Hz, 1H), 7.00 (d, *J =* 8.6 Hz, 1H). | 431 | **B** |
| **16** | 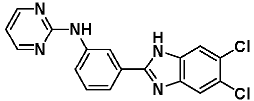 | ¹H NMR (400 MHz, DMSO-*d₆*) δ 13.22 (s, 1H), 9.86 (s, 1H), 8.62 (s, 1H), 8.59-8.41 (m, 2H), 7.96-7.76 (m, 3H), 7.71 (d, *J =* 7.8 Hz, 1H), 7.51-7.45 (t, *J =* 8.0 Hz, 1H), 6.90 (t, *J* = 4.8 Hz, 1H). | 356 | **A** |
| **17** | 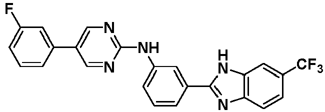 | ¹H NMR (400 MHz, DMSO-*d₆*) δ 13.34 (s, 1H), 10.11 (s, 1H), 8.96 (s, 2H), 8.72 (s, 1H), 8.38-7.90 (m, 2H), 7.85-7.72 (m, 2H), 7.72-7.60 (m, 2H), 7.60-7.47 (m, 3H), 7.25-7.18 (t, *J =* 2.4 Hz, 1H). | 450 | **A** |
| **18** | 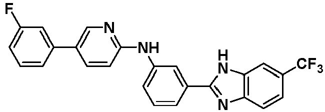 | ¹H NMR (400 MHz, DMSO-*d₆*) δ 13.35 (s, 1H), 9.53 (s, 1H), 8.63 (s, 1H), 8.59 (s, 1H), 8.09-7.95 (m, 3H), 7.78 (s, 1H), 7.72-7.70 (m, 1H), 7.70-7.44 (m, 5H), 7.19-7.13 (m, 1H), 7.00 (dd, *J =* 2.1, 8.7 Hz, 1H). | 449 | **A** |
| **19** | 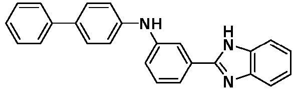 | ¹H NMR (400 MHz, DMSO-*d₆*) δ 12.90 (s, 1H), 8.56 (s, 1H), 8.02 (s, 1H), 7.93-7.50 (m, 8H), 7.49-7.37 (m, 2H), 7.37-7.07 (m, 6H). | 362 | **A** |
| **20** | 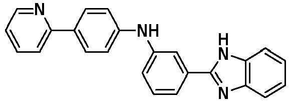 | ¹H NMR (400 MHz, DMSO-*d₆*) δ 12.92 (s, 1H), 8.67 (s, 1H), 8.62 (dd, *J =* 0.9, 3.9 Hz, 1H), 8.06-8.04 (m, 3H), 7.89 (d, *J* = 8.1 Hz, 1H), 7.86-7.80 (m, 1H), 7.77-7.50 (m, 3H), 7.46 (t, *J =* 7.9 Hz, 1H), 7.34-7.11 (m, 6H). | 363 | **A** |
| **21** | 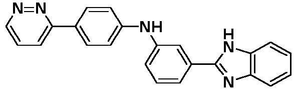 | ¹H NMR (400 MHz, DMSO-*d₆*) δ 12.91 (s, 1H), 9.11 (s, 1H), 8.79 (s, 1H), 8.17-8.12 (m, 3H), 8.08-8.07 (m, 1H), 7.74-7.70 (m, 3H), 7.66-7.46 (m, 2H), 7.31-7.26 (m, 3H), 7.21-7.05 (m, 2H). | 364 | **A** |
| **22** | 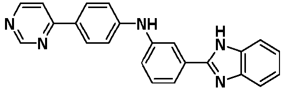 | ¹H NMR (400 MHz, DMSO-*d₆*) δ 12.92 (s, 1H), 9.14 (s, 1H), 8.91 (s, 1H), 8.75 (d, *J =* 5.5 Hz, 1H), 8.18 (d, *J* = 8.8 Hz, 2H), 8.08 (s, 1H), 7.99-7.97 (m, 1H), 7.74 (d, *J* = 8.0 Hz, 1H), 7.65-7.51 (m, 2H), 7.49 (t, *J =* 8.0 Hz, 1H), 7.33-7.14 (m, 5H). | 364 | **A** |
| **23** | 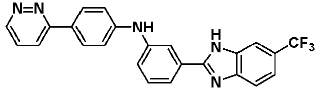 | ¹H NMR (400 MHz, DMSO-*d₆*) δ 13.37 (s, 1H), 9.13 (dd, *J =* 1.5, 4.9 Hz, 1H), 8.84 (s, 1H), 8.20-8.04 (m, 4H), 7.96 (s, 1H), 7.88-7.66 (m, 3H), 7.60-7.45 (m, 2H), 7.40-7.26 (m, 3H). | 432 | **A** |
| **24** | 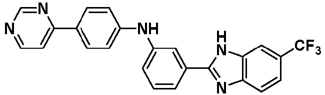 | ¹H NMR (400 MHz, DMSO-*d₆*) δ 13.37 (s, 1H), 9.15 (s, 1H), 8.95 (s, 1H), 8.75 (d, *J =* 5.5 Hz, 1H), 8.19 (d, *J* = 8.8 Hz, 2H), 8.11 (s, 1H), 8.07-7.87 (m, 2H), 7.87-7.67 (m, 2H), 7.58-7.44 (m, 2H), 7.34-7.32 (m, 1H), 7.27 (d, *J =* 8.8 Hz, 2H). | 432 | **A** |
| **25** | 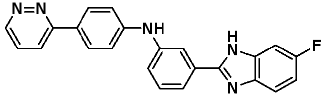 | ¹H NMR (400 MHz, DMSO-*d₆*) δ 13.04 (s, 1H), 9.12 (t, *J =* 4.6 Hz, 1H), 8.81 (s, 1H), 8.44-8.09 (m, 3H), 8.05 (d, *J =* 1.9 Hz, 1H), 8.00-7.54 (m, 2H), 7.50-7.19 (m, 6H), 7.11-7.04 (m, 1H). | 382 | **A** |
| **26** | 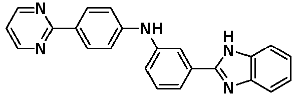 | ¹H NMR (400 MHz, DMSO-*d₆*) δ 12.91 (s, 1H), 8.90-8.75 (m, 3H), 8.33 (d, *J =* 8.8 Hz, 2H), 8.07 (s, 1H), 7.72-7.48 (m, 4H), 7.33 (t, *J =* 4.8 Hz, 1H), 7.30-7.14 (m, 5H). | 364 | **A** |
| **27** | 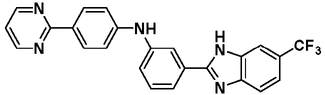 | ¹H NMR (400 MHz, DMSO-*d₆*) δ 13.36 (s, 1H), 8.86-8.83 (m, 3H), 8.34 (d, *J =* 8.8 Hz, 2H), 8.09 (dd, *J* = 2.0, 2.0 Hz, 1H), 7.96-7.60 (m, 3H), 7.60-7.39 (m, 2H), 7.37-7.30 (m, 2H), 7.26 (d, *J* = 8.8 Hz, 2H). | 432 | **A** |
| **28** | 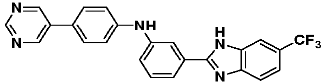 | ¹H NMR (400 MHz, DMSO-*d₆*) δ 13.37 (s, 1H), 9.22-9.10 (m, 3H), 8.75 (s, 1H), 8.07-8.03 (m, 2H), 7.95-7.71 (m, 4H), 7.55-7.65 (m, 2H), 7.64-7.27 (m, 3H). | 432 | **A** |
| **29** | 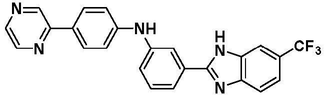 | ¹H NMR (400 MHz, DMSO-*d ₆*) δ 13.39-13.33 (m, 1H), 9.20 (s, 1H), 8.83 (s, 1H), 8.67-8.63 (m, 1H), 8.52-8.49 (m, 1H), 8.14-7.81 (m, 4H), 7.76-7.70 (m, 2H), 7.57-7.48 (m, 2H), 7.34-7.26 (m, 3H). | 432 | **A** |
| **30** | 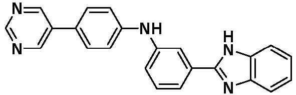 | ¹H NMR (400 MHz, DMSO-*d₆*) δ 12.90 (s, 1H), 9.20-9.08 (m, 3H), 8.71 (s, 1H), 8.05 (dd, *J* = 2.0, 2.0 Hz, 1H), 7.78 (d, *J* = 8.6 Hz, 2H), 7.72-7.61 (m, 2H), 7.54-7.51 (m, 1H), 7.48-7.44 (m, 1H), 7.29 (d, *J* = 8.6 Hz, 2H), 7.26-7.14 (m, 3H). | 364 | **A** |
| **31** | 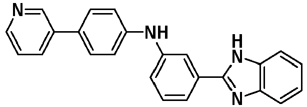 | ¹H NMR (400 MHz, DMSO-*d₆*) δ 12.89 (s, 1H), 8.89 (d, *J* = 1.8 Hz, 1H), 8.63 (s, 1H), 8.51 (dd, *J =* 1.6, 4.7 Hz, 1H), 8.07-8.03 (m, 2H), 7.72-7.63 (m, 4H), 7.53-7.42 (m, 3H), 7.30-7.25 (m, 2H), 7.22 (dd, *J =* 1.8, 7.8 Hz, 3H). | 363 | **A** |
| **32** | 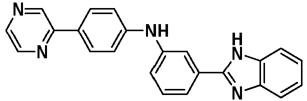 | ¹H NMR (400 MHz, DMSO-*d₆*) δ 12.90 (s, 1H), 9.20 (s, 1H), 8.79 (s, 1H), 8.65 (t, *J =* 2.0 Hz, 1H), 8.50 (d, *J* = 2.5 Hz, 1H), 8.14-8.08 (m, 2H), 8.07 (s, 1H), 7.72-7.70 (m, 1H), 7.67-7.66 (m, 1H), 7.54-7.52 (m, 1H), 7.50-7.46 (m, 1H), 7.29-7.16 (m, 5H). | 364 | **A** |
| **33** | 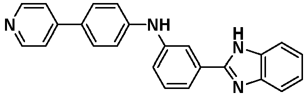 | ¹H NMR (400 MHz, DMSO-*d₆*) δ 12.90 (s, 1H), 8.74 (s, 1H), 8.58 (d, *J* = 1.2 Hz, 2H), 8.05 (s, 1H), 7.87-7.76 (m, 2H), 7.75-7.65 (m, 3H), 7.65-7.50 (m, 3H), 7.47 (dd, *J =* 7.9, 7.9 Hz, 2H), 7.33-7.10 (m, 3H). | 363 | **A** |
| **34** | 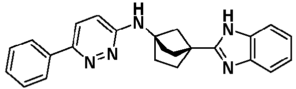 | ¹H NMR (400 MHz, DMSO-*d₆*) δ 12.28 (s, 1H), 7.99 (d, *J =* 7.3 Hz, 2H), 7.84-7.81 (m, 1H), 7.55 (d, *J* = 6.9 Hz, 1H), 7.48 (t, *J =* 7.4 Hz, 2H), 7.45-7.37 (m, 2H), 7.28 (s, 1H), 7.14 (t, *J =* 4.3 Hz, 2H), 6.96 (d, *J =* 1.2 Hz, 1H), 2.33 (s, 2H), 2.28 (d, *J* = 8.3 Hz, 4H), 1.95 (d, *J =* 7.8 Hz, 4H). | 382 | **A** |
| **35** | 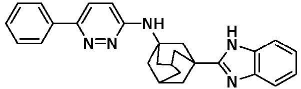 | ¹H NMR (400 MHz, DMSO-*d₆*) δ 12.13 (s, 1H), 7.98-7.95 (m, 2H), 7.78 (d, *J* = 9.6 Hz, 1H), 7.54-7.52 (m, 1H), 7.50-7.35 (m, 4H), 7.15-7.07 (m, 2H), 6.93 (d, *J* = 9.2 Hz, 1H), 6.71 (s, 1H), 2.56 (s, 2H), 2.32-2.14 (m, 4H), 2.14-2.12 (m, 2H), 2.05 (s, 4H), 1.82-1.72 (m, 2H). | 422 | **A** |
| **36** | 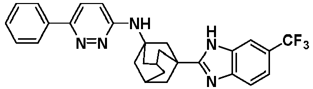 | ¹H NMR (400 MHz, DMSO-*d₆*) δ 12.62 (s, 1H), 7.97 (d, *J =* 8.0 Hz, 2H), 7.81-7.77 (m, 2H), 7.66 (s, 1H), 7.46 (t, *J* = 7.4 Hz, 3H), 7.39 (t, *J =* 7.3 Hz, 1H), 6.94 (d, *J =* 9.2 Hz, 1H), 6.73 (s, 1H), 2.57 (s, 2H), 2.37-2.30 (m, 4H), 2.15-2.05 (m, 6H), 1.83-1.77 (m, 2H). | 490 | **A** |
| **37** | 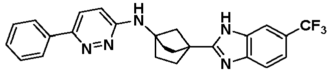 | ¹H NMR (400 MHz, DMSO-*d₆*) δ 12.77 (s, 1H), 8.00 (d, *J* = 7.9 Hz, 2H), 7.95-7.44 (m, 6H), 7.40 (t, *J* = 7.3 Hz, 1H), 7.29 (s, 1H), 6.96 (d, *J* = 9.4 Hz, 1H), 2.51 (s, 2H), 2.37-2.28 (m, 4H), 2.12-1.74 (m, 4H). | 450 | **A** |
| **38** | 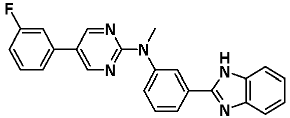 | ¹H NMR (400 MHz, DMSO-*d₆*) δ 13.00 (s, 1H), 8.83 (s, 2H), 8.19 (s, 1H), 8.06 (d, *J =* 7.8 Hz, 1H), 7.93-7.44 (m, 7H), 7.43-7.02 (m, 3H), 3.62 (s, 3H). | 396 | **A** |
| **39** | 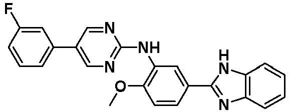 | ¹H NMR (400 MHz, DMSO-*d₆*) δ 12.77 (s, 1H), 8.92 (s, 2H), 8.84 (d, *J* = 1.8 Hz, 1H), 8.60 (s, 1H), 7.91-7.88 (m, 1H), 7.78-7.57 (m, 3H), 7.57-7.42 (m, 2H), 7.26 (d, *J =* 8.4 Hz, 1H), 7.23-7.05 (m, 3H), 3.95 (s, 3H). | 412 | **A** |
| **40** | 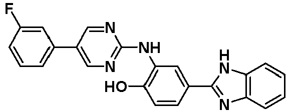 | ¹H NMR (400 MHz, DMSO-*d₆*) δ 14.90 (s, 1H), 11.26 (s, 1H), 8.91 (s, 2H), 8.80-8.75 (m, 2H), 7.88 (d, *J* = 6.4 Hz, 1H), 7.86-7.76 (m, 2H), 7.66-7.63 (m, 1H), 7.63-7.44 (m, 4H), 7.35-7.18 (m, 2H). | 398 | **E** |
| **41** | 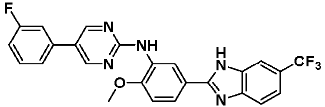 | ¹H NMR (400 MHz, DMSO-*d₆*) δ 13.24 (s, 1H), 8.92 (s, 2H), 8.88 (s, 1H), 8.64 (s, 1H), 7.95-7.84 (m, 2H), 7.75 (d, *J* = 7.9 Hz, 1H), 7.67-7.61 (m, 2H), 7.58-7.44 (m, 2H), 7.30 (d, *J =* 8.8 Hz, 1H), 7.29-7.19 (m, 1H), 3.96 (s, 3H). | 480 | **A** |
| **42** | 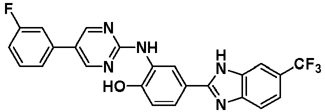 | ¹H NMR (400 MHz, DMSO-*d₆*) δ 13.12 (s, 1H), 10.63 (s, 1H), 8.92 (s, 2H), 8.80 (s, 1H), 8.58 (s, 1H), 7.99-7.82 (m, 1H), 7.82-7.61 (m, 4H), 7.56-7.47 (m, 2H), 7.23-7.18 (m, 1H), 7.07 (d, *J* = 8.4 Hz, 1H). | 466 | **A** |
| **43** | 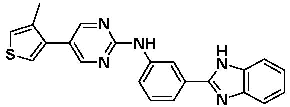 | ¹H NMR (400 MHz, DMSO-*d₆*) δ 12.88 (s, 1H), 9.96 (s, 1H), 8.66 (s, 2H), 8.63 (t, *J =* 1.9 Hz, 1H), 7.92-7.88 (m, 1H), 7.75-7.66 (m, 2H), 7.64 (d, *J* = 3.3 Hz, 1H), 7.54 (dd, *J =* 1.7, 6.9 Hz, 1H), 7.49 (t, *J =* 7.9 Hz, 1H), 7.35-7.33 (m, 1H), 7.26-7.17 (m, 2H), 2.30 (s, 3H). | 384 | **A** |
| **44** | 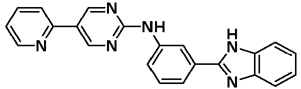 | ¹H NMR (400 MHz, DMSO-*d₆*) δ 12.93 (s, 1H), 10.14 (s, 1H), 9.23 (s, 2H), 8.73-8.61 (m, 2H), 8.03 (d, *J* = 8.0 Hz, 1H), 7.94-7.84 (m, 2H), 7.77 (d, *J* = 7.9 Hz, 1H), 7.67-7.54 (m, 2H), 7.50 (dd, *J =* 7.9, 7.9 Hz, 1H), 7.38-7.35 (m, 1H), 7.31-7.13 (m, 2H). | 365 | **A** |
| **45** | 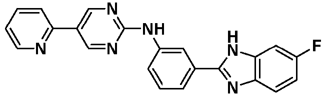 | ¹H NMR (400 MHz, DMSO-*d₆*) δ 10.13 (s, 1H), 9.21 (s, 2H), 8.68-8.65 (m, 1H), 8.65-8.62 (m, 1H), 8.04-7.99 (m, 1H), 7.93-7.88 (m, 2H), 7.76-7.71 (m, 1H), 7.65-7.57 (m, 1H), 7.51 (t, *J* = 7.9 Hz, 1H), 7.44-7.35 (m, 2H), 7.12-7.05 (m, 1H). | 383 | **A** |
| **46** | 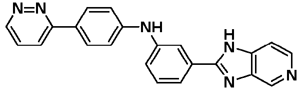 | ¹H NMR (400 MHz, DMSO-*d₆*) δ 13.36 (s, 1H), 9.12 (dd, *J* = 1.6, 4.8 Hz, 1H), 8.94 (s, 1H), 8.83 (s, 1H), 8.31 (d, *J* = 5.5 Hz, 1H), 8.19-8.02 (m, 4H), 7.79-7.66 (m, 2H), 7.61-7.58 (m, 1H), 7.52 (t, *J* = 7.5 Hz, 1H), 7.39-7.28 (m, 3H). | 365 | **A** |
| **47** | 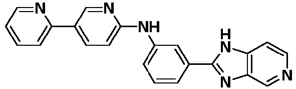 | ¹H NMR (400 MHz, DMSO-*d₆*) δ 14.67 (s, 1H), 9.71 (s, 1H), 9.46 (s, 1H), 8.97 (d, *J =* 2.4 Hz, 1H), 8.83 (s, 1H), 8.66 (d, *J* = 4.5 Hz, 1H), 8.62 (d, *J* = 6.5 Hz, 1H), 8.35 (dd, *J* = 2.5, 8.8 Hz, 1H), 8.17 (d, *J* = 6.4 Hz, 1H), 8.05-7.91 (m, 2H), 7.89-7.85 (m, 1H), 7.84 (d, *J =* 7.6 Hz, 1H), 7.62-7.57 (m, 1H), 7.37-7.33 (m, 1H), 7.04 (d, *J* = 8.8 Hz, 1H). | 365 | **A** |
| **48** | 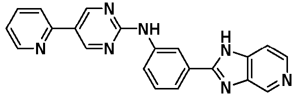 | ¹H NMR (400 MHz, DMSO-*d₆*) δ 13.46 (s, 1H), 10.18 (s, 1H), 9.24 (s, 2H), 8.99 (s, 1H), 8.76 (t, *J* = 1.8 Hz, 1H), 8.69-8.66 (m, 1H), 8.35 (d, *J =* 5.1 Hz, 1H), 8.06-8.02 (m, 1H), 7.96-7.88 (m, 2H), 7.84-7.79 (m, 1H), 7.64 (d, *J* = 5.0 Hz, 1H), 7.54 (t, *J* = 7.9 Hz, 1H), 7.39-7.35 (m, 1H). | 366 | **A** |
| **49** | 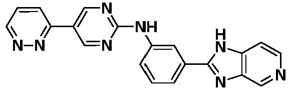 | ¹H NMR (400 MHz, DMSO-*d₆*) δ 14.15 (s, 1H), 10.35 (s, 1H), 9.32-9.31 (m, 2H), 9.23 (dd, *J =* 1.6, 4.8 Hz, 1H), 9.19-9.15 (m, 1H), 8.82 (s, 1H), 8.45 (d, *J* = 5.5 Hz, 1H), 8.32 (dd, *J =* 1.4, 8.8 Hz, 1H), 8.00-7.96 (m, 1H), 7.88-7.80 (m, 3H), 7.59 (t, *J =* 8.0 Hz, 1H). | 367 | **A** |
| **50** | 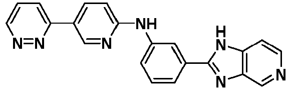 | ¹H NMR (400 MHz, DMSO-*d₆*) δ 13.99 (s, 1H), 9.72 (s, 1H), 9.26-9.06 (m, 2H), 9.03 (d, *J =* 2.4 Hz, 1H), 8.73 (s, 1H), 8.53-8.37 (m, 2H), 8.25 (dd, *J =* 1.5, 8.8 Hz, 1H), 7.95 (dd, *J =* 1.6, 8.2 Hz, 1H), 7.87-7.73 (m, 3H), 7.56 (t, *J =* 7.9 Hz, 1H), 7.07 (d, *J =* 8.6 Hz, 1H). | 366 | **A** |
| **51** | 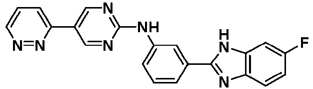 | ¹H NMR (400 MHz, DMSO-*d₆*) δ 10.18 (s, 1H), 9.28 (s, 2H), 9.21 (dd, *J =* 1.4, 4.9 Hz, 1H), 8.66 (s, 1H), 8.30 (dd, *J =* 1.4, 8.7 Hz, 1H), 7.93-7.66 (m, 3H), 7.52 (dd, *J* = 5.0, 8.6 Hz, 1H), 7.45 (t, *J* = 8.0 Hz, 1H), 7.31 (dd, *J* = 1.9, 10.1 Hz, 1H), 6.97-6.91 (m, 1H). | 384 | **A** |
| **52** | 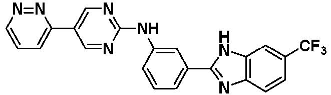 | ¹H NMR (400 MHz, DMSO-*d₆*) δ 13.34 (s, 1H), 10.30 (s, 1H), 9.31 (s, 2H), 9.22 (dd, *J* = 1.9, 5.1 Hz, 1H), 8.74 (s, 1H), 8.31 (dd, *J* = 1.6, 8.7 Hz, 1H), 7.98-7.77 (m, 5H), 7.57 (t, *J =* 7.9 Hz, 2H). | 434 | **A** |
| **53** | 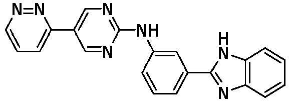 | ¹H NMR (400 MHz, DMSO-*d₆*) δ 10.45 (s, 1H), 9.33 (s, 2H), 9.24 (dd, *J =* 1.5, 5.0 Hz, 1H), 8.76-8.73 (m, 1H), 8.32 (dd, *J* = 1.5, 8.6 Hz, 1H), 8.00-7.96 (m, 1H), 7.85-7.77 (m, 4H), 7.67 (t, *J* = 8.0 Hz, 1H), 7.52-7.48 (m, 2H). | 366 | **A** |
| **54** | 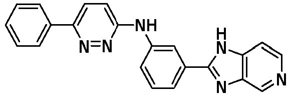 | ¹H NMR (400 MHz, DMSO-*d ₆*) δ 13.37-13.33 (m, 1H), 9.66 (s, 1H), 9.01 (s, 1H), 8.84 (s, 1H), 8.32 (d, *J* = 5.2 Hz, 1H), 8.14-8.04 (m, 3H), 7.91 (d, *J =* 8.3 Hz, 1H), 7.79-7.78 (m, 1H), 7.61-7.43 (m, 5H), 7.30 (d, *J* = 9.4 Hz, 1H). | 365 | **A** |
| **55** | 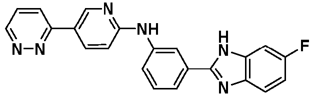 | ¹H NMR (400 MHz, DMSO-*d₆*) δ 9.83 (s, 1H), 9.20 (dd, *J =* 1.7, 5.0 Hz, 1H), 9.03 (d, *J* = 2.3 Hz, 1H), 8.68 (t, *J* = 1.6 Hz, 1H), 8.46 (dd, *J =* 2.5, 8.8 Hz, 1H), 8.28 (dd, *J =* 1.5, 8.6 Hz, 1H), 7.92-7.88 (m, 1H), 7.83-7.72 (m, 3H), 7.65-7.59 (m, 2H), 7.36-7.29 (m, 1H), 7.11 (d, *J* = 8.7 Hz, 1H). | 383 | **A** |
| **56** | 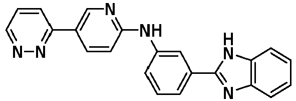 | ¹H NMR (400 MHz, DMSO-*d₆*) δ 9.83 (s, 1H), 9.19 (dd, *J =* 1.5, 4.9 Hz, 1H), 9.05 (d, *J* = 2.4 Hz, 1H), 8.72 (t, *J* = 1.7 Hz, 1H), 8.46 (dd, *J =* 2.5, 8.8 Hz, 1H), 8.25 (dd, *J =* 1.5, 8.8 Hz, 1H), 7.93-7.89 (m, 1H), 7.83-7.73 (m, 4H), 7.65 (t, *J* = 8.5 Hz, 1H), 7.56-7.50 (m, 2H), 7.11 (d, *J =* 8.8 Hz, 1H). | 365 | **A** |
| **57** | 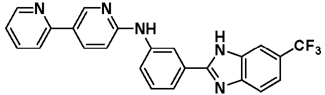 | ¹H NMR (400 MHz, (400 MHz, DMSO-*d₆*) δ 13.36 (s, 1H), 9.58 (s, 1H), 8.97 (d, *J =* 2.3 Hz, 1H), 8.66-8.61 (m, 2H), 8.33 (dd, *J =* 2.5, 8.8 Hz, 1H), 7.98-7.88 (m, 3H), 7.88-7.82 (m, 1H), 7.82-7.69 (m, 2H), 7.60-7.39 (m, 2H), 7.33-7.28 (m, 1H), 7.01 (d, *J* = 8.8 Hz, 1H). | 432 | **A** |
| **58** | 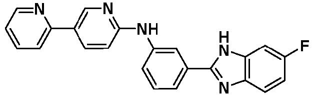 | ¹H NMR (400 MHz, DMSO-*d₆*) δ 13.00 (s, 1H), 9.53 (s, 1H), 8.96 (d, *J* = 2.4 Hz, 1H), 8.64 (dd, *J* = 0.9, 4.8 Hz, 1H), 8.55 (s, 1H), 8.32 (dd, *J =* 2.5, 8.8 Hz, 1H), 7.97-7.88 (m, 1H), 7.87-7.84 (m, 2H), 7.71-7.65 (m, 1H), 7.58-7.41 (m, 2H), 7.34-7.28 (m, 2H), 7.13-7.03 (m, 1H), 7.00 (d, *J* = 8.6 Hz, 1 H). | 382 | **A** |
| **59** | 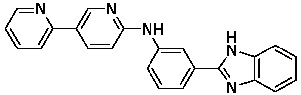 | ¹H NMR (400 MHz, DMSO-*d₆*) δ 12.86 (s, 1H), 9.52 (s, 1H), 8.96 (d, *J* = 2.3 Hz, 1H), 8.64 (d, *J =* 4.0 Hz, 1H), 8.56 (t, *J =* 1.8 Hz, 1H), 8.32 (dd, *J* = 2.5, 8.8 Hz, 1H), 7.98-7.83 (m, 3H), 7.72-7.66 (m, 2H), 7.54 (q, *J* = 2.8 Hz, 1H), 7.48 (t, *J =* 7.9 Hz, 1H), 7.33-7.28 (m, 1H), 7.26-7.17 (m, 2H), 7.00 (d, *J* = 8.8 Hz, 1H). | 364 | **A** |
| **60** | 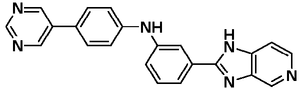 | ¹H NMR (400 MHz, DMSO-*d₆*) δ 13.50 (s, 1H), 9.14-9.11 (m, 3H), 8.97 (s, 1H), 8.75 (s, 1H), 8.34-8.33 (m, 1H), 8.09 (s, 1H), 8.00-7.78 (m, 2H), 7.74 (d, *J =* 7.9 Hz, 1H), 7.63-7.62 (m, 1H), 7.58-7.40 (m, 1H), 7.36-7.15 (m, 3H). | 365 | **A** |
| **61** | 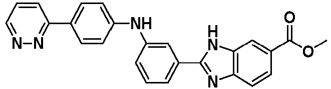 | ¹H NMR (400 MHz, DMSO-*d₆*) δ 9.15 (dd, *J* = 1.5, 4.9 Hz, 1H), 8.88 (s, 1H), 8.23-8.19 (m, 2H), 8.16-8.11 (m, 2H), 8.08 (t, *J* = 2.7 Hz, 1H), 7.90 (dd, *J =* 1.6, 8.4 Hz, 1H), 7.79-7.72 (m, 3H), 7.54 (dd, *J =* 7.9, 7.9 Hz, 1H), 7.37-7.23 (m, 3H), 3.90 (s, 3H). | 422 | **A** |
| **62** | 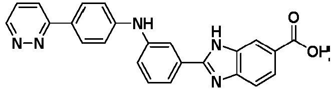 | ¹H NMR (400 MHz, DMSO-*d₆*) δ 13.22 (s, 1H), 12.73 (s, 1H), 9.12 (dd, *J =* 1.4, 4.8 Hz, 1H), 8.81 (s, 1H), 8.25-8.07 (m, 5H), 7.89-7.82 (m, 1H), 7.75-7.59 (m, 3H), 7.51 (t, *J* = 7.8 Hz, 1H), 7.34-7.28 (m, 3H). | 408 | **C** |
| **63** | 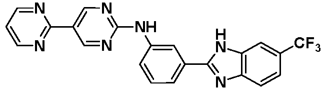 | ¹H NMR (400 MHz, DMSO-*d₆*) δ 13.36 (s, 1H), 10.37 (s, 1H), 9.39 (s, 2H), 8.91 (d, *J =* 4.9 Hz, 2H), 8.70 (s, 1H), 7.99-7.94 (m, 2H), 7.86-7.81 (m, 2H), 7.56 (t, *J* = 7.9 Hz, 2H), 7.46 (t, *J =* 4.9 Hz, 1H). | 434 | **A** |
| **64** | 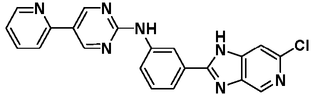 | ¹H NMR (400 MHz, DMSO-*d ₆* ) δ 13.56-13.50 (m, 1H), 10.20 (s, 1H), 9.26 (s, 2H), 8.80-8.75 (m, 2H), 8.68 (d, *J* = 4.0 Hz, 1H), 8.05-8.02 (m, 1H), 7.97-7.88 (m, 2H), 7.83-7.61 (m, 2H), 7.55 (t, *J =* 7.9 Hz, 1H), 7.39-7.35 (m, 1H). | 400 | **A** |
| **65** | 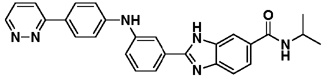 | ¹H NMR (400 MHz, DMSO-*d₆*) δ 13.11 (s, 1H), 9.13 (dd, *J =* 1.5, 4.9 Hz, 1H), 8.81 (s, 1H), 8.23-8.08 (m, 6H), 7.81-7.69 (m, 3H), 7.69-7.46 (m, 2H), 7.36-7.26 (m, 3H), 4.19-4.09 (m, 1H), 1.20 (d, *J =* 6.5 Hz, 6H). | 449 | **D** |
| **66** | 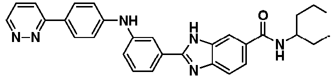 | ¹H NMR (400 MHz, DMSO-*d₆*) δ 13.08 (d, *J =* 2.4 Hz, 1H), 9.12 (dd, *J* = 1.5, 4.9 Hz, 1H), 8.80 (s, 1H), 8.24-8.00 (m, 6H), 7.79-7.47 (m, 5H), 7.29 (d, *J* = 8.8 Hz, 3H), 3.83-3.78 (m, 1H), 1.91-1.68 (m, 4H), 1.65-1.59 (m, 1H), 1.41-1.27 (m, 4H), 1.24-1.11 (m, 1H). | 489 | **D** |
| **67** | 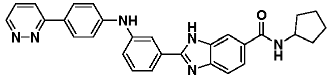 | ¹H NMR (400 MHz, DMSO-*d₆*) δ 13.10 (d, *J =* 3.6 Hz, 1H), 9.12 (dd, *J* = 1.3, 4.8 Hz, 1H), 8.81 (s, 1H), 8.32-8.00 (m, 6H), 7.78-7.47 (m, 5H), 7.32-7.27 (m, 3H), 4.30-4.23 (m, 1H), 1.95-1.87 (m, 2H), 1.76-1.68 (m, 2H), 1.63-1.52 (m, 4H). | 475 | **D** |
| **68** | 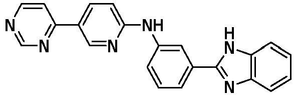 | ¹H NMR (400 MHz, DMSO-*d₆*) δ 12.88 (s, 1H), 9.74 (s, 1H), 9.18 (d, *J =* 1.3 Hz, 1H), 9.11 (d, *J =* 2.3 Hz, 1H), 8.79 (d, *J =* 5.5 Hz, 1H), 8.58 (t, *J* = 1.8 Hz, 1H), 8.42 (dd, *J =* 2.5, 8.9 Hz, 1H), 8.07 (dd, *J =* 1.4, 5.5 Hz, 1H), 7.92-7.88 (m, 1H), 7.76-7.72 (m, 1H), 7.69-7.47 (m, 3H), 7.22 (d, *J =* 4.3 Hz, 2H), 7.03 (d, *J =* 8.5 Hz, 1H). | 365 | **B** |
| **69** | 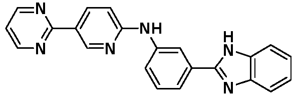 | ¹H NMR (400 MHz, DMSO-*d₆*) δ 12.91 (s, 1H), 9.67 (s, 1H), 9.22 (d, *J* = 2.4 Hz, 1H), 8.86 (d, *J =* 4.9 Hz, 2H), 8.54 (t, *J =* 1.8 Hz, 1H), 8.50 (dd, *J* = 2.5, 8.8 Hz, 1H), 7.90-7.86 (m, 1H), 7.80-7.53 (m, 3H), 7.53-7.47 (m, 1H), 7.39-7.36 (m, 1H), 7.26-7.18 (m, 2H), 7.01 (d, *J =* 8.8 Hz, 1H). | 365 | **B** |
| **70** | 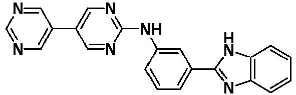 | ¹H NMR (400 MHz, DMSO-*d₆*) δ 12.87 (s, 1H), 10.14 (s, 1H), 9.24 (s, 2H), 9.20 (s, 1H), 9.04 (s, 2H), 8.69 (t, *J* = 5.0 Hz, 1H), 7.89-7.86 (m, 1H), 7.77-7.75 (m, 1H), 7.69-7.67 (m, 1H), 7.53-7.49 (m, 2H), 7.24-7.22 (m, 2H) | 366 | **B** |
| **71** | 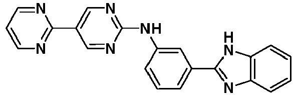 | ¹H NMR (400 MHz, DMSO-*d₆*) δ 12.88 (s, 1H), 10.30 (s, 1H), 9.38 (s, 2H), 8.91 (d, *J =* 4.9 Hz, 2H), 8.64 (t, *J* = 1.8 Hz, 1H), 7.92 (dd, *J =* 1.4, 8.3 Hz, 1H), 7.81-7.79 (m, 1H), 7.75-7.55 (m, 2H), 7.52 (t, *J* = 8.1 Hz, 1H), 7.46 (t, *J =* 4.9 Hz, 1H), 7.22 (d, *J =* 4.9 Hz, 2H). | 366 | **B** |
| **72** | 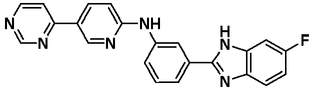 | ¹H NMR (400 MHz, DMSO-*d₆*) δ 13.01 (s, 1H), 9.75 (s, 1H), 9.18 (s, 1H), 9.11 (d, *J =* 2.4 Hz, 1H), 8.79 (d, *J* = 5.4 Hz, 1H), 8.57 (s, 1H), 8.42 (dd, *J =* 2.5, 8.8 Hz, 1H), 8.07 (dd, *J =* 1.2, 5.4 Hz, 1H), 7.89 (t, *J =* 5.6 Hz, 1H), 7.75-7.31 (m, 4H), 7.13-7.01 (m, 2H). | 383 | **B** |
| **73** | 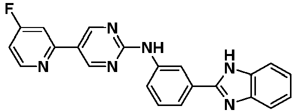 | ¹H NMR (400 MHz, DMSO-*d₆*) δ 12.87 (s, 1H), 10.20 (s, 1H), 9.26 (s, 2H), 8.72-8.68 (m, 2H), 8.02 (dd, *J* = 2.4, 11.0 Hz, 1H), 7.90-7.86 (m, 1H), 7.79-7.76 (m, 1H), 7.71-7.67 (m, 1H), 7.56-7.49 (m, 2H), 7.33-7.18 (m, 3H). | 383 | **B** |
| **74** | 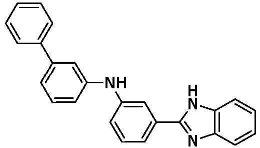 | ¹H NMR (400 MHz, DMSO-*d₆*) δ 12.86 (s, 1H), 8.50 (s, 1H), 8.05-8.02 (m, 1H), 7.68-7.61 (m, 4H), 7.53-7.35 (m, 7H), 7.25-7.16 (m, 5H). | 362 | **B** |
| **75** | 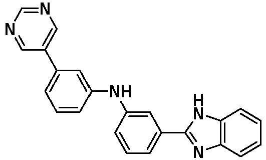 | ¹H NMR (400 MHz, DMSO-*d₆*) δ 12.88 (s, 1H), 9.20 (s, 1H), 9.13 (s, 2H), 8.60 (s, 1H), 8.06-8.04 (m, 1H), 7.68-7.64 (m, 2H), 7.54-7.42 (m, 4H), 7.31-7.16 (m, 5H). | 364 | **B** |
| 76 | 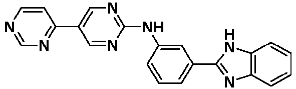 | ¹H NMR (400 MHz, DMSO-*d₆*) δ 12.88 (s, 1H), 10.36 (s, 1H), 9.34 (s, 2H), 9.23 (d, *J =* 1.1 Hz, 1H), 8.86 (d, *J* = 5.5 Hz, 1H), 8.69 (t, *J =* 1.8 Hz, 1H), 8.15 (dd, *J =* 1.4, 5.4 Hz, 1H), 7.90-7.86 (m, 1H), 7.82-7.78 (m, 1H), 7.71-7.67 (m, 1H), 7.56-7.50 (m, 2H), 7.26-7.18 (m, 2H). | 384 | **B** |
| **77** | 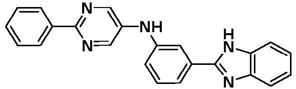 | ¹H NMR (400 MHz, DMSO-*d₆*) δ 12.92 (s, 1H), 8.88 (s, 1H), 8.79 (s, 2H), 8.36-8.33 (m, 2H), 8.04 (s, 1H), 7.76 (d, *J* = 7.9 Hz, 1H), 7.68 (d, *J =* 7.4 Hz, 1H), 7.55-7.46 (m, 5H), 7.29-7.17 (m, 3H). | 364 | **B** |
| **78** | 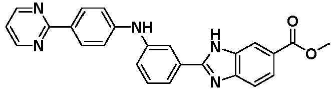 | ¹H NMR (400 MHz, DMSO-*d₆*) δ 13.36 (s, 1H), 8.87-8.82 (m, 3H), 8.33 (d, *J =* 8.8 Hz, 2H), 8.20 (s, 1H), 8.10-8.07 (m, 1H), 7.86 (dd, *J =* 1.5, 8.5 Hz, 1H), 7.76-7.72 (m, 1H), 7.71-7.65 (m, 1H), 7.51 (t, *J* = 7.9 Hz, 1H), 7.33 (t, *J =* 4.8 Hz, 2H), 7.25 (d, *J* = 9.2Hz, 2H), 3.88 (s, 3H). | 422 | **A** |
| **79** | 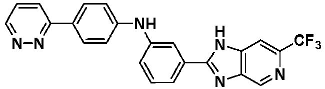 | ¹H NMR (400 MHz, DMSO-*d₆*) δ 13.86 (s, 1H), 9.13 (dd, *J* = 1.3, 4.8 Hz, 1H), 9.08 (s, 1H), 8.87 (s, 1H), 8.19-7.96 (m, 5H), 7.78 (d, *J =* 7.9 Hz, 1H), 7.73 (dd, *J* = 4.9, 8.8 Hz, 1H), 7.55 (t, *J* = 8.0 Hz, 1H), 7.36 (dd, *J =* 1.9, 7.9 Hz, 1H), 7.34-7.29 (m, 2H). | 433 | **A** |
| **80** | 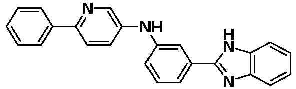 | ¹H NMR (400 MHz, DMSO-*d₆*) δ 12.91 (s, 1H), 8.74 (s, 1H), 8.55 (d, *J* = 2.7 Hz, 1H), 8.08-8.00 (m, 3H), 7.91 (d, *J =* 8.6 Hz, 1H), 7.74-7.62 (m, 3H), 7.53 (dd, *J =* 7.1, 1.7 Hz, 1H), 7.51-7.43 (m, 3H), 7.41-7.33 (m, 1H), 7.27-7.15 (m, 3H). | 363 | **B** |
| **81** | 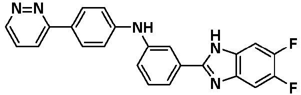 | ¹H NMR (400 MHz, DMSO-*d₆*) δ 13.15 (s, 1H), 9.12 (dd, *J* = 4.8, 1.5 Hz, 1H), 8.79 (s, 1H), 8.19-8.09 (m, 3H), 8.04 (t, *J* = 2.0 Hz, 1H), 7.76-7.56 (m, 4H), 7.48 (t, *J* = 7.9 Hz, 1H), 7.34-7.24 (m, 3H). | 400 | **A** |
| **82** | 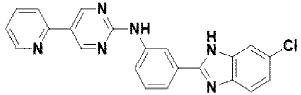 | ¹H NMR (400 MHz, DMSO-*d₆*) δ 13.08 (d, *J* = 14.1 Hz, 1H), 10.15 (s, 1H), 9.23 (s, 2H), 8.68-8.66 (m, 2H), 8.05-8.01 (m, 1H), 7.94-7.88 (m, 2H), 7.76-7.68 (m, 2H), 7.57-7.49 (m, 2H), 7.39-7.35 (m, 1H), 7.28-7.22 (m, 1H). | 400 | **B** |
| **83** | 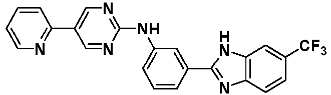 | ¹H NMR (400 MHz, DMSO-*d₆*) δ 13.33 (s, 1H), 10.19 (s, 1H), 9.24 (s, 2H), 8.73 (d, *J =* 7.3 Hz, 1H), 8.69-8.67 (m, 1H), 8.08-7.89 (m, 4H), 7.86-7.72 (m, 2H), 7.58-7.53 (m, 2H), 7.37 (ddd, *J* = 1.0, 4.8, 7.4 Hz, 1H). | 433 | **B** |
| **84** | 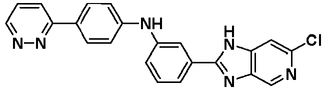 | ¹H NMR (400 MHz, DMSO-*d₆*) δ 13.55 (s, 1H), 9.13 (dd, *J* = 1.4, 4.8 Hz, 1H), 8.85 (s, 1H), 8.73 (d, *J* = 29.4 Hz, 1H), 8.18-8.12 (m, 3H), 8.10 (s, 1H), 7.77-7.61 (m, 3H), 7.52 (dd, *J* = 7.9, 7.9 Hz, 1H), 7.36-7.28 (m, 3H). | 400 | **A** |
| **85** | 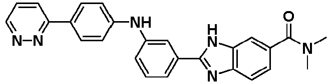 | ¹H NMR (400 MHz, DMSO-*d₆*) δ 13.10 (s, 1H), 9.13 (dd, *J* = 1.4, 4.8 Hz, 1H), 8.82 (s, 1H), 8.18-8.11 (m, 3H), 8.08-8.07 (m, 1H), 7.75-7.67 (m, 3H), 7.58-7.53 (m, 1H), 7.52-7.47 (m, 1H), 7.32-7.23 (m, 4H), 3.02 (s, 6H). | 435 | **D** |
| **86** | 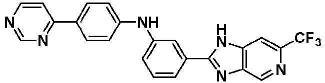 | ¹H NMR (400 MHz, DMSO-*d₆*) δ 13.86 (s, 1H), 9.15 (s, 1H), 9.07 (s, 1H), 8.99 (s, 1H), 8.76 (d, *J* = 5.5 Hz, 1H), 8.20 (d, *J* = 8.8 Hz, 2H), 8.15 (s, 1H), 8.00 (d, *J* = 5.5 Hz, 1H), 7.81 (d, *J =* 7.8 Hz, 1H), 7.56 (dd, *J* = 7.9, 7.9 Hz, 1H), 7.37 (dd, *J* = 1.6, 7.9 Hz, 1H), 7.28 (d, *J* = 8.8 Hz, 2H). | 433 | **A** |
| **87** | 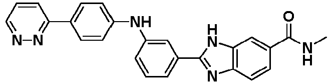 | ¹H NMR (400 MHz, DMSO-*d₆*) δ 13.13 (d, *J* = 6.9 Hz, 1H), 9.13-9.11 (m, 1H), 8.82 (s, 1H), 8.48-8.40 (m, 1H), 8.19-8.11 (m, 4H), 8.08 (s, 1H), 7.79-7.69 (m, 4H), 7.57-7.47 (m, 2H), 7.32-7.28 (m, 3H), 2.82 (d, *J* = 3.8 Hz, 3H). | 421 | **D** |
| **88** | 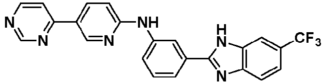 | ¹H NMR (400 MHz, DMSO-*d* ₆) δ 9.79 (s, 1H), 9.15 (dd, *J* = 1.9, 28.6 Hz, 2H), 8.80 (d, *J* = 5.1 Hz, 1H), 8.63 (t, *J* = 1.6 Hz, 1H), 8.43 (dd, *J* = 2.7, 8.8 Hz, 1H), 8.08 (dd, *J =* 1.4, 5.5 Hz, 1H), 8.00-7.93 (m, 2H), 7.81-7.76 (m, 2H), 7.55 (t, *J* = 7.8 Hz, 2H), 7.04 (d, *J* = 8.8 Hz, 1H). | 433 | **A** |
| **89** | 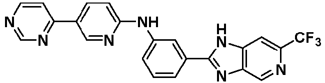 | ¹H NMR (400 MHz, DMSO-*d₆*) δ 13.83 (s, 1H), 9.82 (s, 1H), 9.19 (d, *J* = 1.1 Hz, 1H), 9.12 (d, *J* = 2.5 Hz, 1H), 9.09 (s, 1H), 8.80 (d, *J* = 5.5 Hz, 1H), 8.72 (s, 1H), 8.43 (dd, *J* = 2.4, 8.8 Hz, 1H), 8.08 (dd, *J* = 1.5, 5.6 Hz, 2H), 7.99 - 7.96 (m, 1H), 7.82 (d, *J* = 7.9 Hz, 1H), 7.57 (dd, *J* = 7.9, 7.9 Hz, 1H), 7.04 (d, *J* = 8.9 Hz, 1H). | 434 | **A** |
| **90** | 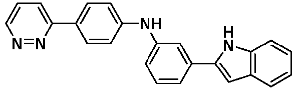 | ¹H NMR (400 MHz, DMSO-*d₆*) δ 11.53 (s, 1H), 9.11 (dd, *J* = 1.4, 4.8 Hz, 1H), 8.69 (s, 1H), 8.16-8.09 (m, 3H), 7.73-7.67 (m, 2H), 7.54 (d, *J* = 7.8 Hz, 1H), 7.46-7.38 (m, 3H), 7.27 (d, *J* = 8.9 Hz, 2H), 7.12-7.07 (m, 2H), 7.03-6.98 (m, 1H), 6.85 (d, *J* = 1.5 Hz, 1H). | 363 | **B** |
| **91** | 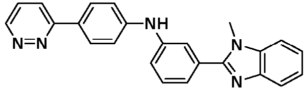 | ¹H NMR (400 MHz, DMSO-*d₆*) δ 9.12 (dd, *J* = 1.4, 4.9 Hz, 1H), 8.82 (s, 1H), 8.16-8.10 (m, 3H), 7.73-7.70 (m, 2H), 7.65-7.61 (m, 2H), 7.51 (t, *J* = 7.8 Hz, 1H), 7.39-7.23 (m, 6H), 3.93 (s, 3H). | 378 | **A** |
| **92** | 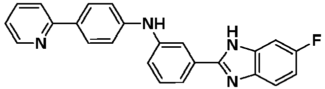 | ¹H NMR (400 MHz, DMSO-*d₆*) δ 13.01 (s, 1H), 8.67-8.61 (m, 2H), 8.07-8.01 (m, 3H), 7.91-7.80 (m, 2H), 7.69-7.23 (m, 1H), 7.11-7.01 (m, 1H). | 381 | **A** |
| **93** | 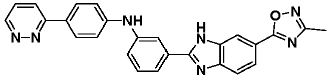 | ¹H NMR (400 MHz, DMSO-*d₆*) δ 13.38 (s, 1H), 9.13 (dd, *J* = 1.5, 4.9 Hz, 1H), 8.83 (s, 1H), 8.30 (s, 1H), 8.18-8.09 (m, 4H), 7.96 (d, *J* = 9.0 Hz, 1H), 7.80-7.70 (m, 3H), 7.53 (t, *J* = 7.9 Hz, 1H), 7.34-7.30 (m, 3H), 2.44 (s, 3H). | 446 | **E** |
| **94** | 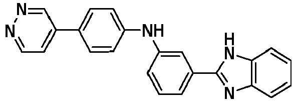 | ¹H NMR (400 MHz, DMSO-*d₆*) δ 12.89 (s, 1H), 9.63 (dd, *J* = 1.2, 2.6 Hz, 1H), 9.18 (dd, *J* = 1.3, 5.6 Hz, 1H), 8.84 (s, 1H), 8.07 (dd, *J* = 1.8, 1.8 Hz, 1H), 7.97-7.89 (m, 3H), 7.73 (td, *J* = 1.2, 7.6 Hz, 1H), 7.66 (dd, *J* = 1.6, 6.9 Hz, 1H), 7.55-7.46 (m, 2H), 7.30-7.17 (m, 5H). | 364 | **B** |
| **95** | 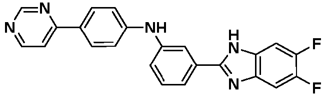 | ¹H NMR (400 MHz, DMSO-*d₆*) δ 13.15 (s, 1H), 9.14 (d, *J* = 1.0 Hz, 1H), 8.89 (s, 1H), 8.75 (d, *J* = 5.5 Hz, 1H), 8.21-8.15 (m, 2H), 8.05 (t, *J* = 1.8 Hz, 1H), 7.98 (dd, *J* = 1.4, 5.6 Hz, 1H), 7.76-7.69 (m, 2H), 7.58-7.47 (m, 2H), 7.30-7.24 (m, 3H). | 400 | **A** |
| **96** | 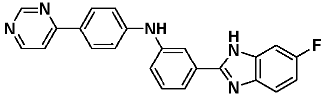 | ¹H NMR (400 MHz, DMSO-*d₆*) δ 13.04 (s, 1H), 9.14 (d, *J* = 1.1 Hz, 1H), 8.89 (s, 1H), 8.75 (d, *J* = 5.5 Hz, 1H), 8.21-8.15 (m, 2H), 8.06 (t, *J* = 1.7 Hz, 1H), 7.98 (dd, *J* = 1.4, 5.6 Hz, 1H), 7.74-7.70 (m, 1H), 7.66-7.24 (m, 6H), 7.07 (t, *J* = 8.6 Hz, 1H). | 382 | **A** |
| **97** | 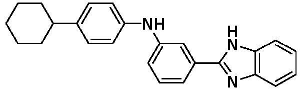 | ¹H NMR (400 MHz, DMSO-*d₆*) δ 12.81 (s, 1H), 8.24 (s, 1H), 7.91 (t, *J* = 2.0 Hz, 1H), 7.64 (dd, *J* = 1.5, 7.0 Hz, 1H), 7.57-7.50 (m, 2H), 7.36 (t, *J* = 7.7 Hz, 1H), 7.23-7.13 (m, 4H), 7.12-7.07 (m, 3H), 2.47-2.42 (m, 1H), 1.82 (d, *J* = 8.3 Hz, 4H), 1.72 (d, *J* = 12.3 Hz, 1H), 1.46-1.18 (m, 5H). | 368 | **B** |
| **98** | 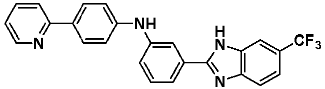 | ¹H NMR (400 MHz, DMSO-*d₆*) δ 8.72 (s, 1H), 8.62 (ddd, *J* = 1.0, 1.8, 4.9 Hz, 1H), 8.08-8.04 (m, 3H), 7.97-7.85 (m, 3H), 7.78 (d, *J* = 10.3 Hz, 1H), 7.71 (d, *J* = 8.0 Hz, 1H), 7.56-7.47 (m, 2H), 7.31-7.25 (m, 4H). | 431 | **A** |
| **99** | 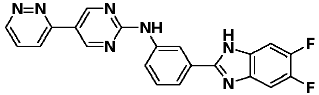 | ¹H NMR (400 MHz, DMSO-*d₆*) δ 13.14 (s, 1H), 10.27 (s, 1H), 9.30-9.19 (m, 3H), 8.67 (t, *J =* 1.7 Hz, 1H), 8.32-8.29 (m, 1H), 7.93-7.88 (m, 1H), 7.84-7.72 (m, 3H), 7.61-7.41 (m, 2H). | 402 | **A** |
| **100** | 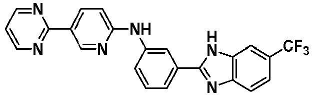 | ¹H NMR (400 MHz, DMSO-*d₆*) δ 13.33 (s, 1H), 9.72 (s, 1H), 9.23 (d, *J* = 1.6 Hz, 1H), 8.87 (d, *J* = 4.9 Hz, 2H), 8.61 (td, *J* = 2.1, 8.3 Hz, 1H), 8.51 (dd, *J* = 2.3, 8.8 Hz, 1H), 8.06-7.73 (m, 4H), 7.58-7.51 (m, 2H), 7.39 (t, *J =* 5.0 Hz, 1H), 7.02 (d, *J* = 8.8 Hz, 1H). | 433 | **B** |
| **101** | 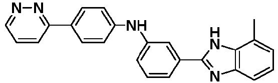 | ¹H NMR (400 MHz, DMSO-*d₆*) δ 9.12 (dd, *J* = 4.8, 1.5 Hz, 1H), 8.83 (s, 1H), 8.19-8.05 (m, 4H), 7.78-7.68 (m, 2H), 7.51 (t, *J =* 7.9 Hz, 1H), 7.43 (d, *J =* 8.0 Hz, 1H), 7.35-7.27 (m, 3H), 7.16 (t, *J =* 7.7 Hz, 1H), 7.06 (d, *J* = 7.3 Hz, 1H), 2.59 (s, 3H). | 378 | **A** |
| **102** | 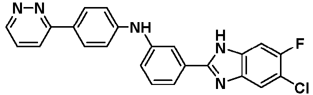 | ¹H NMR (400 MHz, DMSO-*d₆*) δ 9.13 (dd, *J* = 4.9, 1.5 Hz, 1H), 8.80 (s, 1H), 8.17 (dd, *J =* 8.8, 1.5 Hz, 1H), 8.17-8.09 (m, 2H), 8.05 (t, *J =* 2.0 Hz, 1H), 7.82-7.67 (m, 3H), 7.64 (d, *J =* 9.6 Hz, 1H), 7.50 (t, *J* = 7.9 Hz, 1H), 7.34-7.25 (m, 3H). | 416 | **A** |
| **103** | 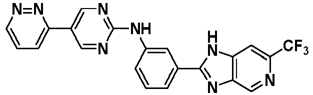 | ¹H NMR (400 MHz, DMSO-*d₆*) δ 13.84 (s, 1H), 10.35 (s, 1H), 9.31 (s, 2H), 9.22 (dd, *J* = 1.5, 4.9 Hz, 1H), 9.09 (d, *J* = 40.6 Hz, 1 H), 8.81 (s, 1H), 8.31 (dd, *J* = 1.3, 8.8 Hz, 1H), 8.22-7.95 (m, 2H), 7.86-7.80 (m, 2H), 7.59 (t, *J =* 7.9 Hz, 1H). | 435 | **A** |
| **104** | 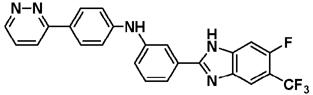 | ¹H NMR (400 MHz, DMSO-*d₆*) δ 13.56 (s, 1H), 9.13 (dd, *J =* 4.9, 1.5 Hz, 1H), 8.85 (s, 1H), 8.20-8.06 (m, 4H), 7.99-7.94 (m, 1H), 7.77-7.64 (m, 3H), 7.51 (t, *J =* 7.9 Hz, 1H), 7.36-7.26 (m, 3H). | 450 | **A** |
| **105** | 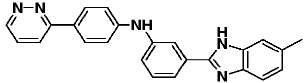 | ¹H NMR (400 MHz, DMSO-*d₆*) δ 9.13 (dd, *J* = 4.8, 1.5 Hz, 1H), 8.80 (s, 1H), 8.19-8.09 (m, 3H), 8.04 (t, *J =* 2.0 Hz, 1H), 7.76-7.65 (m, 2H), 7.54-7.44 (m, 2H), 7.40 (s, 1H), 7.33-7.24 (m, 3H), 7.07 (d, *J =* 8.2 Hz, 1H), 2.44 (s, 3H). | 378 | **A** |
| **106** | 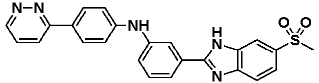 | ¹H NMR (400 MHz, DMSO-*d₆*) δ 9.13 (dd, *J* = 4.9, 1.5 Hz, 1H), 8.85 (s, 1H), 8.20-8.07 (m, 5H), 7.85-7.69 (m, 4H), 7.53 (t, *J =* 7.9 Hz, 1H), 7.37-7.27 (m, 3H), 3.25 (s, 3H). | 442 | **A** |
| **107** | 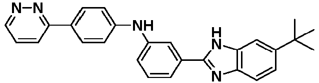 | ¹H NMR (400 MHz, DMSO-*d₆*) δ 9.14 (dd, *J* = 4.8, 1.5 Hz, 1H), 8.88 (s, 1H), 8.21-8.10 (m, 3H), 8.03 (t, *J =* 2.0 Hz, 1H), 7.78-7.65 (m, 2H), 7.65-7.58 (m, 2H), 7.55 (t, *J =* 7.9 Hz, 1H), 7.50-7.43 (m, 1H), 7.33 (dd, *J =* 9.0, 2.3 Hz, 3H), 1.38 (s, 9H). | 420 | **A** |
| **108** | 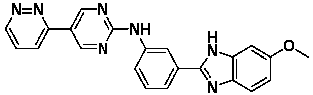 | ¹H NMR (400 MHz, DMSO-*d₆*) δ 10.25 (s, 1H), 9.30 (s, 2H), 9.21 (dd, *J* = 1.5, 4.9 Hz, 1H), 8.64 (t, *J* = 1.9 Hz, 1H), 8.30 (dd, *J =* 1.5, 8.6 Hz, 1H), 7.87 (dd, *J* = 1.4, 8.1 Hz, 1H), 7.81 (dd, *J* = 4.9, 8.7 Hz, 1H), 7.73 (d, *J* = 7.9 Hz, 1H), 7.50 (t, *J* = 8.1 Hz, 2H), 7.11 (s, 1H), 6.86 (dd, *J* = 2.4, 8.8 Hz, 1H), 3.82 (s, 3H). | 396 | **A** |
| **109** | 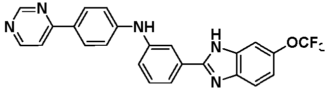 | ¹H NMR (400 MHz, DMSO-*d₆*) δ 13.19 (s, 1H), 9.14 (d, *J* = 1.1 Hz, 1H), 8.91 (s, 1H), 8.75 (d, *J =* 5.5 Hz, 1H), 8.18 (d, *J* = 8.8 Hz, 2H), 8.07 (s, 1H), 7.98 (dd, *J* = 1.4, 5.6 Hz, 1H), 7.75-7.47 (m, 4H), 7.32-7.19 (m, 4H). | 448 | **A** |
| **111** | 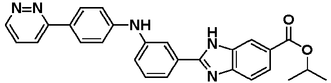 | ¹H NMR (400 MHz, DMSO-*d₆*) δ 9.15 (dd, *J* = 1.5, 4.9 Hz, 1H), 8.89 (s, 1H), 8.23-8.19 (m, 2H), 8.16-8.11 (m, 2H), 8.07 (t, *J* = 2.0 Hz, 1H), 7.89 (dd, *J* = 1.6, 8.4 Hz, 1H), 7.77 (dd, *J* = 4.9, 8.8 Hz, 1H), 7.72 (d, *J* = 8.0 Hz, 2H), 7.54 (t, *J* = 7.9 Hz, 1H), 7.36-7.29 (m, 3H), 5.22-5.12 (m, 1H), 1.36 (d, *J* = 6.3 Hz, 6H). | 450 | **A** |
| **112** | 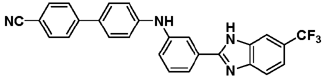 | ¹H NMR (400 MHz, DMSO-*d₆*) δ 13.33 (d, *J* = 4.5 Hz, 1H), 8.75 (d, *J* = 8.4 Hz, 1H), 8.06-7.68 (m, 10H), 7.56-7.46 (m, 2H), 7.31-7.24 (m, 3H). | 455 | **B** |
| **113** | 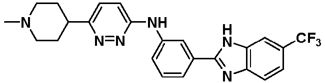 | ¹H NMR (400 MHz, DMSO-*d₆*) δ 9.41 (s, 1H), 8.75 (s, 1H), 8.00-7.76 (m, 3H), 7.73 (d, *J* = 8.0 Hz, 1H), 7.56-7.44 (m, 3H), 7.15 (d, *J* = 9.1 Hz, 1H), 2.89 (d, *J* = 11.4 Hz, 2H), 2.76-2.67 (m, 1H), 2.21 (s, 3H), 1.99 (dt, *J* = 3.0, 11.6 Hz, 2H), 1.90-1.71 (m, 4H). | 453 | **B** |
| **114** | 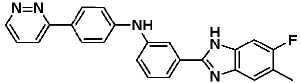 | ¹H NMR (400 MHz, DMSO-*d₆*) δ 9.13 (d, *J* = 4.6 Hz, 1H), 8.81 (s, 1H), 8.19-8.08 (m, 3H), 8.02 (t, *J* = 2.0 Hz, 1H), 7.77-7.64 (m, 2H), 7.53-7.45 (m, 2H), 7.39 (d, *J =* 10.0 Hz, 1H), 7.34-7.25 (m, 3H), 2.36 (d, *J =* 2.1 Hz, 3H). | 396 | **A** |
| **115** | 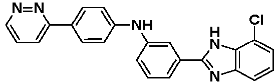 | ¹H NMR (400 MHz, DMSO-*d₆*) δ 9.12 (dd, *J* = 4.8, 1.5 Hz, 1H), 8.84 (s, 1H), 8.20-8.06 (m, 4H), 7.78-7.69 (m, 2H), 7.57-7.46 (m, 2H), 7.36-7.14 (m, 5H). | 398 | **A** |
| **116** | 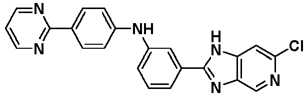 | ¹H NMR (400 MHz, DMSO-*d₆*) δ 13.52 (s, 1H), 8.87 (s, 1H), 8.83 (d, *J* = 4.9 Hz, 2H), 8.74 (s, 1H), 8.35-8.30 (m, 2H), 8.09 (t, *J =* 1.9 Hz, 1H), 7.76-7.71 (m, 1H), 7.66 (s, 1H), 7.55-7.49 (m, 1H), 7.36-7.32 (m, 2H), 7.25 (d, *J* = 8.8 Hz, 2H). | 400 | **A** |
| **117** | 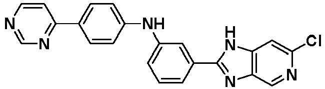 | ¹H NMR (400 MHz, DMSO-*d₆*) δ 13.56 (d, *J* = 26.0 Hz, 1H), 9.14 (d, *J* = 1.3 Hz, 1H), 8.95 (s, 1H), 8.79-8.69 (m, 2H), 8.18 (d, *J =* 8.8 Hz, 2H), 8.10 (s, 1H), 7.98 (dd, *J* = 1.4, 5.5 Hz, 1H), 7.79-7.58 (m, 2H), 7.53 (t, *J =* 8.0 Hz, 1H), 7.34 (d, *J* = 9.1 Hz, 1H), 7.26 (td, *J* = 2.5, 9.8 Hz, 2H). | 400 | **A** |
| **118** | 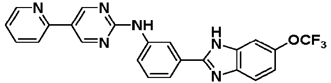 | ¹H NMR (400 MHz, DMSO-*d₆*) δ 10.16 (s, 1H), 9.23 (s, 2H), 8.72-8.64 (m, 2H), 8.03 (dt, *J =* 8.1, 1.1 Hz, 1H), 7.95-7.87 (m, 2H), 7.76 (dt, *J =* 7.8, 1.3 Hz, 1H), 7.73-7.57 (m, 2H), 7.52 (t, *J =* 7.9 Hz, 1H), 7.37 (ddd, *J =* 7.5, 4.8, 1.1 Hz, 1H), 7.22 (d, *J* = 8.7 Hz, 1H). | 449 | **A** |
| **119** | 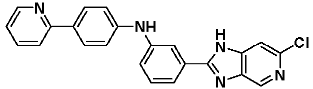 | ¹H NMR (400 MHz, DMSO-*d₆*) δ 13.54 (s, 1H), 8.76-8.60 (m, 3H), 8.06 (d, *J* = 8.4 Hz, 3H), 7.92-7.88 (m, 1H), 7.86-7.80 (m, 1H), 7.70 (d, *J* = 7.6 Hz, 1H), 7.66 (s, 1H), 7.50 (t, *J* = 8.0 Hz, 1H), 7.33-7.23 (m, 4H). | 399 | **A** |
| **120** | 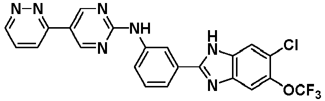 | ¹H NMR (400 MHz, DMSO-*d₆*) δ 13.36 (s, 1H), 10.31 (s, 1H), 9.30 (s, 2H), 9.22 (dd, *J* = 1.7, 5.0 Hz, 1H), 8.71 (d, *J* = 7.3 Hz, 1H), 8.31 (dd, *J* = 1.4, 8.7 Hz, 1H), 7.97-7.89 (m, 2H), 7.84-7.77 (m, 3H), 7.55 (t, *J* = 7.9 Hz, 1H). | 485 | **A** |
| **121** | 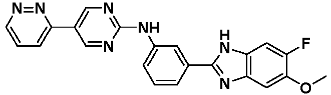 | ¹H NMR (400 MHz, DMSO-*d₆*) δ d 10.28 (s, 1H), 9.30 (s, 2H), 9.23-9.18 (m, 1H), 8.64 (s, 1H), 8.30 (dd, *J* = 1.4, 8.7 Hz, 1H), 7.89-7.86 (m, 1H), 7.83-7.80 (m, 1H), 7.72 (d, *J* = 7.9 Hz, 1H), 7.29 (d, *J* = 7.5 Hz, 1H), 3.91 (s, 3H). | 414 | **A** |
| **122** | 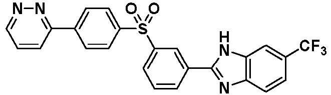 | ¹H NMR (400 MHz, DMSO-*d₆*) δ 13.68 (s, 1H), 9.28 (d, *J* = 4.4 Hz, 1H), 8.86 (s, 1H), 8.53 (d, *J* = 8.0 Hz, 1H), 8.42 (d, *J* = 8.4 Hz, 2H), 8.31 (d, *J* = 8.0 Hz, 1H), 8.23 (d, *J =* 8.4 Hz, 2H), 8.19 (d, *J* = 8.4 Hz, 1H), 8.02 (s, 1H), 7.90-7.83 (m, 3H), 7.57 (d, *J* = 8.0 Hz, 1H). | 481 | **F** |
| | | ¹⁹F-NMR (377 MHz, DMSO-*d₆*) δ -58.97. | | |
| **123** | 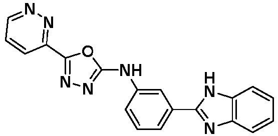 | ¹H NMR (400 MHz, DMSO-*d₆*) δ 12.94 (s, 1H), 11.19 (br, 1H), 9.39-9.38 (m, 1H), 8.55 (s, 1H), 8.37 (d, *J* = 1.6 Hz, 1H), 7.93 (dd, *J* = 8.4 Hz, 5.2 Hz, 1H), 7.82-7.77 (m, 2H), 7.81 (d, *J* = 8.0 Hz, 1H), 7.78-7.70 (m, 2H), 7.25-7.19 (m, 2H). | 356 | **F** |
| **124** | 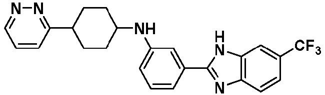 | ¹H NMR (400 MHz, DMSO-*d₆*) δ 13.18 (s, 1H), 9.10 (s, 1H), 7.98-7.70 (m, 4H), 7.68-7.62 (m, 2H), 7.54-7.50 (m, 1H), 7.46-7.24 (m, 1H), 6.86-6.78 (m, ¹H ), 5.97-5.82 (m, 1H), 3.79-3.33 (m, 1H), 3.06-2.92 (m, ¹H ), 2.20-2.09 (m, 2H), 2.06-1.78 (m, 4H), 1.44-1.23 (m, 1H) | 438 | **F** |
| | | **¹⁹F** NMR (376 MHz, DMSO-*d₆*) δ -58.78. | | |
| **125** | 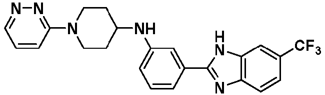 | ¹H NMR (400 MHz, DMSO- *d₆*) δ 13.19 (br, 1H), 8.53 (d, *J* = 4.4 Hz, 1H), 8.00-7.76 (m, 2H), 7.53-7.49 (m, 2H), 7.38-7.35 (m, 2H), 7.29-7.26 (m, 2H), 6.80 (d, *J* = 8.0 Hz, 1H), 5.86 (d, *J* = 8.0 Hz, 1H), 4.36-4.32 (m, 2H), 3.70-3.68 (m, 1H), 3.23-3.18 (m, 2H), 2.08-2.04 (m, 2H), 1.49-1.41 (m, 2H). | 439 | **F** |
| | | ¹⁹F NMR (376 MHz, DMSO-*d₆*) δ -58.81. | | |
| **126** | 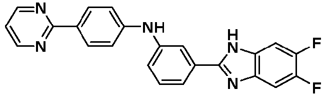 | ¹H NMR (400 MHz, DMSO-*d₆*) δ 8.84-8.80 (m, 3H), 8.32 (d, *J* = 9.3 Hz, 2H), 8.03-8.02 (m, 1H), 7.70-7.61 (m, 3H), 7.51-7.46 (m, 1H), 7.34-7.22 (m, 4H). | 400 | **A** |
| **150** | 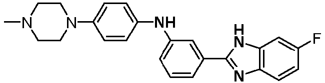 | ¹H NMR (400 MHz, DMSO-*d₆*) δ 12.91 (s, 1H), 8.02 (d, *J* = 2.2 Hz, 1H), 7.85-7.69 (m, 1H), 7.68-7.21 (m, 4H), 7.13-6.84 (m, 6H), 3.13-3.08 (m, 4H), 2.57-2.53 (m, 4H), 2.29 (s, 3H). | 402 | **B** |
| **151** | 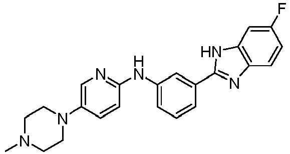 | ¹H NMR (400 MHz, DMSO-*d₆*) δ 12.94 (s, 1H), 8.96 (s, 1H), 8.40 (s, 1H), 7.89 (d, *J =* 3.0 Hz, 1H), 7.78-7.70 (m, 1H), 7.66-7.24 (m, 5H), 7.12-7.00 (m, 1H), 6.84 (d, *J* = 8.9 Hz, 1H), 3.17-2.95 (m, 4H), 2.49-2.40 (m, 4H), 2.23 (s, 3H). | 403 | **B** |
| **152** | 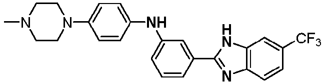 | ¹H NMR (400 MHz, DMSO-*d₆*) δ 13.24 (s, 1H), 8.06 (s, 1H), 8.02-7.65 (m, 3H), 7.55-7.31 (m, 3H), 7.10-6.91 (m, 5H), 3.13-3.05 (m, 4H), 2.49-2.44 (m, 4H), 2.23 (s, 3H). | 452 | **B** |
| **153** | 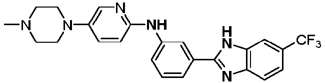 | ¹H NMR (400 MHz, DMSO-*d₆*) δ 13.32-13.24 (m, 1H), 9.04 (s, 1H), 8.52-8.43 (m, 1H), 8.05-7.69 (m, 4H), 7.64-7.50 (m, 2H), 7.46-7.39 (m, 2H), 6.86 (d, *J* = 9.0 Hz, 1H), 3.19-3.03 (m, 4H), 2.82-2.57 (m, 4H), 2.36 (s, 3H). | 453 | **B** |
| **154** | 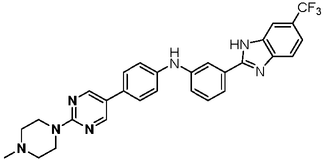 | ¹H NMR (400 MHz, DMSO-*d₆*) δ 13.33 (s, 1H), 8.69 (s, 2H), 8.58 (s, 1H), 8.07-7.68 (m, 3H), 7.65 (d, *J* = 7.7 Hz, 1H), 7.62-7.55 (m, 2H), 7.53 (d, *J* = 8.5 Hz, 1H), 7.45 (t, *J =* 7.9 Hz, 1H), 7.27-7.19 (m, 3H), 3.81-3.73 (m, 4H), 2.42-2.35 (m, 4H), 2.22 (s, 3H). | 530 | **B** |
| **155** | 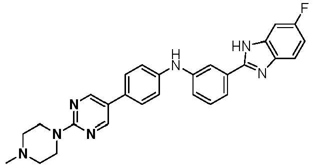 | ¹H NMR (400 MHz, DMSO-*d₆*) δ 12.98 (s, 1H), 8.68 (s, 2H), 8.52 (d, *J* = 2.2 Hz, 1H), 7.98-7.94 (m, 1H), 7.68-7.47 (m, 4H), 7.47-7.15 (m, 5H), 7.11-6.95 (m, 1H), 3.81-3.73 (m, 4H), 2.41-2.34 (m, 4H), 2.22 (s, 3H). | 480 | **B** |
| **156** | 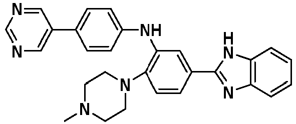 | ¹H NMR (400 MHz, CDCl₃) δ 9.16 (s, 1H), 8.94-8.86 (m, 2H), 8.10 (d, *J* = 2.0 Hz, 1H), 7.71-7.51 (m, 3H), 7.46 (t, *J* = 8.0 Hz, 2H), 7.25-7.23 (m, 5H), 7.21-7.16 (m, 1H), 6.62 (s, 1H), 3.09-2.96 (m, 4H), 2.73-2.54 (m, 4H), 2.40 (s, 3H). | 462 | **A** |
| **157** | 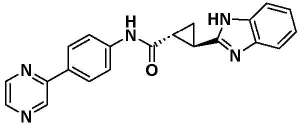 | ¹H NMR (400 MHz, DMSO-*d₆*) δ 12.49 (s, 1H), 10.64 (s, 1H), 9.22 (d, *J* = 1.5 Hz, 1H), 8.68 (dd, *J* = 1.5, 2.5 Hz, 1H), 8.56 (d, *J =* 2.5 Hz, 1H), 8.12 (d, *J* = 8.8 Hz, 2H), 7.80-7.75 (m, 2H), 7.54-7.37 (m, 2H), 7.16-7.05 (m, 2H), 2.61 - 2.54 (m, 2H), 1.66 - 1.58 (m, 2H). | 356 | **A** |
| **158** | 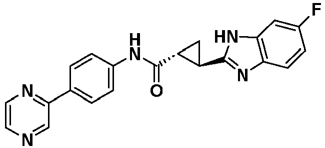 | ¹H NMR (400 MHz, DMSO-*d₆*) δ 12.61 (s, 1H), 10.64 (s, 1H), 9.23 (d, *J* = 1.6 Hz, 1H), 8.72-8.66 (m, 1H), 8.57 (d, *J* = 2.5 Hz, 1H), 8.18-8.10 (m, 2H), 7.78 (d, *J* = 8.8 Hz, 2H), 7.53-7.41 (m, 1H), 7.34-7.24 (m, 1H), 7.03-6.95 (m, 1H), 3.41-3.37 (m, 2H), 2.62-2.55 (m, 2H). | 374 | **A** |
| **159** | 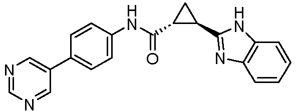 | ¹H NMR (400 MHz, DMSO-*d₆*) δ 12.47 (s, 1H), 10.59 (s, 1H), 9.16-9.11 (m, 3H), 7.82-7.75 (m, 4H), 7.54-7.39 (m, 2H), 7.18-7.09 (m, 2H), 2.62-2.56 (m, 2H), 1.66-1.57 (m, 2H). | 356 | **A** |
| **160** | 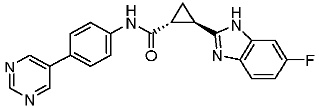 | ¹H NMR (400 MHz, DMSO-*d₆*) δ 12.60 (s, 1H), 10.59 (s, 1H), 9.21-9.10 (m, 3H), 7.85-7.73 (m, 4H), 7.51-7.41 (m, 1H), 7.29 (dd, *J* = 2.5, 9.8 Hz, 1H), 6.99 (td, *J* = 2.5, 9.4 Hz, 1H), 2.63-2.55 (m, 2H), 1.67-1.56 (m, 2H). | 374 | **A** |
| **161** | 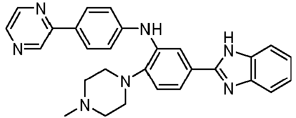 | ¹H NMR (400 MHz, DMSO-*d₆*) δ 12.76 (s, 1H), 9.18 (d, *J* = 1.5 Hz, 1H), 8.63 (dd, *J =* 1.5, 2.5 Hz, 1H), 8.48 (d, *J* = 2.5 Hz, 1H), 8.12 (d, *J* = 2.0 Hz, 1H), 8.09-8.04 (m, 2H), 7.80 (dd, *J* = 2.0, 8.3 Hz, 1H), 7.67 (s, 1H), 7.63- 7.45 (m, 2H), 7.24-7.10 (m, 5H), 3.05-2.91 (m, 4H), 2.49-2.41 (m, 4H), 2.21 (s, 3H). | 462 | **A** |
| **162** | 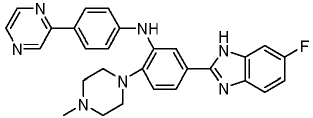 | ¹H NMR (400 MHz, DMSO-*d ₆* ) δ 13.05-12.69 (m, 1H), 9.17 (d, *J* = 1.5 Hz, 1H), 8.65-8.60 (m, 1H), 8.49-8.46 (m, 1H), 8.12-8.04 (m, 3H), 7.83-7.74 (m, 1H), 7.67 (s, 1H), 7.63-7.37 (m, 2H), 7.28-7.19 (m, 3H), 7.03 (t, *J* = 9.5 Hz, 1H), 3.04-2.94 (m, 4H), 2.50-2.41 (m, 4H), 2.20 (s, 3H). | 480 | **A** |
| **163** | 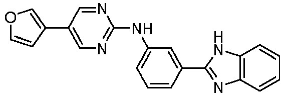 | ¹H NMR (400 MHz, DMSO-*d₆*) δ 12.84 (s, 1H), 9.91 (s, 1H), 8.85 (s, 2H), 8.69 (t, *J =* 2.0 Hz, 1H), 8.29-8.22 (m, 1H), 7.83-7.77 (m, 2H), 7.74-7.43 (m, 4H), 7.24-7.17 (m, 2H), 7.09-7.03 (m, 1H). | 354 | **B** |
| **164** | 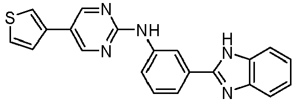 | ¹H NMR (400 MHz, DMSO-*d₆*) δ 12.85 (s, 1H), 9.94 (s, 1H), 8.96 (s, 2H), 8.68 (t, *J =* 1.9 Hz, 1H), 7.97 (dd, *J* = 1.4, 2.9 Hz, 1H), 7.88-7.82 (m, 1H), 7.75-7.45 (m, 6H), 7.25-7.16 (m, 2H). | 370 | **B** |
| **165** | 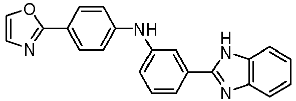 | ¹H NMR (400 MHz, DMSO-*d₆*) δ 12.88 (s, 1H), 8.81 (s, 1H), 8.14-8.10 (m, 1H), 8.08-8.03 (m, 1H), 7.92-7.85 (m, 2H), 7.77-7.70 (m, 1H), 7.69-7.45 (m, 3H), 7.32-7.14 (m, 6H). | 353 | **B** |
| **166** | 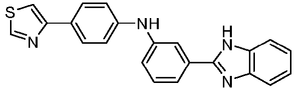 | ¹H NMR (400 MHz, DMSO-*d₆*) δ 12.86 (s, 1H), 9.16 (d, *J* = 1.9 Hz, 1H), 8.56 (s, 1H), 8.01 (t, *J* = 2.0 Hz, 1H), 7.97 (d, *J* = 1.9 Hz, 1H), 7.94-7.90 (m, 2H), 7.68-7.47 (m, 4H), 7.43 (t, *J* = 7.9 Hz, 1H), 7.25-7.17 (m, 4H). | 369 | **B** |
| **167** | 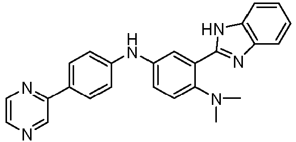 | ¹H NMR (400 MHz, DMSO-*d₆*) δ 12.87 (s, 1H), 9.18 (d, *J* = 1.6 Hz, 1H), 8.71-8.60 (m, 2H), 8.48 (d, *J* = 2.5 Hz, 1H), 8.12-8.03 (m, 3H), 7.69-7.62 (m, 2H), 7.45-7.36 (m, 1H), 7.31-7.14 (m, 5H), 2.72 (s, 6H). | 407 | **A** |
| **168** | 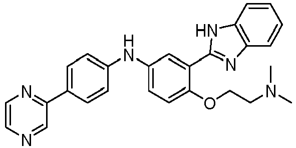 | ¹H NMR (400 MHz, DMSO-*d₆*) δ 13.38 (s, 1H), 9.17 (d, *J* = 1.5 Hz, 1H), 8.63 (dd, *J =* 1.5, 2.6 Hz, 1H), 8.58 (s, 1H), 8.48 (d, *J =* 2.5 Hz, 1H), 8.16 (s, 1H), 8.06 (d, *J* = 8.9 Hz, 2H), 7.63 (s, 2H), 7.34-7.28 (m, 2H), 7.22 (s, 2H), 7.15 (d, *J* = 8.8 Hz, 2H), 4.39 (s, 2H), 2.85 (s, 2H), 2.47-2.39 (m, 6H) | 451 | **B** |
| **169** | 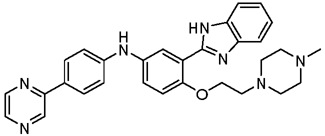 | ¹H NMR (400 MHz, DMSO-*d₆*) δ 12.07 (s, 1H), 9.17 (d, *J* = 1.6 Hz, 1H), 8.65-8.60 (m, 1H), 8.56 (s, 1H), 8.48 (d, *J* = 2.5 Hz, 1H), 8.18 (d, *J* = 2.5 Hz, 1H), 8.09-8.03 (m, 2H), 7.71-7.62 (m, 2H), 7.34-7.21 (m, 4H), 7.18-7.04 (m, 2H), 4.38 (t, *J* = 5.4 Hz, 2H), 2.99-2.57 (m, 10H), 2.40-2.28 (m, 3H). | 506 | **B** |
| **170** | 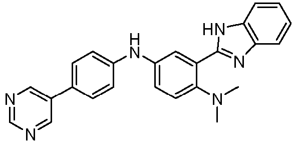 | ¹H NMR (400 MHz, DMSO-*d₆*) δ 12.65 (s, 1H), 9.13-9.07 (m, 3H), 8.53 (s, 1H), 8.08 (d, *J* = 2.8 Hz, 1H), 7.78-7.70 (m, 2H), 7.64 (t, *J* = 8.7 Hz, 2H), 7.35 (d, *J* = 8.6 Hz, 1H), 7.28-7.15 (m, 5H), 2.67 (s, 6H). | 407 | **A** |
| **171** | 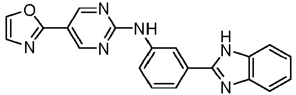 | ¹H NMR (400 MHz, DMSO-*d₆*) δ 12.87 (s, 1H), 10.32 (s, 1H), 9.05 (s, 2H), 8.60 (t, *J =* 1.9 Hz, 1H), 8.25 (d, *J* = 0.8 Hz, 1H), 7.90-7.86 (m, 1H), 7.81-7.78 (m, 1H), 7.70-7.66 (m, 1H), 7.55-7.48 (m, 2H), 7.41-7.39 (m, 1H), 7.26-7.17 (m, 2H). | 355 | **B** |
| **172** | 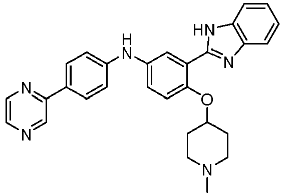 | ¹H NMR (400 MHz, DMSO-*d₆*) δ 11.78 (s, 1H), 9.15 (d, *J* = 1.6 Hz, 1H), 8.64-8.59 (m, 1H), 8.53 (s, 1H), 8.46 (d, *J* = 2.5 Hz, 1H), 8.08-8.02 (m, 3H), 7.67-7.61 (m, 2H), 7.32-7.25 (m, 2H), 7.24-7.16 (m, 2H), 7.13 (d, *J* = 8.6 Hz, 2H), 4.48-4.39 (m, 1H), 2.70-2.59 (m, 3H), 2.18-2.08 (m, 4H), 2.00-1.87 (m, 4H). | 477 | **B** |
| **173** | 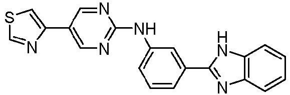 | ¹H NMR (400 MHz, DMSO-*d₆*) δ 12.85 (s, 1H), 10.05 (s, 1H), 9.27 (d, *J* = 1.9 Hz, 1H), 9.12 (s, 2H), 8.67 (t, *J* = 1.9 Hz, 1H), 8.22 (d, *J* = 2.0 Hz, 1H), 7.91-7.82 (m, 1H), 7.74 (d, *J* = 7.7 Hz, 1H), 7.71-7.45 (m, 3H), 7.21 (s, 2H). | 371 | **B** |
| **174** | 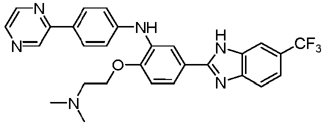 | ¹H NMR (400 MHz, DMSO-*d₆*) δ 13.16 (s, 1H), 9.19 (d, *J* = 1.6 Hz, 1H), 8.67-8.60 (m, 1H), 8.49 (d, *J* = 2.5 Hz, 1H), 8.19 (d, *J =* 2.2 Hz, 1H), 8.14-8.00 (m, 3H), 7.92-7.79 (m, 2H), 7.71 (d, *J* = 8.5 Hz, 1H), 7.47 (d, *J* = 8.4 Hz, 1H), 7.33-7.19 (m, 3H), 4.22 (t, *J* = 5.9 Hz, 2H), 2.71-2.64 (m, 2H), 2.23 (s, 6H). | 519 | **B** |
| **175** | 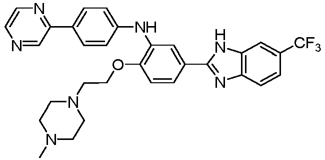 | ¹H NMR (400 MHz, DMSO-*d₆*) δ 13.18 (s, 1H), 9.18 (d, *J* = 1.6 Hz, 1H), 8.66-8.63 (m, 1H), 8.49 (d, *J* = 2.6 Hz, 1H), 8.17 (d, *J =* 2.2 Hz, 1H), 8.13-8.04 (m, 2H), 7.92-7.81 (m, 3H), 7.72 (d, *J* = 8.4 Hz, 1H), 7.48 (dd, *J* = 1.7, 8.6 Hz, 1H), 7.30 (d, *J* = 8.6 Hz, 1H), 7.22-7.18 (m, 2H), 4.24 (t, *J* = 5.9 Hz, 2H), 2.73 (t, *J* = 5.9 Hz, 2H), 2.47-2.19 (m, 8H), 2.09 (s, 3H). | 574 | **B** |
| **176** | 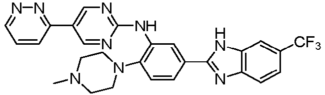 | ¹H NMR (400 MHz, DMSO-*d₆*) δ 13.29 (d, *J* = 6.6 Hz, 1H), 9.31 (s, 2H), 9.22 (dd, *J =* 1.6, 4.9 Hz, 1H), 9.04-8.98 (m, 1H), 8.80 (s, 1H), 8.36-8.26 (m, 1H), 8.06-7.67 (m, 4H), 7.52 (t, *J* = 8.3 Hz, 1H), 7.40 (d, *J* = 8.4 Hz, 1H), 3.04-2.96 (m, 4H), 2.67-2.55 (m, 4H), 2.30 (s, 3H). | 532 | **A** |
| **177** | 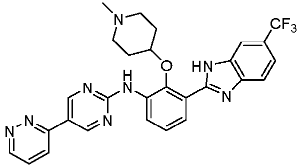 | ¹H NMR (400 MHz, DMSO-*d₆*) δ 12.72 (s, 1H), 9.30-9.17 (m, 4H), 8.31-8.22 (m, 1H), 8.08-7.77 (m, 5H), 7.60-7.51 (m, 1H), 7.36 (t, *J =* 7.9 Hz, 1H), 3.86-3.72 (m, 1H), 1.96 (s, 3H), 1.74-1.49 (m, 6H), 1.38-1.09 (m, 2H). | 547 | **B** |
| **178** | 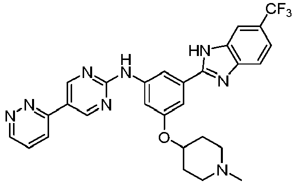 | ¹H NMR (400 MHz, DMSO-*d₆*) δ 13.07 (s, 1H), 10.24 (s, 1H), 9.30 (d, *J* = 5.0 Hz, 2H), 9.22 (dd, *J* = 1.6, 4.9 Hz, 1H), 8.31 (d, *J =* 9.0 Hz, 2H), 7.87-7.63 (m, 4H), 7.54 (s, 1H), 7.42 (s, 1H), 4.52-4.41 (m, 1H), 2.73-2.63 (m, 2H), 2.28-2.14 (m, 5H), 2.12-1.98 (m, 2H), 1.79-1.66 (m, 2H). | 547 | **B** |
| **179** | 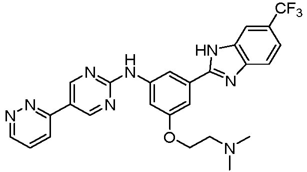 | ¹H NMR (400 MHz, DMSO-*d₆*) δ 13.63-13.50 (m, 1H), 10.34 (s, 1H), 9.36-9.29 (m, 2H), 9.23 (dd, *J* = 1.6, 4.9 Hz, 1H), 8.38-8.28 (m, 2H), 8.08-7.70 (m, 4H), 7.63-7.49 (m, 2H), 4.53-4.36 (m, 2H), 3.52-3.44 (m, 2H), 2.81 (s, 6H). | 521 | **B** |
| **200** | 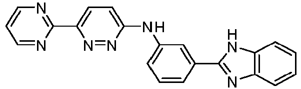 | ¹H NMR (400 MHz, DMSO-*d₆*) δ 10.01 (s, 1H), 9.00 (d, *J* = 5.2 Hz, 2H), 8.85 (s, 1H), 8.38 (d, *J =* 9.2 Hz, 1H), 7.89 (d, *J =* 8.0 Hz, 1H), 7.83-7.77 (m, 3H), 7.67 (t, *J* = 7.8 Hz, 1H), 7.59 (t, *J* = 4.8 Hz, 1H), 7.47-7.45 (m, 2H), 7.40 (d, *J* = 9.6 Hz, 1H). | 366 | **A** |
| **201** | 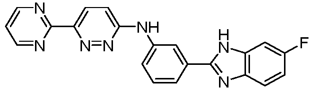 | ¹H NMR (400 MHz, DMSO-*d₆*) δ 9.96 (s, 1H), 9.00 (d, *J* = 4.0 Hz, 2H), 8.81 (s, 1H), 8.38 (d, *J* = 9.2 Hz, 1H), 7.86 (d, *J =* 8.4 Hz, 1H), 7.78 (d, *J* = 8.0 Hz, 1H), 7.71-7.68 (m, 1H), 7.62-7.59 (m, 2H), 7.50 (d, *J =* 9.2 Hz, 1H), 7.38 (d, *J* = 9.2 Hz, 1H), 7.19 (t, *J =* 9.4 Hz, 1H). | 384 | **A** |
| **202** | 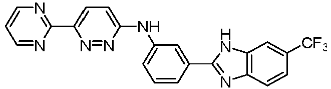 | ¹H NMR (400 MHz, DMSO-*d₆*) δ 13.42 (s, 1H), 9.41 (s, 1H), 9.05 (d, *J* = 4.8 Hz, 2H), 8.90 (s, 1H), 8.40 (d, *J* = 9.6 Hz, 1H), 8.16 (s, 1H), 7.97 (d, *J =* 7.6 Hz, 1H), 7.87 (d, *J* = 7.6 Hz, 1H), 7.80 (s, 1H), 7.66-7.59 (m, 3H), 7.39 (d, *J* =9.6 Hz, 1H). | 434 | **A** |
| **203** | 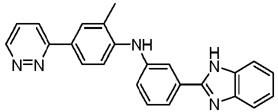 | ¹H NMR (400 MHz, DMSO-*d₆*) δ 12.84 (s, 1H), 9.13 (d, *J =* 3.6 Hz, 1H), 8.17 (d, *J* = 8.4 Hz, 1H), 8.09 (s, 1H), 7.97-7.88 (m, 3H), 7.74-7.64 (m, 3H), 7.51-7.37 (m, 3H), 7.19 (d, *J =* 5.6 Hz, 3H), 2.38 (s, 3H). | 378 | **A** |
| **204** | 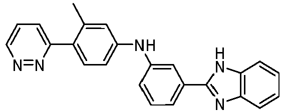 | ¹H NMR (400 MHz, DMSO-*d₆*) δ 9.20 (dd, *J* = 1.2, 4.8 Hz, 1H), 8.78 (s, 1H), 8.00 (s, 1H), 7.86-7.75 (m, 4H), 7.67 (d, *J* = 8.0 Hz, 1H), 7.58 (t, *J* = 8.0 Hz, 1H), 7.51-7.37 (m, 4H), 7.21-7.15 (m, 2H), 2.36 (s, 3H). | 378 | A |
| **205** | 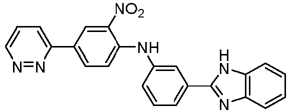 | ¹H NMR (400 MHz, DMSO-*d₆*) δ 12.97 (s, 1H), 9.75 (s, 1H), 9.20 (dd, *J =* 1.2, 4.8 Hz, 1H), 8.98 (d, *J* = 2.0 Hz, 1H), 8.36 (dd, *J =* 2.0, 8.8 Hz, 1H), 8.28 (dd, *J* = 1.6, 8.8 Hz, 1H), 8.21 (s, 1H), 8.06 (d, *J* = 8.0 Hz, 1H), 7.78 (dd, *J =* 4.8, 8.8 Hz, 1H), 7.68-7.63 (m, 2H), 7.53 (t, *J* = 6.0 Hz, 2H), 7.43 (d, *J =* 9.2 Hz, 1H), 7.25-7.18 (m, 2H). | 409 | **A** |
| **206** | 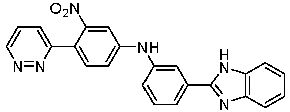 | ¹H NMR (400 MHz, DMSO-*d₆*) δ 12.93 (s, 1H), 9.21-9.17 (m, 2H), 8.09 (s, 1H), 7.97 (dd, *J =* 1.6, 8.4 Hz, 1H), 7.84-7.79 (m, 2H), 7.69 (d, *J* = 8.4 Hz, 1H), 7.62 (d, *J =* 2.4 Hz, 1H), 7.57-7.49 (m, 3H), 7.34 (dd, *J* = 1.6, 8.0 Hz, 1H), 7.21 (d, *J =* 5.2 Hz, 2H). | 409 | **A** |
| **207** | 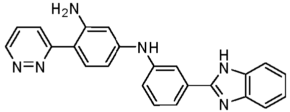 | ¹H NMR (400 MHz, DMSO-*d₆*) δ 12.86 (s, 1H), 9.00 (dd, *J =* 1.6, 4.8 Hz, 1H), 8.48 (s, 1H), 8.09-8.02 (m, 2H), 7.68-7.65 (m, 3H), 7.59 (d, *J =* 8.8 Hz, 1H), 7.51 (d, *J =* 6.8 Hz, 1H), 7.45 (t, *J* = 7.8 Hz, 1H), 7.25-7.18 (m, 3H), 7.02 (s, 2H), 6.61 (d, *J* =2.0 Hz, 1H), 6.48 (dd, *J =* 2.4, 8.8 Hz, 1H). | 379 | **A** |
| **208** | 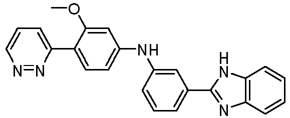 | ¹H NMR (400 MHz, DMSO-*d₆*) δ 12.90 (s, 1H), 9.08 (d, *J* = 4.8 Hz, 1H), 8.76 (s, 1H), 8.11 (s, 1H), 8.04 (d, *J* = 8.4 Hz, 1H), 7.82 (d, *J* = 8.8 Hz, 1H), 7.71 (d, *J* =7.6 Hz, 1H), 7.66-7.63 (m, 2H), 7.53-7.46 (m, 2H), 7.29-7.18 (m, 3H), 6.92-6.90 (m, 2H), 3.84 (s, 3H). | 394 | **A** |
| **209** | 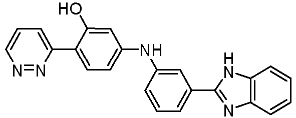 | ¹H NMR (400 MHz, DMSO-*d₆*) δ 9.05 (d, *J* =3.6 Hz, 1H), 8.76 (s, 1H), 8.41 (d, *J* =8.8 Hz, 1H), 8.08 (s, 1H), 7.93 (d, *J* =8.8 Hz, 1H), 7.81-7.73 (m, 2H), 7.59-7.57 (m, 2H), 7.47 (t, *J* = 7.8 Hz, 1H), 7.24 (d, *J* = 8.8 Hz, 1H), 7.19-7.16 (m, 2H), 6.74-6.69 (m, 2H). | 380 | **A** |
| **210** | 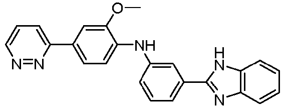 | ¹H NMR (400 MHz, DMSO-*d₆*) δ 12.85 (s, 1H), 9.14 (dd, *J =* 1.2, 4.8 Hz, 1H), 8.22 (dd, *J* = 1.2, 8.4 Hz, 1H), 8.05 (t, *J =* 1.8 Hz, 1H), 7.94 (s, 1H), 7.90 (d, *J =* 2.0 Hz, 1H), 7.75-7.64 (m, 4H), 7.51 (d, *J =* 6.8 Hz, 1H), 7.46-7.41 (m, 2H), 7.29 (dd, *J =* 1.2, 8.0 Hz, 1H), 7.23-7.16 (m, 2H), 4.00 (s, 3H). | 394 | **A** |
| **211** | 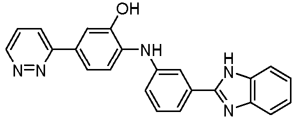 | ¹H NMR (400 MHz, DMSO-*d₆*) δ 12.83 (s, 1H), 9.97 (s, 1H), 9.11 (dd, *J =* 1.6, 4.8 Hz, 1H), 8.14 (s, 1H), 8.09 (dd, *J =* 1.2, 8.4 Hz, 1H), 8.00 (t, *J =* 1.8 Hz, 1H), 7.82 (d, *J* =2.0 Hz, 1H), 7.76 (s, 1H), 7.71 (dd, *J =* 4.8, 8.8 Hz, 1H), 7.63 (d, *J =* 7.6 Hz, 2H), 7.55 (dd, *J =* 2.0, 8.4 Hz, 1H), 7.43-7.37 (m, 2H), 7.25 (dd, *J* = 1.2, 8.0 Hz, 1H), 7.19 (d, *J =* 4.8 Hz, 2H). | 380 | **A** |
| **212** | 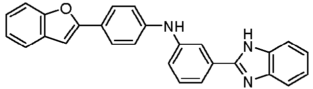 | ¹H NMR (400 MHz, DMSO-*d₆*) δ 12.89 (s, 1H), 8.72 (s, 1H), 8.05 (s, 1H), 7.84 (d, *J =* 8.8 Hz, 2H), 7.71-7.58 (m, 5H), 7.46 (t, *J =* 7.6 Hz, 1H), 7.29-7.19 (m, 8H). | 402 | **A** |
| **213** | 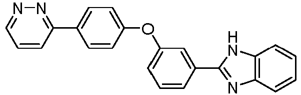 | ¹H NMR (400 MHz, DMSO-*d₆*) δ 9.20 (dd, *J =* 4.8, 1.2 Hz, 1H), 8.25-8.22 (m, 3H), 8.03 (d, *J =* 7.6 Hz, 1H), 7.90 (t, *J =* 1.8 Hz, 1H), 7.78 (dd, *J =* 8.4, 4.8 Hz, 1H), 7.63-7.54 (m, 3H), 7.28-7.24 (m, 3H), 7.16 (dd, *J* = 6.0, 3.2 Hz, 2H). | 365 | **A** |
| **214** | 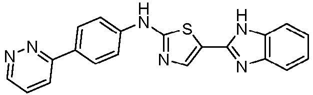 | ¹H NMR (400 MHz, DMSO-*d₆*) δ 12.95 (s, 1H), 10.88 (s, 1H), 9.16 (d, *J =* 4.0 Hz, 1H), 8.19 (d, *J =* 8.4 Hz, 3H), 8.05 (s, 1H), 7.87 (d, *J =* 8.8 Hz, 2H), 7.75 (dd, *J* = 8.8, 5.2 Hz, 1H), 7.54 (t, *J =* 8.0 Hz, 2H), 7.19 (dd, *J* = 6.0, 3.2 Hz, 2H). | 371 | **A** |
| **215** | 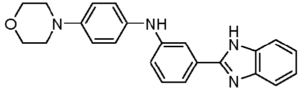 | ¹H NMR (400 MHz, DMSO-*d₆*) δ 8.33 (s, 1H), 7.81-7.73 (m, 3H), 7.54-7.51 (m, 4H), 7.17 (s, 3H), 7.03 (s, 2H), 3.78 (s, 4H), 3.12 (s, 4H). | 371 | **A** |
| **216** | 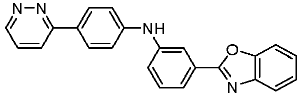 | ¹H NMR (400 MHz, DMSO-*d₆*) δ 9.13 (dd, *J =* 1.6, 5.2 Hz, 1H), 8.89 (s, 1H), 8.18-8.13 (m, 3H), 8.02 (t, *J =* 1.8 Hz, 1H), 7.83-7.80 (m, 2H), 7.74-7.71 (m, 2H), 7.54 (t, *J =* 7.8 Hz, 1H), 7.46-7.40 (m, 3H), 7.31 (d, *J =* 8.8 Hz, 2H). | 365 | **A** |
| **217** | 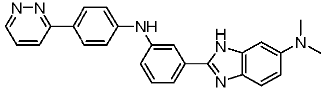 | ¹H NMR (400 MHz, DMSO-*d₆*) δ 12.45 (s, 1H), 9.11 (d, *J* = 4.8 Hz, 1H), 8.73 (s, 1H), 8.15-7.99 (m, 4H), 7.73-7.61 (m, 2H), 7.47-7.19 (m, 5H), 6.96-6.70 (m, 2H), 2.93 (s, 6H). | 407 | **A** |
| **218** | 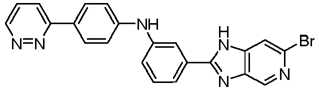 | ¹H NMR (400 MHz, DMSO-*d₆*) δ 13.54 (bs, 1H), 9.13 (d, *J* = 4.0 Hz, 1H), 8.83 (s, 1H), 8.73 (s, 1H), 8.16-8.09 (m, 4H), 7.79-7.70 (m, 3H), 7.51 (t, *J* = 8.0 Hz, 1H), 7.34-7.29 (m, 3H). | 443 | **A** |
| **219** | 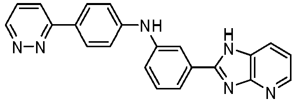 | ¹H NMR (400 MHz, DMSO-*d₆*) δ13.44 (bs, 1H), 9.12 (d, *J* = 4.8 Hz, 1H), 8.79 (s, 1H), 8.34 (d, *J =* 4.8 Hz, 1H), 8.15-8.11 (m, 4H), 8.00 (d, *J =* 7.6 Hz, 1H), 7.77-7.69 (m, 2H), 7.48 (t, *J =* 8.0 Hz, 1H), 7.31-7.22 (m, 4H). | 365 | **A** |
| **220** | 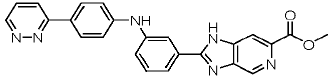 | ¹H NMR (400 MHz, DMSO-*d₆*) δ13.70 (s, 1H), 9.12 (dd, *J =* 1.2, 4.8 Hz, 1H), 9.01 (s, 1H), 8.85 (s, 1H), 8.27 (s, 1H), 8.17-8.12 (m, 4H), 7.77-7.70 (m, 2H), 7.53 (d, *J =* 7.8 Hz, 1H), 7.36-7.30 (m, 3H), 3.90 (s, 3H). | 423 | **A** |
| **221** | 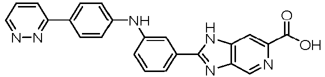 | ¹H NMR (400 MHz, DMSO-*d₆*) δ 13.71 (s, 1H), 9.12 (d, *J* = 4.4 Hz, 1H), 9.04 (s, 1H), 8.85 (s, 1H), 8.34-8.12 (m, 5H), 7.77-7.70 (m, 2H), 7.54 (t, d, *J* = 7.8 Hz, 1H), 7.36-7.30 (m, 3H). | 409 | **A** |
| **222** | 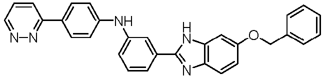 | ¹H NMR (400 MHz, DMSO-*d₆*) δ12.74 (s, 1H), 9.11 (dd, *J* = 4.8, 1.6 Hz, 1H), 8.75 (s, 1H), 8.16-8.11 (m, 3H), 8.02 (s, 1H), 7.73-7.64 (m, 2H), 7.50-7.38 (m, 6H), 7.35-7.22 (m, 4H), 7.04 (bs, 1H), 6.91 (d, *J* = 8.8 Hz, 1H), 5.16 (s, 2H). | 470 | **A** |
| **223** | 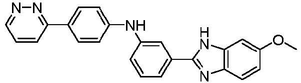 | ¹H NMR (400 MHz, DMSO-*d₆*) δ12.73 (s, 1H), 9.11 (dd, *J =* 1.2 Hz, 4.8 Hz, 1H), 8.75 (s, 1H), 8.15-8.11 (m, 3H), 8.03 (s, 1H), 7.73-7.65 (m, 2H), 7.47-7.43 (m, 2H), 7.30-7.22 (m, 3H), 7.07 (s, 1H), 6.83 (dd, *J =* 2.0, 8.8 Hz, 1H), 3.81 (s, 3H). | 394 | **A** |
| **224** | 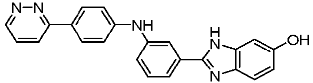 | ¹H NMR (400 MHz, DMSO-*d₆*) δ12.52 (s, 1H), 9.11 (d, *J* = 4.4 Hz, 1H), 8.74 (s, 1H), 8.20-8.10 (m, 4H), 7.99 (s, 1H), 7.71 (dd, *J* = 4.8, 8.4 Hz, 1H), 7.62 (d, *J =* 7.6 Hz, 1H), 7.46-7.37 (m, 2H), 7.29-7.20 (m, 3H), 6.89 (s, 1H), 6.68 (dd, *J =* 1.6, 8.4 Hz, 1H). | 380 | **A** |
| **225** | 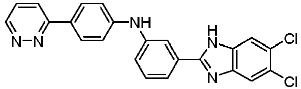 | ¹H NMR (400 MHz, DMSO-*d₆*) δ 9.12 (dd, *J =* 4.8, 1.2Hz, 1H), 8.80 (s, 1H), 8.16-8.11 (m, 3H), 8.05 (s, 1H), 7.84 (bs, 2H), 7.74-7.68 (m, 2H), 7.49 (t, *J =* 8.0 Hz, 1H), 7.31-7.27 (m, 3H). | 432 | **A** |
| **226** | 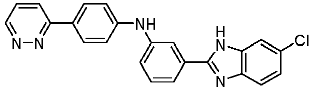 | ¹H NMR (400 MHz, DMSO-*d₆*) δ13.10 (d, *J* = 13.6 Hz, 1H), 9.12 (dd, *J* = 4.8, 1.2Hz, 1H), 8.78 (s, 1H), 8.16-8.11 (m, 3H), 8.05 (s, 1H), 7.73-7.65 (m, 3H), 7.54-7.46 (m, 2H), 7.29-7.19 (m, 4H). | 398 | **A** |
| **227** | 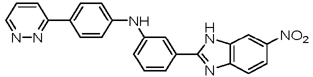 | ¹H NMR (400 MHz, DMSO-*d₆*) δ 9.12 (dd, *J* = 1.2, 4.8 Hz, 1H), 8.82 (s, 1H), 8.47 (d, *J* = 2.0 Hz, 1H), 8.16-8.11 (m, 5H), 7.77-7.70 (m, 3H), 7.51 (t, *J* = 8.0 Hz, 1H), 7.34-7.29 (m, 3H). | 409 | **A** |
| **228** | 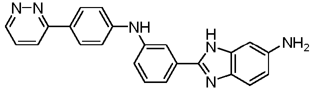 | ¹H NMR (400 MHz, DMSO-*d₆*) δ 9.11 (d, *J* = 1.6, 4.8 Hz, 1H), 8.70 (s, 1H), 8.15-8.10 (m, 4H), 7.96 (s, 1H), 7.70 (dd, *J* = 4.8, 8.8 Hz, 1H), 7.60 (d, *J* = 7.6 Hz, 1H), 7.42 (t, *J* = 7.8 Hz, 1H), 7.28-7.26 (m, 3H), 7.19 (dd, *J =* 1.6, 8.0 Hz, 1H), 6.68 (s, 1H), 6.53 (dd, *J* = 1.6, 8.4 Hz, 1H). | 379 | **A** |
| **229** | 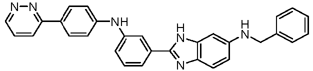 | ¹H NMR (400 MHz, DMSO-*d₆*) δ 12.38 (s, 1H), 9.11 (d, *J* = 4.8 Hz, 1H), 8.69 (s, 1H), 8.14-8.09 (m, 3H), 7.94 (s, 1H), 7.72-7.68 (m, 1H), 7.57 (d, *J* = 7.6 Hz, 1H), 7.42-7.40 (m, 3H), 7.34-7.30 (m, 3H), 7.27-7.16 (m, 4H), 6.66 (d, *J* = 8.8 Hz, 1H), 6.51 (s, 1H), 6.21 (s, 1H), 4.32 (s, 2H). | 469 | **A** |
| **230** | 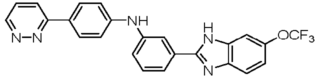 | ¹H NMR (400 MHz, DMSO-*d₆*) δ13.18 (s, 1H), 9.12 (d, *J* = 4.8 Hz, 1H), 8.79 (s, 1H), 8.16-8.06 (m, 4H), 7.73-7.47 (m, 5H), 7.29 (d, *J =* 8.8 Hz, 3H), 7.20 (d, *J =* 8.4 Hz, 1H). | 448 | **A** |
| **231** | 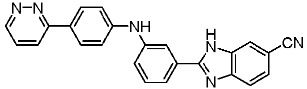 | ¹H NMR (400 MHz, DMSO-*d₆*) δ 9.13 (dd, *J* = 1.2, 4.4 Hz, 1H), 8.83 (s, 1H), 8.18-8.10 (m, 5H), 7.77-7.72 (m, 3H), 7.61 (dd, *J =* 1.6, 8.4 Hz, 1H), 7.51 (t, *J* = 8.0 Hz, 1H), 7.34-7.29 (m, 3H). | 389 | **A** |
| **232** | 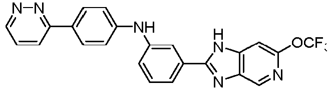 | ¹H NMR (400 MHz, DMSO-*d₆*) δ 9.12 (d, *J* = 4.8 Hz, 1H), 8.83 (s, 1H), 8.70 (s, 1H), 8.17-8.10 (m, 4H), 7.75-7.71 (m, 2H), 7.52 (t, *J =* 7.8 Hz, 1H), 7.45 (s, 1H), 7.34-7.29 (m, 3H). | 449 | **A** |
| **234** | 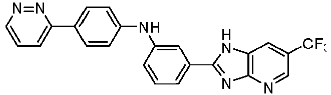 | ¹H NMR (400 MHz, DMSO-*d₆*) δ 13.98 (s, 1H), 9.12 (d, *J* = 4.0 Hz, 1H), 8.84 (s, 1H), 8.71 (s, 1H), 8.41 (s, 1H), 8.15-8.12 (m, 4H), 7.80 (d, *J =* 7.6 Hz, 1H), 7.72 (dd, *J* = 4.8, 8.8 Hz, 1H), 7.52 (t, *J* = 7.6 Hz, 1H), 7.36-7.30 (m, 3H). | 433 | **A** |
| **235** | 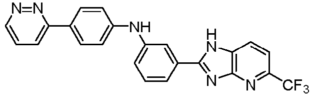 | ¹H NMR (400 MHz, DMSO-*d₆*) δ 14.01 (s, 1H), 9.12 (d, *J* = 4.4 Hz, 1H), 8.86 (s, 1H), 8.23-8.13 (m, 5H), 7.79-7.70 (m, 3H), 7.53 (t, *J* =7.8 Hz, 1H), 7.36-7.31 (m, 3H). | 433 | **A** |
| **238** | 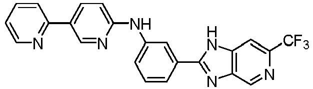 | ¹H NMR (400 MHz, DMSO-*d₆*) δ 9.59 (s, 1H), 9.07 (s, 1H), 8.97 (d, *J* = 2.4 Hz, 1H), 8.70-8.64 (m, 2H), 8.33 (dd, *J =* 2.4, 8.8 Hz, 1H), 8.06 (s, 1H), 7.96 (d, *J* = 8.0 Hz, 2H), 7.88-7.84 (m, 1H), 7.78 (d, *J* = 8.0 Hz, 1H), 7.53 (t, *J* = 8.0 Hz, 1H), 7.32-7.29 (m, 1H), 7.01 (d, *J =* 8.8 Hz, 1H). | 433 | **A** |
| **242** | 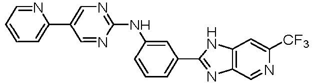 | ¹H NMR (400 MHz, DMSO-*d₆*) δ 10.22 (s, 1H), 9.24 (s, 2H), 9.09 (s, 1H), 8.80 (s, 1H), 8.68 (d, *J* = 8.0 Hz, 1H),8.09-7.83 (m, 5H), 7.57 (t, *J =* 7.8 Hz, 1H), 7.39-7.36 (m, 1H). | 434 | **A** |
| **243** | 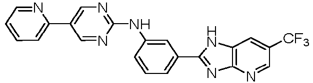 | ¹H NMR (400 MHz, DMSO-*d*₆) δ 14.06 (s, 1H), 10.20 (s, 1H), 9.23 (s, 2H), 8.79-8.23 (m, 3H), 8.51 (s, 1H), 8.04-7.86 (m, 4H), 7.56 (t, *J =* 6.8 Hz, 1H), 7.56 (t, *J =* 5.8 Hz, 1H). | 434 | **A** |
| **244** | 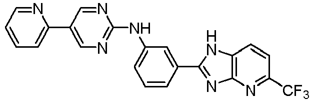 | ¹H NMR (400 MHz, DMSO-*d*₆) δ 14.07 (s, 1H), 13.63 (s, 1H), 10.22 (s, 1H), 9.24 (s, 2H), 8.84 (s, 1H), 8.68 (d, *J* = 4.4 Hz, 1H), 8.26 (s, 1H), 8.04 (d, *J* = 7.6 Hz, 1H), 7.97 (d, *J* = 8.4 Hz, 1H), 7.93-7.89 (m, 1H), 7.85 (d, *J =* 7.6 Hz, 1H), 7.75 (d, *J =* 8.0 Hz, 1H), 7.56 (t, *J* = 7.8 Hz, 1H), 7.37 (dd, *J* = 6.4, 4.8 Hz, 1H), | 434 | **A** |
| **247** | 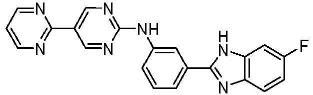 | ¹H NMR (400 MHz, DMSO-*d₆*) δ 10.24 (bs, 1H), 9.36 (s, 2H), 8.90 (d, *J* = 4.8 Hz, 2H), 8.61 (s, 1H), 7.88 (d, *J* = 8.4 Hz, 1H), 7.80 (d, *J =* 7.6 Hz, 1H), 7.56-7.53 (m, 1H), 7.49-7.43 (m, 2H), 7.33 (d, *J* = 9.2 Hz, 1H), 6.98 (t, *J =* 8.8 Hz, 1H). | 384 | **A** |
| **249** | 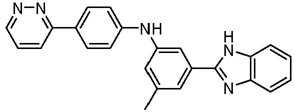 | ¹H NMR (400 MHz, DMSO-*d₆*) δ 13.04 (s, 1H), 9.12 (s, 1H), 8.17 (s, 1H), 8.16-8.11 (m, 3H), 7.87 (s, 1H), 7.72 (s, 1H), 7.59-7.57 (m, 3H), 7.29-7.22 (m, 4H), 7.09 (s, 1H), 2.40 (s, 3H). | 378 | **A** |
| **250** | 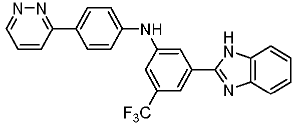 | ¹H NMR (400 MHz, DMSO-*d₆*) δ 13.13 (s, 1H), 9.16-9.10 (m, 2H), 8.32 (s, 1H), 8.19 (d, *J =* 8.8 Hz, 3H), 8.00 (s, 1H), 7.76-7.69 (m, 2H), 7.56 (d, *J* = 7.6 Hz, 1H), 7.46 (s, 1H), 7.37 (d, *J =* 8.8 Hz, 2H), 7.28-7.20 (m, 2H). | 432 | **A** |
| **251** | 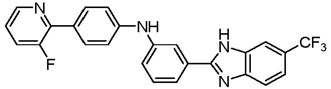 | ¹H NMR (400 MHz, DMSO-*d₆*) δ 13.33 (d, *J* = 4.8 Hz, 1H), 8.76 (s, 1H), 8.52-8.50 (m, 1H), 8.08 (t, *J* = 1.6 Hz, 1H), 8.04 (s, 1H), 7.92 (d, *J =* 7.6 Hz, 2H), 7.88-7.81 (m, 1H), 7.79-7.71 (m, 2H), 7.55-7.48 (m, 2H), 7.41-7.37 (m, 1H), 7.32-7.26 (m, 3H). | 449 | **A** |
| **252** | 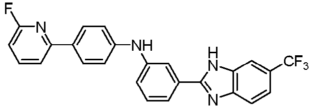 | ¹H NMR (400 MHz, DMSO-*d₆*) δ 13.33 (s, 1H), 8.79 (s, 1H), 8.08-7.72 (m, 8H), 7.54-7.48 (m, 2H), 7.31-7.23 (m, 3H), 7.01 (dd, *J* = 2.8, 8.0 Hz, 1H). | 449 | **A** |
| **253** | 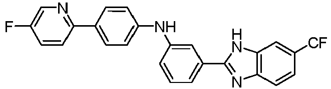 | ¹H NMR (400 MHz, DMSO-*d₆*) δ 13.33 (s, 1H), 8.71 (s, 1H), 8.60 (d, *J* =2.8 Hz, 1H), 8.07-7.86 (m, 5H), 7.83-7.70 (m, 3H), 7.56-7.47 (m, 2H), 7.30-7.25 (m, 3H). | 449 | **A** |
| **254** | 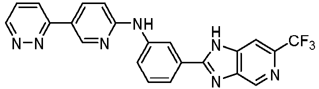 | ¹H NMR (400 MHz, DMSO-*d₆*) δ 13.82 (s, 1H), 9.72 (s, 1H), 9.17-9.02 (m, 3H), 8.72 (s, 1H), 8.43 (dd, *J* = 2.0, 8.4 Hz, 1H), 8.24 (d, *J* = 8.0 Hz, 1H), 8.07 (s, 1H), 7.98 (d, *J =* 8.0 Hz, 1H), 7.81-7.74 (m, 2H), 7.56 (t, *J =* 7.6 Hz, 1H), 7.07 (d, *J =* 8.4 Hz, 1H). | 434 | **A** |
| **255** | 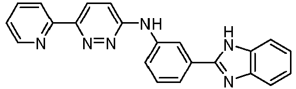 | ¹H NMR (400 MHz, DMSO-*d₆*) δ 9.82 (s, 1H), 8.79 (s, 1H), 8.71 (d, *J* = 3.2 Hz, 1H), 8.48 (d, *J =* 7.6 Hz, 1H), 8.39 (d, *J =* 8.8 Hz, 1H), 7.99 (t, *J* = 7.4 Hz, 1H), 7.90 (d, *J =* 8.0 Hz, 1H), 7.78-7.69 (m, 3H), 7.60 (t, *J =* 8.0 Hz, 1H), 7.50-7.35 (m, 4H). | 365 | **A** |
| **256** | 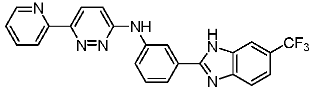 | ¹H NMR (400 MHz, DMSO-*d₆*) δ 13.36 (s, 1H), 9.79 (s, 1H), 8.82 (s, 1H), 8.71 (d, *J =* 4.4 Hz, 1H), 8.49 (d, *J* = 8.0 Hz, 1H), 8.38 (d, *J =* 9.2 Hz, 1H), 8.08-7.93 (m, 3H), 7.85-7.74 (m, 2H), 7.60-7.46 (m, 3H),7.35 (d, *J =* 9.6 Hz, 1H). | 433 | **A** |
| **257** | 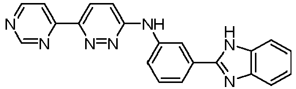 | ¹H NMR (400 MHz, DMSO-*d₆*) δ 12.91 (s, 1H), 9.94 (s, 1 H),9.31 (s, 1H), 8.96 (d, *J =* 5.2 Hz, 1H), 8.75 (s, 1H), 8.49-8.39 (m, 2H), 7.88 (d, *J =* 7.6 Hz, 1H), 7.80 (d, *J =* 7.2 Hz, 1H), 7.70 (d, *J* = 7.6 Hz, 1H), 7.57-7.54 (m, 2H), 7.38 (d, *J* = 9.2 Hz, 1H), 7.22 (t, *J =* 6.6 Hz, 2H). | 366 | **A** |
| **258** | 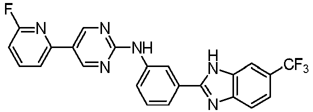 | ¹H NMR (400 MHz, DMSO-*d₆*) δ 10.27 (s, 1H), 9.20 (s, 2H), 8.73 (s, 1H), 8.10 (dd, *J =* 16.0, 8.0 Hz, 1H), 8.00-7.91 (m, 3H), 7.81 (d, *J =* 7.6 Hz, 2H), 7.56-7.53 (m, 2H), 7.14 (dd, *J =* 8.0, 2.4 Hz, 1H). | 451 | **A** |
| **259** | 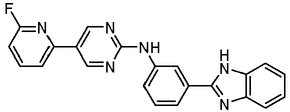 | ¹H NMR (400 MHz, DMSO-*d₆*) δ 10.40 (s, 1H), 9.22 (s, 2H), 8.75 (s, 1H), 8.11 (dd, *J =* 16.0, 8.0 Hz, 1H), 8.01-7.93 (m, 2H), 7.80-7.77 (m, 3H), 7.64 (t, *J* = 8.0 Hz, 1H), 7.48-7.46 (m, 2H), 7.15 (dd, *J =* 8.0, 2.4 Hz, 1H). | 383 | **A** |
| **260** | 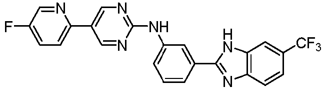 | ¹H NMR (400 MHz, DMSO-*d₆*) δ 13.30 (s, 1H), 10.17 (s, 1H), 9.20 (s, 2H), 8.68 (t, *J =* 10.0 Hz, 2H), 8.11 (dd, *J =* 8.8, 4.4 Hz, 1H), 7.95-7.78 (m, 5H), 7.56-7.52 (m, 2H). | 451 | **A** |
| **261** | 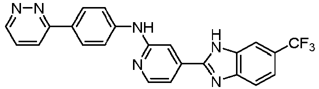 | ¹H NMR (400 MHz, DMSO-*d₆*) δ 13.62 (bs, 1H), 9.69 (s, 1H), 9.14 (d, *J* = 4.4 Hz, 1H), 8.44 (d, *J =* 5.2 Hz, 1H), 8.18-8.13 (m, 3H), 8.03 (s, 1H), 7.93 (d, *J =* 8.4 Hz, 2H), 7.85-7.79 (m, 2H), 7.75-7.71 (m, 1H), 7.59 (d, *J* = 8.0 Hz, 1H), 7.54 (d, *J =* 5.2 Hz, 1H). | 433 | **B** |
| **262** | 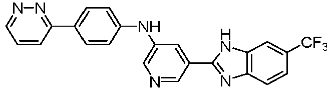 | ¹H NMR (400 MHz, DMSO-*d₆*) δ 13.55 (s, 1H), 9.14 (d, *J* = 4.4 Hz, 1H), 9.01 (s, 1H), 8.90 (s, 1H), 8.52 (s, 1H), 8.36 (s, 1H), 8.18-8.16 (m, 3H), 7.98 (s, 1H), 7.80 (d, *J* = 8.4 Hz, 1H), 7.75-7.72 (m, 1H), 7.54 (d, *J* = 8.4 Hz, 1H), 7.35 (d, *J =* 8.4 Hz, 2H). | 433 | **B** |
| **263** | 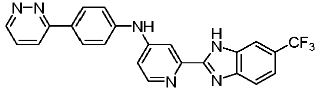 | ¹H NMR (400 MHz, DMSO-*d₆*) δ 13.43 (bs, 1H), 9.44 (s, 1H), 9.18 (d, *J* = 4.0 Hz, 1H), 8.45 (d, *J =* 5.6 Hz, 1H), 8.23 (d, *J =* 8.4 Hz, 3H), 8.06-7.87 (m, 2H), 7.82-7.70 (m, 2H), 7.56-7.51 (m, 1H), 7.46 (d, *J* = 8.8 Hz, 2H), 7.19 (dd, *J =* 5.6, 2.4 Hz, 1H). | 433 | B |
| **264** | 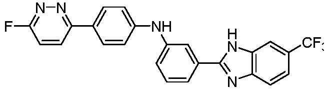 | ¹H NMR (400 MHz, DMSO-*d₆*) δ 13.34 (s, 1H), 8.83 (s, 1H), 8.43-8.39 (m, 1H), 8.09-8.04 (m, 3H), 7.85-7.71 (m, 4H), 7.54-7.49 (m, 2H), 7.32-7.27 (m, 3H). | 450 | B |
| **265** | 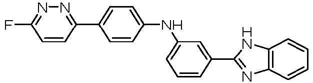 | ¹H NMR (400 MHz, DMSO-*d₆*) δ 12.89 (s, 1H), 8.79 (s, 1H), 8.42-8.38 (m, 1H), 8.09-8.06 (m, 3H), 7.74-7.70 (m, 2H), 7.64-7.45 (m, 3H), 7.29-7.25 (m, 3H), 7.20-7.19 (m, 2H). | 382 | **B** |
| **266** | 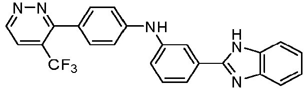 | ¹H NMR (400 MHz, DMSO-*d₆*) δ 9.54 (d, *J* = 5.2 Hz, 1H), 8.77 (s, 1H), 8.19 (d, *J =* 5.2 Hz, 1H), 8.08 (t, *J =* 2.0 Hz, 1H), 7.72 (d, *J* = 8.0 Hz, 1H), 7.60-7.58 (m, 2H), 7.52-7.45 (m, 3H), 7.29-7.26 (m, 3H), 7.20-7.17 (m, 2H). | 432 | **B** |
| **267** | 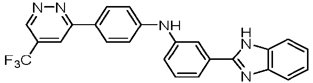 | ¹H NMR (400 MHz, DMSO-*d₆*) δ 12.90 (s, 1H), 9.53 (d, *J* = 1.6 Hz, 1H), 8.88 (s, 1H), 8.55 (d, *J =* 0.8 Hz, 1H), 8.26 (d, *J =* 8.8 Hz, 2H), 8.09 (t, *J =* 1.6 Hz, 1H), 7.73 (d, *J =* 8.0 Hz, 1H), 7.66 (d, *J* = 7.2 Hz, 1H), 7.53-7.47 (m, 2H), 7.31-7.17 (m, 5H). | 432 | **B** |
| **268** | 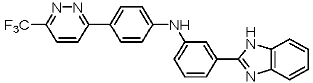 | ¹H NMR (400 MHz, DMSO-*d₆*) δ 12.90 (s, 1H), 8.92 (s, 1H), 8.43 (d, *J* =9.2 Hz, 1H), 8.24-8.21 (m, 3H), 8.09 (s, 1H), 7.74 (d, *J* =8.0 Hz, 1H), 7.65-7.47 (m, 3H), 7.32-7.28 (m, 3H), 7.21 (d, *J* =5.2 Hz, 2H). | 432 | **B** |
| **269** | 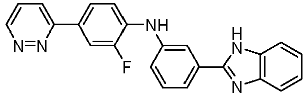 | ¹H NMR (400 MHz, DMSO-*d₆*) δ 13.20 (s, 1H), 9.17 (dd, *J* = 4.8, 1.2 Hz, 1H), 8.54 (s, 1H), 8.23 (dd, *J =* 8.4, 1.2 Hz, 1H), 8.11 (dd, *J* = 13.2, 2.0 Hz, 1H), 8.00-7.98 (m, 2H), 7.78-7.73 (m, 2H), 7.61-7.59 (m, 2H), 7.53-7.47 (m, 2H), 7.27-7.21 (m, 3H). | 382 | **B** |
| **270** | 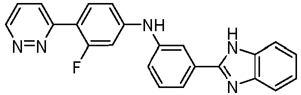 | ¹H NMR (400 MHz, DMSO-*d₆*) δ 12.91 (s, 1H), 9.17 (dd, *J* = 4.8, 1.6 Hz, 1H), 8.98 (s, 1H), 8.08 (s, 1H), 8.01-7.96 (m, 2H), 7.78-7.72 (m, 2H), 7.63-7.49 (m, 3H), 7.30 (dd, *J* = 8.0, 2.0 Hz, 1H), 7.21-7.20 (m, 2H), 7.12 (dd, *J =* 8.4, 2.0 Hz, 1H), 7.02 (dd, *J =* 14.4, 2.0 Hz, 1H). | 382 | **B** |
| **271** | 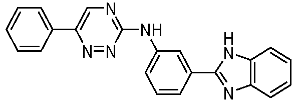 | ¹H NMR (400 MHz, DMSO-*d₆*) δ 12.89 (s, 1H), 10.42 (s, 1H), 9.12 (s, 1H), 8.75 (s, 1H), 8.13 (d, *J =* 8.0 Hz, 2H), 7.85 (d, *J =* 8.4 Hz, 1H), 7.80 (d, *J* = 7.6 Hz, 1H), 7.69 (d, *J =* 7.6 Hz, 1H), 7.58-7.49 (m, 5H), 7.25-7.18 (m, 2H). | 365 | **B** |
| **272** | 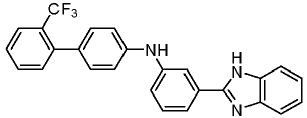 | ¹H NMR (400 MHz, DMSO-*d₆*) δ 12.87 (s, 1H), 8.55 (s, 1H), 8.01 (t, *J* = 1.6 Hz, 1H), 7.82 (d, *J =* 7.2 Hz, 1H), 7.72-7.63 (m, 3H), 7.59-7.51 (m, 2H), 7.44-7.42 (m, 2H), 7.25-7.19 (m, 7H). | 430 | **B** |
| **273** | 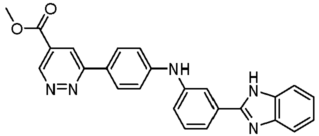 | ¹H NMR (400 MHz, DMSO-*d₆*) δ 12.91 (bs, 1H), 9.43 (d, *J* = 1.6 Hz, 1H), 8.85 (s, 1H), 8.42 (d, *J =* 1.6 Hz, 1H), 8.21-8.18 (m, 2H), 8.08 (t, *J* = 1.6 Hz, 1H), 7.73 (d, *J =* 7.6 Hz, 1H), 7.59 (bs, 2H), 7.49 (t, *J =* 8.0 Hz, 1H), 7.31-7.27 (m, 3H), 7.21-7.19 (m, 2H), 3.97 (s, 3H). | 422 | **B** |
| **274** | 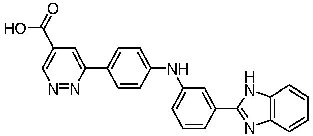 | ¹H NMR (400 MHz, DMSO-*d₆*) δ 13.02 (bs, 1H), 9.41 (d, *J* = 1.6 Hz, 1H), 8.82 (s, 1H), 8.38 (d, *J =* 2.0 Hz, 1H), 8.18 (d, *J =* 8.4 Hz, 2H), 8.08 (s, 1H), 7.72 (d, *J* = 7.6 Hz, 1H), 7.59 (bs, 2H), 7.48 (t, *J =* 8.0 Hz, 1H), 7.31-7.26 (m, 3H), 7.22-7.18 (m, 2H). | 408 | B |
| **275** | 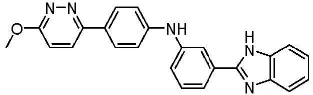 | ¹H NMR (400 MHz, DMSO-*d₆*) δ 12.87 (s, 1H), 8.68 (s, 1H), 8.11-8.00 (m, 4H), 7.69-7.64 (m, 2H), 7.52-7.43 (m, 2H), 7.27-7.19 (m, 6H), 4.06 (s, 3H). | 394 | B |
| **276** | 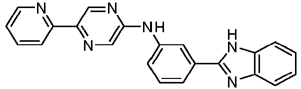 | ¹H NMR (400 MHz, DMSO-*d₆*) δ 12.89 (s, 1H), 10.00 (s, 1H), 9.13 (d, *J* = 1.2 Hz, 1H), 8.66 (dd, *J* = 4.8, 0.8 Hz, 1H), 8.57 (t, *J =* 2.0 Hz, 1H), 8.38 (d, *J* = 1.2 Hz, 1H), 8.19 (d, *J =* 8.0 Hz, 1H), 7.94-7.89 (m, 2H), 7.77 (d, *J =* 8.0 Hz, 1H), 7.68 (d, *J =* 6.8 Hz, 1H), 7.55-7.50 (m, 2H), 7.40-7.36 (m, 1H), 7.25-7.18 (m, 2H). | 365 | **B** |
| **277** | 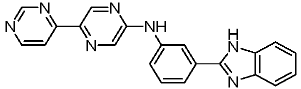 | ¹H NMR (400 MHz, DMSO-*d₆*) δ 12.91 (s, 1H), 10.25 (s, 1H), 9.22 (d, *J =* 1.2 Hz, 1H), 9.20 (d, *J =* 1.2 Hz, 1H), 8.88 (d, *J* =5.2 Hz, 1H), 8.59 (t, *J* = 2.0 Hz, 1H), 8.41 (d, *J =* 1.2 Hz, 1H), 8.16 (dd, *J* = 5.2, 1.2 Hz, 1H), 7.92 (dd, *J =* 8.0, 1.6 Hz, 1H), 7.81 (d, *J =* 8.0 Hz, 1H), 7.68 (d, *J =* 7.2 Hz, 1H), 7.56-7.52 (m, 2H), 7.25-7.18 (m, 2H). | 366 | **B** |
| **278** | 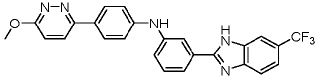 | ¹H NMR (400 MHz, DMSO-*d₆*) δ 13.26 (bs, 1H), 8.72 (s, 1H), 8.11-8.01 (m, 4H), 7.93 (s, 1H), 7.75 (d, *J* = 8.4 Hz, 1H), 7.71 (d, *J =* 7.6 Hz, 1H), 7.51-7.46 (m, 2H), 7.28-7.25 (m, 4H), 4.06 (s, 3H). | 462 | **B** |
| **279** | 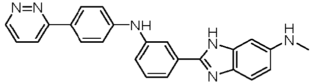 | ¹H NMR (400 MHz, DMSO-*d₆*) δ 12.38 (d, *J* = 46.8 Hz, 1H), 9.11 (dd, *J =* 4.8 Hz, 1H), 8.72 (d, *J* = 7.2 Hz, 1H), 8.15-8.10 (m, 3H), 7.98 (d, *J =* 16.4 Hz, 1H), 7.72-7.58 (m, 2H), 7.43-7.17 (m, 5H), 6.67-6.48 (m, 2H), 5.61-5.34 (m, 1H), 2.72 (d, *J* = 4.4 Hz, 3H). | 393 | **A** |
| **280** | 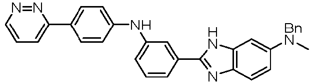 | ¹H NMR (400 MHz, DMSO-*d₆*) δ 12.45 (d, *J* = 55.6 Hz, 1H), 9.11 (dd, *J* = 4.8, 1.2 Hz, 1H), 8.71 (d, *J* = 6.0 Hz, 1H), 8.14-8.09 (m, 3H), 7.98 (d, *J =* 15.6 Hz, 1H), 7.72-7.59 (m, 2H), 7.45-7.39 (m, 2H), 7.33-7.17 (m, 8H), 6.93-6.65 (m, 2H), 4.58 (d, *J =* 20.4 Hz, 2H), 3.02 (d, *J =* 26 Hz, 3H) | 483 | **A** |
| **281** | 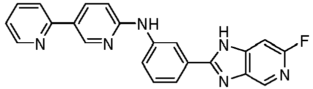 | ¹H NMR (400 MHz, DMSO-*d₆*) δ 13.44 (s, 1H), 9.56 (s, 1H), 8.96 (d, *J* = 2.4 Hz, 1H), 8.64-8.48 (m, 3H), 8.33 (dd, *J =* 8.8, 2.8 Hz, 1H), 7.96-7.83 (m, 3H), 7.72 (t, *J* = 6.0 Hz, 1H), 7.51 (t, *J* = 8.0 Hz, 1H), 7.36-7.21 (m, 2H), 7.00 (t, *J* = 8.8 Hz, 1 H). | 383 | **A** |
| **282** | 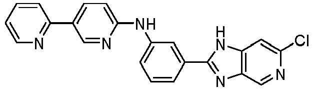 | ¹H NMR (400 MHz, DMSO-*d₆*) δ 13.51 (s, 1H), 9.57 (s, 1H), 8.96 (d, *J* = 2.0 Hz, 1H), 8.74 (s, 1H), 8.65 (s, 2H), 8.32 (dd, *J =* 8.8, 2.4 Hz, 1H), 7.97-7.84 (m, 3H), 7.74 (d, *J =* 7.6 Hz, 1H), 7.66 (s, 1H), 7.52 (t, *J =* 7.8 Hz, 1H), 7.30 (dd, *J* = 6.8, 4.8 Hz, 1H), 7.00 (d, *J* = 8.8 Hz, 1H). | 399 | **A** |
| **283** | 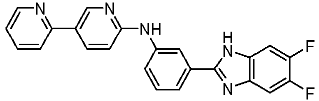 | ¹H NMR (400 MHz, DMSO-*d₆*) δ 9.56 (s, 1H), 8.95 (s, 1H), 8.64 (d, *J* = 2.4 Hz, 1H), 8.55 (s, 1H), 8.32 (d, *J* = 8.0 Hz, 1H), 7.98-7.87 (m, 3H), 7.66-7.63 (m, 3H), 7.49 (t, *J =* 7.6 Hz, 1H), 7.33 (s, 1H), 7.00 (d, *J* = 8.8 Hz, 1H). | 400 | **A** |
| **284** | 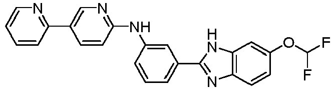 | ¹H NMR (400 MHz, DMSO-*d₆*) δ 13.00 (d, *J* = 8.0 Hz, 1H), 9.53 (d, *J* = 2.4 Hz, 1H), 8.96 (d, *J =* 2.4 Hz, 1H), 8.64-8.63 (m, 1H), 8.56 (d, *J =* 1.8 Hz, 1H), 8.32 (dd, *J* = 8.8, 2.4 Hz, 1H), 7.96-7.83 (m, 3H), 7.71-7.39 (m, 4H), 7.32-7.20 (m, 2H), 7.09-6.99 (m, 2H). | 430 | **A** |
| **285** | 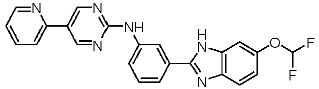 | ¹H NMR (400 MHz, DMSO-*d₆*) δ 13.00 (d, *J* = 7.2 Hz, 1H), 10.14 (s, 1H), 9.23 (s, 2H), 8.67 (s, 2H), 8.03 (d, *J* = 8.0 Hz, 1H), 7.90 (t, *J =* 6.8 Hz, 2H), 7.76-7.21 (m, 6H), 7.09-7.02 (m, 1H). | 431 | **A** |
| **286** | 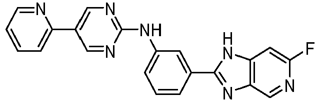 | ¹H NMR (400 MHz, DMSO-*d₆*) δ 10.18 (s, 1H), 9.213 (s, 2H), 8.75 (s, 1H), 8.67 (d, *J =* 4.0 Hz, 1H), 8.56 (s, 1H), 8.03 (d, *J* = 8.0 Hz, 1H), 7.95-7.88 (m, 2H), 7.80 (d, *J* = 8.0 Hz, 1H), 7.54 (t, *J* = 8.0 Hz, 1H), 7.36 (dd, *J* = 6.8, 4.8 Hz, 1H), 7.27 (s, 1H). | 384 | **A** |
| **287** | 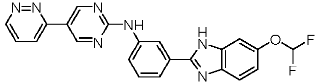 | ¹H NMR (400 MHz, DMSO-*d*₆) δ 13.02 (s, 1H), 10.26 (s, 1H), 9.30 (s, 2H), 9.22 (d, *J =* 4.4 Hz, 1H), 8.68 (s, 1H), 8.30 (d, *J* = 8.8 Hz, 1H), 7.91 (d, *J =* 8.0 Hz, 1H), 7.83-7.76 (m, 2H), 7.54-7.22 (m, 4H), 7.05 (t, *J =* 7.6 Hz, 1H). | 432 | **A** |
| **288** | 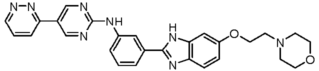 | ¹H NMR (400 MHz, DMSO-*d₆*) δ 12.71 (d, *J* = 12.4 Hz, 1H), 10.23 (d, *J =* 4.4 Hz, 1H), 9.29 (s, 2H), 9.21 (d, *J* = 3.6 Hz, 1H), 8.63 (s, 1H), 8.30 (d, *J* = 7.6 Hz, 1H), 7.87-7.72 (m, 3H), 7.56-7.39 (m, 2H), 7.12 (d, *J =* 87.2 Hz, 1H), 6.85 (t, *J* = 9.6 Hz, 1H), 4.15 (s, 2H), 3.60 (t, *J =* 4.6 Hz, 4H), 3.33 (s, 4H), 2.74 (t, *J =* 5.2 Hz, 2H). | 495 | **A** |
| **289** | 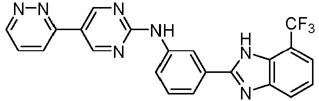 | ¹H NMR (400 MHz, DMSO-*d₆*) δ 13.39 (s, 1H), 10.29 (s, 1H), 9.28 (s, 2H), 9.22 (d, *J =* 4.8 Hz, 1H), 8.62 (s, 1H), 8.30 (d, *J* = 8.8 Hz, 1H), 8.04 (d, *J =* 7.6 Hz, 1H), 7.85-7.79 (m, 3H), 7.58-7.54 (m, 2H), 7.39 (t, *J =* 7.8 Hz, 1H). | 434 | **A** |
| **290** | 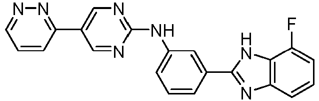 | ¹H NMR (400 MHz, DMSO-*d₆*) δ 13.19 (s, 1H), 10.26 (s, 1H), 9.30 (s, 2H), 9.21 (dd, *J* = 4.8, 1.2 Hz, 1H), 8.70 (s, 1H), 8.31 (dd, *J* = 8.8, 1.2 Hz, 1H), 7.94 (d, *J =* 8.4 Hz, 1H), 7.81 (dd, *J* = 8.4, 4.8 Hz, 2H), 7.53 (t, *J =* 7.8 Hz, 1H), 7.40 (s, 1H), 7.23-7.18 (m, 1H), 7.03 (t, *J =* 9.8 Hz, 1H). | 384 | **A** |
| **291** | 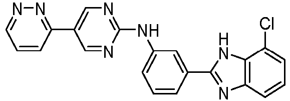 | ¹H NMR (400 MHz, DMSO-*d₆*) δ 10.27 (s, 1H), 9.30 (s, 2H), 9.21 (dd, *J =* 4.8, 1.2 Hz, 1H), 8.66 (s, 1H), 8.30 (dd, *J =* 8.8, 1.2 Hz, 1H), 7.99 (d, *J* = 8.0 Hz, 1H), 7.82-7.79 (m, 2H), 7.54 (t, *J* = 8.0 Hz, 2H), 7.30-7.20 (m, 2H). | 400 | **A** |
| **292** | 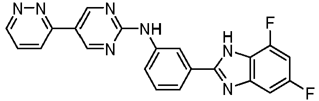 | ¹H NMR (400 MHz, DMSO-*d₆*) δ 13.39 (d, *J* = 52.8 Hz, 1H), 10.26 (s, 1H), 9.30-9.21 (m, 3H), 8.71-8.67 (m, 1H), 8.31 (dd, *J* = 8.8, 1.6 Hz, 1H), 7.95-7.75 (m, 3H), 7.53 (t, *J* = 8.0 Hz, 1H), 7.41-7.05 (m, 2H). | 402 | **A** |
| **293** | 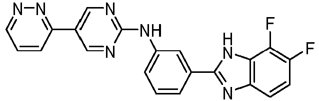 | ¹H NMR (400 MHz, DMSO-*d₆*) δ 13.28 (s, 1H), 10.27 (s, 1H), 9.30 (s, 2H), 9.21 (dd, *J* = 4.8, 1.2 Hz, 1H), 8.70 (s, 1H), 8.31 (d, *J =* 7.6 Hz, 1H), 7.95 (d, *J* = 8.0 Hz, 1H), 7.82-7.79 (m, 2H), 7.54 (t, *J =* 8.0 Hz, 1H), 7.38-7.23 (m, 2H). | 402 | **A** |
| **294** | 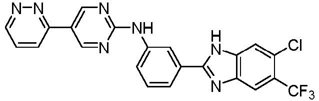 | ¹H NMR (400 MHz, DMSO-*d₆*) δ 10.33 (s, 1H), 9.31 (s, 2H), 9.23 (dd, *J =* 4.8, 1.2 Hz, 1H), 8.73 (s, 1H), 8.33 (dd, *J =* 8.8, 1.6 Hz, 1H), 8.08 (s, 1H), 7.98-7.92 (m, 2H), 7.86-7.80 (m, 2H), 7.57 (t, *J =* 7.6 Hz, 1H). | 468 | **A** |
| **295** | 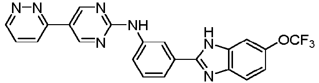 | ¹H NMR (400 MHz, DMSO-*d₆*) δ 10.30 (s, 1H), 9.31 (s, 2H), 9.22 (dd, *J =* 4.8, 1.2 Hz, 1H), 8.71 (t, *J* = 1.8 Hz, 1H), 8.31 (dd, *J =* 8.4, 1.2 Hz, 1H), 7.93 (dd, *J* = 8.0, 1.2 Hz, 1H), 7.83-7.77 (m, 2H), 7.71 (d, *J* = 8.8 Hz, 1H), 7.63 (s, 1H), 7.55 (d, *J* = 7.8 Hz, 1H), 7.25 (dd, *J =* 8.4, 1.2 Hz, 1H). | 450 | **A** |
| **296** | 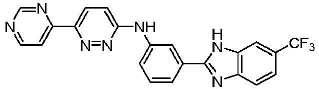 | ¹H NMR (400 MHz, DMSO-*d₆*) δ 10.00 (s, 1H), 9.32 (s, 1H), 8.96 (d, *J* = 5.6 Hz, 1H), 8.80 (s, 1H), 8.47 (d, *J* = 4.2 Hz, 1H), 8.41 (d, *J* = 9.2 Hz, 1H), 7.99 (s, 1H), 7.94 (d, *J* = 8.0 Hz, 1H), 7.83 (t, *J =* 8.6 Hz, 2H), 7.59 (dd, *J =* 16.4, 8.0 Hz, 2H), 7.39 (d, *J* = 9.2 Hz, 1H). | 434 | **A** |
| **297** | 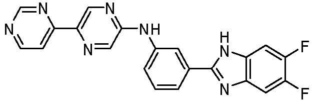 | ¹H NMR (400 MHz, DMSO-*d₆*) δ 13.17 (s, 1H), 10.26 (s, 1H), 9.21 (d, *J =* 15.2 Hz, 2H), 8.88 (d, *J* = 5.2 Hz, 1H), 8.59 (s, 1H), 8.41 (s, 1H), 8.16 (d, *J* = 4.8 Hz, 1H), 7.91 (d, *J* = 7.6 Hz, 1H), 7.76 (dd, *J* = 17.6, 7.6 Hz, 2H), 7.56 (dd, *J =* 15.6, 7.6 Hz, 2H). | 402 | **A** |
| **298** | 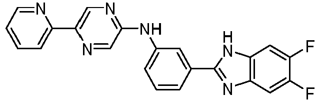 | ¹H NMR (400 MHz, DMSO-*d₆*) δ 10.04 (s, 1H), 9.13 (s, 1H), 8.66 (d, *J* = 4.0 Hz, 1H), 8.58 (s, 1H), 8.38 (s, 1H), 8.21 (d, *J* = 7.2 Hz, 1H), 7.97-7.88 (m, 2H), 7.74 (d, *J =* 7.2 Hz, 1H), 7.66 (t, *J* = 8.2 Hz, 2H), 7.53 (t, *J* = 7.8 Hz, 2H), 7.41 (t, *J =* 5.6 Hz, 1H). | 401 | **A** |
| **299** | 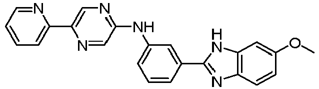 | ¹H NMR (400 MHz, DMSO-*d₆*) δ 12.83 (s, 1H), 10.09 (s, 1H), 9.12 (s, 1H), 8.66 (d, *J =* 4.4 Hz, 1H), 8.55 (s, 1H), 8.42 (s, 1H), 8.19 (d, *J =* 8.0 Hz, 1H), 7.94-7.87 (m, 2H), 7.74 (d, *J* = 7.6 Hz, 1H), 7.56-7.37 (m, 3H), 7.19-7.02 (m, 1H), 6.85 (d, *J* = 7.6 Hz, 1H), 3.82 (s, 3H). | 395 | **A** |
| **300** | 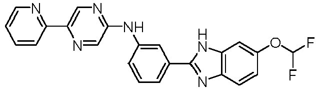 | ¹H NMR (400 MHz, DMSO-*d₆*) δ 13.04 (s, 1H), 10.01 (s, 1H), 9.13 (s, 1H), 8.66 (d, *J =* 4.4 Hz, 1H), 8.57 (s, 1H), 8.38 (s, 1H), 8.19 (d, *J* = 7.6 Hz, 1H), 7.92 (t, *J* = 7.0 Hz, 2H), 7.77-7.22 (m, 6H), 7.07 (s, 1H). | 431 | **A** |
| **301** | 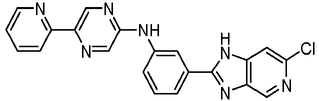 | ¹H NMR (400 MHz, DMSO-*d₆*) δ 13.56 (s, 1H), 10.06 (s, 1H), 9.13 (s, 1H), 8.79-8.66 (m, 3H), 8.38 (s, 1H), 8.19 (d, *J* = 8.0 Hz, 1H), 7.96-7.91 (m, 2H), 7.81-7.55 (m, 3H), 7.39 (t, *J =* 6.0 Hz, 1H). | 400 | **A** |
| **302** | 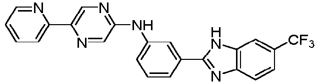 | ¹H NMR (400 MHz, DMSO-*d₆*) δ 13.35 (s, 1H), 10.05 (s, 1H), 9.13 (s, 1H), 8.66-8.62 (m, 2H), 8.38 (s, 1H), 8.19 (d, *J* = 7.6 Hz, 1H), 8.05-7.90 (m, 3H), 7.85-7.74 (m, 2H), 7.56 (t, *J* = 8.0 Hz, 2H), 7.39 (dd, *J* = 6.8, 5.6 Hz, 1H). | 433 | **A** |
| **303** | 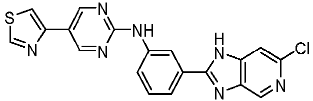 | ¹H NMR (400 MHz, DMSO-*d₆*) δ 13.49 (s, 1H), 10.12 (s, 1H), 9.27 (s, 1H), 9.13 (s, 2H), 8.79-8.69 (m, 2H), 8.23 (s, 1H), 7.93 (s, 1H), 7.78-7.53 (m, 3H). | 406 | **A** |
| **304** | 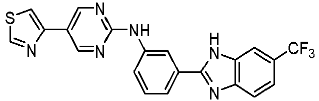 | ¹H NMR (400 MHz, DMSO-*d₆*) δ 13.33 (s, 1H), 10.10 (s, 1H), 9.27 (d, *J* = 2.0 Hz, 1H), 9.13 (s, 2H), 8.72 (s, 1H), 8.23 (d, *J* = 2.0 Hz, 1H), 7.97-7.77 (m, 4H), 7.56-7.51 (m, 2H). | 439 | **A** |
| **305** | 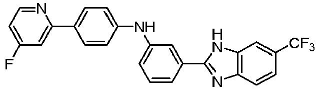 | ¹H NMR (400 MHz, DMSO-*d₆*) δ 13.34 (s, 1H), 8.76 (s, 1H), 8.63 (dd, *J =* 9.2, 4.2 Hz, 1H), 8.10-8.07 (m, 3H), 7.95-7.71 (m, 4H), 7.51 (dd, *J =* 18.0, 10.0 Hz, 2H), 7.31-7.17 (m, 4H). | 449 | **B** |
| **306** | 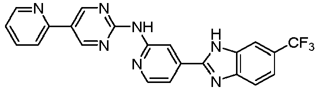 | ¹H NMR (400 MHz, DMSO-*d₆*) δ 13.67 (s, 1H), 10.49 (s, 1H), 9.32 (s, 2H), 9.08 (s, 1H), 8.70 (d, *J =* 4.0 Hz, 1H), 8.54 (d, *J =* 5.6 Hz, 1H), 8.10-7.85 (m, 4H), 7.76 (dd, *J* = 4.8, 1.2 Hz, 1H), 7.61 (d, *J* = 8.4 Hz, 1H), 7.40 (dd, *J =* 7.6, 5.6 Hz, 1H). | 434 | **B** |
| **307** | 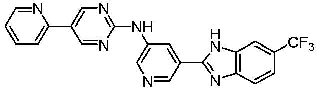 | ¹H NMR (400 MHz, DMSO-*d₆*) δ 13.55 (s, 1H), 10.40 (s, 1H), 9.28 (s, 2H), 9.12 (t, *J =* 4.4 Hz, 1H), 9.05 (t, *J* = 4.4 Hz, 1H), 8.97 (d, *J =* 1.6 Hz, 1H), 8.69-8.68 (m, 1H), 8.06-7.80 (m, 4H), 7.58 (d, *J =* 8.0 Hz, 1H), 7.40-7.37 (m, 1H). | 434 | **B** |
| **308** | 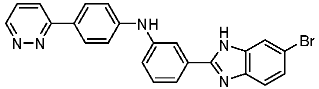 | ¹H NMR (400 MHz, DMSO-*d₆*) δ 13.08 (d, *J* = 18 Hz, 1H), 9.12 (dd, *J* =4.8, 1.2 Hz, 1H), 8.78 (s, 1H), 8.16-8.11 (m, 3H), 8.05 (s, 1H), 7.86-7.67 (m, 3H), 7.63-7.46 (m, 2H), 7.37-7.28 (m, 4H). | 442 | **A** |
| **309** | 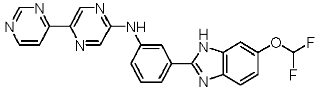 | ¹H NMR (400 MHz, DMSO-*d*₆) δ 13.10 (s, 1H), 10.28 (s, 1H), 9.22 (d, *J* = 1.2 Hz, 1H), 9.19 (d, *J* = 1.6 Hz, 1H), 8.88 (d, *J* = 5.2 Hz, 1H), 8.59 (d, *J* = 2.0 Hz, 1H), 8.42 (d, *J =* 1.2 Hz, 1H), 8.16 (dd, *J* = 5.2, 1.2 Hz, 1H), 7.93 (d, *J* = 8.4 Hz, 1H), 7.80 (d, *J* = 8.0 Hz, 1H), 7.65-7.41 (m, 3H), 7.22-7.04 (m, 2H). | 432 | **A** |
| **310** | 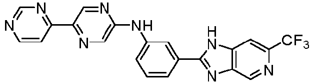 | ¹H NMR (400 MHz, DMSO-*d*₆) δ 13.86 (s, 1H), 10.32 (s, 1H), 9.22 (dd, *J* = 11.2, 1.2 Hz, 2H), 9.08 (s, 1H), 8.89 (d, *J* = 5.2 Hz, 1H), 8.72 (s, 1H), 8.43 (d, *J* = 1.2 Hz, 1H), 8.17 (dd, *J* = 5.2, 1.2 Hz, 1H), 8.08 (s, 1H), 7.99 (dd, *J* = 8.0, 2.0 Hz, 1H), 7.89 (d, *J =* 7.6 Hz, 1H), 7.61 (t, *J* = 7.8 Hz, 1H) | 435 | **A** |
| **311** | 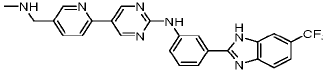 | ¹H NMR (400 MHz, DMSO-*d*₆) δ 10.15 (s, 1H), 9.22 (s, 2H), 8.73 (s, 1H), 8.60 (s, 1H), 7.99-7.92 (m, 3H), 7.85-7.78 (m, 3H), 7.56-7.52 (m, 2H), 3.73 (s, 2H), 2.30 (s, 3H). | 476 | **B** |
| **312** | 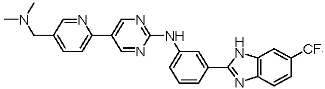 | ¹H NMR (400 MHz, DMSO-*d*₆) δ 13.32 (s, 1H), 10.16 (s, 1H), 9.22 (s, 2H), 8.72 (s, 1H), 8.56 (s, 1H), 8.05-7.73 (m, 6H), 7.54 (t, *J =* 7.8 Hz, 2H), 3.48 (s, 2H), 2.19 (s, 6H). | 490 | **F** |
| **313** | 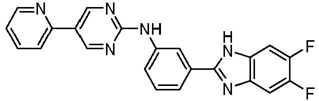 | ¹H NMR (400 MHz, DMSO-*d*₆) δ 13.12 (s, 1H), 10.13 (s, 1H), 9.22 (s, 2H), 8.68-8.65 (m, 2H), 8.02 (d, *J* = 8.0 Hz, 1H), 7.92-7.88 (m, 2H), 7.73 (d, *J* = 8.0 Hz, 2H), 7.58-7.48 (m, 2H), 7.36 (dd, *J* = 6.4, 4.4 Hz, 1H). | 401 | **A** |
| **314** | 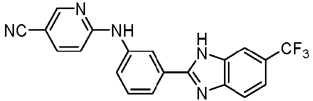 | ¹H NMR (400 MHz, DMSO-*d*₆) δ 13.34 (s, 1H), 10.01 (s, 1H), 8.64 (s, 1H), 8.51 (s, 1H), 8.03-7.72 (m, 5H), 7.55 (t, *J* = 7.8 Hz, 2H), 6.96 (d, *J* = 8.8 Hz, 1H). | 380 | **B** |
| **315** | 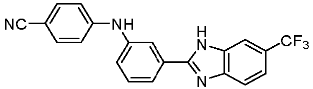 | ¹H NMR (400 MHz, DMSO-*d*₆) δ 13.35 (s, 1H), 9.13 (s, 1H), 8.08-7.65 (m, 6H), 7.55 (t, *J* = 7.8 Hz, 2H), 7.33 (dd, *J* = 8.0, 1.2 Hz, 1H), 7.18 (dd, *J* = 9.2, 2.4 Hz, 2H) | 379 | **B** |
| **316** | 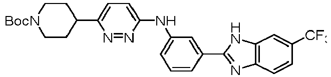 | ¹H NMR (400 MHz, DMSO-*d*₆) δ 13.30 (s, 1H), 9.43 (s, 1H), 8.75 (s, 1H), 8.07-7.72 (m, 4H), 7.55-7.46 (m, 3H), 7.15 (t, *J* = 9.2 Hz, 1H), 4.08 (d, *J* = 12.0 Hz, 2H), 2.99-2.86 (m, 3H), 1.87 (d, *J* = 11.2 Hz, 2H), 1.67-1.56 (m, 2H), 1.43 (s, 9H). | 539 | **B** |
| **317** | 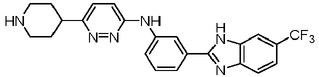 | ¹H NMR (400 MHz, DMSO-*d*₆) δ 9.42 (s, 1H), 8.75 (s, 1H), 7.97 (s, 1H), 7.86 (d, *J =* 8.0 Hz, 1H), 7.79 (d, *J* = 8.4 Hz, 1H), 7.73 (d, *J* = 7.6 Hz, 1H), 7.54-7.42 (m, 3H), 7.15 (d, *J* = 9.2 Hz, 1H), 3.09 (d, *J* = 11.6 Hz, 2H), 2.90-2.83 (m, 1H), 2.66 (t, *J* = 11.2 Hz, 2H), 1.80-1.61 (m, 4H). | 439 | **F** |
| **320** | 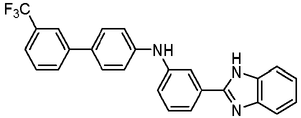 | ¹H NMR (400 MHz, DMSO-*d*₆) δ 12.94 (s, 1H), 8.63 (s, 1H), 8.04-7.94 (m, 3H), 7.73-7.58 (m, 7H), 7.47-7.20 (m, 6H). | 430 | **B** |
| **321** | 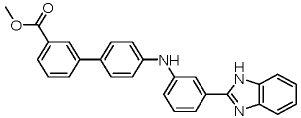 | ¹H NMR (400 MHz, DMSO-*d*₆) δ 12.86 (s, 1H), 8.60 (s, 1H), 8.19 (s, 1H), 8.03 (s, 1H), 7.95-7.88 (m, 2H), 7.68-7.58 (m, 5H), 7.53-7.42 (m, 2H), 7.28-7.18 (m, 5H), 3.90 (s, 3H). | 420 | **B** |
| **322** | 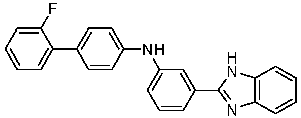 | ¹H NMR (400 MHz, DMSO-*d*₆) δ 12.88 (s, 1H), 8.58 (s, 1H), 8.02 (s, 1H), 7.66-7.49 (m, 6H), 7.44 (t, *J* = 7.8 Hz, 1H), 7.38-7.18 (m, 8H). | 380 | **B** |
| **323** | 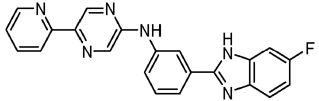 | ¹H NMR (400 MHz, DMSO-*d*₆) δ 10.01 (s, 1H), 9.12 (d, *J* = 1.6 Hz, 1H), 8.67-8.65 (m, 1H), 8.57 (t, *J* = 3.2 Hz, 1H), 8.38 (d, *J =* 1.2 Hz, 1H), 8.19 (d, *J* = 8.0 Hz, 1H), 7.94-7.89 (m, 2H), 7.75 (d, *J* = 7.6 Hz, 1H), 7.62-7.51 (m, 2H), 7.42-7.37 (m, 2H), 7.10-7.05 (m, 1H). | 383 | **A** |
| **324** | 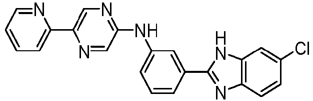 | ¹H NMR (400 MHz, DMSO-*d*₆) δ 10.12 (s, 1H), 9.16 (s, 1H), 8.68 (d, *J* = 4.8 Hz, 1H), 8.62 (s, 1H), 8.40 (s, 1H), 8.26 (d, *J* = 8.0 Hz, 1H), 8.02 (t, *J* = 7.4 Hz, 1H), 7.92 (dd, *J* = 8.4, 1.6 Hz, 1H), 7.77 (d, *J* = 7.6 Hz, 1H), 7.70 (d, *J* = 1.6 Hz, 1H), 7.66 (d, *J* = 8.8 Hz, 1H), 7.57 (d, *J* = 8.0 Hz, 1H), 7.46 (d, *J =* 6.0 Hz, 1H), 7.30 (dd, *J* = 8.4, 1.6 Hz, 1H). | 399 | **A** |
| **325** | 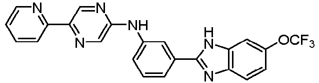 | ¹H NMR (400 MHz, DMSO-*d*₆) δ 13.20 (s, 1H), 10.03 (s, 1H), 9.12 (d, *J* = 1.2 Hz,1H), 8.66 (d, *J* = 4.4 Hz, 1H), 8.60 (s, 1H), 8.38 (d, *J* = 0.8 Hz, 1H), 8.19 (d, *J* = 8.0 Hz, 1H), 7.94-7.90 (m, 2H), 7.78-7.52 (m, 4H), 7.39 (dd, *J* = 6.4, 4.8 Hz, 1H), 7.22 (d, *J* = 8.8 Hz, 1H). | 449 | **A** |
| **326** | 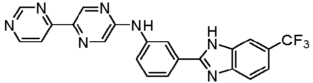 | ¹H NMR (400 MHz, DMSO-*d*₆) δ 10.30 (s, 1H), 9.23 (d, *J* = 1.2 Hz, 1H), 9.20 (d, *J =* 1.2 Hz, 1H), 8.89 (d, *J* = 5.2 Hz,1H), 8.64 (s, 1H), 8.42 (d, *J* = 1.2 Hz, 1H), 8.17 (dd, *J =* 5.2, 1.2 Hz, 1H), 7.98 (dd, *J* = 8.0, 1.6 Hz, 2H), 7.85-7.79 (m, 2H), 7.58 (dd, *J* = 16.0, 7.6 Hz, 2H). | 434 | **A** |
| **327** | 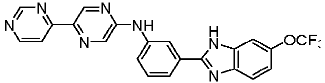 | ¹H NMR (400 MHz, DMSO-*d*₆) δ 10.28 (s, 1H), 9.23 (d, *J* = 1.2 Hz, 1H), 9.20 (d, *J =* 1.2 Hz, 1H), 8.88 (d, *J* = 5.2 Hz,1H), 8.62 (t, *J* = 1.6 Hz, 1H), 8.42 (d, *J* = 1.6 Hz, 1H), 8.16 (dd, *J* = 5.2, 1.2 Hz, 1H), 7.94 (dd, *J =* 8.4, 2.0 Hz, 1H), 7.81 (d, *J* = 8.4 Hz, 1H), 7.69-7.55 (m, 3H), 7.22 (d, *J* = 8.4 Hz, 1H). | 450 | **A** |
| **328** | 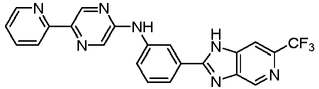 | ¹H NMR (400 MHz, DMSO-*d*₆) δ 10.18 (s, 1H), 9.17 (s, 1H), 9.10 (s, 1H), 8.73 (s, 1H), 8.69 (d, *J* = 4.4 Hz, 1H), 8.41 (s, 1H), 8.30 (d, *J* = 8.0 Hz, 1H), 8.09-8.04 (m, 2H), 7.97 (dd, *J* = 8.0, 1.6 Hz, 1H), 7.87 (d, *J* = 8.0 Hz, 1H), 7.61 (t, *J* = 8.0 Hz, 1H), 7.50 (t, *J* = 6.0 Hz, 1H). | 434 | **A** |
| **329** | 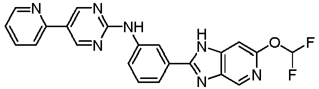 | ¹H NMR (400 MHz, DMSO-*d*₆) δ 13.40 (s, 1H), 10.18 (s, 1H), 9.23 (s, 2H), 8.75-8.56 (m, 3H), 8.04-7.52 (m, 6H), 7.38-7.14 (m, 2H). | 432 | **A** |
| **330** | 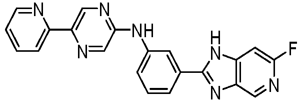 | ¹H NMR (400 MHz, DMSO-*d*₆) δ 10.15 (s, 1H), 9.16 (s, 1H), 8.68 (s, 2H), 8.57 (s, 1H), 8.41 (s, 1H), 8.28 (d, *J* = 7.6 Hz, 1H), 8.04 (t, *J* = 7.6 Hz, 1H), 7.94 (d, *J* = 8.4 Hz, 1H), 7.81 (d, *J* = 7.6 Hz, 1H), 7.58 (t, *J* = 8.0 Hz, 1H), 7.48 (t, *J* = 5.8 Hz, 1H), 7.28 (s, 1H). | 384 | **A** |
| **331** | 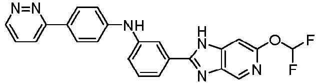 | ¹H NMR (400 MHz, DMSO-*d*₆) δ 13.44 (d, *J* = 16.4 Hz, 1H), 9.12 (dd, *J* = 4.4, 0.8 Hz, 1H), 8.82 (d, *J* = 7.6 Hz, 1H), 8.65 (s, 1H), 8.17-8.08 (m, 4H), 7.91-7.49 (m, 4H), 7.35-7.14 (m, 4H). | 431 | **A** |
| **332** | 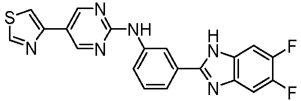 | ¹H NMR (400 MHz, DMSO-*d*₆) δ 13.15 (s, 1H), 10.06 (s, 1H), 9.26 (d, *J* = 2.0 Hz, 1H), 9.12 (s, 2H), 8.66 (s, 1H), 8.22 (d, *J* = 1.6 Hz, 1H), 7.87 (d, *J* = 8.0 Hz, 1H), 7.73-7.57 (m, 3H), 7.49 (t, *J* = 8.0 Hz, 1H). | 407 | **A** |
| **333** | 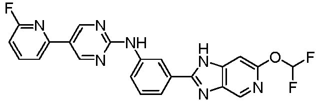 | ¹H NMR (400 MHz, DMSO-*d*₆) δ 10.28 (s, 1H), 9.20 (s, 2H), 8.76 (s, 1H), 8.64 (s, 1H), 8.10 (dd, *J* = 16.0, 8.0 Hz, 1H), 7.99 (dd, *J* = 7.6, 2.4 Hz, 1H), 7.92 (d, *J* = 1.6 Hz, 1H), 7.90 (s, 1H), 7.80 (d, *J* = 8.0 Hz, 1H), 7.72-7.52 (m, 2H), 7.20 (s, 1H), 7.14 (dd, *J* = 8.4, 2.4 Hz, 1H). | 450 | **A** |
| **334** | 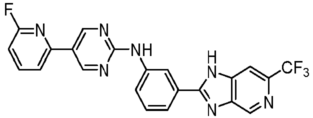 | ¹H NMR (400 MHz, DMSO-*d*₆) δ 13.88 (s, 1H), 10.30 (s, 1H), 9.21 (s, 2H), 9.07 (s, 1H), 8.80 (s, 1H), 8.11-8.07 (m, 2H), 7.99 (d, *J =* 7.2 Hz, 1H), 7.94 (d, *J* = 7.6 Hz, 1H), 7.86 (d, *J* = 8.0 Hz, 1H), 7.56 (t, *J* = 8.0 Hz, 1H), 7.14 (d, *J* = 8.0 Hz, 1H). | 452 | **A** |
| **335** | 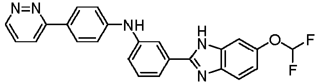 | ¹H NMR (400 MHz, DMSO-*d*₆) δ 13.03 (s, 1H), 9.12 (dd, *J* = 4.8, 1.2 Hz, 1H), 8.79 (s, 1H), 8.16-8.12 (m, 3H), 8.05 (s, 1H), 7.73-7.68 (m, 2H), 7.61 (s, 1H), 7.50-7.21 (m, 6H), 7.07-7.02 (m, 1H). | 430 | **A** |
| **336** | 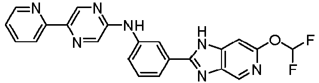 | ¹H NMR (400 MHz, DMSO-*d*₆) δ 13.45 (d, *J* = 16.8 Hz, 1H), 10.05 (d, *J* = 8.0 Hz, 1H), 9.13 (d, *J* = 0.8 Hz, 1H), 8.67-8.57 (m, 3H), 8.38 (d, *J* = 1.2 Hz, 1H), 8.19 (d, *J* = 8.0 Hz, 1H), 7.97-7.54 (m, 5H), 7.39 (dd, *J* = 6.8, 4.8 Hz, 1H), 7.33-7.16 (m, 1H). | 432 | **A** |

### Experimental Example 1: Efficacy test of antithrombosis substance (ferric chloride induced carotid artery thrombosis model)

### 1. Experimental materials and equipment

Isoflurane (Hana Pharm, cat. #9008), iron(III) chloride (Sigma-Aldrich, cat. # 157740), dimethyl sulfoxide (Sigma-Aldrich, cat. # D5879), poly(ethylene glycol), polyethylene glycol 400 with average Mn 400 (PEG400, Sigma-Aldrich, cat. # 202398), Hydroxypropyl-beta-cyclodextrin (HP-β-CD, TCI, cat. # H0979)

### 2. Laboratory animals

As experimental animals, 8-week-old male SD rats (ORIENT BIO.) acclimated for two weeks were obtained, the number of animals was confirmed at the time of acquisition, general symptom observation and weight measurement were performed, and the inspection report provided by the animal supplier was stored as basic test data. All animals were observed for general symptoms once a day during the quarantine and acclimatization period. The rats were raised in polycarbonate breeding cages under aseptic conditions at a temperature (22 ± 3°C), relative humidity of 30-70%, ventilation frequency of 10 to 15 times/hour, and a 12-hour cycle of day and night (illumination of 150 to 300 Lux). The breeding cages were changed once/week and drinking bottles were changed at least twice/week. Animals were fed a laboratory grade solid diet (Teklad Certified Irradiated Global 18% Protein Rodent Diet 2918C, Envigo RMS, Inc., U.S.A.) ad libitum in a feeder.

After final inspection, the groups were separated to ensure that the mean weight of each group was as evenly distributed as possible according to the results of the ranked weight measurements, and the animals remaining after separation were excluded from the study and euthanized by inhalation of carbon dioxide (CO₂) gas.

### 3. Experimental procedure

### 3-1. Preparation of control substance and test substance

The control substance 1 (Rivaroxaban), control substance 2 (Edoxaban) and the test substance were weighed by the required amount, DMSO in an amount of 5% of the required solution was added, followed by sonication for 2 minutes to confirm that the substance was dissolved. After that, PEG400 in an amount of 50% was added and the resulting mixture was sonicated for 2 minutes. A 20% HP-β-CD-containing distilled water solution in an amount of 45% was added thereto and completely dissolved by sonication for 2 minutes. The test substances were prepared and used immediately prior to administration to test animals.

### 3-2. Preparation of inducing substance (50% FeCl₃ solution)

The required amount of the inducing substance was weighed, and water for injection was added and vortexed. Thereafter, water for injection was added to prepare a required liquid amount.

### 3-3. Test substance administration

The test substance was orally administered 2 hours before induction of thrombosis by setting the administration amount to 10 mL/kg and connecting a sonde for oral administration to a disposable syringe. The control substance was orally administered 25 minutes before induction of thrombosis by setting the administration amount to 10 mL/kg and connecting a sonde for oral administration to a disposable syringe. Normal control groups and negative control groups received equal amounts of excipients (5% DMSO, 50% PEG400, 45%(distilled water solution containing 20% HP-β-CD)) orally.

### 3-4. Thrombus induction and blood vessel weight measurement

The test animals were anesthetized with isoflurane and the carotid artery was exposed. Whatman No. 1 filter paper (4.2 mm²) soaked in 50% FeCl₃ was placed on the carotid artery for 10 minutes. After 10 minutes, the paper was removed, FeCl₃ remaining in the blood vessel was washed with PBS, and the blood vessel was left for 20 minutes and separated. The G1 group was equally treated with Whatman No. 1 filter paper (4.2 mm²) soaked in PBS. 20 minutes after removing the Whatman No. 1 filter paper, both ends of the blood vessels were tied together using 4-0 silk-ligature and the blood vessel was separated. At this time, the length of the blood vessel and the length of the ligature were the same. The ligature was removed, then both ends of the blood vessels were gently brought into contact to remove the blood in the blood vessels with Kimwipes, and the weight was measured. Next, an image was taken and the length was measured using the Image J program.

### 3-5. End of test

The blood vessel weight and blood vessel length were measured and photographed, and the test was terminated. The test results were expressed by calculating the inhibition rate (%) using the index value (vessel weight (mg) /vessel length (mm)) as follows.

Inhibition rate (%) = [(Inducing control average Index value - Normal control average Index value) - (Test group average Index value - Normal control average Index value)] / (Inducing control average Index value - Normal control average Index value) X 100

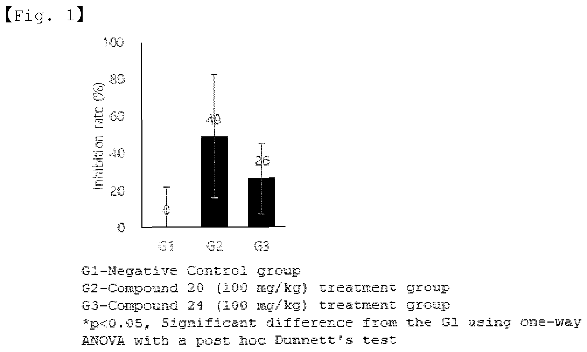

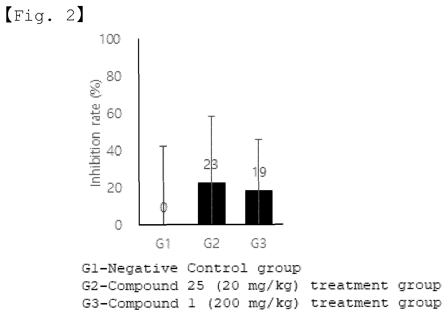

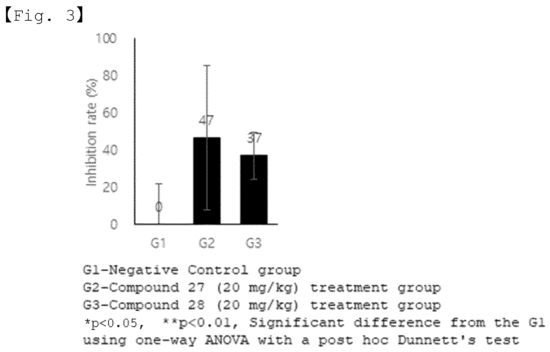

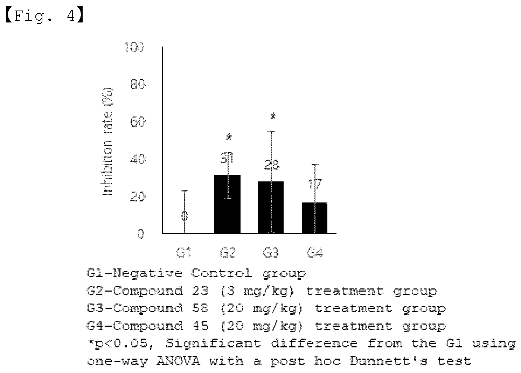

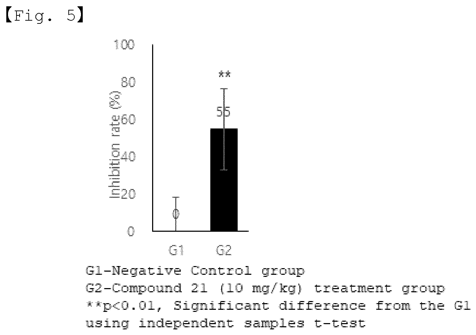

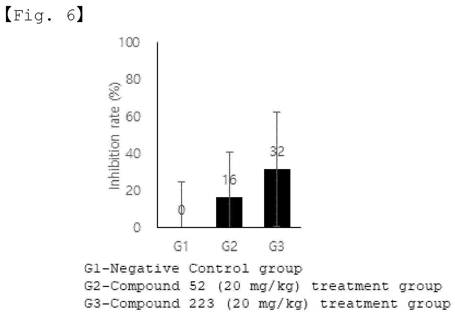

The test results (Inhibitioni rate, %) for each Example compound treatment group are shown in FIG. 1 to 6, and the Example compounds administered in each figure are shown in Table 3 below.

**[Table 3]**

| **FIG.** | **Compound No.** | **FIG.** | **Compound No.** |
|---|---|---|---|
| **1** | 20, 24 | **4** | 23, 58, 45 |
| **2** | 25, 1 | **5** | 21 |
| **3** | 27, 28 | **6** | 52, 223 |

### EXPERIMENTAL EXAMPLE 2: Lact C2 assay (measuring phosphatidylserine externalization scramblase function)

### 1. Materials and equipment

Ionomycin (Alomonelab, cat. # I-700), DAPI (Sigma- Aldrich, cat. # D8417), paraformaldehyde (Biosesang, cat. #P2031), Lionheart FX automated fluorescence microscope (BioTek), Gen5^{™} software program (BioTek), and Image J analysis software program.

### 2. Cell culture

Fisher rat thyroid (FRT) cells expressing human ANO6 (variant 5; GenBank accession no. NP_001191732.1) were cultured at 37°C under 5% CO₂ conditions in DMEM/Ham's F-12 (1:1) medium supplemented with 10% Fetal Bovine Serum, 2 mM L-549 glutamine, 100 units/mL penicillin, and 100 µg/mL streptomycin.

### 3. Test method

Fischer Rat Thyroid (FRT) cells (2 × 10⁴ cells/well) expressing human ANO6 (variant 5) were cultured in a 96-well microplate for 24 hours. After the incubation, the cells were treated with compounds dissolved in DMSO to 1% v/v in each well at each concentration (50 nM, 100 nM, and 1 µM) and reacted for 10 minutes. Thereafter, each well was treated with ionomycin to a final concentration of 10 µM, allowed to react for 10 minutes, and then washed with 200 µL PBS. After washing, each well was treated with 50 µL of Lactadherin-C2 (Lact-C2)-GFP mixed in DMEM medium to a final concentration of 500 nM to stain phosphatidylserine, and cultured at 37°C for 10 minutes. After completion of the reaction, the cells were washed with 200 µL PBS, and fixed with 4% paraformaldehyde for 5 minutes at room temperature. For morphological analysis, DAPI solution was added, and the cells were incubated for 15 minutes, followed by washing with PBS. The fluorescence intensity of Lact-C2-GFP bound to phosphatidylserine on the cell surface was measured using an automated fluorescence microscope. For quantitative analysis of the fluorescence intensity of Lact-C2-GFP, BioTek Gen5^{™} software and Image J analysis software programs were used.

Phosphatidylserine externalization inhibitory activity (%) by the compound was calculated through the following analysis method. Specifically, the background fluorescence value was subtracted from the measured Lact-C2-GFP fluorescence value, and the fluorescence value of the ionomycin non-treatment group and the ionomycin-treatment group in the same row where each test group was located in a 96-well microplate were calculated. In order to calculate the inhibitory activity (%) by the compound, the relative inhibitory activity was calculated by setting the compound and ionomycin non-treatment group to 100% inhibitory activity and the ionomycin alone treatment group to 0% inhibitory activity. To derive the results, the test was repeated three times, and the average values of the results were classified according to the range below and shown in Table 4 below (A: > 60% inhibition, B: 30 to 60% inhibition, N/A: no test).

**[Table 4]**

| **Compound No.** | **% Inhibition 1 µM** | **% Inhibition 100 nM** | **% Inhibition 50 nM** |
|---|---|---|---|
| **1** | A | A | B |
| **2** | A | A | N/A |
| **3** | A | A | A |
| **4** | A | A | B |
| **6** | A | A | N/A |
| **7** | A | A | A |
| **8** | A | A | A |
| **9** | A | A | A |
| **10** | A | A | A |
| **11** | A | B | N/A |
| **12** | A | A | A |
| **13** | A | A | A |
| **14** | A | A | A |
| **15** | A | A | A |
| **17** | A | A | A |
| **18** | A | A | A |
| **19** | A | A | B |
| **20** | A | A | A |
| **21** | A | A | A |
| **22** | A | A | A |
| **23** | A | A | A |
| **24** | A | A | A |
| **25** | A | A | A |
| **26** | A | A | A |
| **27** | A | A | A |
| **28** | N/A | A | A |
| **29** | N/A | A | A |
| **30** | N/A | B | B |
| **31** | N/A | A | B |
| **32** | N/A | B | B |
| **33** | N/A | B | B |
| **43** | N/A | A | A |
| **44** | N/A | A | A |
| **45** | N/A | A | A |
| **51** | N/A | A | B |
| **52** | N/A | A | A |
| **53** | N/A | B | B |
| **55** | N/A | A | A |
| **56** | N/A | A | B |
| **57** | N/A | A | A |
| **58** | N/A | A | A |
| **59** | N/A | A | A |
| **61** | N/A | A | A |
| **63** | N/A | A | A |
| **64** | N/A | B | B |
| 68 | N/A | A | A |
| **69** | N/A | B | N/A |
| **72** | N/A | A | A |
| **76** | N/A | A | B |
| **78** | A | N/A | N/A |
| **79** | N/A | N/A | A |
| **81** | N/A | N/A | A |
| **82** | N/A | N/A | A |
| **83** | N/A | N/A | A |
| **84** | A | N/A | N/A |
| **86** | A | N/A | N/A |
| **88** | A | N/A | A |
| **89** | A | N/A | B |
| **92** | A | N/A | A |
| **93** | A | N/A | B |
| **94** | A | N/A | N/A |
| **95** | A | N/A | A |
| **96** | A | N/A | A |
| **97** | A | N/A | N/A |
| **98** | A | N/A | A |
| **99** | A | N/A | B |
| **100** | A | N/A | A |
| **101** | A | N/A | A |
| **102** | A | N/A | A |
| **103** | A | N/A | N/A |
| **104** | A | N/A | A |
| **105** | A | N/A | A |
| **107** | A | N/A | B |
| **108** | B | N/A | N/A |
| **109** | A | N/A | A |
| **111** | A | N/A | B |
| **114** | A | N/A | B |
| **115** | A | N/A | N/A |
| **117** | A | N/A | N/A |
| **118** | A | N/A | A |
| **119** | A | N/A | B |
| **120** | A | N/A | A |
| **163** | A | N/A | N/A |
| **164** | A | N/A | B |
| **165** | A | N/A | B |
| **166** | A | N/A | A |
| **171** | B | N/A | N/A |
| **174** | B | N/A | N/A |
| **175** | A | N/A | N/A |
| **176** | B | N/A | N/A |
| **203** | A | N/A | B |
| **204** | A | N/A | N/A |
| **207** | A | N/A | N/A |
| **208** | A | N/A | N/A |
| **210** | B | N/A | N/A |
| **217** | A | N/A | B |
| **218** | N/A | N/A | B |
| **219** | A | N/A | N/A |
| **222** | A | N/A | A |
| **223** | A | N/A | A |
| **225** | N/A | N/A | A |
| **226** | N/A | N/A | A |
| **227** | A | N/A | A |
| **229** | A | N/A | N/A |
| **230** | A | N/A | A |
| **231** | A | N/A | N/A |
| **232** | A | N/A | A |
| **234** | A | N/A | B |
| **235** | A | N/A | B |
| **238** | A | N/A | A |
| **242** | A | N/A | A |
| **243** | A | N/A | B |
| **244** | A | N/A | B |
| **247** | B | N/A | N/A |
| **251** | A | N/A | N/A |
| **252** | A | N/A | A |
| **253** | A | N/A | A |
| **254** | A | N/A | A |
| **255** | A | N/A | N/A |
| **256** | A | N/A | N/A |
| **257** | B | N/A | N/A |
| **258** | A | N/A | A |
| **259** | A | N/A | B |
| **260** | A | N/A | B |
| **261** | A | N/A | B |
| **262** | N/A | N/A | A |
| **263** | B | N/A | N/A |
| **264** | N/A | N/A | A |
| **265** | N/A | N/A | A |
| **269** | A | N/A | B |
| **270** | N/A | N/A | A |
| **271** | B | N/A | N/A |
| **272** | A | N/A | N/A |
| **275** | A | N/A | N/A |
| **276** | N/A | N/A | A |
| **277** | N/A | N/A | A |
| **278** | N/A | N/A | A |
| **279** | B | N/A | N/A |
| **280** | A | N/A | B |
| **281** | A | N/A | A |
| **282** | A | N/A | B |
| **283** | A | N/A | A |
| **284** | A | N/A | A |
| **285** | A | N/A | A |
| **286** | A | N/A | N/A |
| **287** | B | N/A | N/A |
| **289** | B | N/A | N/A |
| **290** | B | N/A | N/A |
| **292** | B | N/A | N/A |
| **294** | A | N/A | B |
| **295** | A | N/A | B |
| **296** | B | N/A | N/A |
| **297** | A | N/A | A |
| **298** | A | N/A | B |
| **299** | A | N/A | N/A |
| **300** | A | N/A | B |
| **301** | A | N/A | N/A |
| **302** | A | N/A | A |
| **303** | A | N/A | N/A |
| **304** | A | N/A | B |
| **305** | A | N/A | B |
| **306** | B | N/A | N/A |
| **307** | B | N/A | N/A |
| **308** | A | N/A | A |
| **314** | A | N/A | A |
| **315** | A | N/A | N/A |
| **322** | A | N/A | N/A |

## Claims

1. A compound represented by the following Chemical Formula 1, a tautomer thereof, a seteroisomer thereof, or a pharmaceutically acceptable salt thereof: 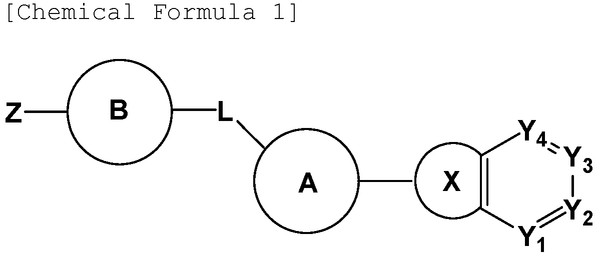 in Chemical Formula 1 above,
ring X is a 5-6 membered heteroaryl, a 5-6 membered heterocycloalkyl, or a 5-6 membered heterocycloalkenyl, wherein at least one H of the 5-6 membered heteroaryl, 5-6 membered heterocycloalkyl, or 5-6 membered heterocycloalkenyl ring may be substituted with -C₁₋₆alkyl, -benzyl, -C₁₋₆haloalkyl, -(CH₂)n-Rₑ, - O-(CH₂)n-Rₑ, or -halo;
Y₁ to Y₄ are each independently CR_{Y} or N;
R_{Y} is -H, -C₁₋₆alkyl, -C₁₋₆aminoalkyl, -C₁₋₆hydroxyalkyl, -C₁₋₆haloalkyl, -CN, -NO₂, -NRₐR_{b}, -OR_{c}, -C(=O)-R_{d}, -(CH₂)n-Rₑ, -O-(CH₂)n-Rₑ, -S(=O)₂-C₁₋₆alkyl, -halo, or 5-6 membered heteroaryl, wherein at least one H of the 5-6 membered heteroaryl may be substituted with -C₁₋₆alkyl;
ring A and ring B are each independently aryl, heteroaryl, cycloalkyl, or heterocycloalkyl, wherein the aryl, heteroaryl, cycloalkyl, or heterocycloalkyl is a single ring or multiple rings, and at least one H of the aryl, heteroaryl, cycloalkyl, or heterocycloalkyl ring may be substituted with -C₁₋₆alkyl, -C₁₋₆aminoalkyl, -C₁₋₆hydroxyalkyl, -C₁₋₆haloalkyl, -CN, -NO₂, -NRₐR_{b}, - OR_{c}, -C(=O)-R_{d}, -(CH₂)n-Rₑ, -O-(CH₂)n-Rₑ, -halo, or 5-6 membered heterocycloalkyl, wherein at least one H of the 5-6 membered heterocycloalkyl may be substituted with -C₁₋₆alkyl;
L is -CH₂-, -NR_{L}-, -C≡C-NR_{L}, -O-, -C(=O)-, -C(=O)O-, - OC(=O)-, -C(=O)NR_{L}-, -NR_{L}C(=O)-, -S-, -S(=O)₂-, -S(=O)₂-NR_{L}-, or - NR_{L}-S(=O)₂-;
R_{L} is -H or -C₁₋₆alkyl;
Z is -H, -C₁₋₆alkyl, -CN, -C₂alkynyl, aryl, heteroaryl, cycloalkyl, or heterocycloalkyl, wherein at least one H of the - C₂alkynyl, aryl, heteroaryl, cycloalkyl, or heterocycloalkyl ring may be substituted with -C₁₋₆alkyl, -C₁₋₆aminoalkyl, -C₁₋₆hydroxyalkyl, -C₁₋₆haloalkyl, -CN, -NO₂, -NRₐR_{b}, -OR_{c}, -C(=O)-R_{d}, - (CH₂)n-Rₑ, -O-(CH₂)n-Rₑ, -halo, or 5-6 membered heterocycloalkyl, wherein at least one H of the 5-6 membered heterocycloalkyl may be substituted with -C₁₋₆alkyl;
Rₐ and R_{b} are each independently -H, -C₁₋₆alkyl, or -benzyl;
R_{c} is -H, -C₁₋₆alkyl, -C₁₋₆haloalkyl, -(CH₂)n-Rₑ, -benzyl, or heterocycloalkyl, wherein at least one H of the heterocycloalkyl may be substituted with -C₁₋₆alkyl;
R_{d} is -H, -OH, -O-C₁₋₆alkyl, -NH₂, -NH-C₁₋₆alkyl, -N(C₁₋₆alkyl) (C₁₋₆alkyl), -NH-C₃₋₆cycloalkyl , or -NH-aryl;
Rₑ is -C₁₋₆aminoalkyl, -NH₂, -NH-C₁₋₆alkyl, -N(C₁₋₆alkyl)(C₁₋₆alkyl), heteroaryl, heterocycloalkyl, or heterocycloalkenyl, wherein the heteroaryl, heterocycloalkyl, or heterocycloalkenyl is a single ring or multiple rings, and at least one H of the heteroaryl, heterocycloalkyl, or heterocycloalkenyl may be substituted with -C₁₋₆alkyl; and
n is 0, 1, 2, 3 or 4.

2. The compound represented by Chemical Formula 1, the tautomer thereof, the stereoisomer thereof, or the pharmaceutically acceptable salt thereof according to claim 1, wherein
the compound represented by Chemical Formula 1 is a compound represented by the following Chemical Formula 1-1; 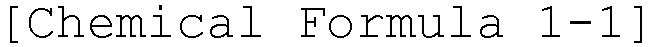 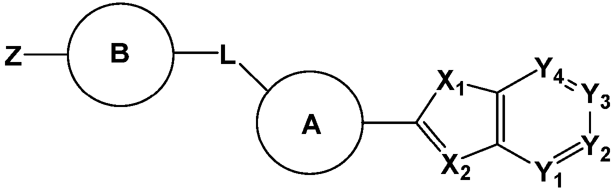 in Chemical Formula 1-1 above,
X₁ is CR₁R₂, NR₃, O, or S;
X₂ is CR₄ or N;
R₁ to R₄ are each independently -H or -C₁₋₆alkyl;
Y₁ to Y₄ are each independently CR_{Y} or N;
R_{Y} is -H, -C₁₋₆alkyl, -C₁₋₆aminoalkyl, -C₁₋₆hydroxyalkyl, -C₁₋₆haloalkyl, -CN, -NO₂, -NRₐR_{b}, -OR_{c}, -C(=O)-R_{d}, -S(=O)₂-C₁₋₆lkyl,-halo, or 5-6 membered heteroaryl, wherein at least one H of the 5-6 membered heteroaryl may be substituted with -C₁₋₆alkyl;
ring A and ring B are each independently aryl, heteroaryl, cycloalkyl, or heterocycloalkyl, wherein the aryl, heteroaryl, cycloalkyl, or heterocycloalkyl is a single ring or multiple rings, and at least one H of the aryl, heteroaryl, cycloalkyl, or heterocycloalkyl ring may be substituted with -C₁₋₆alkyl, -C₁₋₆aminoalkyl, -C₁₋₆hydroxyalkyl, -C₁₋₆haloalkyl, -CN, -NO₂, -NRₐR_{b},-OR_{c}, -C(=O)-R_{d}, -halo, or 5-6 membered heterocycloalkyl, wherein at least one H of the 5-6 membered heterocycloalkyl may be substituted with -C₁₋₆alkyl;
L is -CH₂-, -NR_{L}-, -C≡C-NR_{L}, -O-, -C(=O)-, -C(=O)O-,-OC(=O)-, -C(=O)NR_{L}-, -NR_{L}-C(=O)-, -S-, -S(=O)₂-, -S(=O)₂-NR_{L}-, or-NR_{L}-S(=O)₂-;
R_{L} is -H or -C₁₋₆alkyl;
Z is -H, -CN, -C₂alkynyl, aryl, heteroaryl, cycloalkyl, or heterocycloalkyl, wherein at least one H of the -C₂alkynyl, aryl, heteroaryl, cycloalkyl, or heterocycloalkyl ring may be substituted with -C₁₋₆alkyl, -C₁₋₆aminoalkyl, -C₁₋₆hydroxyalkyl, -C₁₋₆haloalkyl, -CN, -NO₂, -NRₐR_{b}, -OR_{c}, -C(=O)-R_{d}, -O-(CH₂)n-Rₑ-, -halo, or 5-6 membered heterocycloalkyl, wherein at least one H of the 5-6 membered heterocycloalkyl ring may be substituted with -C₁₋₆alkyl;
Rₐ and R_{b} are each independently -H, -C₁₋₆alkyl, or -benzyl;
R_{c} is -H, -C₁₋₆alkyl, -C₁₋₆haloalkyl, -(CH₂)n-Rₑ, -benzyl, or heterocycloalkyl, wherein at least one H of the heterocycloalkyl ring may be substituted with -C₁₋₆alkyl;
R_{d} is -H, -OH, -O-C₁₋₆alkyl, -NH₂, -NH-C₁₋₆alkyl, -N(C₁₋₆alkyl) (C₁₋₆alkyl), -NH-C₃₋₆cycloalkyl , or -NH-aryl;
Rₑ is -NH₂, -NH-C₁₋₆alkyl, -N(C₁₋₆alkyl)(C₁₋₆alkyl), heteroaryl, heterocycloalkyl, or heterocycloalkenyl, wherein at least one H of the heteroaryl, heterocycloalkyl, or heterocycloalkenyl ring may be substituted with -C₁₋₆alkyl; and
n is 0, 1, 2, 3, or 4.

3. The compound represented by Chemical Formula 1, the tautomer thereof, the stereoisomer thereof, or the pharmaceutically acceptable salt thereof according to claim 2, wherein
X₁ is NR₃ or O;
X₂ is CR₄ or N;
R₃ and R₄ are each independently -H or -C₁₋₆alkyl;
Y₁ and Y₄ are each independently CR_{Y};
Y₂ and Y₃ are each independently CR_{Y} or N, wherein when ring X₁ is S, one of Y₂ and Y₃ is N, when ring X₁ is O and X₂ is CR₄, one of Y₂ and Y₃ is N;
R_{Y} is -H, -C₁₋₆alkyl, -C₁₋₆haloalkyl, -CN, -NO₂, -NRₐR_{b}, -OR_{c}, -C(=O)-R_{d}, -S(=O)₂-C₁₋₆alkyl, -halo, or 5-6 membered heteroaryl, wherein at least one H of the 5-6 membered heteroayl may be substituted with -C₁₋₆alkyl.

4. The compound represented by Chemical Formula 1, the tautomer thereof, the stereoisomer thereof, or the pharmaceutically acceptable salt thereof according to claim 2, wherein
ring A is phenyl, 5-6 membered heteroaryl, or 5-6 membered cycloalkyl, wherein at least one H of the phenyl, 5-6 membered heteroaryl, or 5-6 membered cycloalkyl ring may be substituted with -C₁₋₆haloalkyl, -NO₂, -NRₐR_{b}, -OR_{c}, -halo, or 5-6 membered heterocycloalkyl, wherein at least one H of the 5-6 membered heterocycloalkyl may be substituted with -C₁₋₆alkyl.

5. The compound represented by Chemical Formula 1, the tautomer thereof, the stereoisomer thereof, or the pharmaceutically acceptable salt thereof according to claim 2, wherein
L is -NR_{L}-, -C≡C-NR_{L}, -O-, or -S(=O)₂-; and
R_{L} is -H or -C₁₋₆alkyl.

6. The compound represented by Chemical Formula 1, the tautomer thereof, the stereoisomer thereof, or the pharmaceutically acceptable salt thereof according to claim 2, wherein
ring B is phenyl, 5-6 membered heteroaryl, or 5-6 membered heterocycloalkyl, wherein at least one H of the phenyl, 5-6 membered heteroaryl, or 5-6 membered heterocycloalkyl ring may be substituted with -C₁₋₆alkyl, -C₁₋₆haloalkyl, -NO₂, -NRₐR_{b}, -OR_{c}, or - halo.

7. The compound represented by Chemical Formula 1, the tautomer thereof, the stereoisomer thereof, or the pharmaceutically acceptable salt thereof according to claim 2, wherein
Z is -CN, phenyl, 5-6 membered heteroaryl, 5-6 membered cycloalkyl, or 5-6 membered heterocycloalkyl, wherein at least one H of the phenyl, 5-6 membered heteroaryl, 5-6 membered cycloalkyl, or 5-6 membered heterocycloalkyl ring may be substituted with -C₁₋₆alkyl, -C₁₋₆haloalkyl, -CN, -OR_{c}, -C(=O)-R_{d}, -(CH₂)n-Rₑ, -halo, or 5-6 membered heterocycloalkyl, wherein at least one H of the 5-6 membered heterocycloalkyl may be substituted with -C₁₋₆alkyl, wherein when the ring B is phenyl, 6 membered heteroaryl, or 6 membered heterocycloalkyl, Z is connected to p-position with respect to L.

8. The compound represented by Chemical Formula 1, the tautomer thereof, the stereoisomer thereof or the pharmaceutically acceptable salt thereof according to claim 1, wherein
the compound represented by Chemical Formula 1 above is selected from the group consisting of the following compounds:
| | |
|---|---|
| **1** | 6-Phenyl-*N*-{3-[6-(trifluoromethyl)-1*H-*benzo[*d*]imidazol-2-yl]phenyl}pyridazin-3-amine |
| **2** | *N*-[3-(1H-benzo[*d*]imidazol-2-yl)phenyl]-6-phenylpyridazin-3-amine |
| **3** | *N*-[3-(6-tert-butyl-1*H*-benzo[*d*]imidazol-2-yl)phenyl]-6-phenylpyridazin-3-amine |
| **4** | *N*-[3-(6-bromo-5-fluoro-1*H*-benzo[*d*]imidazol-2-yl)phenyl]-6-phenylpyridazin-3-amine |
| **5** | 5,6-Dichloro-2-{3-[(4-phenylpiperazin-1-yl)methyl]phenyl}-1*H-*benzo[*d*]imidazole |
| **6** | 6-Phenyl-*N*-{3-[6-(trifluoromethoxy)-1*H*-benzo[*d*]imidazol-2-yl]phenyl}pyridazin-3-amine |
| **7** | *N*-[3-(5,6-dichloro-1H-benzo[*d*]imidazol-2-yl)phenyl]-6-phenylpyridazin-3-amine |
| **8** | *N*-{3-[6-chloro-5-(trifluoromethyl)-1*H*-benzo[*d*]imidazol-2-yl]phenyl}-6-phenylpyridazin-3-amine |
| **9** | *N*-{3-[6-bromo-5-(trifluoromethyl)-1*H*-benzo[*d*]imidazol-2-yl]phenyl}-6-phenylpyridazin-3-amine |
| **10** | *N*-{3-[6-chloro-5-(trifluoromethoxy)-1*H*-benzo[*d*]imidazol-2-yl]phenyl}-6-phenylpyridazin-3-amine |
| **11** | *N*-[3-(7-bromo-5-fluoro-1*H*-benzo[*d*]imidazol-2-yl)phenyl]-6-phenylpyridazin-3-amine |
| **12** | *N*-[3-(6-chloro-1H*-*benzo[*d*]imidazol-2-yl)phenyl]-6-phenylpyridazin-3-amine |
| **13** | *N*-[3-(5,6-dichloro-1*H*-benzo[*d*]imidazol-2-yl)phenyl]-5-phenylpyridin-2-amine |
| **14** | 5-Phenyl-*N*-{3-[6-(trifluoromethyl)-1*H*-benzo[*d*]imidazol-2-yl]phenyl}pyrimidin-2-amine |
| **15** | 5-Phenyl-*N*-{3-[6-(trifluoromethyl)-1*H*-benzo[*d*]imidazol-2-yl]phenyl}pyridin-2-amine |
| **16** | *N*-[3-(5,6-dichloro-1*H*-benzo[*d*]imidazol-2-yl)phenyl]pyrimidin-2-amine |
| **17** | 5-(3-Fluorophenyl)-*N*-{3-[6-(trifluoromethyl)-1*H*-benzo[*d*]imidazol-2-yl]phenyl}pyrimidin-2-amine |
| **18** | 5-(3-Fluorophenyl)-N-{3-[6-(trifluoromethyl)-1H-benzo[d]imidazol-2-yl]phenyl}pyridin-2-amine |
| **19** | *N*-[3-(1H-benzo[*d*]imidazol-2-yl)phenyl]-[1,1'-biphenyl]-4-amine |
| **20** | *N*-[3-(1H-benzo[*d*]imidazol-2-yl)phenyl]-4-(pyridin-2-yl)aniline |
| **21** | *N*-[3-(1H-benzo[*d*]imidazol-2-yl)phenyl]-4-(pyridazin-3-yl)aniline |
| **22** | *N*-[3-(1H-benzo[*d*]imidazol-2-yl)phenyl]-4-(pyrimidin-4-yl)aniline |
| **23** | *N*-[4-(pyridazin-3-yl)phenyl]-3-[6-(trifluoromethyl)-1*H-*benzo[*d*]imidazol-2-yl]aniline |
| **24** | *N*-[4-(pyrimidin-4-yl)phenyl]-3-[6-(trifluoromethyl)-1*H-*benzo[*d*]imidazol-2-yl]aniline |
| **25** | 3-(6-Fluoro-1*H*-benzo[*d*]imidazol-2-yl)-*N*-[4-(pyridazin-3-yl)phenyl]aniline |
| **26** | *N*-[3-(1*H*-benzo[*d*]imidazol-2-yl)phenyl]-4-(pyrimidin-2-yl)aniline |
| **27** | *N*-[4-(pyrimidin-2-yl)phenyl]-3-[6-(trifluoromethyl)-1*H-*benzo[*d*]imidazol-2-yl]aniline |
| **28** | *N*-[4-(pyrimidin-5-yl)phenyl]-3-[6-(trifluoromethyl)-1*H-*benzo[*d*]imidazol-2-yl]aniline |
| **29** | *N*-[4-(pyrazin-2-yl)phenyl]-3-[6-(trifluoromethyl)-1*H-*benzo[*d*]imidazol-2-yl]aniline |
| **30** | *N*-[3-(1*H*-benzo[*d*]imidazol-2-yl)phenyl]-4-(pyrimidin-5-yl)aniline |
| **31** | *N*-[3-(1*H*-benzo[*d*]imidazol-2-yl)phenyl]-4-(pyridin-3-yl)aniline |
| **32** | *N*-[3-(1*H*-benzo[*d*]imidazol-2-yl)phenyl]-4-(pyrazin-2-yl)aniline |
| **33** | *N*-[3-(1*H*-benzo[*d*]imidazol-2-yl)phenyl]-4-(pyridin-4-yl)aniline |
| **34** | 6-Phenyl-*N*-[(1s,4s)-4-(1*H*-benzo[*d*]imidazol-2-yl)bicyclo[2.2.1]heptan-1-yl]pyridazin-3-amine |
| **35** | *N*-[3-(1*H*-benzo[d]imidazol-2-yl)adamantan-1-yl]-6-phenylpyridazin-3-amine |
| **36** | 6-Phenyl-*N*-{3-[6-(trifluoromethyl)-1*H*-benzo[*d*]imidazol-2-yl]adamantan-1-yl}pyridazin-3-amine |
| **37** | 6-Phenyl-*N*-[(1s,4s)-4-[6-(trifluoromethyl)-1*H*-benzo[*d*]imidazol-2-yl]bicyclo[2.2.1]heptan-1-yl]pyridazin-3-amine |
| **38** | *N*-[3-(1*H*-benzo[*d*]imidazol-2-yl)phenyl]-5-(3-fluorophenyl)-*N-*methylpyrimidin-2-amine |
| **39** | *N*-[5-(1*H*-benzo[*d*]imidazol-2-yl)-2-methoxyphenyl]-5-(3-fluorophenyl)pyrimidin-2-amine |
| **40** | 4-(1*H*-benzo[*d*]imidazol-2-yl)-2-{[5-(3-fluorophenyl)pyrimidin-2-yl]amino}phenol |
| **41** | 5-(3-Fluorophenyl)-*N*-{2-methoxy-5-[6-(trifluoromethyl)-1H-benzo[d]imidazol-2-yl]phenyl}pyrimidin-2-amine |
| **42** | 2-{[5-(3-Fluorophenyl)pyrimidin-2-yl]amino}-4-[6-(trifluoromethyl)-1*H*-benzo[*d*]imidazol-2-yl]phenol |
| **43** | *N*-[3-(1H-benzo[*d*]imidazol-2-yl)phenyl]-5-(4-methylthiophen-3-yl)pyrimidin-2-amine |
| **44** | *N*-[3-(1H-benzo[*d*]imidazol-2-yl)phenyl]-5-(pyridin-2-yl)pyrimidin-2-amine |
| **45** | *N*-[3-(6-fluoro-1*H*-benzo[*d*]imidazol-2-yl)phenyl]-5-(pyridin-2-yl)pyrimidin-2-amine |
| **46** | *N*-(3-{1*H*-imidazolo[4,5-c]pyridin-2-yl}phenyl)-4-(pyridazin-3-yl)aniline |
| **47** | *N*-(3-{1*H*-imidazolo[4,5-c]pyridin-2-yl}phenyl)-[2,3'-bipyridin]-6'-amine |
| **48** | *N*-(3-{1*H*-imidazolo[4,5-c]pyridin-2-yl}phenyl)-5-(pyridin-2-yl)pyrimidin-2-amine |
| **49** | *N*-(3-{1*H*-imidazolo[4,5-c]pyridin-2-yl}phenyl)-5-(pyridazin-3-yl)pyrimidin-2-amine |
| **50** | *N*-(3-{1*H*-imidazolo[4,5-c]pyridin-2-yl}phenyl)-5-(pyridazin-3-yl)pyridin-2-amine |
| **51** | *N*-[3-(6-fluoro-1*H*-benzo[d]imidazol-2-yl)phenyl]-5-(pyridazin-3-yl)pyrimidin-2-amine |
| **52** | 5-(Pyridazin-3-yl)-*N*-{3-[6-(trifluoromethyl)-1*H*-benzo[*d*]imidazol-2-yl]phenyl}pyrimidin-2-amine |
| **53** | *N*-[3-(1*H*-benzo[*d*]imidazol-2-yl)phenyl]-5-(pyridazin-3-yl)pyrimidin-2-amine |
| **54** | *N*-(3-{1*H*-imidazolo[4,5-c]pyridin-2-yl}phenyl)-6-phenylpyridazin-3-amine |
| **55** | *N*-[3-(6-fluoro-1*H*-benzo[*d*]imidazol-2-yl)phenyl]-5-(pyridazin-3-yl)pyridin-2-amine |
| **56** | *N*-[3-(1*H*-benzo[*d*]imidazol-2-yl)phenyl]-5-(pyridazin-3-yl)pyridin-2-amine |
| **57** | *N*-{3-[6-(trifluoromethyl)-1*H*-benzo[*d*]imidazol-2-yl]phenyl}-[2,3'-bipyridin]-6'-amine |
| **58** | *N*-[3-(6-fluoro-1*H*-benzo[*d*]imidazol-2-yl)phenyl]-[2,3'-bipyridin]-6'-amine |
| **59** | *N*-[3-(1*H*-benzo[d]imidazol-2-yl)phenyl]-[2,3'-bipyridin]-6'-amine |
| **60** | *N*-(3-{1*H*-imidazolo[4,5-c]pyridin-2-yl}phenyl)-4-(pyrimidin-5-yl)aniline |
| **61** | Methyl 2-(3-{[4-(pyridazin-3-yl)phenyl]amino}phenyl)-1*H-*benzo[*d*]imidazol-6-carboxylate |
| **62** | 2-(3-{[4-(Pyridazin-3-yl)phenyl]amino}phenyl)-1*H*-benzo[*d*]imidazol-6-carboxylic acid |
| **63** | *N*-{3-[6-(trifluoromethyl)-1*H*-benzo[d]imidazol-2-yl]phenyl}-[2,5'-bipyrimidin]-2'-amine |
| **64** | *N*-(3-{6-chloro-1*H*-imidazolo[4,5-c]pyridin-2-yl}phenyl)-5-(pyridin-2-yl)pyrimidin-2-amine |
| **65** | *N*-(propan-2-yl)-2-(3-{[4-(pyridazin-3-yl)phenyl]amino}phenyl)-1*H-*benzo[*d*]imidazol-6-carboxamide |
| **66** | *N*-cyclohexyl-2-(3-{[4-(pyridazin-3-yl)phenyl]amino}phenyl)-1*H-*benzo[*d*]imidazol-6-carboxamide |
| **67** | *N*-cyclopentyl-2-(3-{[4-(pyridazin-3-yl)phenyl]amino}phenyl)-1*H-*benzo[*d*]imidazol-6-carboxamide |
| **68** | *N*-[3-(1*H*-benzo[d]imidazol-2-yl)phenyl]-5-(pyrimidin-4-yl)pyridin-2-amine |
| **69** | *N*-[3-(1*H*-benzo[*d*]imidazol-2-yl)phenyl]-5-(pyrimidin-2-yl)pyridin-2-amine |
| **70** | *N*-[3-(1*H*-benzo[*d*]imidazol-2-yl)phenyl]-[5,5'-bipyrimidin]-2-amine |
| **71** | *N*-[3-(1H-benzo[*d*]imidazol-2-yl)phenyl]-[2,5'-bipyrimidin]-2'-amine |
| **72** | *N*-[3-(6-fluoro-1*H*-benzo[*d*]imidazol-2-yl)phenyl]-5-(pyrimidin-4-yl)pyridin-2-amine |
| **73** | *N*-[3-(1H-benzo[*d*]imidazol-2-yl)phenyl]-5-(4-fluoropyridin-2-yl)pyrimidin-2-amine |
| **74** | *N*-[3-(1H-benzo[*d*]imidazol-2-yl)phenyl]-[1,1'-biphenyl]-3-amine |
| **75** | *N*-[3-(1H-benzo[*d*]imidazol-2-yl)phenyl]-3-(pyrimidin-5-yl)aniline |
| **76** | *N*-[3-(1H-benzo[d]imidazol-2-yl)phenyl]-[4,5'-bipyrimidin]-2'-amine |
| **77** | *N*-[3-(1H-benzo[d]imidazol-2-yl)phenyl]-2-phenylpyrimidin-5-amine |
| **78** | Methyl 2-(3-{[4-(pyrimidin-2-yl)phenyl]amino}phenyl)-1*H-*benzo[*d*]imidazol-6-carboxylate |
| **79** | *N*-[4-(pyridazin-3-yl)phenyl]-3-[6-(trifluoromethyl)-1*H-*imidazolo[4,5-c]pyridin-2-yl]aniline |
| **80** | *N*-[3-(1*H*-benzo[*d*]imidazol-2-yl)phenyl]-6-phenyl-pyridin-3-amine |
| **81** | 3-(5,6-difluoro-1*H*-benzo[*d*]imidazol-2-yl)-*N*-(4-pyridazin-3-ylphenyl)aniline |
| **82** | *N*-[3-(6-chloro-1*H*-benzo[*d*]imidazol-2-yl)phenyl]-5-(2-pyridyl)pyrimidin-2-amine |
| **83** | 5-(2-pyridyl)-*N*-[3-[6-(trifluoromethyl)-1*H*-benzo[*d*]imidazol-2-yl]phenyl]pyrimidin-2-amine |
| **84** | 3-(6-chloro-1*H*-imidazo[4,5-*c*]pyridin-2-yl)-*N*-(4-pyridazin-3-ylphenyl)aniline |
| **85** | *N*,*N*-dimethyl-2-(3-((4-(pyridazin-3-yl)phenyl)amino)phenyl)-1H-benzo[d]imidazole-6-carboxamide |
| **86** | *N*-(4-pyrimidin-4-ylphenyl)-3-[6-(trifluoromethyl)-1*H*-imidazo[4,5-*c*]pyridin-2-yl]aniline |
| **87** | *N*-methyl-2-(3-((4-(pyridazin-3-yl)phenyl)amino)phenyl)-1*H-*benzo[*d*]imidazole-6-carboxamide |
| **88** | 5-pyrimidin-4-yl-*N*-[3-[6-(trifluoromethyl)-1*H*-benzo[*d*]imidazol-2-yl]phenyl]pyridin-2-amine |
| **89** | 5-pyrimidin-4-yl-*N*-[3-[6-(trifluoromethyl)-1*H*-imidazo[4,5-*c*]pyridin-2-yl]phenyl]pyridin-2-amine |
| **90** | 3-(1*H*-indol-2-yl)-*N*-(4-pyridazin-3-ylphenyl)aniline |
| **91** | 3-(1-methylbenzimidazol-2-yl)-*N*-(4-pyridazin-3-ylphenyl)aniline |
| **92** | 3-(6-fluoro-1*H*-benzo[*d*]imidazol-2-yl)-*N*-[4-(2-pyridyl)phenyl]aniline |
| **93** | 3-[6-(3-methyl-1,2,4-oxadiazol-5-yl)-1*H*-benzo[*d*]imidazol-2-yl]-*N-*(4-pyridazin-3-ylphenyl)aniline |
| **94** | 3-(1*H*-benzo[*d*]imidazol-2-yl)-*N*-(4-pyridazin-4-ylphenyl)aniline |
| **95** | 3-(5,6-difluoro-1*H*-benzo[d]imidazol-2-yl)-*N*-(4-pyrimidin-4-ylphenyl)aniline |
| **96** | 3-(6-fluoro-1*H*-benzo[*d*]imidazol-2-yl)-*N*-(4-pyrimidin-4-ylphenyl)aniline |
| **97** | 3-(1*H*-benzo[*d*]imidazol-2-yl)-*N*-(4-cyclohexylphenyl)aniline |
| **98** | *N*-[4-(2-pyridyl)phenyl]-3-[6-(trifluoromethyl)-1*H*-benzo[*d*]imidazol-2-yl]aniline |
| **99** | *N*-[3-(5,6-difluoro-1*H*-benzo[*d*]imidazol-2-yl)phenyl]-5-pyridazin-3-yl-pyrimidin-2-amine |
| **100** | 5-pyrimidin-2-yl-*N*-[3-[6-(trifluoromethyl)-1*H*-benzo[*d*]imidazol-2-yl]phenyl]pyridin-2-amine |
| **101** | 3-(7-methyl-1*H*-benzo[*d*]imidazol-2-yl)-N-(4-pyridazin-3-ylphenyl)aniline |
| **102** | 3-(5-chloro-6-fluoro-1*H*-benzo[*d*]imidazol-2-yl)-*N*-(4-pyridazin-3-ylphenyl)aniline |
| **103** | 5-pyridazin-3-yl-*N*-[3-[6-(trifluoromethyl)-1*H*-imidazo[4,5-*c*]pyridin-2-yl]phenyl]pyrimidin-2-amine |
| **104** | 3-[6-fluoro-5-(trifluoromethyl)-1*H*-benzo[*d*]imidazol-2-yl]-*N*-(4-pyridazin-3-ylphenyl)aniline |
| **105** | 3-(6-methyl-1*H*-benzo[*d*]imidazol-2-yl)-*N*-(4-pyridazin-3-ylphenyl)aniline |
| **106** | 3-(6-methylsulfonyl-1*H*-benzo[*d*]imidazol-2-yl)-*N*-(4-pyridazin-3-ylphenyl)aniline |
| **107** | 3-(6-tert-butyl-1*H*-benzo[d]imidazol-2-yl)-*N*-(4-pyridazin-3-ylphenyl)aniline |
| **108** | *N*-[3-(6-methoxy-1*H*-benzo[*d*]imidazol-2-yl)phenyl]-5-pyridazin-3-yl-pyrimidin-2-amine |
| **109** | *N*-(4-pyrimidin-4-ylphenyl)-3-[6-(trifluoromethoxy)-1*H-*benzo[*d*]imidazol-2-yl]aniline |
| **111** | isopropyl 2-(3-((4-(pyridazin-3-yl)phenyl)amino)phenyl)-1*H-*benzo[*d*]imidazole-6-carboxylate |
| **112** | 4-[4-[3-[6-(trifluoromethyl)-1*H*-benzo[*d*]imidazol-2-yl]anilino]phenyl]benzonitrile |
| **113** | 6-(1-methyl-4-piperidyl)-*N*-[3-[6-(trifluoromethyl)-1*H-*benzo[*d*]imidazol-2-yl]phenyl]pyridazin-3-amine |
| **114** | 3-(6-fluoro-5-methyl-1*H*-benzo[*d*]imidazol-2-yl)-*N*-(4-pyridazin-3-ylphenyl)aniline |
| **115** | 3-(7-chloro-1*H*-benzo[*d*]imidazol-2-yl)-*N*-(4-pyridazin-3-ylphenyl)aniline |
| **116** | 3-(6-chloro-1*H*-imidazo[4,5-*c*]pyridin-2-yl)-*N*-(4-pyrimidin-2-ylphenyl)aniline |
| **117** | 3-(6-chloro-1*H*-imidazo[4,5-*c*]pyridin-2-yl)-*N*-(4-pyrimidin-4-ylphenyl)aniline |
| **118** | 5-(2-pyridyl)-*N*-[3-[6-(trifluoromethoxy)-1*H*-benzo[*d*]imidazol-2-yl]phenyl]pyrimidin-2-amine |
| **119** | 3-(6-chloro-1*H*-imidazo[4,5-*c*]pyridin-2-yl)-*N*-[4-(2-pyridyl)phenyl]aniline |
| **120** | *N*-[3-[6-chloro-5-(trifluoromethoxy)-1*H*-benzo[*d*]imidazol-2-yl]phenyl]-5-pyridazin-3-yl-pyrimidin-2-amine |
| **121** | *N*-[3-(6-fluoro-5-methoxy-1*H*-benzo[*d*]imidazol-2-yl)phenyl]-5-pyridazin-3-yl-pyrimidin-2-amine |
| **122** | 2-[3-(4-pyridazin-3-ylphenyl)sulfonylphenyl]-6-(trifluoromethyl)-1*H*-benzo[d]imidazole |
| **123** | *N*-[3-(1H-benzo[*d*]imidazol-2-yl)phenyl]-5-pyridazin-3-yl-1,3,4-oxadiazol-2-amine |
| **124** | *N*-(4-pyridazin-3-ylcyclohexyl)-3-[6-(trifluoromethyl)-1*H-*benzo[*d*]imidazol-2-yl]aniline |
| **125** | 1-pyridazin-3-yl-*N*-[3-[6-(trifluoromethyl)-1*H*-benzo[*d*]imidazol-2-yl]phenyl]piperidin-4-amine |
| **126** | 3-(5,6-difluoro-1*H*-benzo[*d*]imidazol-2-yl)-*N*-(4-(pyrimidin-2-yl)phenyl)aniline |
| **150** | 3-(6-fluoro-1*H*-benzo[*d*]imidazol-2-yl)-N-[4-(4-methylpiperazin-1-yl)phenyl]aniline |
| **151** | *N*-[3-(6-fluoro-1*H*-benzo[*d*]imidazol-2-yl)phenyl]-5-(4-methylpiperazin-1-yl)pyridin-2-amine |
| **152** | *N*-[4-(4-methylpiperazin-1-yl)phenyl]-3-[6-(trifluoromethyl)-1*H-*benzo[*d*]imidazol-2-yl]aniline |
| **153** | 5-(4-methylpiperazin-1-yl)-*N*-[3-[6-(trifluoromethyl)-1*H-*benzo[*d*]imidazol-2-yl]phenyl]pyridin-2-amine |
| **154** | *N*-[4-[2-(4-methylpiperazin-1-yl)pyrimidin-5-yl]phenyl]-3-[6-(trifluoromethyl)-1*H*-benzo[*d*]imidazol-2-yl]aniline |
| **155** | 3-(5-fluoro-1*H*-benzo[*d*]imidazol-2-yl)-*N*-[4-[2-(4-methylpiperazin-1-yl)pyrimidin-5-yl]phenyl]aniline |
| **156** | 5-(1*H*-benzo[*d*]imidazol-2-yl)-2-(4-methylpiperazin-1-yl)-*N*-(4-pyrimidin-5-ylphenyl)aniline |
| **157** | (1*R*,2*R*)-2-(1*H*-benzo[*d*]imidazol-2-yl)-N-(4-pyrazin-2-ylphenyl)cyclopropanecarboxamide |
| **158** | (1*R*,2*R*)-2-(5-fluoro-1*H*-benzo[d]imidazol-2-yl)-*N*-(4-pyrazin-2-ylphenyl)cyclopropanecarboxamide |
| **159** | (1*R*,2*R*)-2-(1*H*-benzo[*d*]imidazol-2-yl)-*N*-(4-pyrimidin-5-ylphenyl)cyclopropanecarboxamide |
| **160** | (1*R*,2*R*)-2-(5-fluoro-1*H*-benzo[*d*]imidazol-2-yl)-*N*-(4-pyrimidin-5-ylphenyl)cyclopropanecarboxamide |
| **161** | 5-(1*H*-benzo[*d*]imidazol-2-yl)-2-(4-methylpiperazin-1-yl)-*N*-(4-pyrazin-2-ylphenyl)aniline |
| **162** | 5-(6-fluoro-1*H*-benzo[*d*]imidazol-2-yl)-2-(4-methylpiperazin-1-yl)-*N-*(4-pyrazin-2-ylphenyl)aniline |
| **163** | *N*-[3-(1*H*-benzo[*d*]imidazol-2-yl)phenyl]-5-(3-furyl)pyrimidin-2-amine |
| **164** | *N*-[3-(1H-benzo[*d*]imidazol-2-yl)phenyl]-5-(3-thienyl)pyrimidin-2-amine |
| **165** | 3-(1*H*-benzo[*d*]imidazol-2-yl)-*N*-(4-oxazol-2-ylphenyl)aniline |
| **166** | 3-(1*H*-benzo[*d*]imidazol-2-yl)-*N*-(4-thiazol-4-ylphenyl)aniline |
| **167** | 2-(1*H*-benzo[*d*]imidazol-2-yl)-*N*¹,*N*¹-dimethyl-*N*⁴-(4-pyrazin-2-ylphenyl)benzene-1,4-diamine |
| **168** | 3-(1*H*-benzo[*d*]imidazol-2-yl)-4-[2-(dimethylamino)ethoxy]-*N*-(4-pyrazin-2-ylphenyl)aniline |
| **169** | 3-(1*H*-benzo[*d*]imidazol-2-yl)-4-[2-(4-methylpiperazin-1-yl)ethoxy]-*N*-(4-pyrazin-2-ylphenyl)aniline |
| **170** | 2-(1*H*-benzo[*d*]imidazol-2-yl)-*N*¹,*N*¹-dimethyl-*N*⁴-(4-pyrimidin-5-ylphenyl)benzene-1,4-diamine |
| **171** | *N*-[3-(1*H*-benzo[*d*]imidazol-2-yl)phenyl]-5-oxazol-2-yl-pyrimidin-2-amine |
| **172** | 3-(1*H*-benzo[*d*]imidazol-2-yl)-4-[(1-methyl-4-piperidyl)oxy]-*N*-(4-pyrazin-2-ylphenyl)aniline |
| **173** | *N*-[3-(1*H*-benzo[*d*]imidazol-2-yl)phenyl]-5-thiazol-4-yl-pyrimidin-2-amine |
| **174** | 2-[2-(dimethylamino)ethoxy]-*N*-(4-pyrazin-2-ylphenyl)-5-[6-(trifluoromethyl)-1*H*-benzo[*d*]imidazol-2-yl]aniline |
| **175** | 2-[2-(4-methylpiperazin-1-yl)ethoxy]-*N*-(4-pyrazin-2-ylphenyl)-5-[6-(trifluoromethyl)-1*H*-benzo[*d*]imidazol-2-yl]aniline |
| **176** | *N*-[2-(4-methylpiperazin-1-yl)-5-[6-(trifluoromethyl)-1*H-*benzo[*d*]imidazol-2-yl]phenyl]-5-pyridazin-3-yl-pyrimidin-2-amine |
| **177** | *N*-[2-[(1-methyl-4-piperidyl)oxy]-3-[6-(trifluoromethyl)-1*H-*benzo[*d*]imidazol-2-yl]phenyl]-5-pyridazin-3-yl-pyrimidin-2-amine |
| **178** | *N*-[3-[(1-methyl-4-piperidyl)oxy]-5-[6-(trifluoromethyl)-1*H-*benzo[*d*]imidazol-2-yl]phenyl]-5-pyridazin-3-yl-pyrimidin-2-amine |
| **179** | *N*-[3-[2-(dimethylamino)ethoxy]-5-[6-(trifluoromethyl)-1*H-*benzo[*d*]imidazol-2-yl]phenyl]-5-pyridazin-3-yl-pyrimidin-2-amine |
| **200** | *N*-(3-(1*H*-benzo[*d*]imidazol-2-yl)phenyl)-6-(pyrimidin-2-yl)pyridazin-3-amine |
| **201** | *N*-(3-(6-fluoro-1*H*-benzo[*d*]imidazol-2-yl) phenyl)-6-(pyrimidin-2-yl)pyridazin-3-amine |
| **202** | 6-(pyrimidin-2-yl)-*N*-(3-(6-(trifluoromethyl)-1*H*-benzo[*d*]imidazol-2-yl)phenyl)pyridazin-3-amine |
| **203** | *N*-(3-(1*H*-benzo[*d*]imidazol-2-yl)phenyl)-2-methyl-4-(pyridazin-3-yl)aniline |
| **204** | *N*-(3-(1*H*-benzo[*d*]imidazol-2-yl)phenyl)-3-methyl-4-(pyridazin-3-yl)aniline |
| **205** | *N*-(3-(1*H*-benzo[*d*]imidazol-2-yl)phenyl)-2-nitro-4-(pyridazin-3-yl)aniline |
| **206** | *N*-(3-(1*H*-benzo[*d*]imidazol-2-yl)phenyl)-3-nitro-4-(pyridazin-3-yl)aniline |
| **207** | *N¹*-(3-(1*H*-benzo[*d*]imidazol-2-yl)phenyl)-4-(pyridazin-3-yl)benzene-1,3-diamine |
| **208** | *N*-(3-(1*H*-benzo[*d*]imidazol-2-yl)phenyl)-3-methoxy-4-(pyridazin-3-yl)aniline |
| **209** | 5-((3-(1*H*-benzo[*d*]imidazol-2-yl)phenyl)amino)-2-(pyridazin-3-yl)phenol |
| **210** | *N*-(3-(1*H*-benzo[*d*]imidazol-2-yl)phenyl)-2-methoxy-4-(pyridazin-3-yl)aniline |
| **211** | 2-((3-(1*H*-benzo[*d*]imidazol-2-yl)phenyl)amino)-5-(pyridazin-3-yl)phenol |
| **212** | 3-(1*H*-benzo[*d*]imidazol-2-yl)-*N*-(4-(benzofuran-2-yl)phenyl)aniline |
| **213** | 2-(3-(4-(pyridazin-3-yl)phenoxy)phenyl)-1*H*-benzo[*d*]imidazole |
| **214** | 5-(1*H*-benzo[*d*]imidazol-2-yl)-*N*-(4-(pyridazin-3-yl)phenyl)thiazol-2-amine |
| **215** | 3-(1*H*-benzo[*d*]imidazol-2-yl)-*N*-(4-morpholinophenyl)aniline |
| **216** | 3-(benzo[d]oxazol-2-yl)-*N*-(4-(pyridazin-3-yl)phenyl)aniline |
| **217** | *N*,*N*-dimethyl-2-(3-((4-(pyridazin-3-yl)phenyl)amino)phenyl)-1*H-*benzo[*d*]imidazol-6-amine |
| **218** | 3-(6-bromo-1*H*-imidazo[4,5-*c*]pyridin-2-yl)-*N*-(4-(pyridazin-3-yl)phenyl)aniline |
| **219** | 3-(1*H*-imidazo[4,5-b]pyridin-2-yl)-*N*-(4-(pyridazin-3-yl)phenyl)aniline |
| **220** | methyl 2-(3-((4-(pyridazin-3-yl)phenyl)amino)phenyl)-1*H-*imidazo[4,5-*c*]pyridine-6-carboxylate |
| **221** | 2-(3-((4-(pyridazin-3-yl) phenyl)amino)phenyl)-1*H*-imidazo[4,5-c]pyridine-6-carboxylic acid |
| **222** | 3-(6-(benzyloxy)-1*H*-benzo[*d*]imidazol-2-yl)-*N*-(4-(pyridazin-3-yl)phenyl)aniline |
| **223** | 3-(6-methoxy-1*H*-benzo[*d*]imidazol-2-yl)-*N*-(4-(pyridazin-3-yl)phenyl)aniline |
| **224** | 2-(3-((4-(pyridazin-3-yl)phenyl)amino)phenyl)-1*H*-benzo[*d*]imidazol-6-ol |
| **225** | 3-(5,6-dichloro-1*H*-benzo[d]imidazol-2-yl)-*N*-(4-(pyridazin-3-yl)phenyl)aniline |
| **226** | 3-(6-chloro-1*H*-benzo[d]imidazol-2-yl)-*N*-(4-(pyridazin-3-yl)phenyl)aniline |
| **227** | 3-(6-nitro-1*H*-benzo[d]imidazol-2-yl)-*N*-(4-(pyridazin-3-yl)phenyl)aniline |
| **228** | 2-(3-((4-(pyridazin-3-yl)phenyl)amino)phenyl)-1*H*-benzo[d]imidazol-6-amine |
| **229** | *N*-benzyl-2-(3-((4-(pyridazin-3-yl)phenyl)amino)phenyl)-1H-benzo[*d*]imidazol-6-amine |
| **230** | *N*-(4-(pyridazin-3-yl)phenyl)-3-(6-(trifluoromethoxy)-1H-benzo[*d*]imidazol-2-yl)aniline |
| **231** | 2-(3-((4-(pyridazin-3-yl)phenyl)amino)phenyl)-1*H*-benzo[*d*]imidazole-6-carbonitrile |
| **232** | *N*-(4-(pyridazin-3-yl)phenyl)-3-(6-(trifluoromethoxy)-1*H-*imidazo[4,5-*c*]pyridin-2-yl)aniline |
| **234** | *N*-(4-(pyridazin-3-yl)phenyl)-3-(6-(trifluoromethyl)-1*H*-imidazo[4,5-*b*]pyridin-2-yl)aniline |
| **235** | *N*-(4-(pyridazin-3-yl)phenyl)-3-(5-(trifluoromethyl)-1*H*-imidazo[4,5-*b*]pyridin-2-yl)aniline |
| **238** | *N*-(3-(6-(trifluoro methyl)-1*H*-imidazo [4,5-*c*]pyridin-2-yl) phenyl)-[2,3'-bipyridin]-6'-amine |
| **242** | 5-(pyridin-2-yl)-*N*-(3-(6-(trifluoromethyl)-1*H*-imidazo[4,5-*c*]pyridin-2-yl)phenyl)pyrimidin-2-amine |
| **243** | 5-(pyridin-2-yl)-*N*-(3-(6-(trifluoromethyl)-1*H*-imidazo[4,5-*b*]pyridin-2-yl)phenyl)pyrimidin-2-amine |
| **244** | 5-(pyridin-2-yl)-*N*-(3-(5-(trifluoromethyl)-1*H*-imidazo[4,5-*b*]pyridin-2-yl)phenyl)pyrimidin-2-amine |
| **247** | *N*-(3-(6-fluoro-1*H*-benzo[*d*]imidazol-2-yl)phenyl)-[2,5'-bipyrimidin]-2'-amine |
| **249** | 3-(1*H*-benzo[*d*]imidazol-2-yl)-5-methyl-*N*-(4-(pyridazin-3-yl)phenyl)aniline |
| **250** | 3-(1*H*-benzo[d]imidazol-2-yl)-*N*-(4-(pyridazin-3-yl) phenyl)-5-(trifluoromethyl)aniline |
| **251** | *N*-(4-(3-fluoropyridin-2-yl)phenyl)-3-(6-(trifluoromethyl)-1H-benzo[*d*]imidazol-2-yl)aniline |
| **252** | *N*-(4-(6-fluoropyridin-2-yl)phenyl)-3-(6-(trifluoromethyl)-1*H-*benzo[*d*]imidazol-2-yl)aniline |
| **253** | *N*-(4-(5-fluoropyridin-2-yl)phenyl)-3-(6-(trifluoromethyl)-1*H-*benzo[*d*]imidazol-2-yl)aniline |
| **254** | 5-(pyridazin-3-yl)-*N*-(3-(6-(trifluoromethyl)-1*H*-imidazo[4,5-*c*]pyridin-2-yl)phenyl)pyridin-2-amine |
| **255** | *N*-(3-(1*H*-benzo[*d*] imidazol-2-yl)phenyl)-6-(pyridin-2-yl)pyrimidin-3-amine |
| **256** | 6-(pyridin-2-yl)-*N*-(3-(6-(trifluoromethyl)-1*H*-benzo[*d*]imidazol-2-yl)phenyl)pyridazin-3-amine |
| **257** | *N*-(3-(1*H*-benzo[*d*]imidazol-2-yl)phenyl)-6-(pyrimidin-4-yl)pyridazin-3-amine |
| **258** | 5-(6-fluoropyridin-2-yl)-*N*-(3-(6-(trifluoromethyl)-1*H-*benzo[*d*]imidazol-2-yl)phenyl)pyrimidin-2-amine |
| **259** | *N*-(3-(1*H*-benzo[*d*]imidazol-2-yl)phenyl)-5-(6-fluoropyridin-2-yl) pyrimidin-2-amine |
| **260** | 5-(5-fluoropyridin-2-yl)-*N*-(3-(6-(trifluoromethyl)-1*H-*benzo[*d*]imidazol-2-yl)phenyl)pyrimidin-2-amine |
| **261** | *N*-(4-(pyridazin-3-yl) phenyl)-4-(6-(trifluoromethyl)-1*H-*benzo[*d*]imidazol-2-yl)pyridin-2-amine |
| **262** | *N*-(4-(pyridazin-3-yl) phenyl)-5-(6-(trifluoromethyl)-1*H-*benzo[d]imidazol-2-yl)pyridin-3-amine |
| **263** | *N*-(4-(pyridazin-3-yl) phenyl)-2-(6-(trifluoromethyl)-1*H-*benzo[d]imidazol-2-yl)pyridin-4-amine |
| **264** | *N*-(4-(6-fluoropyridazin-3-yl)phenyl)-3-(6-(trifluoromethyl)-1*H-*benzo[d]imidazol-2-yl) aniline |
| **265** | 3-(1*H*-benzo[*d*]imidazol-2-yl)-*N*-(4-(6-fluoropyridazin-3-yl)phenyl)aniline |
| **266** | 3-(1*H*-benzo[*d*]imidazole-2-yl)-*N*-(4-(4-(trifluoromethyl)pyridazin-3-yl)phenyl)aniline |
| **267** | 3-(1*H*-benzo[*d*]imidazol-2-yl)-*N*-(4-(5-(trifluoromethyl)pyridazin-3-yl)phenyl)aniline |
| **268** | 3-(1*H*-benzo[*d*]imidazol-2-yl)-*N*-(4-(6-(trifluoromethyl)pyridazin-3-yl)phenyl)aniline |
| **269** | *N*-(3-(1*H*-benzo[*d*]imidazol-2-yl)phenyl)-2-fluoro-4-(pyridazin-3-yl)aniline |
| **270** | *N*-(3-(1*H*-benzo[*d*]imidazol-2-yl)phenyl)-3-fluoro-4-(pyridazin-3-yl)aniline |
| **271** | *N*-(3-(1*H*-benzo[*d*]imidazol-2-yl)phenyl)-6-phenyl-1,2,4-triazin-3-amine |
| **272** | *N*-(3-(1*H*-benzo[*d*]imidazol-2-yl)phenyl)-2'-(trifluoromethyl)-[1,1'-biphenyl]-4-amine |
| **273** | methyl 6-(4-((3-(1*H*-benzo[*d*]imidazol-2-yl) phenyl)amino)phenyl)pyridazine-4-carboxylate |
| **274** | 6-(4-((3-(1*H*-benzo[d]imidazol-2-yl)phenyl) amino)phenyl)pyridazine-4-carboxylic acid |
| **275** | 3-(1*H*-benzo[*d*] imidazol-2-yl)-*N*-(4-(6-methoxypyridazin-3-yl)phenyl)aniline |
| **276** | *N*-(3-(1*H*-benzo[*d*] imidazol-2-yl)phenyl)-5-(pyridin-2-yl)pyrazin-2-amine |
| **277** | *N*-(3-(1*H*-benzo[*d*] imidazol-2-yl)phenyl)-5-(pyrimidin-4-yl)pyrazin-2-amine |
| **278** | *N*-(4-(6-methoxypyridazin-3-yl)phenyl)-3-(6-(trifluoromethyl)-1*H-*benzo[d]imidazol-2-yl)aniline |
| **279** | *N*-methyl-2-(3-((4-(pyridazin-3-yl)phenyl) amino)phenyl)-1*H-*benzo[d]imidazol-6-amine |
| **280** | *N*-benzyl-*N*-methyl-2-(3-((4-(pyridazin-3-yl)phenyl)amino)phenyl)-1*H-*benzo[*d*]imidazol-6-amine |
| **281** | *N*-(3-(6-fluoro-1*H*-imidazo[4,5-*c*]pyridin-2-yl)phenyl)-[2,3'-bipyridin]-6'-amine |
| **282** | *N*-(3-(6-chloro-1*H*-imidazo[4,5-*c*]pyridin-2-yl)phenyl)-[2,3'-bipyridin]-6'-amine |
| **283** | *N*-(3-(5,6-difluoro-1*H*-benzo[*d*]imidazol-2-yl) phenyl)-[2,3'-bipyridin] -6'-amine |
| **284** | *N*-(3-(6-(difluoromethoxy)-1*H*-benzo[*d*]imidazol-2-yl)phenyl)-[2,3'-bipyridin]-6'-amine |
| **285** | *N*-(3-(6-(difluoromethoxy)-1*H*-benzo[*d*]imidazol-2-yl)phenyl)-5-(pyridin-2-yl)pyrimidin-2-amine |
| **286** | *N*-(3-(6-fluoro-1*H*-imidazo[4,5-*c*]pyridin-2-yl)phenyl)-5-(pyridin-2-yl)pyrimidin-2-amine |
| **287** | *N*-(3-(6-(difluoromethoxy)-1*H*-benzo[*d*]imidazol-2-yl)phenyl)-5-(pyridazin-3-yl)pyrimidin-2-amine |
| **288** | *N*-(3-(6-(2-morpholinoethoxy)-1*H*-benzo[*d*]imidazol-2-yl)phenyl)-5-(pyridazin-3-yl)pyrimidin-2-amine |
| **289** | 5-(pyridazin-3-yl)-*N*-(3-(7-(trifluoromethyl)-1*H*-benzo[*d*]imidazol-2-yl) phenyl)pyrimidin-2-amine |
| **290** | *N*-(3-(7-fluoro-1*H*-benzo[*d*]imidazol-2-yl)phenyl)-5-(pyridazin-3-yl)pyrimidin-2-amine |
| **291** | *N*-(3-(7-chloro-1*H*-benzo[*d*]imidazol-2-yl)phenyl)-5-(pyridazin-3-yl)pyrimidin-2-amine |
| **292** | *N*-(3-(5,7-difluoro-1*H*-benzo[*d*]imidazol-2-yl)phenyl)-5-(pyridazin-3-yl)pyrimidin-2-amine |
| **293** | *N*-(3-(6,7-difluoro-1*H*-benzo[*d*]imidazol-2-yl)phenyl)-5-(pyridazin-3-yl)pyrimidin-2-amine |
| **294** | *N*-(3-(6-chloro-5-(trifluoromethyl)-1*H*-benzo[*d*]imidazol-2-yl)phenyl)-5-(pyridazin-3-yl)pyrimidin-2-amine |
| **295** | 5-(pyridazin-3-yl)-*N*-(3-(6-(trifluoromethoxy)-1*H*-benzo[*d*]imidazol-2-yl)phenyl)pyrimidin-2-amine |
| **296** | 6-(pyrimidin-4-yl)-*N*-(3-(6-(trifluoromethyl)-1*H*-benzo[*d*]imidazol-2-yl)phenyl)pyridazin-3-amine |
| **297** | *N*-(3-(5,6-difluoro-1*H*-benzo[*d*]imidazol-2-yl) phenyl)-5-(pyrimidin-4-yl)pyrazin-2-amine |
| **298** | *N*-(3-(5,6-difluoro-1*H*-benzo[*d*]imidazol-2-yl) phenyl)-5-(pyridin-2-yl)pyrazin-2-amine |
| **299** | *N*-(3-(6-methoxy-1*H*-benzo[*d*]imidazol-2-yl) phenyl)-5-(pyridin-2-yl)pyrazin-2-amine |
| **300** | *N*-(3-(6-(difluoromethoxy)-1*H*-benzo[*d*]imidazol-2-yl)phenyl)-5-(pyridin-2-yl)pyridazin-2-amine |
| **301** | *N*-(3-(6-chloro-1*H*-imidazo[4,5-*c*]pyridin-2-yl)phenyl)-5-(pyridin-2-yl)pyrazin-2-amine |
| **302** | 5-(pyridin-2-yl)-*N*-(3-(6-(trifluoromethyl)-1*H*-benzo[*d*]imidazol-2-yl)phenyl)pyrazin-2-amine |
| **303** | *N*-(3-(6-chloro-1*H*-imidazo[4,5-*c*]pyridin-2-yl)phenyl)-5-(thiazol-4-yl)pyrimidin-2-amine |
| **304** | 5-(thiazol-4-yl)-*N*-(3-(6-(trifluoromethyl)-1*H*-benzo[*d*]imidazol-2-yl)phenyl)pyrimidin-2-amine |
| **305** | *N*-(4-(4-fluoropyridin-2-yl)phenyl)-3-(6-(trifluoromethyl)-1*H-*benzo[*d*]imidazol-2-yl)aniline |
| **306** | 5-(pyridin-2-yl)-N-(4-(6-(trifluoromethyl)-1*H*-benzo[*d*]imidazol-2-yl)pyridin-2-yl)pyrimidin-2-amine |
| **307** | 5-(pyridin-2-yl)-*N*-(5-(6-(trifluoromethyl)-1*H*-benzo[*d*]imidazol-2-yl) pyridin-3-yl)pyrimidin-2-amine |
| **308** | 3-(6-bromo-1*H*-benzo[*d*]imidazol-2-yl)-*N*-(4-(pyridazin-3-yl) phenyl)aniline |
| **309** | *N*-(3-(6-(difluoromethoxy)-1*H*-benzo[*d*]imidazol-2-yl) phenyl)-5-(pyrimidin-4-yl)pyrazin-2-amine |
| **310** | 5-(pyrimidin-4-yl)-*N*-(3-(6-(trifluoromethyl)-1*H*-imidazo[4,5-*c*] pyridin-2-yl)phenyl)pyrazin-2-amine |
| **311** | 5-(5-((methylamino)methyl)pyridin-2-yl)-*N*-(3-(6-(trifluoromethyl)-1*H*-benzo[*d*]imidazol-2-yl)phenyl)pyrimidin-2-amine |
| **312** | 5-(5-((dimethylamino)methyl)pyridin-2-yl)-*N*-(3-(6-(trifluoromethyl)-1*H*-benzo[*d*]imidazol-2-yl)phenyl)pyrimidin-2-amine |
| **313** | *N*-(3-(5,6-difluoro-1*H*-benzo[*d*]imidazol-2-yl)phenyl)-5-(pyridin-2-yl)pyrimidin-2-amine |
| **314** | 6-((3-(6-(trifluoromethyl)-1*H*-benzo[*d*]imidazol-2-yl)phenyl)amino)nicotinonitrile |
| **315** | 4-((3-(6-(trifluoromethyl)-1*H*-benzo[*d*]imidazol-2-yl)phenyl)amino)benzonitrile |
| **316** | *tert*-butyl 4-(6-((3-(6-(trifluoromethyl)-1*H*-benzo[*d*]imidazol-2-yl)phenyl)amino)pyridazine-3-yl)piperidine-1-carboxylate |
| **317** | 6-(piperidin-4-yl)-*N*-(3-(6-(trifluoromethyl)-1*H*-benzo[*d*]imidazol-2-yl)phenyl)pyridazin-3-amine |
| **320** | *N*-(3-(1*H*-benzo[*d*]imidazol-2-yl)phenyl)-3'-(trifluoromethyl)-[1,1'-biphenyl]-4-amine |
| **321** | methyl 4'-((3-(1*H*-benzo[*d*]imidazol-2-yl-phenyl)amino)-[1,1'-biphenyl]-3-carboxylate |
| **322** | *N*-(3-(1*H*-benzo[*d*]imidazol-2-yl)phenyl)-2'-fluoro-[1,1'-biphenyl]-4-amine |
| **323** | *N*-(3-(6-fluoro-1*H*-benzo[*d*]imidazol-2-yl)phenyl)-5-(pyridin-2-yl)pyrazin-2-amine |
| **324** | *N*-(3-(6-chloro-1*H*-benzo[*d*]imidazol-2-yl)phenyl)-5-(pyridin-2-yl)pyrazin-2-amine |
| **325** | 5-(pyridin-2-yl)-*N*-(3-(6-(trifluoromethoxy)-1*H*-benzo[*d*]imidazol-2-yl)phenyl)pyrazin-2-amine |
| **326** | 5-(pyrimidin-4-yl)-*N*-(3-(6-(trifluoromethyl)-1*H*-benzo[*d*]imidazol-2-yl)phenyl)pyrazin-2-amine |
| **327** | 5-(pyrimidin-4-yl)-*N*-(3-(6-(trifluoromethoxy)-1*H*-benzo[*d*]imidazol-2- yl)phenyl)pyrazin-2-amine |
| **328** | 5-(pyridin-2-yl)-*N*-(3-(6-(trifluoromethoxy)-1*H*-imidazo[4,5-*c*]pyridin-2-yl)phenyl)pyrazin-2-amine |
| **329** | *N*-(3-(6-(difluoromethoxy)-1*H*-imidazo[4,5-*c*]pyridin-2-yl)phenyl)-5-(pyridin-2-yl)pyrimidin-2-amine |
| **330** | *N*-(3-(6-fluoro-1*H*-imidazo[4,5-*c*]pyridin-2-yl)phenyl)-5-(pyridin-2-yl)pyrazin-2-amine |
| **331** | 3-(6-(difluoromethoxy)-1*H*-imidazo[4,5-*c*] pyridin-2-yl)-N-(4-(pyridazin-3-yl)phenyl)aniline |
| **332** | *N*-(3-(5,6-difluoro-1*H*-benzo[*d*]imidazol-2-yl)phenyl)-5-(thiazol-4-yl)pyrimidin-2-amine |
| **333** | *N*-(3-(6-(difluoromethoxy)-1*H*-imidazo[4,5-*c*]pyridin-2-yl)phenyl)-5-(6-fluoropyridin-2-yl)pyrimidin-2-amine |
| **334** | 5-(6-fluoropyridin-2-yl)-*N*-(3-(6-(trifluoromethyl)-1*H*-imidazo[4,5-*c*]pyridin-2-yl)phenyl)pyrimidin-2-amine |
| **335** | 3-(6-(difluoromethoxy)-1*H*-benzo[*d*]imidazol-2-yl)-*N*-(4-(pyridazin-3-yl)phenyl)aniline |
| **336** | *N*-(3-(6-(difluoromethoxy)-1*H*-imidazo[4,5-*c*]pyridin-2-yl)phenyl)-5-(pyridin-2-yl)pyrazin-2-amine |

9. A pharmaceutical composition comprising the compound, the tautomer thereof, the stereoisomer thereof, or the pharmaceutically acceptable salt thereof according to any one of claims 1 to 8 as an active ingredient.

10. A pharmaceutical composition for treating or preventing thrombosis or thrombosis-related diseases, comprising the compound, the tautomer thereof, the stereoisomer thereof, or the pharmaceutically acceptable salt thereof according to any one of claims 1 to 8 as an active ingredient.

11. The pharmaceutical composition according to claim 10, wherein the thrombosis or thrombosis-related disease is at least one disease selected from the group consisting of
acute myocardial infarction; atrial fibrillation; unstable angina; chronic stable angina; transient ischemic attack; stroke; peripheral vascular disease; pre-eclampsia; eclampsia; deep vein thrombosis; embolism; cancer-related thrombosis; disseminated intravascular coagulation; thrombotic thrombocytopenia; and thrombotic or restenotic complications that occur after invasive procedures resulting from angioplasty, carotid endarterectomy, post coronary artery bypass graft (CABG) surgery, vascular graft surgery, stent placements and insertion of endovascular devices and prostheses.

12. Use of the compound represented by Chemical Formula I, the tautomer thereof, the stereoisomer thereof, or the pharmaceutically acceptable salt thereof according to any one of Claims 1 to 8 for preparing a medicament for preventing or treating the thrombosis or thrombosis-related diseases.

13. A method for preventing or treating the thrombosis or thrombosis-related diseases comprising administering a therapeutically effective amount of the compound represented by Chemical Formula I, the tautomer thereof, the stereoisomer thereof, or the pharmaceuticaaly acceptable salt thereof according to any one of Claims 1 to 8 as an active ingredient.
